# EUROPEAN PATENT APPLICATION

(11) **EP 4 385 514 A2**
(43) Date of publication of application: **19.06.2024**
(21) Application number: 24159518.0
(22) Date of filing: 28.09.2018
(51) Int. Cl.: A61K 31/7088

(54) **FORMULATIONS**

(30) Priority: 29.09.2017 US 201762566240 P
(62) Divisional of application: 18800355.2
(71) Applicant: Intellia Therapeutics, Inc., Cambridge, MA 02139 (US)
(72) Inventor: WOOD, Kristy, M., Wellesley, 02481 (US); GARDNER, Noah, Paul, Holden, 01520 (US); SHAH, Ruchi, Rudraprasad, Framingham, 01701 (US); SCULLY, Stephen, S, Belmont, 02478 (US); MAJZOUB, Ramsey, Boston, 02114 (US)
(74) Representative: Schlich, George

(57) **Abstract**

The invention provides lipid nanoparticle-based compositions with improved properties for delivery of biologically active agents, engineered cells, and methods for delivery of the agents.

## Description

The present application claims the benefit of priority to U.S. Provisional Patent Application No. 62/566,240, filed September 29, 2017, the contents of which are incorporated herein by reference in their entirety.

Lipid nanoparticle ("LNP") compositions with improved properties for delivery of biologically active agents, in particular RNAs, mRNAs, and guide RNAs are provided herein. The LNP compositions facilitate delivery of RNA agents across cell membranes, and in particular embodiments, they introduce components and compositions for gene editing into living cells.

Biologically active agents that are particularly difficult to deliver to cells include proteins, nucleic acid-based drugs, and derivatives thereof. Compositions for delivery of promising gene editing technologies into cells, such as for delivery of CRISPR/Cas9 system components, are of particular interest.

A number of components and systems for editing genes in cells *in vivo* now exist, providing tremendous potential for treating diseases. CRISPR/Cas gene editing systems are active as ribonucleoprotein complexes in a cell. An RNA-directed nuclease binds to and directs cleavage of a DNA sequence in the cell. This site-specific nuclease activity facilitates gene editing through the cell's own natural processes. For example, the cell responds to double-stranded DNA breaks (DSBs) with an error-prone repair process known as non-homologous end joining ("NHEJ"). During NHEJ, nucleotides may be added or removed from the DNA ends by the cell, resulting in a sequence altered from the cleaved sequence. In other circumstances, cells repair DSBs by homology-directed repair ("HDR") or homologous recombination ("HR") mechanisms, in which an endogenous or exogenous template can be used to direct repair of the break. Several of these editing technologies take advantage of cellular mechanisms for repairing single-stranded breaks (SSBs) or DSBs.

Compositions for delivery of the protein and nucleic acid components of CRISPR/Cas to a cell, such as a cell in a patient, are needed. In particular, compositions for delivering mRNA encoding the CRISPR protein component, and for delivering CRISPR guide RNAs are of particular interest. Compositions with useful properties for in vitro and in vivo delivery that can stabilize and deliver RNA components, are also of particular interest.

We herein provide lipid nanoparticle-based compositions with useful properties, in particular for delivery of CRISPR/Cas gene editing components.

In certain embodiments, the LNP compositions comprise: an RNA component; and a lipid component, wherein the lipid component comprises: (1) about 50-60 mol-% amine lipid; (2) about 8-10 mol-% neutral lipid; and (3) about 2.5-4 mol-% PEG lipid, wherein the remainder of the lipid component is helper lipid, and wherein the N/P ratio of the LNP composition is about 6. In additional embodiments, the LNP compositions comprise (1) an RNA component; (2) about 50-60 mol-% amine lipid; (3) about 27-39.5 mol-% helper lipid; (4) about 8-10 mol-% neutral lipid; and (5) about 2.5-4 mol-% PEG lipid, wherein the N/P ratio of the LNP composition is about 5-7.

In other embodiments, the LNP compositions comprise an RNA component and a lipid component, wherein the lipid component comprises: (1) about 50-60 mol-% amine lipid; (2) about 5-15 mol-% neutral lipid; and (3) about 2.5-4 mol-% PEG lipid, wherein the remainder of the lipid component is helper lipid, and wherein the N/P ratio of the LNP composition is about 3-10. In additional embodiments, the LNP compositions comprise a lipid component that includes (1) about 40-60 mol-% amine lipid; (2) about 5-15 mol-% neutral lipid; and (3) about 2.5-4 mol-% PEG lipid, wherein the remainder of the lipid component is helper lipid, and wherein the N/P ratio of the LNP composition is about 6. In another embodiment, the LNP compositions comprise a lipid component that includes (1) about 50-60 mol-% amine lipid; (2) about 5-15 mol-% neutral lipid; and (3) about 1.5-10 mol-% PEG lipid, wherein the remainder of the lipid component is helper lipid, and wherein the N/P ratio of the LNP composition is about 6.

In some embodiments, the LNP compositions comprise an RNA component and a lipid component, wherein the lipid component comprises: (1) about 40-60 mol-% amine lipid; (2) about 0-5 mol-% neutral lipid, e.g., phospholipid; and (3) about 1.5-10 mol-% PEG lipid, wherein the remainder of the lipid component is helper lipid, and wherein the N/P ratio of the LNP composition is about 3-10. In some embodiments, the LNP compositions comprise an RNA component and a lipid component, wherein the lipid component comprises: (1) about 40-60 mol-% amine lipid; (2) less than about 1 mol-% neutral lipid, e.g., phospholipid; and (3) about 1.5-10 mol-% PEG lipid, wherein the remainder of the lipid component is helper lipid, and wherein the N/P ratio of the LNP composition is about 3-10. In certain embodiments, the LNP composition is essentially free of neutral lipid. In some embodiments, the LNP compositions comprise an RNA component and a lipid component, wherein the lipid component comprises: (1) about 40-60 mol-% amine lipid; and (2) about 1.5-10 mol-% PEG lipid, wherein the remainder of the lipid component is helper lipid, wherein the N/P ratio of the LNP composition is about 3-10, and wherein the LNP composition is free of neutral lipid, e.g., phospholipid. In certain embodiments, the LNP composition is essentially free of or free of a neutral phospholipid. In certain embodiments, the LNP composition is essentially free of or free of a neutral lipid, e.g., phospholipid.

In certain embodiments, the RNA component comprises an mRNA, such as an RNA-guided DNA-binding agent (e.g., a Cas nuclease or Class 2 Cas nuclease). In certain embodiments, the RNA component comprises a gRNA.

### BRIEF DESCRIPTION OF DRAWINGS

Fig. 1 shows the percentage of TTR gene editing achieved in mouse liver after delivery of CRISPR/Cas gene editing components Cas9 mRNA and gRNA in LNP compositions as indicated at a single dose of 1 mpk (Fig. 1A)) or 0.5 mpk (Fig. 1B).
Fig. 2 shows particle distribution data for LNP compositions comprising Cas9 mRNA and gRNA.
Fig. 3 depicts physicochemical properties of LNP compositions, comparing log differential molar mass (Fig. 3A) and average molecular weight measurements (Fig. 3B) for the compositions.
Fig. 4 shows polydispersity calculations in Fig. 4A and Burchard-Stockmeyer analysis in Fig. 4B, analyzing the LNP compositions of Fig. 3.
Fig. 5 provides the results of an experiment evaluating the effect of LNP compositions with increased PEG lipid concentrations on serum TTR knockdown, gene editing in the liver, and cytokine MCP-1 levels after a single dose administration in rats. Fig. 5A graphs serum TTR levels; Fig. 5B graphs percent editing in liver samples; and Fig. 5C provides MCP-1 levels in pg/mL.
Fig. 6 shows that LNP compositions maintain potency for gene editing with various PEG lipids (as measured by serum TTR levels (Figs. 6A and 6B) and percent editing (Fig. 6C).
Fig. 7 shows that Lipid A analogs effectively deliver gene editing cargos in LNP compositions as measured by % liver editing after a single dose administration in mouse.
Fig. 8 shows a dose response curve of percent editing with various LNP compositions in primary cyno hepatocytes.
Fig. 9A and Fig. 9B show serum TTR and percent editing results when the ratio of gRNA to mRNA varies, and Fig. 9C and Fig. 9D show serum TTR and percent editing results in liver when the amount of Cas9 mRNA is held constant and gRNA varies following a single dose administration in mouse.
Fig. 10A and Fig. 10B show serum TTR and liver editing results after administration of LNP compositions with and without neutral lipid.

### DETAILED DESCRIPTION

The present disclosure provides embodiments of lipid nanoparticle (LNP) compositions of RNAs, including CRISPR/Cas component RNAs (the "cargo") for delivery to a cell and methods for their use. The LNP compositions may exhibit improved properties as compared to prior delivery technologies. The LNP composition may contain an RNA component and a lipid component, as defined herein. In certain embodiments, the RNA component includes a Cas nuclease, such as a Class 2 Cas nuclease. In certain embodiments, the cargo or RNA component includes an mRNA encoding a Class 2 Cas nuclease and a guide RNA or nucleic acids encoding guide RNAs. Methods of gene editing and methods of making engineered cells are also provided.

### CRISPR/Cas Cargo

The CRISPR/Cas cargo delivered via LNP formulation may include an mRNA molecule encoding a protein of interest. For example, an mRNA for expressing a protein such as green fluorescent protein (GFP), and RNA-guided DNA-binding agent, or a Cas nuclease is included. LNP compositions that include a Cas nuclease mRNA, for example a Class 2 Cas nuclease mRNA that allows for expression in a cell of a Cas9 protein are provided. Further, the cargo may contain one or more guide RNAs or nucleic acids encoding guide RNAs. A template nucleic acid, e.g., for repair or recombination, may also be included in the composition or a template nucleic acid may be used in the methods described herein.

"mRNA" refers to a polynucleotide that comprises an open reading frame that can be translated into a polypeptide (i.e., can serve as a substrate for translation by a ribosome and amino-acylated tRNAs). mRNA can comprise a phosphate-sugar backbone including ribose residues or analogs thereof, e.g., 2'-methoxy ribose residues. In some embodiments, the sugars of an mRNA phosphate-sugar backbone consist essentially of ribose residues, 2'-methoxy ribose residues, or a combination thereof. In general, mRNAs do not contain a substantial quantity of thymidine residues (e.g., 0 residues or fewer than 30, 20, 10, 5, 4, 3, or 2 thymidine residues; or less than 10%, 9%, 8%, 7%, 6%, 5%, 4%, 4%, 3%, 2%, 1%, 0.5%, 0.2%, or 0.1% thymidine content). An mRNA can contain modified uridines at some or all of its uridine positions.

### CRISPR/Cas Nuclease Systems

One component of the disclosed formulations is an mRNA encoding RNA-guided DNA-binding agent, such as a Cas nuclease.

As used herein, an "RNA-guided DNA binding agent" means a polypeptide or complex of polypeptides having RNA and DNA binding activity, or a DNA-binding subunit of such a complex, wherein the DNA binding activity is sequence-specific and depends on the sequence of the RNA. Exemplary RNA-guided DNA binding agents include Cas cleavases/nickases and inactivated forms thereof ("dCas DNA binding agents"). "Cas nuclease", as used herein, encompasses Cas cleavases, Cas nickases, and dCas DNA binding agents. Cas cleavases/nickases and dCas DNA binding agents include a Csm or Cmr complex of a type III CRISPR system, the Cas10, Csm1, or Cmr2 subunit thereof, a Cascade complex of a type I CRISPR system, the Cas3 subunit thereof, and Class 2 Cas nucleases. As used herein, a "Class 2 Cas nuclease" is a single-chain polypeptide with RNA-guided DNA binding activity. Class 2 Cas nucleases include Class 2 Cas cleavases/nickases (e.g., H840A, D10A, or N863A variants), which further have RNA-guided DNA cleavase or nickase activity, and Class 2 dCas DNA binding agents, in which cleavase/nickase activity is inactivated. Class 2 Cas nucleases include, for example, Cas9, Cpfl, C2c1, C2c2, C2c3, HF Cas9 (e.g., N497A, R661A, Q695A, Q926A variants), HypaCas9 (e.g., N692A, M694A, Q695A, H698A variants), eSPCas9(1.0) (e.g, K810A, K1003A, R1060A variants), and eSPCas9(1.1) (e.g., K848A, K1003A, R1060A variants) proteins and modifications thereof. Cpfl protein, Zetsche et al., Cell, 163: 1-13 (2015), is homologous to Cas9, and contains a RuvC-like nuclease domain. Cpfl sequences of Zetsche are incorporated by reference in their entirety. *See, e.g.,* Zetsche, Tables S1 and S3. *See, e.g.,* Makarova et al., Nat Rev Microbiol, 13(11): 722-36 (2015); Shmakov et al., Molecular Cell, 60:385-397 (2015).

In some embodiments, the RNA-guided DNA-binding agent is a Class 2 Cas nuclease. In some embodiments, the RNA-guided DNA-binding agent has cleavase activity, which can also be referred to as double-strand endonuclease activity. In some embodiments, the RNA-guided DNA-binding agent comprises a Cas nuclease, such as a Class 2 Cas nuclease (which may be, e.g., a Cas nuclease of Type II, V, or VI). Class 2 Cas nucleases include, for example, Cas9, Cpfl, C2c1, C2c2, and C2c3 proteins and modifications thereof. Examples of Cas9 nucleases include those of the type II CRISPR systems *of S. pyogenes, S. aureus,* and other prokaryotes (see, e.g., the list in the next paragraph), and modified (e.g., engineered or mutant) versions thereof. See, e.g., U.S. 2016/0312198 A1; U.S. 2016/0312199 A1. Other examples of Cas nucleases include a Csm or Cmr complex of a type III CRISPR system or the Cas10, Csm1, or Cmr2 subunit thereof; and a Cascade complex of a type I CRISPR system, or the Cas3 subunit thereof. In some embodiments, the Cas nuclease may be from a Type-IIA, Type-IIB, or Type-IIC system. For discussion of various CRISPR systems and Cas nucleases see, e.g., Makarova et al., Nat. Rev. Microbiol. 9:467-477 (2011); Makarova et al., Nat. Rev. Microbiol, 13: 722-36 (2015); Shmakov et al., Molecular Cell, 60:385-397 (2015).

Non-limiting exemplary species that the Cas nuclease can be derived from include Streptococcus pyogenes, Streptococcus thermophilus, Streptococcus sp., Staphylococcus aureus, Listeria innocua, Lactobacillus gasseri, Francisella novicida, Wolinella succinogenes, Sutterella wadsworthensis, Gammaproteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria, Acidaminococcus sp., Lachnospiraceae bacterium ND2006, and Acaryochloris marina.

In some embodiments, the Cas nuclease is the Cas9 nuclease from *Streptococcus pyogenes.* In some embodiments, the Cas nuclease is the Cas9 nuclease from *Streptococcus thermophilus.* In some embodiments, the Cas nuclease is the Cas9 nuclease from *Neisseria meningitidis.* In some embodiments, the Cas nuclease is the Cas9 nuclease is from *Staphylococcus aureus.* In some embodiments, the Cas nuclease is the Cpfl nuclease from *Francisella novicida.* In some embodiments, the Cas nuclease is the Cpfl nuclease from *Acidaminococcus sp.* In some embodiments, the Cas nuclease is the Cpfl nuclease from *Lachnospiraceae* bacterium ND2006. In further embodiments, the Cas nuclease is the Cpfl nuclease from *Francisella tularensis, Lachnospiraceae bacterium, Butyrivibrio proteoclasticus, Peregrinibacteria bacterium, Parcubacteria bacterium, Smithella, Acidaminococcus, Candidates Methanoplasma termitum, Eubacterium eligens, Moraxella bovoculi, Leptospira inadai, Porphyromonas crevioricanis, Prevotella disiens,* or *Porphyromonas macacae.* In certain embodiments, the Cas nuclease is a Cpfl nuclease from an *Acidaminococcus* or *Lachnospiraceae.*

Wild type Cas9 has two nuclease domains: RuvC and HNH. The RuvC domain cleaves the non-target DNA strand, and the HNH domain cleaves the target strand of DNA. In some embodiments, the Cas9 nuclease comprises more than one RuvC domain and/or more than one HNH domain. In some embodiments, the Cas9 nuclease is a wild type Cas9. In some embodiments, the Cas9 is capable of inducing a double strand break in target DNA. In certain embodiments, the Cas nuclease may cleave dsDNA, it may cleave one strand of dsDNA, or it may not have DNA cleavase or nickase activity. An exemplary Cas9 amino acid sequence is provided as SEQ ID NO: 3. An exemplary Cas9 mRNA ORF sequence, which includes start and stop codons, is provided as SEQ ID NO: 4. An exemplary Cas9 mRNA coding sequence, suitable for inclusion in a fusion protein, is provided as SEQ ID NO: 10.

In some embodiments, chimeric Cas nucleases are used, where one domain or region of the protein is replaced by a portion of a different protein. In some embodiments, a Cas nuclease domain may be replaced with a domain from a different nuclease such as Fok1. In some embodiments, a Cas nuclease may be a modified nuclease.

In other embodiments, the Cas nuclease may be from a Type-I CRISPR/Cas system. In some embodiments, the Cas nuclease may be a component of the Cascade complex of a Type-I CRISPR/Cas system. In some embodiments, the Cas nuclease may be a Cas3 protein. In some embodiments, the Cas nuclease may be from a Type-III CRISPR/Cas system. In some embodiments, the Cas nuclease may have an RNA cleavage activity.

In some embodiments, the RNA-guided DNA-binding agent has single-strand nickase activity, i.e., can cut one DNA strand to produce a single-strand break, also known as a "nick." In some embodiments, the RNA-guided DNA-binding agent comprises a Cas nickase. A nickase is an enzyme that creates a nick in dsDNA, i.e., cuts one strand but not the other of the DNA double helix. In some embodiments, a Cas nickase is a version of a Cas nuclease (e.g., a Cas nuclease discussed above) in which an endonucleolytic active site is inactivated, e.g., by one or more alterations (e.g., point mutations) in a catalytic domain. See, e.g., U.S. Pat. No. 8,889,356 for discussion of Cas nickases and exemplary catalytic domain alterations. In some embodiments, a Cas nickase such as a Cas9 nickase has an inactivated RuvC or HNH domain. An exemplary Cas9 nickase amino acid sequence is provided as SEQ ID NO: 6. An exemplary Cas9 nickase mRNA ORF sequence, which includes start and stop codons, is provided as SEQ ID NO: 7. An exemplary Cas9 nickase mRNA coding sequence, suitable for inclusion in a fusion protein, is provided as SEQ ID NO: 11.

In some embodiments, the RNA-guided DNA-binding agent is modified to contain only one functional nuclease domain. For example, the agent protein may be modified such that one of the nuclease domains is mutated or fully or partially deleted to reduce its nucleic acid cleavage activity. In some embodiments, a nickase is used having a RuvC domain with reduced activity. In some embodiments, a nickase is used having an inactive RuvC domain. In some embodiments, a nickase is used having an HNH domain with reduced activity. In some embodiments, a nickase is used having an inactive HNH domain.

In some embodiments, a conserved amino acid within a Cas protein nuclease domain is substituted to reduce or alter nuclease activity. In some embodiments, a Cas nuclease may comprise an amino acid substitution in the RuvC or RuvC-like nuclease domain. Exemplary amino acid substitutions in the RuvC or RuvC-like nuclease domain include D10A (based on the S. *pyogenes* Cas9 protein). *See, e.g.,* Zetsche et al. (2015) Cell Oct 22:163(3): 759-771. In some embodiments, the Cas nuclease may comprise an amino acid substitution in the HNH or HNH-like nuclease domain. Exemplary amino acid substitutions in the HNH or HNH-like nuclease domain include E762A, H840A, N863A, H983A, and D986A (based on the S. *pyogenes* Cas9 protein). *See, e.g.,* Zetsche et al. (2015). Further exemplary amino acid substitutions include D917A, E1006A, and D1255A (based on the *Francisella novicida* U112 Cpfl (FnCpf1) sequence (UniProtKB - A0Q7Q2 (CPF1 FRATN)).

In some embodiments, an mRNA encoding a nickase is provided in combination with a pair of guide RNAs that are complementary to the sense and antisense strands of the target sequence, respectively. In this embodiment, the guide RNAs direct the nickase to a target sequence and introduce a DSB by generating a nick on opposite strands of the target sequence (i.e., double nicking). In some embodiments, use of double nicking may improve specificity and reduce off-target effects. In some embodiments, a nickase is used together with two separate guide RNAs targeting opposite strands of DNA to produce a double nick in the target DNA. In some embodiments, a nickase is used together with two separate guide RNAs that are selected to be in close proximity to produce a double nick in the target DNA.

In some embodiments, the RNA-guided DNA-binding agent lacks cleavase and nickase activity. In some embodiments, the RNA-guided DNA-binding agent comprises a dCas DNA-binding polypeptide. A dCas polypeptide has DNA-binding activity while essentially lacking catalytic (cleavase/nickase) activity. In some embodiments, the dCas polypeptide is a dCas9 polypeptide. In some embodiments, the RNA-guided DNA-binding agent lacking cleavase and nickase activity or the dCas DNA-binding polypeptide is a version of a Cas nuclease (e.g., a Cas nuclease discussed above) in which its endonucleolytic active sites are inactivated, e.g., by one or more alterations (e.g., point mutations) in its catalytic domains. See, e.g., U.S. 2014/0186958 A1; U.S. 2015/0166980 A1. An exemplary dCas9 amino acid sequence is provided as SEQ ID NO: 8. An exemplary Cas9 mRNA ORF sequence, which includes start and stop codons, is provided as SEQ ID NO: 9. An exemplary Cas9 mRNA coding sequence, suitable for inclusion in a fusion protein, is provided as SEQ ID NO: 12.

In some embodiments, the RNA-guided DNA-binding agent comprises one or more heterologous functional domains (e.g., is or comprises a fusion polypeptide).

In some embodiments, the heterologous functional domain may facilitate transport of the RNA-guided DNA-binding agent into the nucleus of a cell. For example, the heterologous functional domain may be a nuclear localization signal (NLS). In some embodiments, the RNA-guided DNA-binding agent may be fused with 1-10 NLS(s). In some embodiments, the RNA-guided DNA-binding agent may be fused with 1-5 NLS(s). In some embodiments, the RNA-guided DNA-binding agent may be fused with one NLS. Where one NLS is used, the NLS may be linked at the N-terminus or the C-terminus of the RNA-guided DNA-binding agent sequence. It may also be inserted within the RNA-guided DNA binding agent sequence. In other embodiments, the RNA-guided DNA-binding agent may be fused with more than one NLS. In some embodiments, the RNA-guided DNA-binding agent may be fused with 2, 3, 4, or 5 NLSs. In some embodiments, the RNA-guided DNA-binding agent may be fused with two NLSs. In certain circumstances, the two NLSs may be the same *(e.g.,* two SV40 NLSs) or different. In some embodiments, the RNA-guided DNA-binding agent is fused to two SV40 NLS sequences linked at the carboxy terminus. In some embodiments, the RNA-guided DNA-binding agent may be fused with two NLSs, one linked at the N-terminus and one at the C-terminus. In some embodiments, the RNA-guided DNA-binding agent may be fused with 3 NLSs. In some embodiments, the RNA-guided DNA-binding agent may be fused with no NLS. In some embodiments, the NLS may be a monopartite sequence, such as, *e.g.,* the SV40 NLS, PKKKRKV or PKKKRRV. In some embodiments, the NLS may be a bipartite sequence, such as the NLS of nucleoplasmin, KRPAATKKAGQAKKKK. In a specific embodiment, a single PKKKRKV NLS may be linked at the C-terminus of the RNA-guided DNA-binding agent. One or more linkers are optionally included at the fusion site.

In some embodiments, the heterologous functional domain may be capable of modifying the intracellular half-life of the RNA-guided DNA binding agent. In some embodiments, the half-life of the RNA-guided DNA binding agent may be increased. In some embodiments, the half-life of the RNA-guided DNA-binding agent may be reduced. In some embodiments, the heterologous functional domain may be capable of increasing the stability of the RNA-guided DNA-binding agent. In some embodiments, the heterologous functional domain may be capable of reducing the stability of the RNA-guided DNA-binding agent. In some embodiments, the heterologous functional domain may act as a signal peptide for protein degradation. In some embodiments, the protein degradation may be mediated by proteolytic enzymes, such as, for example, proteasomes, lysosomal proteases, or calpain proteases. In some embodiments, the heterologous functional domain may comprise a PEST sequence. In some embodiments, the RNA-guided DNA-binding agent may be modified by addition of ubiquitin or a polyubiquitin chain. In some embodiments, the ubiquitin may be a ubiquitin-like protein (UBL). Non-limiting examples of ubiquitin-like proteins include small ubiquitin-like modifier (SUMO), ubiquitin cross-reactive protein (UCRP, also known as interferon-stimulated gene-15 (ISG15)), ubiquitin-related modifier-1 (URM1), neuronal-precursor-cell-expressed developmentally downregulated protein-8 (NEDD8, also called Rub1 in *S. cerevisiae*), human leukocyte antigen F-associated (FAT10), autophagy-8 (ATG8) and -12 (ATG12), Fau ubiquitin-like protein (FUB1), membrane-anchored UBL (MUB), ubiquitin fold-modifier-1 (UFM1), and ubiquitin-like protein-5 (UBL5).

In some embodiments, the heterologous functional domain may be a marker domain. Non-limiting examples of marker domains include fluorescent proteins, purification tags, epitope tags, and reporter gene sequences. In some embodiments, the marker domain may be a fluorescent protein. Non-limiting examples of suitable fluorescent proteins include green fluorescent proteins (*e.g.,* GFP, GFP-2, tagGFP, turboGFP, sfGFP, EGFP, Emerald, Azami Green, Monomeric Azami Green, CopGFP, AceGFP, ZsGreen1 ), yellow fluorescent proteins (*e.g.,* YFP, EYFP, Citrine, Venus, YPet, PhiYFP, ZsYellow1), blue fluorescent proteins (*e.g.,* EBFP, EBFP2, Azurite, mKalamal, GFPuv, Sapphire, T-sapphire,), cyan fluorescent proteins (*e*.*g*., ECFP, Cerulean, CyPet, AmCyan1, Midoriishi-Cyan), red fluorescent proteins (*e.g.,* mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (mOrange, mKO, Kusabira-Orange, Monomeric Kusabira-Orange, mTangerine, tdTomato) or any other suitable fluorescent protein. In other embodiments, the marker domain may be a purification tag and/or an epitope tag. Non-limiting exemplary tags include glutathione-S-transferase (GST), chitin binding protein (CBP), maltose binding protein (MBP), thioredoxin (TRX), poly(NANP), tandem affinity purification (TAP) tag, myc, AcV5, AU1, AU5, E, ECS, E2, FLAG, HA, nus, Softag 1, Softag 3, Strep, SBP, Glu-Glu, HSV, KT3, S, S1, T7, V5, VSV-G, 6xHis, 8xHis, biotin carboxyl carrier protein (BCCP), poly-His, and calmodulin. Non-limiting exemplary reporter genes include glutathione-S-transferase (GST), horseradish peroxidase (HRP), chloramphenicol acetyltransferase (CAT), beta-galactosidase, beta-glucuronidase, luciferase, or fluorescent proteins.

In additional embodiments, the heterologous functional domain may target the RNA-guided DNA-binding agent to a specific organelle, cell type, tissue, or organ. In some embodiments, the heterologous functional domain may target the RNA-guided DNA-binding agent to mitochondria.

In further embodiments, the heterologous functional domain may be an effector domain. When the RNA-guided DNA-binding agent is directed to its target sequence, *e.g.,* when a Cas nuclease is directed to a target sequence by a gRNA, the effector domain may modify or affect the target sequence. In some embodiments, the effector domain may be chosen from a nucleic acid binding domain, a nuclease domain (e.g., a non-Cas nuclease domain), an epigenetic modification domain, a transcriptional activation domain, or a transcriptional repressor domain. In some embodiments, the heterologous functional domain is a nuclease, such as a FokI nuclease. See, e.g., U.S. Pat. No. 9,023,649. In some embodiments, the heterologous functional domain is a transcriptional activator or repressor. See, e.g., Qi et al., "Repurposing CRISPR as an RNA-guided platform for sequence-specific control of gene expression," Cell 152:1173-83 (2013); Perez-Pinera et al., "RNA-guided gene activation by CRISPR-Cas9-based transcription factors," Nat. Methods 10:973-6 (2013); Mali et al., "CAS9 transcriptional activators for target specificity screening and paired nickases for cooperative genome engineering," Nat. Biotechnol. 31:833-8 (2013); Gilbert et al., "CRISPR-mediated modular RNA-guided regulation of transcription in eukaryotes," Cell 154:442-51 (2013). As such, the RNA-guided DNA-binding agent essentially becomes a transcription factor that can be directed to bind a desired target sequence using a guide RNA. In certain embodiments, the DNA modification domain is a methylation domain, such as a demethylation or methyltransferase domain. In certain embodiments, the effector domain is a DNA modification domain, such as a base-editing domain. In particular embodiments, the DNA modification domain is a nucleic acid editing domain that introduces a specific modification into the DNA, such as a deaminase domain. *See, e.g.,* WO 2015/089406; U.S. 2016/0304846. The nucleic acid editing domains, deaminase domains, and Cas9 variants described in WO 2015/089406 and U.S. 2016/0304846 are hereby incorporated by reference.

The nuclease may comprise at least one domain that interacts with a guide RNA ("gRNA"). Additionally, the nuclease may be directed to a target sequence by a gRNA. In Class 2 Cas nuclease systems, the gRNA interacts with the nuclease as well as the target sequence, such that it directs binding to the target sequence. In some embodiments, the gRNA provides the specificity for the targeted cleavage, and the nuclease may be universal and paired with different gRNAs to cleave different target sequences. Class 2 Cas nuclease may pair with a gRNA scaffold structure of the types, orthologs, and exemplary species listed above.

### Guide RNA (gRNA)

In some embodiments of the present disclosure, the cargo for the LNP formulation includes at least one gRNA. The gRNA may guide the Cas nuclease or Class 2 Cas nuclease to a target sequence on a target nucleic acid molecule. In some embodiments, a gRNA binds with and provides specificity of cleavage by a Class 2 Cas nuclease. In some embodiments, the gRNA and the Cas nuclease may form a ribonucleoprotein (RNP), *e.g.,* a CRISPR/Cas complex such as a CRISPR/Cas9 complex which may be delivered by the LNP composition. In some embodiments, the CRISPR/Cas complex may be a Type-II CRISPR/Cas9 complex. In some embodiments, the CRISPR/Cas complex may be a Type-V CRISPR/Cas complex, such as a Cpf1/guide RNA complex. Cas nucleases and cognate gRNAs may be paired. The gRNA scaffold structures that pair with each Class 2 Cas nuclease vary with the specific CRISPR/Cas system.

"Guide RNA", "gRNA", and simply "guide" are used herein interchangeably to refer to either a crRNA (also known as CRISPR RNA), or the combination of a crRNA and a trRNA (also known as tracrRNA). The crRNA and trRNA may be associated as a single RNA molecule (single guide RNA, sgRNA) or in two separate RNA molecules (dual guide RNA, dgRNA). "Guide RNA" or "gRNA" refers to each type. The trRNA may be a naturally-occurring sequence, or a trRNA sequence with modifications or variations compared to naturally-occurring sequences.

As used herein, a "guide sequence" refers to a sequence within a guide RNA that is complementary to a target sequence and functions to direct a guide RNA to a target sequence for binding or modification (e.g., cleavage) by an RNA-guided DNA binding agent. A "guide sequence" may also be referred to as a "targeting sequence," or a "spacer sequence." A guide sequence can be 20 base pairs in length, e.g., in the case of *Streptococcus pyogenes* (i.e., Spy Cas9) and related Cas9 homologs/orthologs. Shorter or longer sequences can also be used as guides, e.g., 15-, 16-, 17-, 18-, 19-, 21-, 22-, 23-, 24-, or 25-nucleotides in length. In some embodiments, the target sequence is in a gene or on a chromosome, for example, and is complementary to the guide sequence. In some embodiments, the degree of complementarity or identity between a guide sequence and its corresponding target sequence may be about 75%, 80%, 85%, 90%, 95%, 96%, 97%, 98%, 99%, or 100%. In some embodiments, the guide sequence and the target region may be 100% complementary or identical. In other embodiments, the guide sequence and the target region may contain at least one mismatch. For example, the guide sequence and the target sequence may contain 1, 2, 3, or 4 mismatches, where the total length of the target sequence is at least 17, 18, 19, 20 or more base pairs. In some embodiments, the guide sequence and the target region may contain 1-4 mismatches where the guide sequence comprises at least 17, 18, 19, 20 or more nucleotides. In some embodiments, the guide sequence and the target region may contain 1, 2, 3, or 4 mismatches where the guide sequence comprises 20 nucleotides.

Target sequences for Cas proteins include both the positive and negative strands of genomic DNA (i.e., the sequence given and the sequence's reverse compliment), as a nucleic acid substrate for a Cas protein is a double stranded nucleic acid. Accordingly, where a guide sequence is said to be "complementary to a target sequence", it is to be understood that the guide sequence may direct a guide RNA to bind to the reverse complement of a target sequence. Thus, in some embodiments, where the guide sequence binds the reverse complement of a target sequence, the guide sequence is identical to certain nucleotides of the target sequence (e.g., the target sequence not including the PAM) except for the substitution of U for T in the guide sequence.

The length of the targeting sequence may depend on the CRISPR/Cas system and components used. For example, different Class 2 Cas nucleases from different bacterial species have varying optimal targeting sequence lengths. Accordingly, the targeting sequence may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length. In some embodiments, the targeting sequence length is 0, 1, 2, 3, 4, or 5 nucleotides longer or shorter than the guide sequence of a naturally-occurring CRISPR/Cas system. In certain embodiments, the Cas nuclease and gRNA scaffold will be derived from the same CRISPR/Cas system. In some embodiments, the targeting sequence may comprise or consist of 18-24 nucleotides. In some embodiments, the targeting sequence may comprise or consist of 19-21 nucleotides. In some embodiments, the targeting sequence may comprise or consist of 20 nucleotides.

In some embodiments, the sgRNA is a "Cas9 sgRNA" capable of mediating RNA-guided DNA cleavage by a Cas9 protein. In some embodiments, the sgRNA is a "Cpfl sgRNA" capable of mediating RNA-guided DNA cleavage by a Cpfl protein. In certain embodiments, the gRNA comprises a crRNA and tracr RNA sufficient for forming an active complex with a Cas9 protein and mediating RNA-guided DNA cleavage. In certain embodiments, the gRNA comprises a crRNA sufficient for forming an active complex with a Cpfl protein and mediating RNA-guided DNA cleavage. *See* Zetsche 2015.

Certain embodiments of the invention also provide nucleic acids, *e.g.,* expression cassettes, encoding the gRNA described herein. A "guide RNA nucleic acid" is used herein to refer to a guide RNA (*e.g.* an sgRNA or a dgRNA) and a guide RNA expression cassette, which is a nucleic acid that encodes one or more guide RNAs.

In some embodiments, the nucleic acid may be a DNA molecule. In some embodiments, the nucleic acid may comprise a nucleotide sequence encoding a crRNA. In some embodiments, the nucleotide sequence encoding the crRNA comprises a targeting sequence flanked by all or a portion of a repeat sequence from a naturally-occurring CRISPR/Cas system. In some embodiments, the nucleic acid may comprise a nucleotide sequence encoding a tracr RNA. In some embodiments, the crRNA and the tracr RNA may be encoded by two separate nucleic acids. In other embodiments, the crRNA and the tracr RNA may be encoded by a single nucleic acid. In some embodiments, the crRNA and the tracr RNA may be encoded by opposite strands of a single nucleic acid. In other embodiments, the crRNA and the tracr RNA may be encoded by the same strand of a single nucleic acid. In some embodiments, the gRNA nucleic acid encodes an sgRNA. In some embodiments, the gRNA nucleic acid encodes a Cas9 nuclease sgRNA. In come embodiments, the gRNA nucleic acid encodes a Cpfl nuclease sgRNA.

The nucleotide sequence encoding the guide RNA may be operably linked to at least one transcriptional or regulatory control sequence, such as a promoter, a 3' UTR, or a 5' UTR. In one example, the promoter may be a tRNA promoter, *e.g.,* tRNA^{Lys3}, or a tRNA chimera. *See* Mefferd et al., RNA. 2015 21: 1683-9; Scherer et al., Nucleic Acids Res. 2007 35: 2620-2628. In certain embodiments, the promoter may be recognized by RNA polymerase III (Pol III). Non-limiting examples of Pol III promoters also include U6 and H1 promoters. In some embodiments, the nucleotide sequence encoding the guide RNA may be operably linked to a mouse or human U6 promoter. In some embodiments, the gRNA nucleic acid is a modified nucleic acid. In certain embodiments, the gRNA nucleic acid includes a modified nucleoside or nucleotide. In some embodiments, the gRNA nucleic acid includes a 5' end modification, for example a modified nucleoside or nucleotide to stabilize and prevent integration of the nucleic acid. In some embodiments, the gRNA nucleic acid comprises a double-stranded DNA having a 5' end modification on each strand. In certain embodiments, the gRNA nucleic acid includes an inverted dideoxy-T or an inverted abasic nucleoside or nucleotide as the 5' end modification. In some embodiments, the gRNA nucleic acid includes a label such as biotin, desthiobioten-TEG, digoxigenin, and fluorescent markers, including, for example, FAM, ROX, TAMRA, and AlexaFluor.

In certain embodiments, more than one gRNA nucleic acid, such as a gRNA, can be used with a CRISPR/Cas nuclease system. Each gRNA nucleic acid may contain a different targeting sequence, such that the CRISPR/Cas system cleaves more than one target sequence. In some embodiments, one or more gRNAs may have the same or differing properties such as activity or stability within a CRISPR/Cas complex. Where more than one gRNA is used, each gRNA can be encoded on the same or on different gRNA nucleic acid. The promoters used to drive expression of the more than one gRNA may be the same or different.

### Modified RNAs

In certain embodiments, the LNP compositions comprise modified RNAs.

Modified nucleosides or nucleotides can be present in an RNA, for example a gRNA or mRNA. A gRNA or mRNA comprising one or more modified nucleosides or nucleotides, for example, is called a "modified" RNA to describe the presence of one or more non-naturally and/or naturally occurring components or configurations that are used instead of or in addition to the canonical A, G, C, and U residues. In some embodiments, a modified RNA is synthesized with a non-canonical nucleoside or nucleotide, here called "modified."

Modified nucleosides and nucleotides can include one or more of: (i) alteration, *e.g.,* replacement, of one or both of the non-linking phosphate oxygens and/or of one or more of the linking phosphate oxygens in the phosphodiester backbone linkage (an exemplary backbone modification); (ii) alteration, *e.g.,* replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.,* removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification). Certain embodiments comprise a 5' end modification to an mRNA, gRNA, or nucleic acid. Certain embodiments comprise a 3' end modification to an mRNA, gRNA, or nucleic acid. A modified RNA can contain 5' end and 3' end modifications. A modified RNA can contain one or more modified residues at non-terminal locations. In certain embodiments, a gRNA includes at least one modified residue. In certain embodiments, an mRNA includes at least one modified residue.

As used herein, a first sequence is considered to "comprise a sequence with at least X% identity to" a second sequence if an alignment of the first sequence to the second sequence shows that X% or more of the positions of the second sequence in its entirety are matched by the first sequence. For example, the sequence AAGA comprises a sequence with 100% identity to the sequence AAG because an alignment would give 100% identity in that there are matches to all three positions of the second sequence. The differences between RNA and DNA (generally the exchange of uridine for thymidine or vice versa) and the presence of nucleoside analogs such as modified uridines do not contribute to differences in identity or complementarity among polynucleotides as long as the relevant nucleotides (such as thymidine, uridine, or modified uridine) have the same complement (e.g., adenosine for all of thymidine, uridine, or modified uridine; another example is cytosine and 5-methylcytosine, both of which have guanosine or modified guanosine as a complement). Thus, for example, the sequence 5'-AXG where X is any modified uridine, such as pseudouridine, N1-methyl pseudouridine, or 5-methoxyuridine, is considered 100% identical to AUG in that both are perfectly complementary to the same sequence (5'-CAU). Exemplary alignment algorithms are the Smith-Waterman and Needleman-Wunsch algorithms, which are well-known in the art. One skilled in the art will understand what choice of algorithm and parameter settings are appropriate for a given pair of sequences to be aligned; for sequences of generally similar length and expected identity >50% for amino acids or >75% for nucleotides, the Needleman-Wunsch algorithm with default settings of the Needleman-Wunsch algorithm interface provided by the EBI at the www.ebi.ac.uk web server is generally appropriate.

### mRNAs

In some embodiments, a composition or formulation disclosed herein comprises an mRNA comprising an open reading frame (ORF) encoding an RNA-guided DNA binding agent, such as a Cas nuclease, or Class 2 Cas nuclease as described herein. In some embodiments, an mRNA comprising an ORF encoding an RNA-guided DNA binding agent, such as a Cas nuclease or Class 2 Cas nuclease, is provided, used, or administered. In some embodiments, the ORF encoding an RNA-guided DNA binding agent is a "modified RNA-guided DNA binding agent ORF" or simply a "modified ORF," which is used as shorthand to indicate that the ORF is modified in one or more of the following ways: (1) the modified ORF has a uridine content ranging from its minimum uridine content to 150% of the minimum uridine content; (2) the modified ORF has a uridine dinucleotide content ranging from its minimum uridine dinucleotide content to 150% of the minimum uridine dinucleotide content; (3) the modified ORF has at least 90% identity to any one of SEQ ID NOs: 1, 4, 7, 9, 10, 11, 12, 14, 15, 17, 18, 20, 21, 23, 24, 26, 27, 29, 30, 50, 52, 54, 65, or 66; (4) the modified ORF consists of a set of codons of which at least 75% of the codons are minimal uridine codon(s) for a given amino acid, e.g. the codon(s) with the fewest uridines (usually 0 or 1 except for a codon for phenylalanine, where the minimal uridine codon has 2 uridines); or (5) the modified ORF comprises at least one modified uridine. In some embodiments, the modified ORF is modified in at least two, three, or four of the foregoing ways. In some embodiments, the modified ORF comprises at least one modified uridine and is modified in at least one, two, three, or all of (1)-(4) above.

"Modified uridine" is used herein to refer to a nucleoside other than thymidine with the same hydrogen bond acceptors as uridine and one or more structural differences from uridine. In some embodiments, a modified uridine is a substituted uridine, i.e., a uridine in which one or more non-proton substituents (e.g., alkoxy, such as methoxy) takes the place of a proton. In some embodiments, a modified uridine is pseudouridine. In some embodiments, a modified uridine is a substituted pseudouridine, i.e., a pseudouridine in which one or more non-proton substituents (e.g., alkyl, such as methyl) takes the place of a proton. In some embodiments, a modified uridine is any of a substituted uridine, pseudouridine, or a substituted pseudouridine.

"Uridine position" as used herein refers to a position in a polynucleotide occupied by a uridine or a modified uridine. Thus, for example, a polynucleotide in which "100% of the uridine positions are modified uridines" contains a modified uridine at every position that would be a uridine in a conventional RNA (where all bases are standard A, U, C, or G bases) of the same sequence. Unless otherwise indicated, a U in a polynucleotide sequence of a sequence table or sequence listing in, or accompanying, this disclosure can be a uridine or a modified uridine.

**Table 1. Minimal Uridine Codons**

| | **Amino Acid** | **Minimal uridine codon** |
|---|---|---|
| A | Alanine | GCA or GCC or GCG |
| G | Glycine | GGA or GGC or GGG |
| V | Valine | GUC or GUA or GUG |
| D | Aspartic acid | GAC |
| E | Glutamic acid | GAA or GAG |
| I | Isoleucine | AUC or AUA or AUG |
| T | Threonine | ACA or ACC or ACG |
| N | Asparagine | AAC |
| K | Lysine | AAG or AAA |
| S | Serine | AGC |
| R | Arginine | AGA or AGG |
| L | Leucine | CUG or CUA or CUC |
| P | Proline | CCG or CCA or CCC |
| H | Histidine | CAC or CAA or CAG |
| Q | Glutamine | CAG or CAA |
| F | Phenylalanine | UUC |
| Y | Tyrosine | UAC |
| C | Cysteine | UGC |
| W | Tryptophan | UGG |
| M | Methionine | AUG |

In any of the foregoing embodiments, the modified ORF may consist of a set of codons of which at least 75%, 80%, 85%, 90%, 95%, 98%, 99%, or 100% of the codons are codons listed in the Table of Minimal Uridine Codons. In any of the foregoing embodiments, the modified ORF may comprise a sequence with at least 90%, 95%, 98%, 99%, or 100% identity to any one of SEQ ID NO: 1, 4, 7, 9, 10, 11, 12, 14, 15, 17, 18, 20, 21, 23, 24, 26, 27, 29, 30, 50, 52, 54, 65, or 66.

In any of the foregoing embodiments, the modified ORF may comprise a sequence with at least 90%, 95%, 98%, 99%, or 100% identity to any one of SEQ ID NO: 1, 4, 7, 9, 10, 11, 12, 14, 15, 17, 18, 20, 21, 23, 24, 26, 27, 29, 30, 50, 52, 54, 65, or 66.

In any of the foregoing embodiments, the modified ORF may have a uridine content ranging from its minimum uridine content to 150%, 145%, 140%, 135%, 130%, 125%, 120%, 115%, 110%, 105%, 104%, 103%, 102%, or 101% of the minimum uridine content.

In any of the foregoing embodiments, the modified ORF may have a uridine dinucleotide content ranging from its minimum uridine dinucleotide content to 150%, 145%, 140%, 135%, 130%, 125%, 120%, 115%, 110%, 105%, 104%, 103%, 102%, or 101% of the minimum uridine dinucleotide content.

In any of the foregoing embodiments, the modified ORF may comprise a modified uridine at least at one, a plurality of, or all uridine positions. In some embodiments, the modified uridine is a uridine modified at the 5 position, e.g., with a halogen, methyl, or ethyl. In some embodiments, the modified uridine is a pseudouridine modified at the 1 position, e.g., with a halogen, methyl, or ethyl. The modified uridine can be, for example, pseudouridine, N1-methyl-pseudouridine, 5-methoxyuridine, 5-iodouridine, or a combination thereof. In some embodiments, the modified uridine is 5-methoxyuridine. In some embodiments, the modified uridine is 5-iodouridine. In some embodiments, the modified uridine is pseudouridine. In some embodiments, the modified uridine is N1-methyl-pseudouridine. In some embodiments, the modified uridine is a combination of pseudouridine and N1-methyl-pseudouridine. In some embodiments, the modified uridine is a combination of pseudouridine and 5-methoxyuridine. In some embodiments, the modified uridine is a combination of N1-methyl pseudouridine and 5-methoxyuridine. In some embodiments, the modified uridine is a combination of 5-iodouridine and N1-methyl-pseudouridine. In some embodiments, the modified uridine is a combination of pseudouridine and 5-iodouridine. In some embodiments, the modified uridine is a combination of 5-iodouridine and 5-methoxyuridine.

In some embodiments, at least 10%, 15%, 20%, 25%, 30%, 35%, 40%, 45%, 50%, 55%, 60%, 65%, 70%, 75%, 80%, 85%, 90%, 95%, 98%, 99%, or 100% of the uridine positions in an mRNA according to the disclosure are modified uridines. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are modified uridines, e.g., 5-methoxyuridine, 5-iodouridine, N1-methyl pseudouridine, pseudouridine, or a combination thereof. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-methoxyuridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-iodouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-methoxyuridine, and the remainder are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-iodouridine, and the remainder are N1-methyl pseudouridine.

In any of the foregoing embodiments, the modified ORF may comprise a reduced uridine dinucleotide content, such as the lowest possible uridine dinucleotide (UU) content, e.g. an ORF that (a) uses a minimal uridine codon (as discussed above) at every position and (b) encodes the same amino acid sequence as the given ORF. The uridine dinucleotide (UU) content can be expressed in absolute terms as the enumeration of UU dinucleotides in an ORF or on a rate basis as the percentage of positions occupied by the uridines of uridine dinucleotides (for example, AUUAU would have a uridine dinucleotide content of 40% because 2 of 5 positions are occupied by the uridines of a uridine dinucleotide). Modified uridine residues are considered equivalent to uridines for the purpose of evaluating minimum uridine dinucleotide content.

In some embodiments, the mRNA comprises at least one UTR from an expressed mammalian mRNA, such as a constitutively expressed mRNA. An mRNA is considered constitutively expressed in a mammal if it is continually transcribed in at least one tissue of a healthy adult mammal. In some embodiments, the mRNA comprises a 5' UTR, 3' UTR, or 5' and 3' UTRs from an expressed mammalian RNA, such as a constitutively expressed mammalian mRNA. Actin mRNA is an example of a constitutively expressed mRNA.

In some embodiments, the mRNA comprises at least one UTR from Hydroxysteroid 17-Beta Dehydrogenase 4 (HSD17B4 or HSD), e.g., a 5' UTR from HSD. In some embodiments, the mRNA comprises at least one UTR from a globin mRNA, for example, human alpha globin (HBA) mRNA, human beta globin (HBB) mRNA, or Xenopus laevis beta globin (XBG) mRNA. In some embodiments, the mRNA comprises a 5' UTR, 3' UTR, or 5' and 3' UTRs from a globin mRNA, such as HBA, HBB, or XBG. In some embodiments, the mRNA comprises a 5' UTR from bovine growth hormone, cytomegalovirus (CMV), mouse Hba-a1, HSD, an albumin gene, HBA, HBB, or XBG. In some embodiments, the mRNA comprises a 3' UTR from bovine growth hormone, cytomegalovirus, mouse Hba-a1, HSD, an albumin gene, HBA, HBB, or XBG. In some embodiments, the mRNA comprises 5' and 3' UTRs from bovine growth hormone, cytomegalovirus, mouse Hba-a1, HSD, an albumin gene, HBA, HBB, XBG, heat shock protein 90 (Hsp90), glyceraldehyde 3-phosphate dehydrogenase (GAPDH), beta-actin, alpha-tubulin, tumor protein (p53), or epidermal growth factor receptor (EGFR).

In some embodiments, the mRNA comprises 5' and 3' UTRs that are from the same source, e.g., a constitutively expressed mRNA such as actin, albumin, or a globin such as HBA, HBB, or XBG.

In some embodiments, the mRNA does not comprise a 5' UTR, e.g., there are no additional nucleotides between the 5' cap and the start codon. In some embodiments, the mRNA comprises a Kozak sequence (described below) between the 5' cap and the start codon, but does not have any additional 5' UTR. In some embodiments, the mRNA does not comprise a 3' UTR, e.g., there are no additional nucleotides between the stop codon and the poly-A tail.

In some embodiments, the mRNA comprises a Kozak sequence. The Kozak sequence can affect translation initiation and the overall yield of a polypeptide translated from an mRNA. A Kozak sequence includes a methionine codon that can function as the start codon. A minimal Kozak sequence is NNNRUGN wherein at least one of the following is true: the first N is A or G and the second N is G. In the context of a nucleotide sequence, R means a purine (A or G). In some embodiments, the Kozak sequence is RNNRUGN, NNNRUGG, RNNRUGG, RNNAUGN, NNNAUGG, or RNNAUGG. In some embodiments, the Kozak sequence is rccRUGg with zero mismatches or with up to one or two mismatches to positions in lowercase. In some embodiments, the Kozak sequence is rccAUGg with zero mismatches or with up to one or two mismatches to positions in lowercase. In some embodiments, the Kozak sequence is gccRccAUGG with zero mismatches or with up to one, two, or three mismatches to positions in lowercase. In some embodiments, the Kozak sequence is gccAccAUG with zero mismatches or with up to one, two, three, or four mismatches to positions in lowercase. In some embodiments, the Kozak sequence is GCCACCAUG. In some embodiments, the Kozak sequence is gccgccRccAUGG with zero mismatches or with up to one, two, three, or four mismatches to positions in lowercase.

In some embodiments, the mRNA comprising an ORF encoding an RNA-guided DNA binding agent comprises a sequence having at least 90% identity to SEQ ID NO: 43, optionally wherein the ORF of SEQ ID NO: 43 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 44, optionally wherein the ORF of SEQ ID NO: 44 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 56, optionally wherein the ORF of SEQ ID NO: 56 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 57, optionally wherein the ORF of SEQ ID NO: 57 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: , optionally wherein the ORF of SEQ ID NO: 58 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 59, optionally wherein the ORF of SEQ ID NO: 59 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 60, optionally wherein the ORF of SEQ ID NO: 60 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprising an ORF encoding an RNA-guided DNA binding agent comprises a sequence having at least 90% identity to SEQ ID NO: 61, optionally wherein the ORF of SEQ ID NO: 61 (i.e., SEQ ID NO: 4) is substituted with an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the mRNA comprises an alternative ORF of any one of SEQ ID NO: 7, 9, 10, 11, 12, 14, 15, 17, 18, 20, 21, 23, 24, 26, 27, 29, 30, 50, 52, 54, 65, or 66.

In some embodiments, the degree of identity to the optionally substituted sequences of SEQ ID NOs 43, 44, or 56-61 is 95%. In some embodiments, the degree of identity to the optionally substituted sequences of SEQ ID NOs 43, 44, or 56-61 is 98%. In some embodiments, the degree of identity to the optionally substituted sequences of SEQ ID NOs 43, 44, or 56-61 is 99%. In some embodiments, the degree of identity to the optionally substituted sequences of SEQ ID NOs 43, 44, or 56-61 is 100%.

In some embodiments, an mRNA disclosed herein comprises a 5' cap, such as a Cap0, Cap1, or Cap2. A 5' cap is generally a 7-methylguanine ribonucleotide (which may be further modified, as discussed below e.g. with respect to ARCA) linked through a 5'-triphosphate to the 5' position of the first nucleotide of the 5'-to-3' chain of the mRNA, i.e., the first cap-proximal nucleotide. In Cap0, the riboses of the first and second cap-proximal nucleotides of the mRNA both comprise a 2'-hydroxyl. In Cap1, the riboses of the first and second transcribed nucleotides of the mRNA comprise a 2'-methoxy and a 2'-hydroxyl, respectively. In Cap2, the riboses of the first and second cap-proximal nucleotides of the mRNA both comprise a 2'-methoxy. See, e.g., Katibah et al. (2014) Proc Natl Acad Sci USA 111(33):12025-30; Abbas et al. (2017) Proc Natl Acad Sci USA 114(11):E2106-E2115. Most endogenous higher eukaryotic mRNAs, including mammalian mRNAs such as human mRNAs, comprise Cap1 or Cap2. Cap0 and other cap structures differing from Cap1 and Cap2 may be immunogenic in mammals, such as humans, due to recognition as "non-self" by components of the innate immune system such as IFIT-1 and IFIT-5, which can result in elevated cytokine levels including type I interferon. Components of the innate immune system such as IFIT-1 and IFIT-5 may also compete with eIF4E for binding of an mRNA with a cap other than Cap1 or Cap2, potentially inhibiting translation of the mRNA.

A cap can be included co-transcriptionally. For example, ARCA (anti-reverse cap analog; Thermo Fisher Scientific Cat. No. AM8045) is a cap analog comprising a 7-methylguanine 3'-methoxy-5'-triphosphate linked to the 5' position of a guanine ribonucleotide which can be incorporated in vitro into a transcript at initiation. ARCA results in a Cap0 cap in which the 2' position of the first cap-proximal nucleotide is hydroxyl. See, e.g., Stepinski et al., (2001) "Synthesis and properties of mRNAs containing the novel 'anti-reverse' cap analogs 7-methyl(3'-O-methyl)GpppG and 7-methyl(3'deoxy)GpppG," RNA 7: 1486-1495. The ARCA structure is shown below.

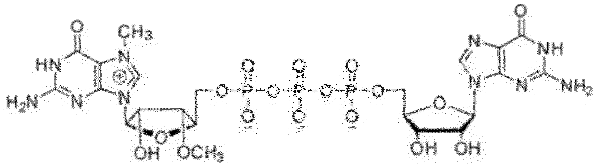

CleanCap^{™} AG (m7G(5')ppp(5')(2'OMeA)pG; TriLink Biotechnologies Cat. No. N-7113) or CleanCap^{™} GG (m7G(5')ppp(5')(2'OMeG)pG; TriLink Biotechnologies Cat. No. N-7133) can be used to provide a Cap1 structure co-transcriptionally. 3'-O-methylated versions of CleanCap^{™} AG and CleanCap^{™} GG are also available from TriLink Biotechnologies as Cat. Nos. N-7413 and N-7433, respectively. The CleanCap^{™} AG structure is shown below.

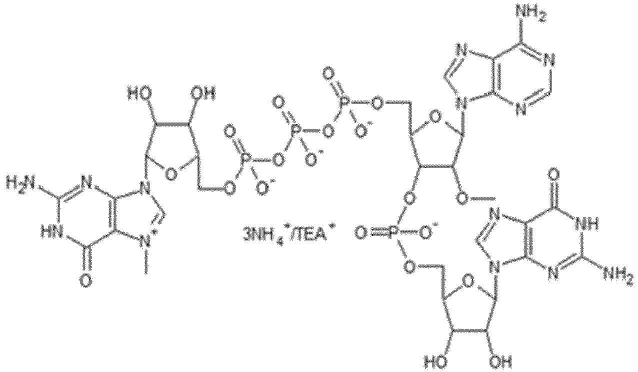

Alternatively, a cap can be added to an RNA post-transcriptionally. For example, Vaccinia capping enzyme is commercially available (New England Biolabs Cat. No. M2080S) and has RNA triphosphatase and guanylyltransferase activities, provided by its D1 subunit, and guanine methyltransferase, provided by its D12 subunit. As such, it can add a 7-methylguanine to an RNA, so as to give Cap0, in the presence of S-adenosyl methionine and GTP. See, e.g., Guo, P. and Moss, B. (1990) Proc. Natl. Acad. Sci. USA 87, 4023-4027; Mao, X. and Shuman, S. (1994) J. Biol. Chem. 269, 24472-24479.

In some embodiments, the mRNA further comprises a poly-adenylated (poly-A) tail. In some embodiments, the poly-A tail comprises at least 20, 30, 40, 50, 60, 70, 80, 90, or 100 adenines, optionally up to 300 adenines. In some embodiments, the poly-A tail comprises 95, 96, 97, 98, 99, or 100 adenine nucleotides. In some instances, the poly-A tail is "interrupted" with one or more non-adenine nucleotide "anchors" at one or more locations within the poly-A tail. The poly-A tails may comprise at least 8 consecutive adenine nucleotides, but also comprise one or more non-adenine nucleotide. As used herein, "non-adenine nucleotides" refer to any natural or non-natural nucleotides that do not comprise adenine. Guanine, thymine, and cytosine nucleotides are exemplary non-adenine nucleotides. Thus, the poly-A tails on the mRNA described herein may comprise consecutive adenine nucleotides located 3' to nucleotides encoding an RNA-guided DNA-binding agent or a sequence of interest. In some instances, the poly-A tails on mRNA comprise non-consecutive adenine nucleotides located 3' to nucleotides encoding an RNA-guided DNA-binding agent or a sequence of interest, wherein non-adenine nucleotides interrupt the adenine nucleotides at regular or irregularly spaced intervals.

In some embodiments, the mRNA further comprises a poly-adenylated (poly-A) tail. In some embodiments, the poly-A tail comprises at least 20, 30, 40, 50, 60, 70, 80, 90, or 100 adenines, optionally up to 300 adenines. In some embodiments, the poly-A tail comprises 95, 96, 97, 98, 99, or 100 adenine nucleotides. In some instances, the poly-A tail is "interrupted" with one or more non-adenine nucleotide "anchors" at one or more locations within the poly-A tail. The poly-A tails may comprise at least 8 consecutive adenine nucleotides, but also comprise one or more non-adenine nucleotide. As used herein, "non-adenine nucleotides" refer to any natural or non-natural nucleotides that do not comprise adenine. Guanine, thymine, and cytosine nucleotides are exemplary non-adenine nucleotides. Thus, the poly-A tails on the mRNA described herein may comprise consecutive adenine nucleotides located 3' to nucleotides encoding an RNA-guided DNA-binding agent or a sequence of interest. In some instances, the poly-A tails on mRNA comprise non-consecutive adenine nucleotides located 3' to nucleotides encoding an RNA-guided DNA-binding agent or a sequence of interest, wherein non-adenine nucleotides interrupt the adenine nucleotides at regular or irregularly spaced intervals.

In some embodiments, the one or more non-adenine nucleotides are positioned to interrupt the consecutive adenine nucleotides so that a poly(A) binding protein can bind to a stretch of consecutive adenine nucleotides. In some embodiments, one or more non-adenine nucleotide(s) is located after at least 8, 9, 10, 11, or 12 consecutive adenine nucleotides. In some embodiments, the one or more non-adenine nucleotide is located after at least 8-50 consecutive adenine nucleotides. In some embodiments, the one or more non-adenine nucleotide is located after at least 8-100 consecutive adenine nucleotides. In some embodiments, the non-adenine nucleotide is after one, two, three, four, five, six, or seven adenine nucleotides and is followed by at least 8 consecutive adenine nucleotides.

The poly-A tail may comprise one sequence of consecutive adenine nucleotides followed by one or more non-adenine nucleotides, optionally followed by additional adenine nucleotides.

In some embodiments, the poly-A tail comprises or contains one non-adenine nucleotide or one consecutive stretch of 2-10 non-adenine nucleotides. In some embodiments, the non-adenine nucleotide(s) is located after at least 8, 9, 10, 11, or 12 consecutive adenine nucleotides. In some instances, the one or more non-adenine nucleotides are located after at least 8-50 consecutive adenine nucleotides. In some embodiments, the one or more non-adenine nucleotides are located after at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 consecutive adenine nucleotides.

In some embodiments, the non-adenine nucleotide is guanine, cytosine, or thymine. In some instances, the non-adenine nucleotide is a guanine nucleotide. In some embodiments, the non-adenine nucleotide is a cytosine nucleotide. In some embodiments, the non-adenine nucleotide is a thymine nucleotide. In some instances, where more than one non-adenine nucleotide is present, the non-adenine nucleotide may be selected from: a) guanine and thymine nucleotides; b) guanine and cytosine nucleotides; c) thymine and cytosine nucleotides; or d) guanine, thymine and cytosine nucleotides. An exemplary poly-A tail comprising non-adenine nucleotides is provided as SEQ ID NO: 62.

In some embodiments, the mRNA is purified. In some embodiments, the mRNA is purified using a precipation method (*e.g.,* LiCl precipitation, alcohol precipitation, or an equivalent method, *e.g.,* as described herein). In some embodiments, the mRNA is purified using a chromatography-based method, such as an HPLC-based method or an equivalent method (*e.g.,* as described herein). In some embodiments, the mRNA is purified using both a precipitation method *(e.g.,* LiCl precipitation) and an HPLC-based method.

In some embodiments, at least one gRNA is provided in combination with an mRNA disclosed herein. In some embodiments, a gRNA is provided as a separate molecule from the mRNA. In some embodiments, a gRNA is provided as a part, such as a part of a UTR, of an mRNA disclosed herein.

### Chemically Modified gRNA

In some embodiments, the gRNA is chemically modified. A gRNA comprising one or more modified nucleosides or nucleotides is called a "modified" gRNA or "chemically modified" gRNA, to describe the presence of one or more non-naturally and/or naturally occurring components or configurations that are used instead of or in addition to the canonical A, G, C, and U residues. In some embodiments, a modified gRNA is synthesized with a non-canonical nucleoside or nucleotide, is here called "modified." Modified nucleosides and nucleotides can include one or more of: (i) alteration, *e.g.,* replacement, of one or both of the non-linking phosphate oxygens and/or of one or more of the linking phosphate oxygens in the phosphodiester backbone linkage (an exemplary backbone modification); (ii) alteration, *e.g.,* replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.,* removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification).

In some embodiments, a gRNA comprises a modified uridine at some or all uridine positions. In some embodiments, the modified uridine is a uridine modified at the 5 position, e.g., with a halogen or C1-C6 alkoxy. In some embodiments, the modified uridine is a pseudouridine modified at the 1 position, e.g., with a C1-C6 alkyl. The modified uridine can be, for example, pseudouridine, N1-methyl-pseudouridine, 5-methoxyuridine, 5-iodouridine, or a combination thereof. In some embodiments the modified uridine is 5-methoxyuridine. In some embodiments the modified uridine is 5-iodouridine. In some embodiments the modified uridine is pseudouridine. In some embodiments the modified uridine is N1-methyl-pseudouridine. In some embodiments, the modified uridine is a combination of pseudouridine and N1-methyl-pseudouridine. In some embodiments, the modified uridine is a combination of pseudouridine and 5-methoxyuridine. In some embodiments, the modified uridine is a combination of N1-methyl pseudouridine and 5-methoxyuridine. In some embodiments, the modified uridine is a combination of 5-iodouridine and N1-methyl-pseudouridine. In some embodiments, the modified uridine is a combination of pseudouridine and 5-iodouridine. In some embodiments, the modified uridine is a combination of 5-iodouridine and 5-methoxyuridine.

In some embodiments, at least 10%, 15%, 20%, 25%, 30%, 35%, 40%, 45%, 50%, 55%, 60%, 65%, 70%, 75%, 80%, 85%, 90%, 95%, 98%, 99%, or 100% of the uridine positions in a gRNA according to the disclosure are modified uridines. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are modified uridines, e.g., 5-methoxyuridine, 5-iodouridine, N1-methyl pseudouridine, pseudouridine, or a combination thereof. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-methoxyuridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-iodouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-methoxyuridine, and the remainder are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-iodouridine, and the remainder are N1-methyl pseudouridine.

Chemical modifications such as those listed above can be combined to provide modified gRNAs comprising nucleosides and nucleotides (collectively "residues") that can have two, three, four, or more modifications. For example, a modified residue can have a modified sugar and a modified nucleobase. In some embodiments, every base of a gRNA is modified, *e.g.,* all bases have a modified phosphate group, such as a phosphorothioate group. In certain embodiments, all, or substantially all, of the phosphate groups of an gRNA molecule are replaced with phosphorothioate groups. In some embodiments, modified gRNAs comprise at least one modified residue at or near the 5' end of the RNA. In some embodiments, modified gRNAs comprise at least one modified residue at or near the 3' end of the RNA.

In some embodiments, the gRNA comprises one, two, three or more modified residues. In some embodiments, at least 5% (*e.g.,* at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%) of the positions in a modified gRNA are modified nucleosides or nucleotides.

Unmodified nucleic acids can be prone to degradation by, *e.g.,* intracellular nucleases or those found in serum. For example, nucleases can hydrolyze nucleic acid phosphodiester bonds. Accordingly, in one aspect the gRNAs described herein can contain one or more modified nucleosides or nucleotides, *e.g.,* to introduce stability toward intracellular or serum-based nucleases. In some embodiments, the modified gRNA molecules described herein can exhibit a reduced innate immune response when introduced into a population of cells, both *in vivo* and *ex vivo.* The term "innate immune response" includes a cellular response to exogenous nucleic acids, including single stranded nucleic acids, which involves the induction of cytokine expression and release, particularly the interferons, and cell death.

In some embodiments of a backbone modification, the phosphate group of a modified residue can be modified by replacing one or more of the oxygens with a different substituent. Further, the modified residue, *e.g.,* modified residue present in a modified nucleic acid, can include the wholesale replacement of an unmodified phosphate moiety with a modified phosphate group as described herein. In some embodiments, the backbone modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. The phosphorous atom in an unmodified phosphate group is achiral. However, replacement of one of the non-bridging oxygens with one of the above atoms or groups of atoms can render the phosphorous atom chiral. The stereogenic phosphorous atom can possess either the "R" configuration (herein Rp) or the "S" configuration (herein Sp). The backbone can also be modified by replacement of a bridging oxygen, (*i.e.,* the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

The phosphate group can be replaced by non-phosphorus containing connectors in certain backbone modifications. In some embodiments, the charged phosphate group can be replaced by a neutral moiety. Examples of moieties which can replace the phosphate group can include, without limitation, *e.g.,* methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Template Nucleic Acid

The compositions and methods disclosed herein may include a template nucleic acid. The template may be used to alter or insert a nucleic acid sequence at or near a target site for a Cas nuclease. In some embodiments, the methods comprise introducing a template to the cell. In some embodiments, a single template may be provided. In other embodiments, two or more templates may be provided such that editing may occur at two or more target sites. For example, different templates may be provided to edit a single gene in a cell, or two different genes in a cell.

In some embodiments, the template may be used in homologous recombination. In some embodiments, the homologous recombination may result in the integration of the template sequence or a portion of the template sequence into the target nucleic acid molecule. In other embodiments, the template may be used in homology-directed repair, which involves DNA strand invasion at the site of the cleavage in the nucleic acid. In some embodiments, the homology-directed repair may result in including the template sequence in the edited target nucleic acid molecule. In yet other embodiments, the template may be used in gene editing mediated by non-homologous end joining. In some embodiments, the template sequence has no similarity to the nucleic acid sequence near the cleavage site. In some embodiments, the template or a portion of the template sequence is incorporated. In some embodiments, the template includes flanking inverted terminal repeat (ITR) sequences.

In some embodiments, the template may comprise a first homology arm and a second homology arm (also called a first and second nucleotide sequence) that are complementary to sequences located upstream and downstream of the cleavage site, respectively. Where a template contains two homology arms, each arm can be the same length or different lengths, and the sequence between the homology arms can be substantially similar or identical to the target sequence between the homology arms, or it can be entirely unrelated. In some embodiments, the degree of complementarity or percent identity between the first nucleotide sequence on the template and the sequence upstream of the cleavage site, and between the second nucleotide sequence on the template and the sequence downstream of the cleavage site, may permit homologous recombination, such as, e.g., high-fidelity homologous recombination, between the template and the target nucleic acid molecule. In some embodiments, the degree of complementarity may be about 50%, 55%, 60%, 65%, 70%, 75%, 80%, 85%, 90%, 95%, 97%, 98%, 99%, or 100%. In some embodiments, the degree of complementarity may be about 95%, 97%, 98%, 99%, or 100%. In some embodiments, the degree of complementarity may be at least 98%, 99%, or 100%. In some embodiments, the degree of complementarity may be 100%. In some embodiments, the percent identity may be about 50%, 55%, 60%, 65%, 70%, 75%, 80%, 85%, 90%, 95%, 97%, 98%, 99%, or 100%. In some embodiments, the percent identity may be about 95%, 97%, 98%, 99%, or 100%. In some embodiments, the percent identity may be at least 98%, 99%, or 100%. In some embodiments, the percent identity may be 100%.

In some embodiments, the template sequence may correspond to, comprise, or consist of an endogenous sequence of a target cell. It may also or alternatively correspond to, comprise, or consist of an exogenous sequence of a target cell. As used herein, the term "endogenous sequence" refers to a sequence that is native to the cell. The term "exogenous sequence" refers to a sequence that is not native to a cell, or a sequence whose native location in the genome of the cell is in a different location. In some embodiments, the endogenous sequence may be a genomic sequence of the cell. In some embodiments, the endogenous sequence may be a chromosomal or extrachromosomal sequence. In some embodiments, the endogenous sequence may be a plasmid sequence of the cell. In some embodiments, the template sequence may be substantially identical to a portion of the endogenous sequence in a cell at or near the cleavage site, but comprise at least one nucleotide change. In some embodiments, editing the cleaved target nucleic acid molecule with the template may result in a mutation comprising an insertion, deletion, or substitution of one or more nucleotides of the target nucleic acid molecule. In some embodiments, the mutation may result in one or more amino acid changes in a protein expressed from a gene comprising the target sequence. In some embodiments, the mutation may result in one or more nucleotide changes in an RNA expressed from the target gene. In some embodiments, the mutation may alter the expression level of the target gene. In some embodiments, the mutation may result in increased or decreased expression of the target gene. In some embodiments, the mutation may result in gene knock-down. In some embodiments, the mutation may result in gene knock-out. In some embodiments, the mutation may result in restored gene function. In some embodiments, editing of the cleaved target nucleic acid molecule with the template may result in a change in an exon sequence, an intron sequence, a regulatory sequence, a transcriptional control sequence, a translational control sequence, a splicing site, or a non-coding sequence of the target nucleic acid molecule, such as DNA.

In other embodiments, the template sequence may comprise an exogenous sequence. In some embodiments, the exogenous sequence may comprise a protein or RNA coding sequence operably linked to an exogenous promoter sequence such that, upon integration of the exogenous sequence into the target nucleic acid molecule, the cell is capable of expressing the protein or RNA encoded by the integrated sequence. In other embodiments, upon integration of the exogenous sequence into the target nucleic acid molecule, the expression of the integrated sequence may be regulated by an endogenous promoter sequence. In some embodiments, the exogenous sequence may provide a cDNA sequence encoding a protein or a portion of the protein. In yet other embodiments, the exogenous sequence may comprise or consist of an exon sequence, an intron sequence, a regulatory sequence, a transcriptional control sequence, a translational control sequence, a splicing site, or a non-coding sequence. In some embodiments, the integration of the exogenous sequence may result in restored gene function. In some embodiments, the integration of the exogenous sequence may result in a gene knock-in. In some embodiments, the integration of the exogenous sequence may result in a gene knock-out.

The template may be of any suitable length. In some embodiments, the template may comprise 10, 15, 20, 25, 50, 75, 100, 150, 200, 500, 1000, 1500, 2000, 2500, 3000, 3500, 4000, 4500, 5000, 5500, 6000, or more nucleotides in length. The template may be a single-stranded nucleic acid. The template can be double-stranded or partially double-stranded nucleic acid. In certain embodiments, the single stranded template is 20, 30, 40, 50, 75, 100, 125, 150, 175, or 200 nucleotides in length. In some embodiments, the template may comprise a nucleotide sequence that is complementary to a portion of the target nucleic acid molecule comprising the target sequence (*i.e.,* a "homology arm"). In some embodiments, the template may comprise a homology arm that is complementary to the sequence located upstream or downstream of the cleavage site on the target nucleic acid molecule.

In some embodiments, the template contains ssDNA or dsDNA containing flanking invert-terminal repeat (ITR) sequences. In some embodiments, the template is provided as a vector, plasmid, minicircle, nanocircle, or PCR product.

### Purification of Nucleic Acids

In some embodiments, the nucleic acid is purified. In some embodiments, the nucleic acid is purified using a precipation method *(̅e.g.,* LiCl precipitation, alcohol precipitation, or an equivalent method, *e.g.,* as described herein). In some embodiments, the nucleic acid is purified using a chromatography-based method, such as an HPLC-based method or an equivalent method (*e.g.,* as described herein). In some embodiments, the nucleic is purified using both a precipitation method (e.g., LiCl precipitation) and an HPLC-based method.

### Target Sequences

In some embodiments, a CRISPR/Cas system of the present disclosure may be directed to and cleave a target sequence on a target nucleic acid molecule. For example, the target sequence may be recognized and cleaved by the Cas nuclease. In certain embodiments, a target sequence for a Cas nuclease is located near the nuclease's cognate PAM sequence. In some embodiments, a Class 2 Cas nuclease may be directed by a gRNA to a target sequence of a target nucleic acid molecule, where the gRNA hybridizes with and the Class 2 Cas protein cleaves the target sequence. In some embodiments, the guide RNA hybridizes with and a Class 2 Cas nuclease cleaves the target sequence adjacent to or comprising its cognate PAM. In some embodiments, the target sequence may be complementary to the targeting sequence of the guide RNA. In some embodiments, the degree of complementarity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. In some embodiments, the percent identity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. In some embodiments, the homology region of the target is adjacent to a cognate PAM sequence. In some embodiments, the target sequence may comprise a sequence 100% complementary with the targeting sequence of the guide RNA. In other embodiments, the target sequence may comprise at least one mismatch, deletion, or insertion, as compared to the targeting sequence of the guide RNA.

The length of the target sequence may depend on the nuclease system used. For example, the targeting sequence of a guide RNA for a CRISPR/Cas system may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length and the target sequence is a corresponding length, optionally adjacent to a PAM sequence. In some embodiments, the target sequence may comprise 15-24 nucleotides in length. In some embodiments, the target sequence may comprise 17-21 nucleotides in length. In some embodiments, the target sequence may comprise 20 nucleotides in length. When nickases are used, the target sequence may comprise a pair of target sequences recognized by a pair of nickases that cleave opposite strands of the DNA molecule. In some embodiments, the target sequence may comprise a pair of target sequences recognized by a pair of nickases that cleave the same strands of the DNA molecule. In some embodiments, the target sequence may comprise a part of target sequences recognized by one or more Cas nucleases.

The target nucleic acid molecule may be any DNA or RNA molecule that is endogenous or exogenous to a cell. In some embodiments, the target nucleic acid molecule may be an episomal DNA, a plasmid, a genomic DNA, viral genome, mitochondrial DNA, or chromosomal DNA from a cell or in the cell. In some embodiments, the target sequence of the target nucleic acid molecule may be a genomic sequence from a cell or in a cell, including a human cell.

In further embodiments, the target sequence may be a viral sequence. In further embodiments, the target sequence may be a pathogen sequence. In yet other embodiments, the target sequence may be a synthesized sequence. In further embodiments, the target sequence may be a chromosomal sequence. In certain embodiments, the target sequence may comprise a translocation junction, e.g., a translocation associated with a cancer. In some embodiments, the target sequence may be on a eukaryotic chromosome, such as a human chromosome. In certain embodiments, the target sequence is a liver-specific sequence, in that it is expressed in liver cells.

In some embodiments, the target sequence may be located in a coding sequence of a gene, an intron sequence of a gene, a regulatory sequence, a transcriptional control sequence of a gene, a translational control sequence of a gene, a splicing site or a non-coding sequence between genes. In some embodiments, the gene may be a protein coding gene. In other embodiments, the gene may be a non-coding RNA gene. In some embodiments, the target sequence may comprise all or a portion of a disease-associated gene. In some embodiments, the target sequence may be located in a non-genic functional site in the genome, for example a site that controls aspects of chromatin organization, such as a scaffold site or locus control region.

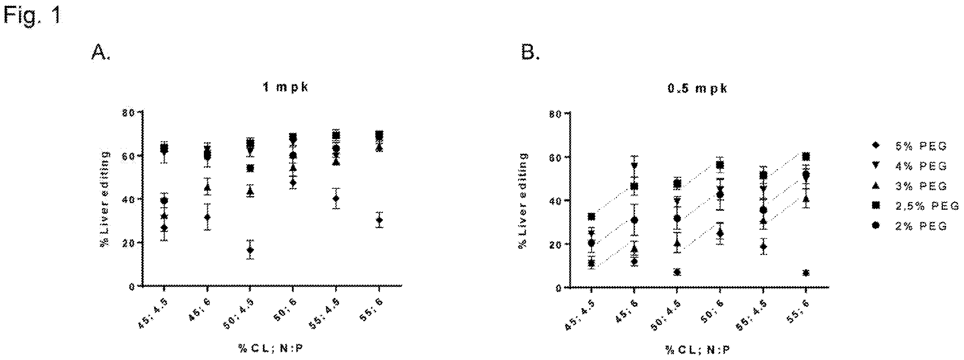

In embodiments involving a Cas nuclease, such as a Class 2 Cas nuclease, the target sequence may be adjacent to a protospacer adjacent motif ("PAM"). In some embodiments, the PAM may be adjacent to or within 1, 2, 3, or 4, nucleotides of the 3' end of the target sequence. The length and the sequence of the PAM may depend on the Cas protein used. For example, the PAM may be selected from a consensus or a particular PAM sequence for a specific Cas9 protein or Cas9 ortholog, including those disclosed in Figure 1 of Ran et al., Nature, 520: 186-191 (2015), and Figure S5 of Zetsche 2015, the relevant disclosure of each of which is incorporated herein by reference. In some embodiments, the PAM may be 2, 3, 4, 5, 6, 7, 8, 9, or 10 nucleotides in length. Non-limiting exemplary PAM sequences include NGG, NGGNG, NG, NAAAAN, NNAAAAW, NNNNACA, GNNNCNNA, TTN, and NNNNGATT (wherein N is defined as any nucleotide, and W is defined as either A or T). In some embodiments, the PAM sequence may be NGG. In some embodiments, the PAM sequence may be NGGNG. In some embodiments, the PAM sequence may be TTN. In some embodiments, the PAM sequence may be NNAAAAW.

### Lipid Formulation

Disclosed herein are various embodiments of LNP formulations for RNAs, including CRISPR/Cas cargos. Such LNP formulations include an "amine lipid", along with a helper lipid, a neutral lipid, and a PEG lipid. In some embodiments, such LNP formulations include an "amine lipid", along with a helper lipid and a PEG lipid. In some embodiments, the LNP formulations include less than 1 percent neutral phospholipid. In some embodiments, the LNP formulations include less than 0.5 percent neutral phospholipid. By "lipid nanoparticle" is meant a particle that comprises a plurality of (*i.e.* more than one) lipid molecules physically associated with each other by intermolecular forces.

### Amine Lipids

The LNP compositions for the delivery of biologically active agents comprise an "amine lipid", which is defined as Lipid A or its equivalents, including acetal analogs of Lipid A.

In some embodiments, the amine lipid is Lipid A, which is (9Z,12Z)-3-((4,4-bis(octyloxy)butanoyl)oxy)-2-((((3-(diethylamino)propoxy)carbonyl)oxy)methyl) propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate. Lipid A can be depicted as:

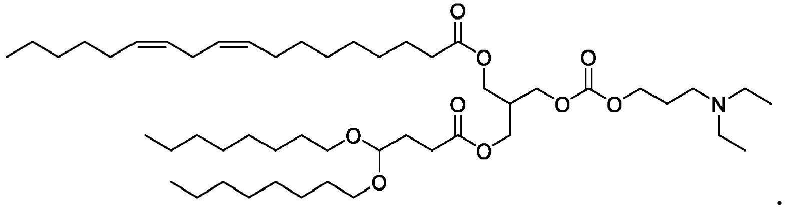

Lipid A may be synthesized according to WO2015/095340 *(e.g.,* pp. 84-86). In certain embodiments, the amine lipid is an equivalent to Lipid A.

In certain embodiments, an amine lipid is an analog of Lipid A. In certain embodiments, a Lipid A analog is an acetal analog of Lipid A. In particular LNP compositions, the acetal analog is a C4-C12 acetal analog. In some embodiments, the acetal analog is a C5-C12 acetal analog. In additional embodiments, the acetal analog is a C5-C10 acetal analog. In further embodiments, the acetal analog is chosen from a C4, C5, C6, C7, C9, C10, C11, and C12 acetal analog.

Amine lipids suitable for use in the LNPs described herein are biodegradable *in vivo* and suitable for delivering a biologically active agent, such as an RNA to a cell. The amine lipids have low toxicity (*e.g.,* are tolerated in an animal model without adverse effect in amounts of greater than or equal to 10 mg/kg of RNA cargo). In certain embodiments, LNPs comprising an amine lipid include those where at least 75% of the amine lipid is cleared from the plasma within 8, 10, 12, 24, or 48 hours, or 3, 4, 5, 6, 7, or 10 days. In certain embodiments, LNPs comprising an amine lipid include those where at least 50% of the mRNA or gRNA is cleared from the plasma within 8, 10, 12, 24, or 48 hours, or 3, 4, 5, 6, 7, or 10 days. In certain embodiments, LNPs comprising an amine lipid include those where at least 50% of the LNP is cleared from the plasma within 8, 10, 12, 24, or 48 hours, or 3, 4, 5, 6, 7, or 10 days, for example by measuring a lipid (e.g., an amine lipid), RNA (e.g., mRNA), or another component. In certain embodiments, lipid-encapsulated versus free lipid, RNA, or nucleic acid component of the LNP is measured.

Lipid clearance may be measured as described in literature. *See* Maier, M.A., et al. Biodegradable Lipids Enabling Rapidly Eliminated Lipid Nanoparticles for Systemic Delivery of RNAi Therapeutics. Mol. Ther. 2013, 21(8), 1570-78 ("*Maier*")*.* For example, in *Maier,* LNP-siRNA systems containing luciferases-targeting siRNA were administered to six- to eight-week old male C57Bl/6 mice at 0.3 mg/kg by intravenous bolus injection *via* the lateral tail vein. Blood, liver, and spleen samples were collected at 0.083, 0.25, 0.5, 1, 2, 4, 8, 24, 48, 96, and 168 hours post-dose. Mice were perfused with saline before tissue collection and blood samples were processed to obtain plasma. All samples were processed and analyzed by LC-MS. Further, *Maier* describes a procedure for assessing toxicity after administration of LNP-siRNA formulations. For example, a luciferase-targeting siRNA was administered at 0, 1, 3, 5, and 10 mg/kg (5 animals/group) via single intravenous bolus injection at a dose volume of 5 mL/kg to male Sprague-Dawley rats. After 24 hours, about 1 mL of blood was obtained from the jugular vein of conscious animals and the serum was isolated. At 72 hours post-dose, all animals were euthanized for necropsy. Assessments of clinical signs, body weight, serum chemistry, organ weights and histopathology were performed. *Although Maier* describes methods for assessing siRNA-LNP formulations, these methods may be applied to assess clearance, pharmacokinetics, and toxicity of administration of LNP compositions of the present disclosure.

The amine lipids may lead to an increased clearance rate. In some embodiments, the clearance rate is a lipid clearance rate, for example the rate at which a lipid is cleared from the blood, serum, or plasma. In some embodiments, the clearance rate is an RNA clearance rate, for example the rate at which an mRNA or a gRNA is cleared from the blood, serum, or plasma. In some embodiments, the clearance rate is the rate at which LNP is cleared from the blood, serum, or plasma. In some embodiments, the clearance rate is the rate at which LNP is cleared from a tissue, such as liver tissue or spleen tissue. In certain embodiments, a high clearance rate leads to a safety profile with no substantial adverse effects. The amine lipids may reduce LNP accumulation in circulation and in tissues. In some embodiments, a reduction in LNP accumulation in circulation and in tissues leads to a safety profile with no substantial adverse effects.

The amine lipids of the present disclosure are ionizable (e.g., may form a salt) depending upon the pH of the medium they are in. For example, in a slightly acidic medium, the amine lipids may be protonated and thus bear a positive charge. Conversely, in a slightly basic medium, such as, for example, blood, where pH is approximately 7.35, the amine lipids may not be protonated and thus bear no charge. In some embodiments, the amine lipids of the present disclosure may be protonated at a pH of at least about 9. In some embodiments, the amine lipids of the present disclosure may be protonated at a pH of at least about 9. In some embodiments, the amine lipids of the present disclosure may be protonated at a pH of at least about 10.

The pH at which an amine lipid is predominantly protonated is related to its intrinsic pKa. In some embodiments, the amine lipids of the present disclosure may each, independently, have a pKa in the range of from about 5.1 to about 7.4. In some embodiments, the amine lipids of the present disclosure may each, independently, have a pKa in the range of from about 5.5 to about 6.6. In some embodiments, the amine lipids of the present disclosure may each, independently, have a pKa in the range of from about 5.6 to about 6.4. In some embodiments, the amine lipids of the present disclosure may each, independently, have a pKa in the range of from about 5.8 to about 6.2. For example, the amine lipids of the present disclosure may each, independently, have a pKa in the range of from about 5.8 to about 6.5. The pKa of an amine lipid can be an important consideration in formulating LNPs as it has been found that cationic lipids with a pKa ranging from about 5.1 to about 7.4 are effective for delivery of cargo in vivo, e.g., to the liver. Furthermore, it has been found that cationic lipids with a pKa ranging from about 5.3 to about 6.4 are effective for delivery in vivo, e.g., to tumors. *See, e.g.,* WO 2014/136086.

### Additional Lipids

"Neutral lipids" suitable for use in a lipid composition of the disclosure include, for example, a variety of neutral, uncharged or zwitterionic lipids. Examples of neutral phospholipids suitable for use in the present disclosure include, but are not limited to, 5-heptadecylbenzene-1,3-diol (resorcinol), dipalmitoylphosphatidylcholine (DPPC), distearoylphosphatidylcholine (DSPC), pohsphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauryloylphosphatidylcholine (DLPC), dimyristoylphosphatidylcholine (DMPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero-3-phosphocholine (DEPC), palmitoyloleoyl phosphatidylcholine (POPC), lysophosphatidyl choline, dioleoyl phosphatidylethanolamine (DOPE), dilinoleoylphosphatidylcholine distearoylphosphatidylethanolamine (DSPE), dimyristoyl phosphatidylethanolamine (DMPE), dipalmitoyl phosphatidylethanolamine (DPPE), palmitoyloleoyl phosphatidylethanolamine (POPE), lysophosphatidylethanolamine and combinations thereof. In one embodiment, the neutral phospholipid may be selected from the group consisting of distearoylphosphatidylcholine (DSPC) and dimyristoyl phosphatidyl ethanolamine (DMPE). In another embodiment, the neutral phospholipid may be distearoylphosphatidylcholine (DSPC). In another embodiment, the neutral phospholipid may be dipalmitoylphosphatidylcholine (DPPC).

"Helper lipids" include steroids, sterols, and alkyl resorcinols. Helper lipids suitable for use in the present disclosure include, but are not limited to, cholesterol, 5-heptadecylresorcinol, and cholesterol hemisuccinate. In one embodiment, the helper lipid may be cholesterol. In one embodiment, the helper lipid may be cholesterol hemisuccinate.

PEG lipids are stealth lipids that alter the length of time the nanoparticles can exist *in vivo* (*e.g.,* in the blood). PEG lipids may assist in the formulation process by, for example, reducing particle aggregation and controlling particle size. PEG lipids used herein may modulate pharmacokinetic properties of the LNPs. Typically, the PEG lipid comprises a lipid moiety and a polymer moiety based on PEG.

In some embodiments, the lipid moiety may be derived from diacylglycerol or diacylglycamide, including those comprising a dialkylglycerol or dialkylglycamide group having alkyl chain length independently comprising from about C4 to about C40 saturated or unsaturated carbon atoms, wherein the chain may comprise one or more functional groups such as, for example, an amide or ester. In some embodiments, the alkyl chain length comprises about C10 to C20. The dialkylglycerol or dialkylglycamide group can further comprise one or more substituted alkyl groups. The chain lengths may be symmetrical or assymetric.

Unless otherwise indicated, the term "PEG" as used herein means any polyethylene glycol or other polyalkylene ether polymer. In one embodiment, PEG moiety is an optionally substituted linear or branched polymer of ethylene glycol or ethylene oxide. In certain embodiments, PEG moiety is Alternatively, the PEG moiety may be substituted, *e.g.,* by one or more alkyl, alkoxy, acyl, hydroxy, or aryl groups. In one embodiment, the PEG moiety includes PEG copolymer such as PEG-polyurethane or PEG-polypropylene (see, *e.g.,* J. Milton Harris, Polyethylene glycol) chemistry: biotechnical and biomedical applications (1992)); alternatively, the PEG moiety does not include PEG copolymers, e.g., it may be a PEG monopolymer. In one embodiment, the PEG has a molecular weight of from about 130 to about 50,000, in a sub-embodiment, about 150 to about 30,000, in a sub-embodiment, about 150 to about 20,000, in a sub-embodiment about 150 to about 15,000, in a sub-embodiment, about 150 to about 10,000, in a sub-embodiment, about 150 to about 6,000, in a sub-embodiment, about 150 to about 5,000, in a sub-embodiment, about 150 to about 4,000, in a sub-embodiment, about 150 to about 3,000, in a sub-embodiment, about 300 to about 3,000, in a sub-embodiment, about 1,000 to about 3,000, and in a sub-embodiment, about 1,500 to about 2,500.

In certain embodiments, the PEG (*e.g.,* conjugated to a lipid moiety or lipid, such as a stealth lipid), is a "PEG-2K," also termed "PEG 2000," which has an average molecular weight of about 2,000 daltons. PEG-2K is represented herein by the following formula (I), wherein n is 45, meaning that the number averaged degree of polymerization comprises about 45 subunits 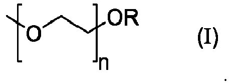 However, other PEG embodiments known in the art may be used, including, *e.g.,* those where the number-averaged degree of polymerization comprises about 23 subunits (n=23), and/or 68 subunits (n=68). In some embodiments, n may range from about 30 to about 60. In some embodiments, n may range from about 35 to about 55. In some embodiments, n may range from about 40 to about 50. In some embodiments, n may range from about 42 to about 48. In some embodiments, n may be 45. In some embodiments, R may be selected from H, substituted alkyl, and unsubstituted alkyl. In some embodiments, R may be unsubstituted alkyl. In some embodiments, R may be methyl.

In any of the embodiments described herein, the PEG lipid may be selected from PEG-dilauroylglycerol, PEG-dimyristoylglycerol (PEG-DMG) (catalog # GM-020 from NOF, Tokyo, Japan), PEG-dipalmitoylglycerol, PEG-distearoylglycerol (PEG-DSPE) (catalog # DSPE-020CN, NOF, Tokyo, Japan), PEG-dilaurylglycamide, PEG-dimyristylglycamide, PEG-dipalmitoylglycamide, and PEG-distearoylglycamide, PEG-cholesterol (1-[8'-(Cholest-5-en-3[beta]-oxy)carboxamido-3',6'-dioxaoctanyl]carbamoyl-[omega]-methyl-poly(ethylene glycol), PEG-DMB (3,4-ditetradecoxylbenzyl-[omega]-methyl-poly(ethylene glycol)ether), 1,2-dimyristoyl-sn-glycero-3-phosphoethanolamine-N-[methoxy(polyethylene glycol)-2000] (PEG2k-DMG) (cat. #880150P from Avanti Polar Lipids, Alabaster, Alabama, USA), 1,2-distearoyl-sn-glycero-3-phosphoethanolamine-N-[methoxy(polyethylene glycol)-2000] (PEG2k-DSPE) (cat. #880120C from Avanti Polar Lipids, Alabaster, Alabama, USA), 1,2-distearoyl-sn-glycerol, methoxypolyethylene glycol (PEG2k-DSG; GS-020, NOF Tokyo, Japan), poly(ethylene glycol)-2000-dimethacrylate (PEG2k-DMA), and 1,2-distearyloxypropyl-3-amine-N-[methoxy(polyethylene glycol)-2000] (PEG2k-DSA). In one embodiment, the PEG lipid may be PEG2k-DMG. In some embodiments, the PEG lipid may be PEG2k-DSG. In one embodiment, the PEG lipid may be PEG2k-DSPE. In one embodiment, the PEG lipid may be PEG2k-DMA. In one embodiment, the PEG lipid may be PEG2k-C-DMA. In one embodiment, the PEG lipid may be compound S027, disclosed in WO2016/010840 at paragraphs [00240] to [00244]. In one embodiment, the PEG lipid may be PEG2k-DSA. In one embodiment, the PEG lipid may be PEG2k-C11. In some embodiments, the PEG lipid may be PEG2k-C14. In some embodiments, the PEG lipid may be PEG2k-C16. In some embodiments, the PEG lipid may be PEG2k-C18.

### LNP Formulations

Embodiments of the present disclosure provide lipid compositions described according to the respective molar ratios of the component lipids in the formulation. In one embodiment, the mol-% of the amine lipid may be from about 30 mol-% to about 60 mol-%. In one embodiment, the mol-% of the amine lipid may be from about 40 mol-% to about 60 mol-%. In one embodiment, the mol-% of the amine lipid may be from about 45 mol-% to about 60 mol-%. In one embodiment, the mol-% of the amine lipid may be from about 50 mol-% to about 60 mol-%. In one embodiment, the mol-% of the amine lipid may be from about 55 mol-% to about 60 mol-%. In one embodiment, the mol-% of the amine lipid may be from about 50 mol-% to about 55 mol-%. In one embodiment, the mol-% of the amine lipid may be about 50 mol-%. In one embodiment, the mol-% of the amine lipid may be about 55 mol-%. In some embodiments, the amine lipid mol-% of the LNP batch will be ±30%, ±25%, ±20%, ±15%, ±10%, ±5%, or ±2.5% of the target mol-%. In some embodiments, the amine lipid mol-% of the LNP batch will be ±4 mol-%, ±3 mol-%, ±2 mol-%, ±1.5 mol-%, ±1 mol-%, ±0.5 mol-%, or ±0.25 mol-% of the target mol-%. All mol-% numbers are given as a fraction of the lipid component of the LNP compositions. In certain embodiments, LNP inter-lot variability of the amine lipid mol-% will be less than 15%, less than 10% or less than 5%.

In one embodiment, the mol-% of the neutral lipid, e.g., neutral phospholipid, may be from about 5 mol-% to about 15 mol-%. In one embodiment, the mol-% of the neutral lipid, e.g., neutral phospholipid, may be from about 7 mol-% to about 12 mol-%. In one embodiment, the mol-% of the neutral lipid, e.g., neutral phospholipid, may be from about 0 mol-% to about 5 mol-%. In one embodiment, the mol-% of the neutral lipid, e.g., neutral phospholipid, may be from about 0 mol-% to about 10 mol-%. In one embodiment, the mol-% of the neutral lipid, e.g., neutral phospholipid, may be from about 5 mol-% to about 10 mol-%. In one embodiment, the mol-% of the neutral lipid, e.g., neutral phospholipid, may be from about 8 mol-% to about 10 mol-%.

In one embodiment, the mol-% of the neutral lipid, e.g., neutral phospholipid, may be about 5 mol-%, about 6 mol-%, about 7 mol-%, about 8 mol-%, about 9 mol-%, about 10 mol-%, about 11 mol-%, about 12 mol-%, about 13 mol-%, about 14 mol-%, or about 15 mol-%. In one embodiment, the mol-% of the neutral lipid, e.g., neutral phospholipid, may be about 9 mol-%.

In one embodiment, the mol-% of the neutral lipid, e.g., neutral phospholipid, may be from about 1 mol-% to about 5 mol-%. In one embodiment, the mol-% of the neutral lipid may be from about 0.1 mol-% to about 1 mol-%. In one embodiment, the mol-% of the neutral lipid such as neutral phospholipid may be about 0.1 mol-%, about 0.2 mol-%, about 0.5 mol-%, 1 mol-%, about 1.5 mol-%, about 2 mol-%, about 2.5 mol-%, about 3 mol-%, about 3.5 mol-%, about 4 mol-%, about 4.5 mol-%, or about 5 mol-%.

In one embodiment, the mol-% of the neutral lipid, e.g., neutral phospholipid, may be less than about 1 mol-%. In one embodiment, the mol-% of the neutral lipid, e.g., neutral phospholipid, may be less than about 0.5 mol-%. In one embodiment, the mol-% of the neutral lipid, e.g., neutral phospholipid, may be about 0 mol-%, about 0.1 mol-%, about 0.2 mol-%, about 0.3 mol-%, about 0.4 mol-%, about 0.5 mol-%, about 0.6 mol-%, about 0.7 mol-%, about 0.8 mol-%, about 0.9 mol-%, or about 1 mol-%. In some embodiments, the formulations disclosed herein are free of neutral lipid (i.e., 0 mol-% neutral lipid). In some embodiments, the formulations disclosed herein are essentially free of neutral lipid (i.e., about 0 mol-% neutral lipid). In some embodiments, the formulations disclosed herein are free of neutral phospholipid (i.e., 0 mol-% neutral phospholipid). In some embodiments, the formulations disclosed herein are essentially free of neutral phospholipid (i.e., about 0 mol-% neutral phospholipid).

In some embodiments, the neutral lipid mol-% of the LNP batch will be ±30%, ±25%, ±20%, ±15%, ±10%, ±5%, or ±2.5% of the target neutral lipid mol-%. In certain embodiments, LNP inter-lot variability will be less than 15%, less than 10% or less than 5%.

In one embodiment, the mol-% of the helper lipid may be from about 20 mol-% to about 60 mol-%. In one embodiment, the mol-% of the helper lipid may be from about 25 mol-% to about 55 mol-%. In one embodiment, the mol-% of the helper lipid may be from about 25 mol-% to about 50 mol-%. In one embodiment, the mol-% of the helper lipid may be from about 25 mol-% to about 40 mol-%. In one embodiment, the mol-% of the helper lipid may be from about 30 mol-% to about 50 mol-%. In one embodiment, the mol-% of the helper lipid may be from about 30 mol-% to about 40 mol-%. In one embodiment, the mol-% of the helper lipid is adjusted based on amine lipid, neutral lipid, and PEG lipid concentrations to bring the lipid component to 100 mol-%. In one embodiment, the mol-% of the helper lipid is adjusted based on amine lipid and PEG lipid concentrations to bring the lipid component to 100 mol-%. In one embodiment, the mol-% of the helper lipid is adjusted based on amine lipid and PEG lipid concentrations to bring the lipid component to at least 99 mol-%. In some embodiments, the helper mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

In one embodiment, the mol-% of the PEG lipid may be from about 1 mol-% to about 10 mol-%. In one embodiment, the mol-% of the PEG lipid may be from about 2 mol-% to about 10 mol-%. In one embodiment, the mol-% of the PEG lipid may be from about 2 mol-% to about 8 mol-%. In one embodiment, the mol-% of the PEG lipid may be from about 2 mol-% to about 4 mol-%. In one embodiment, the mol-% of the PEG lipid may be from about 2.5 mol-% to about 4 mol-%. In one embodiment, the mol-% of the PEG lipid may be about 3 mol-%. In one embodiment, the mol-% of the PEG lipid may be about 2.5 mol-%. In some embodiments, the PEG lipid mol-% of the LNP batch will be ±30%, ±25%, ±20%, ±15%, ±10%, ±5%, or ±2.5% of the target PEG lipid mol-%. In certain embodiments, LNP inter-lot variability will be less than 15%, less than 10% or less than 5%.

In certain embodiments, the cargo includes an mRNA encoding an RNA-guided DNA-binding agent (e.g. a Cas nuclease, a Class 2 Cas nuclease, or Cas9), and a gRNA or a nucleic acid encoding a gRNA, or a combination of mRNA and gRNA. In one embodiment, an LNP composition may comprise a Lipid A or its equivalents. In some aspects, the amine lipid is Lipid A. In some aspects, the amine lipid is a Lipid A equivalent, e.g. an analog of Lipid A. In certain aspects, the amine lipid is an acetal analog of Lipid A. In various embodiments, an LNP composition comprises an amine lipid, a neutral lipid, a helper lipid, and a PEG lipid. In certain embodiments, the helper lipid is cholesterol. In certain embodiments, the neutral lipid is DSPC. In specific embodiments, PEG lipid is PEG2k-DMG. In some embodiments, an LNP composition may comprise a Lipid A, a helper lipid, a neutral lipid, and a PEG lipid. In some embodiments, an LNP composition comprises an amine lipid, DSPC, cholesterol, and a PEG lipid. In some embodiments, the LNP composition comprises a PEG lipid comprising DMG. In certain embodiments, the amine lipid is selected from Lipid A, and an equivalent of Lipid A, including an acetal analog of Lipid A. In additional embodiments, an LNP composition comprises Lipid A, cholesterol, DSPC, and PEG2k-DMG.

In various embodiments, an LNP composition comprises an amine lipid, a helper lipid, a neutral lipid, and a PEG lipid. In various embodiments, an LNP composition comprises an amine lipid, a helper lipid, a neutral phospholipid, and a PEG lipid. In various embodiments, an LNP composition comprises a lipid component that consists of an amine lipid, a helper lipid, a neutral lipid, and a PEG lipid. In various embodiments, an LNP composition comprises an amine lipid, a helper lipid, and a PEG lipid. In certain embodiments, an LNP composition does not comprise a neutral lipid, such as a neutral phospholipid. In various embodiments, an LNP composition comprises a lipid component that consists of an amine lipid, a helper lipid, and a PEG lipid. In certain embodiments, the neutral lipid is chosen from one or more of DSPC, DPPC, DAPC, DMPC, DOPC, DOPE, and DSPE. In certain embodiments, the neutral lipid is DSPC. In certain embodiments, the neutral lipid is DPPC. In certain embodiments, the neutral lipid is DAPC. In certain embodiments, the neutral lipid is DMPC. In certain embodiments, the neutral lipid is DOPC. In certain embodiments, the neutral lipid is DOPE. In certain embodiments, the neutral lipid is DSPE. In certain embodiments, the helper lipid is cholesterol. In specific embodiments, the PEG lipid is PEG2k-DMG. In some embodiments, an LNP composition may comprise a Lipid A, a helper lipid, and a PEG lipid. In some embodiments, an LNP composition may comprise a lipid component that consists of Lipid A, a helper lipid, and a PEG lipid. In some embodiments, an LNP composition comprises an amine lipid, cholesterol, and a PEG lipid. In some embodiments, an LNP composition comprises a lipid component that consists of an amine lipid, cholesterol, and a PEG lipid. In some embodiments, the LNP composition comprises a PEG lipid comprising DMG. In certain embodiments, the amine lipid is selected from Lipid A and an equivalent of Lipid A, including an acetal analog of Lipid A. In certain embodiments, the amine lipid is a C5-C12 or a C4-C12 acetal analog of Lipid A. In additional embodiments, an LNP composition comprises Lipid A, cholesterol, and PEG2k-DMG.

Embodiments of the present disclosure also provide lipid compositions described according to the molar ratio between the positively charged amine groups of the amine lipid (N) and the negatively charged phosphate groups (P) of the nucleic acid to be encapsulated. This may be mathematically represented by the equation N/P. In some embodiments, an LNP composition may comprise a lipid component that comprises an amine lipid, a helper lipid, a neutral lipid, and a PEG lipid; and a nucleic acid component, wherein the N/P ratio is about 3 to 10. In some embodiments, an LNP composition may comprise a lipid component that comprises an amine lipid, a helper lipid, and a PEG lipid; and a nucleic acid component, wherein the N/P ratio is about 3 to 10. In some embodiments, an LNP composition may comprise a lipid component that comprises an amine lipid, a helper lipid, a neutral lipid, and a helper lipid; and an RNA component, wherein the N/P ratio is about 3 to 10. In some embodiments, an LNP composition may comprise a lipid component that comprises an amine lipid, a helper lipid, and a PEG lipid; and an RNA component, wherein the N/P ratio is about 3 to 10. In one embodiment, the N/P ratio may be about 5 to 7. In one embodiment, the N/P ration may be about 3 to 7. In one embodiment, the N/P ratio may be about 4.5 to 8. In one embodiment, the N/P ratio may be about 6. In one embodiment, the N/P ratio may be 6 ± 1. In one embodiment, the N/P ratio may be 6 ± 0.5. In some embodiments, the N/P ratio will be ±30%, ±25%, ±20%, ±15%, ±10%, ±5%, or ±2.5% of the target N/P ratio. In certain embodiments, LNP inter-lot variability will be less than 15%, less than 10% or less than 5%.

In some embodiments, the nucleic acid component, e.g., an RNA component, may comprise an mRNA, such as an mRNA encoding a Cas nuclease. An RNA component includes RNA, optionally with additional nucleic acid and/or protein, e.g., RNP cargo. In one embodiment, RNA comprises a Cas9 mRNA. In some compositions comprising an mRNA encoding a Cas nuclease, the LNP further comprises a gRNA nucleic acid, such as a gRNA. In some embodiments, the RNA component comprises a Cas nuclease mRNA and a gRNA. In some embodiments, the RNA component comprises a Class 2 Cas nuclease mRNA and a gRNA.

In certain embodiments, an LNP composition may comprise an mRNA encoding a Cas nuclease such as a Class 2 Cas nuclease, an amine lipid, a helper lipid, a neutral lipid, and a PEG lipid. In certain embodiments, an LNP composition may comprise an mRNA encoding a Cas nuclease such as a Class 2 Cas nuclease, an amine lipid, a helper lipid, and a PEG lipid. In certain LNP compositions comprising an mRNA encoding a Cas nuclease such as a Class 2 Cas nuclease, the helper lipid is cholesterol. In other compositions comprising an mRNA encoding a Cas nuclease such as a Class 2 Cas nuclease, the neutral lipid is DSPC. In additional embodiments comprising an mRNA encoding a Cas nuclease such as a Class 2 Cas nuclease, the PEG lipid is PEG2k-DMG or PEG2k-C11. In specific compositions comprising an mRNA encoding a Cas nuclease such as a Class 2 Cas nuclease, the amine lipid is selected from Lipid A and its equivalents, such as an acetal analog of Lipid A.

In some embodiments, an LNP composition may comprise a gRNA. In certain embodiments, an LNP composition may comprise an amine lipid, a gRNA, a helper lipid, a neutral lipid, and a PEG lipid. In certain embodiments, an LNP composition may comprise an amine lipid, a gRNA, a helper lipid, and a PEG lipid. In certain LNP compositions comprising a gRNA, the helper lipid is cholesterol. In some compositions comprising a gRNA, the neutral lipid is DSPC. In additional embodiments comprising a gRNA, the PEG lipid is PEG2k-DMG or PEG2k-C11. In certain embodiments, the amine lipid is selected from Lipid A and its equivalents, such as an acetal analog of Lipid A.

In one embodiment, an LNP composition may comprise an sgRNA. In one embodiment, an LNP composition may comprise a Cas9 sgRNA. In one embodiment, an LNP composition may comprise a Cpfl sgRNA. In some compositions comprising an sgRNA, the LNP includes an amine lipid, a helper lipid, a neutral lipid, and a PEG lipid. In some compositions comprising an sgRNA, the LNP includes an amine lipid, a helper lipid, and a PEG lipid. In certain compositions comprising an sgRNA, the helper lipid is cholesterol. In other compositions comprising an sgRNA, the neutral lipid is DSPC. In additional embodiments comprising an sgRNA, the PEG lipid is PEG2k-DMG or PEG2k-C11. In certain embodiments, the amine lipid is selected from Lipid A and its equivalents, such as acetal analogs of Lipid A.

In certain embodiments, an LNP composition comprises an mRNA encoding a Cas nuclease and a gRNA, which may be an sgRNA. In one embodiment, an LNP composition may comprise an amine lipid, an mRNA encoding a Cas nuclease, a gRNA, a helper lipid, a neutral lipid, and a PEG lipid. In one embodiment, an LNP composition may comprise a lipid component consisting of an amine lipid, a helper lipid, a neutral lipid, and a PEG lipid; and a nucleic acid component consisting of an mRNA encoding a Cas nuclease, and a gRNA. In one embodiment, an LNP composition may comprise a lipid component consisting of an amine lipid, a helper lipid, and a PEG lipid; and a nucleic acid component consisting of an mRNA encoding a Cas nuclease, and a gRNA. In certain compositions comprising an mRNA encoding a Cas nuclease and a gRNA, the helper lipid is cholesterol. In some compositions comprising an mRNA encoding a Cas nuclease and a gRNA, the neutral lipid is DSPC. Certain compositions comprising an mRNA encoding a Cas nuclease and a gRNA comprise less than about 1 mol-% neutral lipid, e.g. neutral phospholipid. Certain compositions comprising an mRNA encoding a Cas nuclease and a gRNA comprise less than about 0.5 mol-% neutral lipid, e.g. neutral phospholipid. In certain compositions, the LNP does not comprise a neutral lipid, e.g., neutral phospholipid. In additional embodiments comprising an mRNA encoding a Cas nuclease and a gRNA, the PEG lipid is PEG2k-DMG or PEG2k-C11. In certain embodiments, the amine lipid is selected from Lipid A and its equivalents, such as acetal analogs of Lipid A.

In certain embodiments, the LNP compositions include a Cas nuclease mRNA, such as a Class 2 Cas mRNA and at least one gRNA. In certain embodiments, the LNP composition includes a ratio of gRNA to Cas nuclease mRNA, such as Class 2 Cas nuclease mRNA from about 25:1 to about 1:25. In certain embodiments, the LNP formulation includes a ratio of gRNA to Cas nuclease mRNA, such as Class 2 Cas nuclease mRNA from about 10:1 to about 1:10. In certain embodiments, the LNP formulation includes a ratio of gRNA to Cas nuclease mRNA, such as Class 2 Cas nuclease mRNA from about 8:1 to about 1:8. As measured herein, the ratios are by weight. In some embodiments, the LNP formulation includes a ratio of gRNA to Cas nuclease mRNA, such as Class 2 Cas mRNA from about 5:1 to about 1:5. In some embodiments, ratio range is about 3:1 to 1:3, about 2:1 to 1:2, about 5:1 to 1:2, about 5:1 to 1:1, about 3:1 to 1:2, about 3:1 to 1:1, about 3:1, about 2:1 to 1:1. In some embodiments, the gRNA to mRNA ratio is about 3:1 or about 2:1 In some embodiments the ratio of gRNA to Cas nuclease mRNA, such as Class 2 Cas nuclease is about 1:1. The ratio may be about 25:1, 10:1, 5:1, 3:1, 1:1, 1:3, 1:5, 1:10, or 1:25.

The LNP compositions disclosed herein may include a template nucleic acid. The template nucleic acid may be co-formulated with an mRNA encoding a Cas nuclease, such as a Class 2 Cas nuclease mRNA. In some embodiments, the template nucleic acid may be co-formulated with a guide RNA. In some embodiments, the template nucleic acid may be co-formulated with both an mRNA encoding a Cas nuclease and a guide RNA. In some embodiments, the template nucleic acid may be formulated separately from an mRNA encoding a Cas nuclease or a guide RNA. The template nucleic acid may be delivered with, or separately from the LNP compositions. In some embodiments, the template nucleic acid may be single- or double-stranded, depending on the desired repair mechanism. The template may have regions of homology to the target DNA, or to sequences adjacent to the target DNA.

In some embodiments, LNPs are formed by mixing an aqueous RNA solution with an organic solvent-based lipid solution, e.g., 100% ethanol. Suitable solutions or solvents include or may contain: water, PBS, Tris buffer, NaCl, citrate buffer, ethanol, chloroform, diethylether, cyclohexane, tetrahydrofuran, methanol, isopropanol. A pharmaceutically acceptable buffer, e.g., for *in vivo* administration of LNPs, may be used. In certain embodiments, a buffer is used to maintain the pH of the composition comprising LNPs at or above pH 6.5. In certain embodiments, a buffer is used to maintain the pH of the composition comprising LNPs at or above pH 7.0. In certain embodiments, the composition has a pH ranging from about 7.2 to about 7.7. In additional embodiments, the composition has a pH ranging from about 7.3 to about 7.7 or ranging from about 7.4 to about 7.6. In further embodiments, the composition has a pH of about 7.2, 7.3, 7.4, 7.5, 7.6, or 7.7. The pH of a composition may be measured with a micro pH probe. In certain embodiments, a cryoprotectant is included in the composition. Non-limiting examples of cryoprotectants include sucrose, trehalose, glycerol, DMSO, and ethylene glycol. Exemplary compositions may include up to 10% cryoprotectant, such as, for example, sucrose. In certain embodiments, the LNP composition may include about 1, 2, 3, 4, 5, 6, 7, 8, 9, or 10% cryoprotectant. In certain embodiments, the LNP composition may include about 1, 2, 3, 4, 5, 6, 7, 8, 9, or 10% sucrose. In some embodiments, the LNP composition may include a buffer. In some embodiments, the buffer may comprise a phosphate buffer (PBS), a Tris buffer, a citrate buffer, or mixtures thereof. In certain exemplary embodiments, the buffer comprises NaCl. In certain emboidments, NaCl is omitted. Exemplary amounts of NaCl may range from about 20 mM to about 45 mM. Exemplary amounts of NaCl may range from about 40 mM to about 50 mM. In some embodiments, the amount of NaCl is about 45 mM. In some embodiments, the buffer is a Tris buffer. Exemplary amounts of Tris may range from about 20 mM to about 60 mM. Exemplary amounts of Tris may range from about 40 mM to about 60 mM. In some embodiments, the amount of Tris is about 50 mM. In some embodiments, the buffer comprises NaCl and Tris. Certain exemplary embodiments of the LNP compositions contain 5% sucrose and 45 mM NaCl in Tris buffer. In other exemplary embodiments, compositions contain sucrose in an amount of about 5% w/v, about 45 mM NaCl, and about 50 mM Tris at pH 7.5. The salt, buffer, and cryoprotectant amounts may be varied such that the osmolality of the overall formulation is maintained. For example, the final osmolality may be maintained at less than 450 mOsm/L. In further embodiments, the osmolality is between 350 and 250 mOsm/L. Certain embodiments have a final osmolality of 300 +/- 20 mOsm/L.

In some embodiments, microfluidic mixing, T-mixing, or cross-mixing is used. In certain aspects, flow rates, junction size, junction geometry, junction shape, tube diameter, solutions, and/or RNA and lipid concentrations may be varied. LNPs or LNP compositions may be concentrated or purified, *e.g.,* via dialysis, tangential flow filtration, or chromatography. The LNPs may be stored as a suspension, an emulsion, or a lyophilized powder, for example. In some embodiments, an LNP composition is stored at 2-8° C, in certain aspects, the LNP compositions are stored at room temperature. In additional embodiments, an LNP composition is stored frozen, for example at -20° C or -80° C. In other embodiments, an LNP composition is stored at a temperature ranging from about 0° C to about -80° C. Frozen LNP compositions may be thawed before use, for example on ice, at room temperature, or at 25° C.

The LNPs may be, *e.g.,* microspheres (including unilamellar and multilamellar vesicles, *e.g.,* "liposomes"-lamellar phase lipid bilayers that, in some embodiments, are substantially spherical-and, in more particular embodiments, can comprise an aqueous core, *e.g.,* comprising a substantial portion of RNA molecules), a dispersed phase in an emulsion, micelles, or an internal phase in a suspension.

Moreover, the LNP compositions are biodegradable, in that they do not accumulate to cytotoxic levels *in vivo* at a therapeutically effective dose. In some embodiments, the LNP compositions do not cause an innate immune response that leads to substantial adverse effects at a therapeutic dose level. In some embodiments, the LNP compositions provided herein do not cause toxicity at a therapeutic dose level.

In some embodiments, the pdi may range from about 0.005 to about 0.75. In some embodiments, the pdi may range from about 0.01 to about 0.5. In some embodiments, the pdi may range from about zero to about 0.4. In some embodiments, the pdi may range from about zero to about 0.35. In some embodiments, the pdi may range from about zero to about 0.35. In some embodiments, the pdi may range from about zero to about 0.3. In some embodiments, the pdi may range from about zero to about 0.25. In some embodiments, the pdi may range from about zero to about 0.2. In some embodiments, the pdi may be less than about 0.08, 0.1, 0.15, 0.2, or 0.4.

The LNPs disclosed herein have a size (e.g., Z-average diameter) of about 1 to about 250 nm. In some embodiments, the LNPs have a size of about 10 to about 200 nm. In further embodiments, the LNPs have a size of about 20 to about 150 nm. In some embodiments, the LNPs have a size of about 50 to about 150 nm. In some embodiments, the LNPs have a size of about 50 to about 100 nm. In some embodiments, the LNPs have a size of about 50 to about 120 nm. In some embodiments, the LNPs have a size of about 60 to about 100 nm. In some embodiments, the LNPs have a size of about 75 to about 150 nm. In some embodiments, the LNPs have a size of about 75 to about 120 nm. In some embodiments, the LNPs have a size of about 75 to about 100 nm. Unless indicated otherwise, all sizes referred to herein are the average sizes (diameters) of the fully formed nanoparticles, as measured by dynamic light scattering on a Malvern Zetasizer. The nanoparticle sample is diluted in phosphate buffered saline (PBS) so that the count rate is approximately 200-400 kcps. The data is presented as a weighted-average of the intensity measure (Z-average diameter).

In some embodiments, the LNPs are formed with an average encapsulation efficiency ranging from about 50% to about 100%. In some embodiments, the LNPs are formed with an average encapsulation efficiency ranging from about 50% to about 70%. In some embodiments, the LNPs are formed with an average encapsulation efficiency ranging from about 70% to about 90%. In some embodiments, the LNPs are formed with an average encapsulation efficiency ranging from about 90% to about 100%. In some embodiments, the LNPs are formed with an average encapsulation efficiency ranging from about 75% to about 95%.

In some embodiments, the LNPs are formed with an average molecular weight ranging from about 1.00E+05 g/mol to about 1.00E+10 g/mol. In some embodiments, the LNPs are formed with an average molecular weight ranging from about 5.00E+05 g/mol to about 7.00E+07g/mol. In some embodiments, the LNPs are formed with an average molecular weight ranging from about 1.00E+06 g/mol to about 1.00E+10 g/mol. In some embodiments, the LNPs are formed with an average molecular weight ranging from about 1.00E+07 g/mol to about 1.00E+09 g/mol. In some embodiments, the LNPs are formed with an average molecular weight ranging from about 5.00E+06 g/mol to about 5.00E+09 g/mol.

In some embodiments, the polydispersity (Mw/Mn; the ratio of the weight averaged molar mass (Mw) to the number averaged molar mass (Mn)) may range from about 1.000 to about 2.000. In some embodiments, the Mw/Mn may range from about 1.00 to about 1.500. In some embodiments, the Mw/Mn may range from about 1.020 to about 1.400. In some embodiments, the Mw/Mn may range from about 1.010 to about 1.100. In some embodiments, the Mw/Mn may range from about 1.100 to about 1.350.

### Methods of Engineering Cells; Engineered Cells

The LNP compositions disclosed herein may be used in methods for engineering cells through gene editing, both *in vivo* and *in vitro.* In some embodiments, the methods involve contacting a cell with an LNP composition described herein.

In some embodiments, methods involve contacting a cell in a subject, such as a mammal, such as a human. In some embodiments, the cell is in an organ, such as a liver, such as a mammalian liver, such as a human liver. In some embodiments, the cell is a liver cell, such as a mammalian liver cell, such as a human liver cell. In some embodiments, the cell is a hepatocyte, such as a mammalian hepatocyte, such as a human hepatocyte. In some embodiments, the liver cell is a stem cell. In some embodiments, the human liver cell may be a liver sinusoidal endothelial cell (LSEC). In some embodiments, the human liver cell may be a Kupffer cell. In some embodiments, the human liver cell may be a hepatic stellate cell. In some embodiments, the human liver cell may be a tumor cell. In some embodiments, the human liver cell may be a liver stem cell. In additional embodiments, the cell comprises ApoE-binding receptors. In some embodiments, the liver cell such as a hepatocyte is in situ. In some embodiments, the liver cell such as a hepatocyte is isolated, e.g., in a culture, such as in a primary culture. Also provided are methods corresponding to the uses disclosed herein, which comprise administering the LNP compositions disclosed herein to a subject or contacting a cell such as those described above with the LNP compositions disclosed herein

In some embodiments, engineered cells are provided, for example an engineered cell derived from any one of the cell types in the preceding paragraph. Such engineered cells are produced according to the methods described herein. In some embodiments, the engineered cell resides within a tissue or organ, e.g., a liver within a subject.

In some of the methods and cells described herein, a cell comprises a modification, for example an insertion or deletion ("indel") or substitution of nucleotides in a target sequence. In some embodiments, the modification comprises an insertion of 1, 2, 3, 4 or 5 or more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification comprises an indel which results in a frameshift mutation in a target sequence. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

In some embodiments, a population of cells comprising engineered cells is provided, for example a population of cells comprising cells engineered according to the methods described herein. In some embodiments, the population comprises engineered cells cultured *in vitro.* In some embodiments, the population resides within a tissue or organ, e.g., a liver within a subject. In some embodiments, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% or more of the cells within the population is engineered. In certain embodiments, a method disclosed herein results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% editing efficiency (or "percent editing"), defined by detetion of indels. In other embodiments, a method disclosed herein, results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% DNA modification efficiency, defined by detecting a change in sequence, whether by insertion, deletion, substitution or otherwise. In certain embodiments, a method disclosed herein results in an editing efficiency level or a DNA modification efficiency level of between about 5% to about 100%, about 10% to about 50%, about 20 to about 100%, about 20 to about 80%, about 40 to about 100%, or about 40 to about 80% in a cell population.

In some of the methods and cells described herein, cells within the population comprise a modification, e.g., an indel or substitution at a target sequence. In some embodiments, the modification comprises an insertion of 1, 2, 3, 4 or 5 or more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification results in a frameshift mutation in a target sequence. In some embodiments, the modification comprises an indel which results in a frameshift mutation in a target sequence. In some embodiments, at least 80%, at least 85%, at least 90%, at least 91%, at least 92%, at least 93%, at least 94%, at least 95%, at least 96%, at least 97%, at least 98%, or at least 99% or more of the engineered cells in the population comprise a frameshift mutation. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

### Methods of Gene Editing

The LNP compositions disclosed herein may be used for gene editing *in vivo* and *in vitro.* In one embodiment, one or more LNP compositions described herein may be administered to a subject in need thereof. In one embodiment, one or more LNP compositions described herein may contact a cell. In one embodiment, a therapeutically effective amount of a composition described herein may contact a cell of a subject in need thereof. In one embodiment, a genetically engineered cell may be produced by contacting a cell with an LNP composition described herein. In various embodiments, the methods comprise introducing a template nucleic acid to a cell or subject, as set forth above.

In some embodiments, the methods involve administering the LNP composition to a cell associated with a liver disorder. In some embodiments, the methods involve treating a liver disorder. In certain embodiments, the methods involve contacting a hepatic cell with the LNP composition. In certain embodiments, the methods involve contacting a hepatocyte with the LNP composition. In some embodiments, the methods involve contacting an ApoE binding cell with the LNP composition.

In one embodiment, an LNP composition comprising an mRNA encoding a Class 2 Cas nuclease and a gRNA may be administered to a cell, such as an ApoE binding cell. In additional embodiments, a template nucleic acid is also introduced to the cell. In certain instances, an LNP composition comprising a Class 2 Cas nuclease and an sgRNA may be administered to a cell, such as an ApoE binding cell. In one embodiment, an LNP composition comprising an mRNA encoding a Class 2 Cas nuclease, a gRNA, and a template may be administered to a cell. In certain instances, an LNP composition comprising a Cas nuclease and an sgRNA may be administered to a liver cell. In some cases, the liver cell is in a subject.

In certain embodiments, a subject may receive a single dose of an LNP composition. In other examples, a subject may receive multiple doses of an LNP composition. In some embodiments, the LNP composition is administered 2-5 times. Where more than one dose is administered, the doses may be administered about 1, 2, 3, 4, 5, 6, 7, 14, 21, or 28 days apart; about 2, 3, 4, 5, or 6 months apart; or about 1, 2, 3, 4, or 5 years apart. In certain embodiments, editing improves upon readministration of an LNP composition.

In one embodiment, an LNP composition comprising an mRNA encoding a Cas nuclease such as a Class 2 Cas nuclease, may be administered to a cell, separately from the administration of a composition comprising a gRNA. In one embodiment, an LNP composition comprising an mRNA encoding a Cas nuclease such as a Class 2 Cas nuclease and a gRNA may be administered to a cell, separately from the administration of a template nucleic acid to the cell. In one embodiment, an LNP composition comprising an mRNA encoding a Cas nuclease such as a Class 2 Cas nuclease may be administered to a cell, followed by the sequential administration of an LNP composition comprising a gRNA and then a template to the cell. In embodiments where an LNP composition comprising an mRNA encoding a Cas nuclease is administered before an LNP composition comprising a gRNA, the administrations may be separated by about 4, 6, 8, 12, or 24 hours; or 2, 3, 4, 5, 6, or 7 days.

In one embodiment, the LNP compositions may be used to edit a gene resulting in a gene knockout. In an embodiment, the LNP compositions may be used to edit a gene resulting in gene knockdown in a population of cells. In another embodiment, the LNP compositions may be used to edit a gene resulting in a gene correction. In a further embodiment, the LNP compositions may be used to edit a cell resulting in gene insertion.

In one embodiment, administration of the LNP compositions may result in gene editing which results in persistent response. For example, administration may result in a duration of response of a day, a month, a year, or longer. As used herein, "duration of response" means that, after cells have been edited using an LNP composition disclosed herein, the resulting modification is still present for a certain period of time after administration of the LNP composition. The modification may be detected by measuring target protein levels. The modification may be detected by detecting the target DNA. In some embodiments, the duration of response may be at least 1 week. In other embodiments, the duration of response may be at least 2 weeks. In one embodiment, the duration of response may be at least 1 month. In some embodiments, the duration of response may be at least 2 months. In one embodiment, the duration of response may be at least 4 months. In one embodiment, the duration of response may be at least 6 months. In certain embodiments, the duration of response may be about 26 weeks. In some embodiments, the duration of response may be at least 1 year. In some embodiments, the duration of response may be at least 5 years. In some embodiments, the duration of response may be at least 10 years. In some embodiments, a persistent response is detectable after at least 0.5, 1, 2, 3, 4, 5, 6, 7, 8, 9, 10, 11, 12, 15, 18, 21, or 24 months, either by measuring target protein levels or by detection of the target DNA. In some embodiments, a persistent response is detectable after at least 1, 2, 3, 4, 5, 6, 7, 8, 9, 10, 12, 14, 16, 18, or 20 years, either by measuring target protein levels or by detection of the target DNA.

The LNP compositions can be administered parenterally. The LNP compositions may be administered directly into the blood stream, into tissue, into muscle, or into an internal organ. Administration may be systemic, e.g., to injection or infusion. Administration may be local. Suitable means for administration include intravenous, intraarterial, intrathecal, intraventricular, intraurethral, intrasternal, intracranial, subretinal, intravitreal, intra-anterior chamber, intramuscular, intrasynovial, intradermal, and subcutaneous. Suitable devices for administration include needle (including microneedle) injectors, needle-free injectors, osmotic pumps, and infusion techniques.

The LNP compositions will generally, but not necessarily, be administered as a formulation in association with one or more pharmaceutically acceptable excipients. The term "excipient" includes any ingredient other than the compound(s) of the disclosure, the other lipid component(s) and the biologically active agent. An excipient may impart either a functional (*e.g.* drug release rate controlling) and/or a non-functional (*e.g.* processing aid or diluent) characteristic to the formulations. The choice of excipient will to a large extent depend on factors such as the particular mode of administration, the effect of the excipient on solubility and stability, and the nature of the dosage form.

Parenteral formulations are typically aqueous or oily solutions or suspensions. Where the formulation is aqueous, excipients such as sugars (including but not restricted to glucose, mannitol, sorbitol, *etc.)* salts, carbohydrates and buffering agents (preferably to a pH of from 3 to 9), but, for some applications, they may be more suitably formulated with a sterile non-aqueous solution or as a dried form to be used in conjunction with a suitable vehicle such as sterile, pyrogen-free water (WFI).

While the invention is described in conjunction with the illustrated embodiments, it is understood that they are not intended to limit the invention to those embodiments. On the contrary, the invention is intended to cover all alternatives, modifications, and equivalents, including equivalents of specific features, which may be included within the invention as defined by the appended claims.

Both the foregoing general description and detailed description, as well as the following examples, are exemplary and explanatory only and are not restrictive of the teachings. The section headings used herein are for organizational purposes only and are not to be construed as limiting the desired subject matter in any way. In the event that any literature incorporated by reference contradicts any term defined in this specification, this specification controls. All ranges given in the application encompass the endpoints unless stated otherwise.

It should be noted that, as used in this application, the singular form "a", "an" and "the" include plural references unless the context clearly dictates otherwise. Thus, for example, reference to "a composition" includes a plurality of compositions and reference to "a cell" includes a plurality of cells and the like. The use of "or" is inclusive and means "and/or" unless stated otherwise.

Numeric ranges are inclusive of the numbers defining the range. Measured and measureable values are understood to be approximate, taking into account significant digits and the error associated with the measurement. The term "about" or "approximately" means an acceptable error for a particular value as determined by one of ordinary skill in the art, which depends in part on how the value is measured or determined. The use of a modifier such as "about" before a range or before a list of values, modifies each endpoint of the range or each value in the list. "About" also includes the value or enpoint. For example, "about 50-55" encompasses "about 50 to about 55". Also, the use of "comprise", "comprises", "comprising", "contain", "contains", "containing", "include", "includes", and "including" is not limiting.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; embodiments in the specification that recite "about" various components are also contemplated as "at" the recited components; and embodiments in the specification that recite "consisting essentially of" various components are also contemplated as "consisting of" or "comprising" the recited components (this interchangeability does not apply to the use of these terms in the claims).

### EXAMPLES

### Example 1 - LNP compositions for In Vivo Editing in Mice

Small scale preparations of various LNP compositions were prepared to investigate their properties. In assays for percent liver editing in mice, Cas9 mRNA and chemically modified sgRNA targeting a mouse TTR sequence were formulated in LNPs with varying PEG mol-%, Lipid A mol-%, and N:P ratios as described in Table 2, below.

**Table 2. LNP compositions.**

| **LNP #** | **Lipid A mol-%** | **PEG-DMG mol-%** | **N:P ratio** |
|---|---|---|---|
| **(various)** | **45** | **2, 2.5, 3, 4, 5** | **4.5** |
| **(various)** | **45** | **2, 2.5, 3, 4, 5** | **6** |
| **(various)** | **50** | **2, 2.5, 3, 4, 5** | **4.5** |
| **(various)** | **50** | **2, 2.5, 3, 4, 5** | **6** |
| **(various)** | **55** | **2, 2.5, 3, 4, 5** | **4.5** |
| **(various)** | **55** | **2, 2.5, 3, 4, 5** | **6** |

In Fig. 1, LNP formulations are identified on the X-axis based on their Lipid A mol-% and N:P ratios, labeled "% CL; N:P". As indicated in the legend to Fig. 1, PEG-2k-DMG concentrations of 2, 2.5, 3, 4, or 5 mol-% were formulated with (1) 45 mol-% Lipid A; 4.5 N:P ("45; 4.5"); (2) 45 mol-% Lipid A; 6 N:P ("45; 6"); (3) 50 mol-% Lipid A; 4.5 N:P ("50; 4.5"); (4) 50 mol-% Lipid A; 6 N:P ("50; 6"); (5) 55 mol-% Lipid A; 4.5 N:P ("55; 4.5"); and (6) 55 mol-% Lipid A; 6 N:P ("55; 6"). The DSPC mol-% was kept constant at 9 mol-% and the cholesterol mol-% was added to bring the balance of each formulation lipid component to 100 mol-%. Each of the 30 formulations was formulated as described below, and administered as single dose at 1 mg per kg or 0.5 mg per kg doses of total RNA, (Fig. 1A and Fig. 1B, respectively).

### LNP formulation - NanoAssemblr

The lipid nanoparticle components were dissolved in 100% ethanol with the lipid component molar ratios set forth above. The RNA cargos were dissolved in 25 mM citrate, 100 mM NaCl, pH 5.0, resulting in a concentration of RNA cargo of approximately 0.45 mg/mL. The LNPs were formulated with a lipid amine to RNA phosphate (N:P) molar ratio of about 4.5 or about 6, with the ratio of mRNA to gRNA at 1:1 by weight.

The LNPs were formed by microfluidic mixing of the lipid and RNA solutions using a Precision Nanosystems NanoAssemblr^{™} Benchtop Instrument, according to the manufacturer's protocol. A 2:1 ratio of aqueous to organic solvent was maintained during mixing using differential flow rates. After mixing, the LNPs were collected, diluted in water (approximately 1:1 v/v), held for 1 hour at room temperature, and further diluted with water (approximately 1:1 v/v) before final buffer exchange. The final buffer exchange into 50 mM Tris, 45 mM NaCl, 5% (w/v) sucrose, pH 7.5 (TSS) was completed with PD-10 desalting columns (GE). If required, formulations were concentrated by centrifugation with Amicon 100 kDa centrifugal filters (Millipore). The resulting mixture was then filtered using a 0.2 µm sterile filter. The final LNP was stored at -80°C until further use.

### Formulation Analytics

Dynamic Light Scattering ("DLS") is used to characterize the polydispersity index ("pdi") and size of the LNPs of the present disclosure. DLS measures the scattering of light that results from subjecting a sample to a light source. PDI, as determined from DLS measurements, represents the distribution of particle size (around the mean particle size) in a population, with a perfectly uniform population having a PDI of zero.

Electropheretic light scattering is used to characterize the surface charge of the LNP at a specified pH. The surface charge, or the zeta potential, is a measure of the magnitude of electrostatic repulsion/attraction between particles in the LNP suspension.

Assymetric-Flow Field Flow Fractionation - Multi-Angle Light Scattering (AF4-MALS) is used to separate particles in the formulation by hydrodynamic radius and then measure the molecular weights, hydrodynamic radii and root mean square radii of the fractionated particles. This allows the ability to assess molecular weight and size distributions as well as secondary characteristics such as the Burchard-Stockmeyer Plot (ratio of root mean square ("rms") radius to hydrodynamic radius over time suggesting the internal core density of a particle) and the rms conformation plot (log of rms radius versus log of molecular weight where the slope of the resulting linear fit gives a degree of compactness versus elongation).

Nanoparticle tracking analysis (NTA, Malvern Nanosight) can be used to determine formulation particle size distribution as well as particle concentration. LNP samples are diluted appropriately and injected onto a microscope slide. A camera records the scattered light as the particles are slowly infused through field of view. After the movie is captured, the Nanoparticle Tracking Analysis processes the movie by tracking pixels and calculating a diffusion coefficient. This diffusion coefficient can be translated into the hydrodynamic radius of the particle. The instrument also counts the number of individual particles counted in the analysis to give particle concentration.

Cryo-electron microscopy ("cryo-EM") can be used to determine the particle size, morphology, and structural characteristics of an LNP.

Lipid compositional analysis of the LNPs can be determined from liquid chromotography followed by charged aerosol detection (LC-CAD). This analysis can provide a comparison of the actual lipid content versus the theoretical lipid content.

LNP formulations are analyzed for average particle size, polydispersity index (pdi), total RNA content, encapsulation efficiency of RNA, and zeta potential. LNP formualtions may be further characterized by lipid analysis, AF4-MALS, NTA, and/or cryo-EM. Average particle size and polydispersity are measured by dynamic light scattering (DLS) using a Malvern Zetasizer DLS instrument. LNP samples were diluted 30X in PBS prior to being measured by DLS. Z-average diameter which is an intensity-based measurement of average particle size was reported along with number average diameter and pdi. A Malvern Zetasizer instrument is also used to measure the zeta potential of the LNP. Samples are diluted 1: 17 (50 µL into 800 µL) in 0.1X PBS, pH 7.4 prior to measurement.

A fluorescence-based assay (Ribogreen^{®}, ThermoFisher Scientific) is used to determine total RNA concentration and free RNA. Encapsulation efficiency is calclulated as (Total RNA - Free RNA)/Total RNA. LNP samples are diluted appropriately with 1x TE buffer containing 0.2% Triton-X 100 to determine total RNA or 1x TE buffer to determine free RNA. Standard curves are prepared by utilizing the starting RNA solution used to make the formulations and diluted in 1x TE buffer +/- 0.2% Triton-X 100. Diluted RiboGreen^{®} dye (according to the manufacturer's instructions) is then added to each of the standards and samples and allowed to incubate for approximately 10 minutes at room temperature, in the absence of light. A SpectraMax M5 Microplate Reader (Molecular Devices) is used to read the samples with excitation, auto cutoff and emission wavelengths set to 488 nm, 515 nm, and 525 nm respectively. Total RNA and free RNA are determined from the appropriate standard curves.

Encapsulation efficiency is calclulated as (Total RNA - Free RNA)/Total RNA. The same procedure may be used for determining the encapsulation efficiency of a DNA-based or nucleic acid-containing cargo component. For single-strand DNA Oligreen Dye may be used, and for double-strand DNA, Picogreen Dye.

AF4-MALS is used to look at molecular weight and size distributions as well as secondary statistics from those calculations. LNPs are diluted as appropriate and injected into an AF4 separation channel using an HPLC autosampler where they are focused and then eluted with an exponential gradient in cross flow across the channel. All fluid is driven by an HPLC pump and Wyatt Eclipse Instrument. Particles eluting from the AF4 channel flow through a UV detector, multi-angle light scattering detector, quasi-elastic light scattering detector and differential refractive index detector. Raw data is processed by using a Debeye model to determine molecular weight and rms radius from the detector signals.

Lipid components in LNPs are analyzed quantitatively by HPLC coupled to a charged aerosol detector (CAD). Chromatographic separation of 4 lipid components is achieved by reverse phase HPLC. CAD is a destructive mass-based detector which detects all non-volatile compounds and the signal is consistent regardless of analyte structure.

### Cas9 mRNA and gRNA Cargos

The Cas9 mRNA cargo was prepared by in vitro transcription. Capped and polyadenylated Cas9 mRNA comprising 1XNLS (SEQ ID NO:48) was generated by *in vitro* transcription using a linearized plasmid DNA template and T7 RNA polymerase. Plasmid DNA containing a T7 promoter and a 100 nt poly(A/T) region was linearized by incubating at 37 °C for 2 hrs with XbaI with the following conditions: 200 ng/µL plasmid, 2 U/µL XbaI (NEB), and 1x reaction buffer. The XbaI was inactivated by heating the reaction at 65 °C for 20 min. The linearized plasmid was purified from enzyme and buffer salts using a silica maxi spin column (Epoch Life Sciences) and analyzed by agarose gel to confirm linearization. The IVT reaction to generate Cas9 modified mRNA was incubated at 37 °C for 4 hours in the following conditions: 50 ng/µL linearized plasmid; 2 mM each of GTP, ATP, CTP, and N1-methyl pseudo-UTP (Trilink); 10 mM ARCA (Trilink); 5 U/µL T7 RNA polymerase (NEB); 1 U/µL Murine RNase inhibitor (NEB); 0.004 U/µL Inorganic *E. coli* pyrophosphatase (NEB); and 1x reaction buffer. After the 4 hr incubation, TURBO DNase (ThermoFisher) was added to a final concentration of 0.01 U/µL, and the reaction was incubated for an additional 30 minutes to remove the DNA template. The Cas9 mRNA was purified from enzyme and nucleotides using a MegaClear Transcription Clean-up kit per the manufacturer's protocol (ThermoFisher). Alternatively, the Cas9 mRNA was purified with a LiCl precipitation method.

The sgRNA in this example was chemically synthesized and sourced from a commercial supplier. The sg282 sequence is provided below, with 2'-O-methyl modifications and phosphorothioate linkages as represented below (m = 2'-OMe; * = phosphorothioate):

mU*mU*mA*CAGCCACGUCUACAGCAGUUUUAGAmGmCmUmAmGmAmAmA
mUmAmGmCAAGUUAAAAUAAGGCUAGUCCGUUAUCAmAmCmUmUmGmAm
AmAmAmAmGmUmGmGmCmAmCmCmGmAmGmUmCmGmGmUmGmCmU*mU
*mU*mU. (SEQ ID NO:42).

### LNPs

The final LNPs were characterized to determine the encapsulation efficiency, polydispersity index, and average particle size according to the analytical methods provided above.

The LNPs were dosed to mice (single dose at 1 mg/kg or 0.5 mg/kg) and genomic DNA was isolated for NGS analysis as described below.

### LNP Delivery In Vivo

CD-1 female mice, ranging from 6 to 10 weeks of age were used in each study. Animals were weighed and grouped according to body weight for preparing dosing solutions based on group average weight. LNPs were dosed via the lateral tail vein in a volume of 0.2 mL per animal (approximately 10 mL per kilogram body weight). The animals were observed at approximately 6 hours post dose for adverse effects. Body weight was measured at twenty-four hours post-administration, and animals were euthanized at various time points by exsanguination via cardiac puncture under isoflurane anesthesia. Blood was collected into serum separator tubes or into tubes containing buffered sodium citrate for plasma as described herein. For studies involving *in vivo* editing, liver tissue was collected from the median lobe or from three independent lobes (e.g., the right median, left median, and left lateral lobes) from each animal for DNA extraction and analysis.

Cohorts of mice were measured for liver editing by Next-Generation Sequencing (NGS) and serum TTR levels (data not shown).

### Transthyretin (TTR) ELISA analysis

Blood was collected and the serum was isolated as indicated. The total mouse TTR serum levels were determined using a Mouse Prealbumin (Transthyretin) ELISA Kit (Aviva Systems Biology, Cat. OKIA00111). Rat TTR serum levels were measured using a rat specific ELISA kit (Aviva Systems Biology catalog number OKIA00159) according to manufacture's protocol. Briefly, sera were serial diluted with kit sample diluent to a final dilution of 10,000-fold. This diluted sample was then added to the ELISA plates and the assay was then carried out according to directions.

### NGS Sequencing

In brief, to quantitatively determine the efficiency of editing at the target location in the genome, genomic DNA was isolated and deep sequencing was utilized to identify the presence of insertions and deletions introduced by gene editing.

PCR primers were designed around the target site (e.g., TTR), and the genomic area of interest was amplified. Primer sequences are provided below. Additional PCR was performed according to the manufacturer's protocols (Illumina) to add the necessary chemistry for sequencing. The amplicons were sequenced on an Illumina MiSeq instrument. The reads were aligned to the human reference genome (e.g., hg38) after eliminating those having low quality scores. The resulting files containing the reads were mapped to the reference genome (BAM files), where reads that overlapped the target region of interest were selected and the number of wild type reads versus the number of reads which contain an insertion, substitution, or deletion was calculated.

The editing percentage (e.g., the "editing efficiency" or "percent editing") is defined as the total number of sequence reads with insertions or deletions over the total number of sequence reads, including wild type.

Fig. 1 shows editing percentages in mouse liver as measured by NGS. As shown in Fig. 1A, when 1 mg per kg RNA is dosed, in vivo editing percentages range from about 20% to over 60% liver editing. At a 0.5 mg per kg dose, Fig. 1B, about 10% to 60% liver editing was observed. In this mouse *in vivo* testing, all compositions effectively delivered Cas9 mRNA and gRNA to the liver cells, with evidence of active CRISPR/Cas nuclease activity at the target site measured by NGS for each LNP composition. LNPs containing 5% PEG lipid had lower encapsulation (data not shown), and somewhat reduced potency.

### Example 2 - LNP Composition Analytics

Analytical characterization of LNPs shows improved physicochemical parameters in LNPs formulated with increasing amounts of Lipid A and PEG-lipid. Compositons that comprise either 2 mol-% or 3 mol-% PEG lipid (PEG2k-DMG) are provided in Table 3 below.

**Table 3.**

| | **LNP898** | **LNP897** | **LNP966** | **LNP969** |
|---|---|---|---|---|
| CL/chol./DSPC/PEG (theoretical mol-%) | 45/44/9/2 | 45/43/9/3 | 50/38/9/3 | 55/33/9/3 |
| mRNA | Cas9 SEQ ID NO:48 | Cas9 SEQ ID NO:48 | Cas9 U-dep SEQ ID NO:43 | Cas9 U-dep SEQ ID NO:43 |
| gRNA | G502 SEQ ID NO: 70 | G502 SEQ ID NO: 70 | G534 SEQ ID NO: 72 | G534 SEQ ID NO: 72 |
| N/P | 4.5 | 4.5 | 6.0 | 6.0 |

### LNP Formulation - Cross Flow

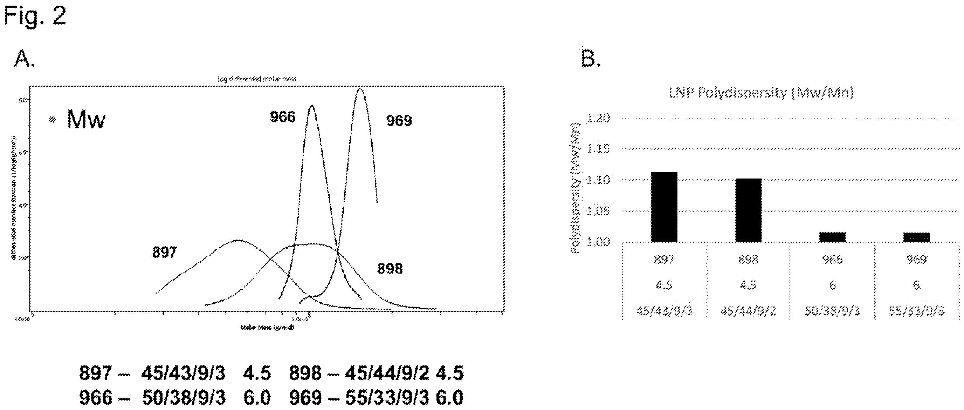

The LNPs were formed by impinging jet mixing of the lipid in ethanol with two volumes of RNA solutions and one volume of water. The lipid in ethanol is mixed through a mixing cross with the two volumes of RNA solution. A fourth stream of water is mixed with the outlet stream of the cross through an inline tee. (*See* WO2016010840 at Fig. 2.) The LNPs were maintained at room temperature for 1 hour, and then further diluted with water (approximately 1: 1 v/v). Diluted LNPs were concentrated using tangential flow filtration on a flat sheet cartridge (Sartorius, 100kD MWCO) and then buffer exchanged by diafiltration into 50 mM Tris, 45 mM NaCl, 5% (w/v) sucrose, pH 7.5 (TSS). Alternatively, the final buffer exchange into TSS was completed with PD-10 desalting columns (GE). If required, formulations were concentrated by centrifugation with Amicon 100 kDa centrifugal filters (Millipore). The resulting mixture was then filtered using a 0.2 µm sterile filter. The final LNP was stored at 4°C or -80°C until further use.

Cas9 mRNA and sgRNA were prepared as in Example 1, except that capped and poly-adenylated Cas9 U-depleted (Cas9 Udep) mRNA comprises SEQ ID N:43. Sg282 is described in Example 1, and the sequence for sg534 ("G534") is provided below:

mA*mC*mG*CAAAUAUCAGUCCAGCGGUUUUAGAmGmCmUmAmGmAmA

mAmUmAmGmCAAGUUAAAAUAAGGCUAGUCCGUUAUCAmAmCmUmUm

GmAmAmAmAmAmGmUmGmGmCmAmCmCmGmAmGmUmCmGmGmUmGm

CmU*mU*mU*mU (SEQ ID NO:72)

LNP formulations were analyzed for average particle size, polydispersity (pdi), total RNA content and encapsulation efficiency of RNA as described in Example 1.

Analysis of average particle size, polydispersity (PDI), total RNA content and encapsulation efficiency of RNA are shown in Table 4. In addition to the theoretical lipid concentrations of the LNP compositions, lipid analysis demonstrated the actual mol-% lipid levels, as indicated in Table 5 below.

**Table 4.**

| **LNP ID** # | **N/P** | **Z-Ave.** (nm) | **PDI** | **Number Ave.** (nm) | **RNA Conc** (mg/mL) | **Encaps.** (%) |
|---|---|---|---|---|---|---|
| LNP898 | 4.5 | 87.91 | 0.030 | 71.33 | 1.53 | 98 |
| LNP897 | 4.5 | 74.05 | 0.036 | 58.55 | 1.43 | 98 |
| LNP966 | 6.0 | 82.78 | 0.010 | 67.86 | 2.12 | 98 |
| LNP969 | 6.0 | 92.97 | 0.042 | 75.52 | 2.09 | 97 |

**Table 5.**

| **LNP ID #** | **Lipid Ratio** (Lipid A/Chol/ DSPC/PEG) (theoretical and actual) | **Lipid A mg/mL** (theoretical and actual) | **Chol mg/mL** (theoretical and actual) | **DSPC mg/mL** (theoretical and actual) | **PEG mg/mL** (theoretical and actual) |
|---|---|---|---|---|---|
| LNP898 | 45/44/9/2 | 18.0 | 8.0 | 3.3 | 2.3 |
| | 46.1/42.6/9.2/2 | 18.3 | 7.7 | 3.4 | 2.4 |
| LNP897 | 45/43/9/3 | 18.0 | 7.8 | 3.3 | 3.5 |
| | 44.8/42.9/9.2/3.1 | 17.8 | 7.7 | 3.4 | 3.6 |
| LNP966 | 50/38/9/3 | 33.4 | 11.5 | 5.6 | 5.8 |
| | 50.0/38.0/8.8/3.1 | 35.6 | 12.3 | 5.8 | 6.5 |
| LNP969 | 55/33/9/3 | 33.4 | 9.1 | 5.1 | 5.3 |
| | 54.8/33.2/8.8/3.2 | 31.6 | 8.7 | 4.7 | 5.4 |

To further analyze the physicochemical properties, LNP897, LNP898, LNP966, and LNP969 were subjected to Asymmetric-Flow Field Flow Fractionation - Multi-Angle Light Scattering (AF4-MALS) analysis. The AF4-MALS instrument measures particle size and molecular weight distribtions, and provides information about particle conformation and density.

LNPs are injected into an AF4 separation channel using an HPLC autosampler where they are focused and then eluted with an exponential gradient in cross flow across the channel. All fluid is driven by an HPLC pump and Wyatt Eclipse Instrument. Particles eluting from the AF4 channel flow through a UV detector, Wyatt Heleos II multi-angle light scattering detector, quasi-elastic light scattering detector and Wyatt Optilab T-rEX differential refractive index detector. Raw data is processed in Wyatt Astra 7 Software by using a Debeye model to determine molecular weight and rms radius from the detector signals.

A log differential molar mass plot for the LNPs is provided as Fig. 2A. In brief, the X-axis indicates molar mass (g/mol), and the Y-axis indicates the differential number fraction. The log differential molar mass plot shows the distribution of the different molecular weights measured for a specific formulation. This gives data towards the mode of the molecular weights as well as the overall distribution of molecular weights within the formulation, which gives a better picture of particle heterogeniety than average molecular weight.

The heterogeniety of the different LNP formulations are determined by measuring the different molar mass moments and calculating the ratio of the weight averaged molar mass (Mw) to the number averaged molar mass (Mn) to give a polydispersity of Mw/Mn. The graph of the polydispersity for these different formulations is provided in Fig 2B.

The data indicate tighter particle distributions with 3 mol-% PEG, and with 50 and 55 mol-% Lipid A at N/P 6.0 as shown in Fig. 2A. This is reflected in a tight polydispersity as shown in Fig. 2B

### Example 3 - AF4 MALS Data - Additional formulations

Analytical characterization of LNPs shows improved physicochemical parameters in LNPs formulated with increasing amounts of Lipid A. Compositons that comprise either 45 mol-%, 50 mol-%, or 55 mol-% Lipid A with two different gRNA are provided in Table 6 below.

**Table 6.**

| | **LNP1021** | **LNP1022** | **LNP1023** | **LNP1024** | **LNP1025** |
|---|---|---|---|---|---|
| CL/chol./DSPC/PEG (theoretical mol-%) | 50/38/9/3 | 55/33/9/3 | 45/43/9/3 | 50/38/9/3 | 55/33/9/3 |
| mRNA | Cas9 Udep SEQ ID NO:43 | Cas9 Udep SEQ ID NO:43 | Cas9 Udep SEQ ID NO:43 | Cas9 Udep SEQ ID NO:43 | Cas9 Udep SEQ ID NO:43 |
| gRNA | G502 SEQ ID NO: 70 | G502 SEQ ID NO:70 | G502 SEQ ID NO:70 | G509 SEQ ID NO:71 | G509 SEQ ID NO:71 |
| N/P | 6.0 | 6.0 | 4.5 | 6.0 | 6.0 |

The LNPs were formed as described in Example 2.

Cas9 mRNA and sgRNA were prepared as described above.

The LNPs compositions were characterized to determine the encapsulation efficiency, polydispersity index, and average particle size as described in Example 1.

Analysis of average particle size, polydispersity (PDI), total RNA content and encapsulation efficiency of RNA are shown in Table 7. In addition to the theoretical lipid concentrations of the LNP compositions, lipid analysis demonstrated the actual mol-% lipid levels, as indicated in Table 8, below.

**Table 7.**

| **LNP ID #** | **N/P** | **Z-Ave.** (nm) | **PDI** | **Number Ave.** (nm) | **RNA Conc.** (mg/mL) | **Encaps.** (%) |
|---|---|---|---|---|---|---|
| LNP1021 | 6.0 | 83.18 | 0.027 | 67.15 | 1.63 | 98 |
| LNP1022 | 6.0 | 94.08 | 0.005 | 78.28 | 1.60 | 97 |
| LNP1023 | 4.5 | 74.01 | 0.017 | 61.11 | 1.61 | 97 |
| LNP1024 | 6.0 | 85.37 | 0.002 | 70.42 | 1.59 | 97 |
| LNP1025 | 6.0 | 94.47 | 0.018 | 77.71 | 1.60 | 98 |

**Table 8.**

| **LNP ID** # | **Lipid Ratio** (Lipid A/Chol/ DSPC/PEG) (theoretical and actual) | **Lipid A mg/mL** (theoretical and actual) | **Chol. mg/mL** (theoretical and actual) | **DSPC mg/mL** (theoretical and actual) | **PEG mg/mL** (theoretical and actual) |
|---|---|---|---|---|---|
| LNP1021 | 50/38/9/3 | 23.6 | 8.1 | 3.9 | 4.1 |
| | 50.9/37.4/8.6/3.1 | 21.6 | 7.2 | 3.4 | 3.8 |
| LNP1022 | 55/33/9/3 | 23.6 | 6.4 | 3.6 | 3.7 |
| | 55.2/33.0/8.7/3.1 | 20.4 | 5.5 | 3.0 | 3.4 |
| LNP1023 | 45/43/9/3 | 17.7 | 7.7 | 3.3 | 3.4 |
| | 45.9/42.4/8.6/3.1 | 15.3 | 6.4 | 2.7 | 3.0 |
| LNP1024 | 50/38/9/3 | 23.6 | 8.1 | 3.9 | 4.1 |
| | 50.5/37.9/8.5/3.0 | 22.4 | 7.6 | 3.5 | 3.9 |
| LNP1024 | 55/33/9/3 | 23.6 | 6.4 | 3.6 | 3.7 |
| | 55.5/33.1/8.5/3.0 | 21.3 | 5.8 | 3.0 | 3.4 |

To further analyze the physicochemical properties, LNP1021, LNP1022, LNP1023, LNP1024 and LNP1025 were subjected to Asymmetric-Flow Field Flow Fractionation - Multi-Angle Light Scattering (AF4-MALS) analysis. The AF4-MALS instrument measures particle size and molecular weight distribtions, and provides information about particle conformation and density.

LNPs were run on AF4-MALS as described in Example 1.

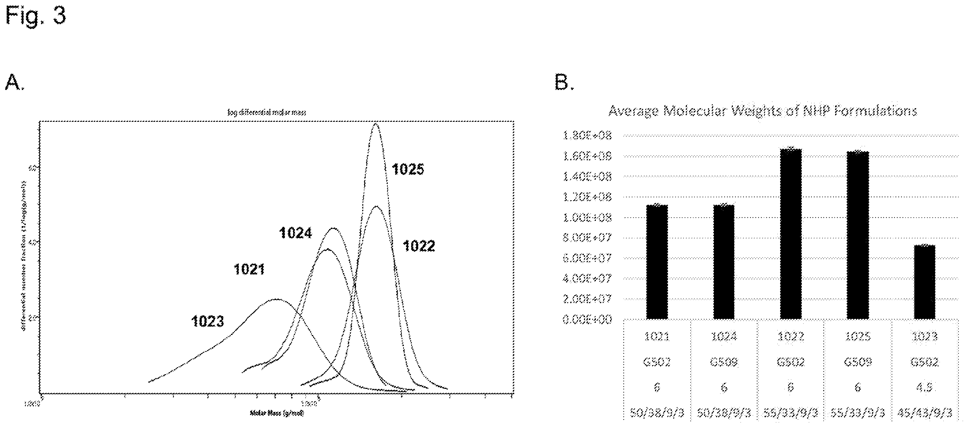

A log differential molar mass plot for the LNPs is provided as Fig. 3A. In brief, the X-axis indicates molar mass (g/mol), and the Y-axis indicates the differential number fraction. The log differential molar mass plot shows the distribution of the different molecular weights calculated for a specific formulation. This gives data towards the mode of the molecular weights as well as the overall distribution of molecular weights within the formulation, which gives a better picture of particle heterogeniety than average molecular weight.

Average molecular weight is plotted in Fig. 3B. The average molecular weight is the average of the entire distribution but gives no information about the shape of that distribution. LNP1022 and LNP1025 have the same average molecular weight but LNP1022 has a slightly broader distribution.

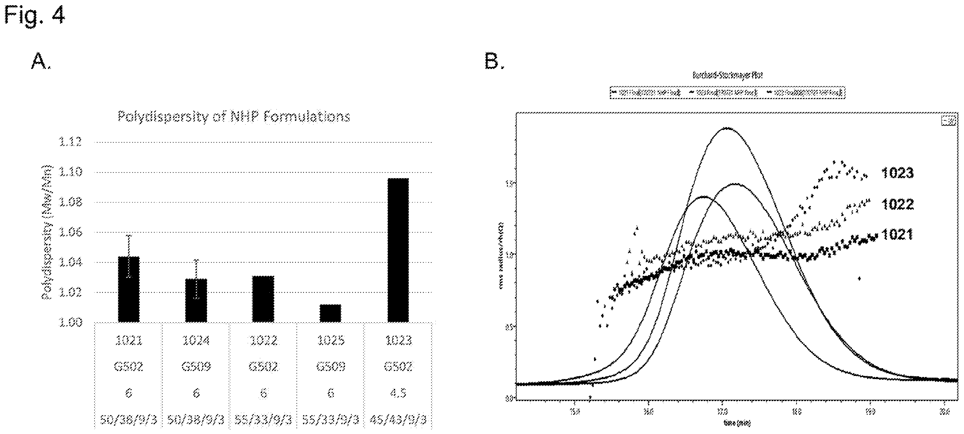

The heterogeniety of the different LNP formulations are calculated by look at the different molar mass moments and calculating the ratio of the weight averaged molar mass (Mw) to the number averaged molar mass (Mn) to give a polydispersity of Mw/Mn. The graph of the polydispersity for these different formulations is provided in Fig. 4A.

Additionally, a Burchard-Stockmeyer plot of the LNP formulations is provided as Fig. 4B. The Burchard-Stockmeyer plot shows the ratio of the rms radius versus the hydrodynamic radius across the elution of the formulation from the AF4 channel. This gives information towards the internal density of a lipid nanoparticle. Figure 4B shows that LNP1021, LNP1022 and LNP1023 have different profiles in this measurement.

### Example 4 - Increased PEG Lipid Maintains Potency with Reduced Cytokine Response

In another study, PEG DMG lipid was compared in LNP formulations comprising 2 mol-% or 3 mol-% of the PEG lipid. Compositons that comprise either 2 mol-%, or 3 mol-%, PEG DMG are provided in Table 9 below.

**Table 9.**

| | **LNP809** | **LNP810** |
|---|---|---|
| CL/chol./DSPC/PEG (theoretical mol-%) | 45/44/9/2 | 45/43/9/3 |
| mRNA | Cas9 SEQ ID NO:48 | Cas9 SEQ ID NO:48 |
| gRNA | G390 SEQ ID NO: 69 | G390 SEQ ID NO: 69 |
| N/P | 4.5 | 4.5 |

The LNPs were formed by the process described in Example 2.

Cas9 mRNA and sgRNA were prepared as in Example 1, with the sequence of sg390 ("G390") provided below:

mG*mC*mC*GAGUCUGGAGAGCUGCAGUUUUAGAmGmCmUmAmGmAmAmA
mUmAmGmCAAGUUAAAAUAAGGCUAGUCCGUUAUCAmAmCmUmUmGmAm
AmAmAmAmGmUmGmGmCmAmCmCmGmAmGmUmCmGmGmUmGmCmU*mU
*mU*mU (SEQ ID NO:69).

LNP formulations were analyzed for average particle size, polydispersity (pdi), total RNA content and encapsulation efficiency of RNA as described in Example 1.

Analysis of average particle size, polydispersity (PDI), total RNA content and encapsulation efficiency of RNA are shown in Table 10. In addition to the theoretical lipid concentrations of the LNP compositions, lipid analysis demonstrated the actual mol-% lipid levels, as indicated in Table 11: below.

**Table 10.**

| **LNP ID** # | **N/P** | **Z-Ave.** (nm) | **PDI** | **Number Ave.** (nm) | **RNA Conc.** (mg/mL) | **Encaps.** (%) |
|---|---|---|---|---|---|---|
| LNP809 | 4.5 | 89.85 | 0.060 | 72.10 | 2.45 | 97 |
| LNP810 | 4.5 | 75.26 | 0.025 | 61.17 | 2.14 | 97 |

**Table 11.**

| **LNP ID** # | **Lipid Ratio** (Lipid A/Chol/ DSPC/PEG) (theoretical and actual) | **Lipid A mg/mL** (theoretical and actual) | **Chol. mg/mL** (theoretical and actual) | **DSPC mg/mL** (theoretical and actual) | **PEG mg/mL** (theoretical and actual) |
|---|---|---|---|---|---|
| LNP809 | 45/44/9/2 | 28.6 | 12.7 | 5.3 | 3.7 |
| | 45.7/43.3/9.0/2.1 | 30.5 | 13.1 | 5.6 | 4.0 |
| LNP810 | 45/43/9/3 | 25.2 | 10.9 | 4.7 | 4.9 |
| | 45.0/42.3/9.7/3.0 | 24.7 | 10.5 | 4.9 | 4.7 |

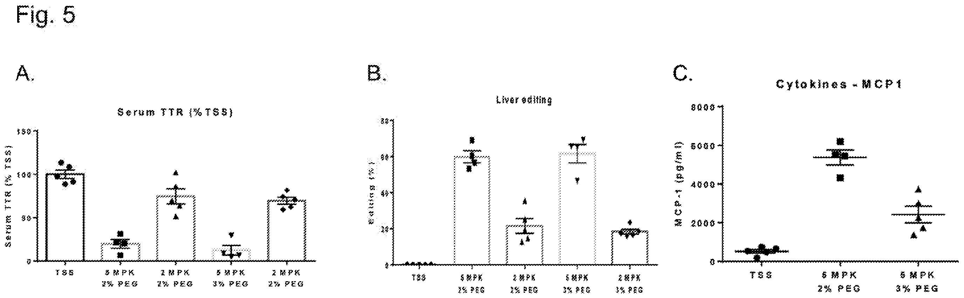

Rat serum cytokines were evaluated using a Luminex magnetic bead multiplex assay (Milliplex MAP magnetic bead assay from Millipore Sigma, catalog number RECYTMAG-65K) analyzing MCP-1, IL-6, TNF-alpha and IFN-gamma. The assay beads were read on the BioRad BioPlex-200 and cytokine concentrations calculated off a standard curve using 4 parameter logistic fit with BioPlex Manager Software version 6.1. Data is graphed in Fig. 5. See Fig. 5A (serum TTR), Fig. 5B (liver editing), and Fig. 5C (cytokine p MCP1).

Rat TTR serum levels were measured using a rat specific ELISA kit (Aviva Systems Biology catalog number OKIA00159) according to manufacture's protocol. Briefly, serums were serially diluted with kit sample diluent to a final dilution of 10,000-fold. This diluted sample was then added to the ELISA plates and the assay was then carried out according to directions.

Genomic DNA was isolated from approximately 10 mg of liver tissue and analyzed using NGS as described above. PCR primer sequences for amplification are described below.

Fig. 5A and Fig. 5B show that serum TTR knockdown and liver editing were sufficient in the 2 mol-% and 3 mol-% PEG formulations. Fig. 5C shows that MCP-1 response is reduced using 3 mol-% PEG formulations.

### Example 5 - LNP Delivery to Non-Human Primates

Three studies were conducted with LNP formulations prepared as described in Example 1. The particular molar amounts and cargos are provided in Tables 12-26. Each formulation containing Cas9 mRNA and guide RNA (gRNA) had a mRNA:gRNA ratio of 1:1 by weight. Doses of LNP (in mg/kg, total RNA content), route of administration and whether animals received pre-treatment of dexamethasone are indicated in the Tables. For animals receiving dexamethasone (Dex) pre-treatment, Dex was administered at 2 mg/kg by IV bolus injection, 1 hour prior to LNP or vehicle administration.

For blood chemistry analysis, blood was drawn from animals at times as indicated in the tables below for each factor that was measured. Cytokine induction was measured in pre- and post-treated NHPs. A minimum of 0.5 mL of whole blood was collected from a peripheral vein of restrained, conscious animals into a 4 mL serum separator tube. Blood was allowed to clot for a minimum of 30 minutes at room temperature followed by centrifugation at 2000 xg for 15 minutes. Serum was aliquoted into 2 polypropylene microtubes of 120 µL each and stored at -60 to -86 °C until analysis. A non-human primate U-Plex Cytokine custom kit from Meso Scale Discovery (MSD) was used for analysis. The following parameters were included in the analysis INF-g, IL-1b, IL-2, IL-4, IL-6, IL-8, IL-10, IL-12p40, MCP-1 and TNF-a, with focus on IL-6 and MCP-1. Kit reagents and standards were prepared as directed in the manufacturer's protocol. NHP serum was used neat. The plates were run on an MSD Sector Imager 6000 with analysis performed with MSD Discovery work bench software Version 4012.

Complement levels were measured in pre- and post-treated animals by enzyme Immunoassay. Whole blood (0.5 mL) was collected from a peripheral vein of restrained, conscious animals into a tube containing 0.5 mL k₂EDTA. Blood was centrifuged at 2000 xg for 15 minutes. Plasma was aliquoted into 2 polypropylene microtubes of 120 µL each and stored at -60 to -86 °C until analysis. A Quidel MicroVue Complement Plus EIA kit (C3a - Cat # A031) or (Bb-Cat # A027) was used for analysis. Kit reagents and standards were prepared as directed in the manufacturer's protocol. The plates were run on an MSD Sector Imager 6000 at optical density at 450 nm. The results were analyzed using a 4-parameter curve fit.

The data for cytokine induction and complement activation are provided in the Tables below. "BLQ" means below the limit of quantification.

**Table 12. Study 1.**

| **Treatment group** | **Molar Ratios (Lipid A, Cholesterol, DSPC, and PEG2k-DMG, respectively** | **N:P** | **Cargo** | **sample size (n)** | **Route** | **Dose level, total RNA content (mg/kg)** | **Dex** |
|---|---|---|---|---|---|---|---|
| (1) TSS (vehicle) | n/a | n/a | n/a | 3 | IV-infusion | n/a | no |
| (2) LNP699 G502 | 45/44/9/2 | 4.5 | Cas9 mRNA (SEQ ID NO:2); G000502 | 3 | IV-infusion | 3 | no |
| (3) LNP688 G506 | 45/44/9/2 | 4.5 | Cas9 mRNA (SEQ ID NO:2); G000506 | 3 | IV-infusion | 3 | no |
| (4) LNP689 G509 | 45/44/9/2 | 4.5 | Cas9 mRNA (SEQ ID NO:2); G000509 | 3 | IV-infusion | 3 | no |
| (5) LNP690 G510 | 45/44/9/2 | 4.5 | Cas9 mRNA (SEQ ID NO:2); G000510 | 3 | IV-infusion | 3 | no |

**Table 13. Study 2.**

| **Treatment group** | **Molar Ratios (Lipid A, Cholesterol, DSPC, and PEG2k-DMG, respectively** | **N:P** | **Cargo** | **sample size (n)** | **Route** | **Dose level, total RNA content (mg/kg)** | **Dex** |
|---|---|---|---|---|---|---|---|
| (1) TSS (vehicle) | n/a | n/a | | 1 | IV-bolus | n/a | yes |
| (2) TSS (vehicle) | n/a | n/a | | 1 | IV-bolus | n/a | no |
| (3) LNP898 G502 | 45/44/9/2 | 4.5 | Cas9 mRNA (SEQ ID NO:2); G000502 | 1 | IV-infusi In | 3 | yes |
| (4) LNP898 G502 | 45/44/9/2 | 4.5 | Cas9 mRNA (SEQ ID NO:2); G000502 | 1 | IV-infusi on | 3 | no |
| (5) LNP897 G502 | 45/43/9/3 | 4.5 | Cas9 mRNA (SEQ ID NO:2); G000502 | 1 | IV-bolus | 3 | yes |
| (6) LNP897 G502 | 45/43/9/3 | 4.5 | Cas9 mRNA (SEQ ID NO:2); G000502 | 1 | IV-bolus | 3 | no |
| (7) LNP897 G502 | 45/43/9/3 | 4.5 | Cas9 mRNA (SEQ ID NO:2); G000502 | 1 | IV-infusi In | 3 | yes |
| (8) LNP897 G502 | 45/43/9/3 | 4.5 | Cas9 mRNA (SEQ ID NO:2); G000502 | 1 | IV - infusi on | 3 | no |
| (9) LNP916 GFP | 45/43/9/3 | 4.5 | eGFP mRNA (SEQ ID NO:) | 1 | IV - infusi on | 6 | yes |
| (10) LNP916 GFP | 45/43/9/3 | 4.5 | eGFP mRNA (SEQ ID NO:) | 1 | IV - infusi on | 6 | no |

**Table 14. Study 3.**

| **Treatment group** | **Molar Ratios (Lipid A, Cholesterol, DSPC, and PEG2k-DMG, respectively** | **N:P** | **Cargo** | **sampl e size (n)** | **Route** | **Dose level, total RNA content (mg/kg)** | **Dex** |
|---|---|---|---|---|---|---|---|
| (1) TSS | n/a | n/a | n/a | 3 | IV-bolus | n/a | no |
| (2) LNP1021 G502 | 50/38/9/3 | 6 | Cas9 mRNA (SEQ ID NO:1); G00050 2 | 3 | IV-bolus | 1 | no |
| (3) LNP1021 G502 | 50/38/9/4 | 6 | Cas9 mRNA (SEQ ID NO:1); G00050 2 | 1 | IV-bolus | 1 | yes |
| (4) LNP1022 G502 | 55/33/9/3 | 6 | Cas9 mRNA (SEQ ID NO:1); G00050 2 | 3 | IV-bolus | 1 | no |
| (5) LNP1023 G502 | 45/43/9/3 | 4.5 | Cas9 mRNA (SEQ ID NO:1); G00050 2 | 3 | IV-bolus | 3 | no |
| (6) LNP1024 G509 | 50/38/9/3 | 6 | Cas9 mRNA (SEQ ID NO:1); G00050 9 | 3 | IV-bolus | 1 | no |
| (7) LNP1024 G509 | 50/38/9/4 | 6 | Cas9 mRNA (SEQ ID NO:1); G00050 9 | 1 | IV-bolus | 1 | yes |
| (8) LNP1025 G509 | 55/33/9/3 | 6 | Cas9 mRNA (SEQ ID NO:1); G00050 9 | 3 | IV-bolus | 1 | no |
| (9) LNP1021 G502 | 50/38/9/3 | 6 | Cas9 mRNA (SEQ ID NO:1); G00050 2 | 1 | IV-bolus | 3 | no |
| (10) LNP1022 G502 | 50/38/9/3 | 6 | Cas9 mRNA (SEQ ID NO:1); G00050 2 | 1 | IV-bolus | 3 | no |

**Table 15. IL-6 measurements from Study 1.**

| **Treatment Group** | **Pre-Bleed** | **6 hour** | **24 hour** |
|---|---|---|---|
| (1) TSS (vehicle) | 5.71±2.70 | 29.1±20.37 | 7.05±3.49 |
| (2) LNP699 G502 | 9.73±8.34 | 1296.41±664.71 | 5.43±7.68 |
| (3) LNP688 G506 | 16.83±4.08 | 1749.47±1727.22 | 38.57±39.39 |
| (4) LNP689 G509 | 18.11±11.51 | 1353.49±766.66 | 32.42±18.40 |
| (5) LNP690 G510 | 13.95±1.85 | 11838±17161.74 | 90.07±96.02 |

**Table 16. MCP-1 measurements from Study 1.**

| **Treatment Group** | **Pre-Bleed** | **6 hour** | **24 hour** |
|---|---|---|---|
| (1) TSS (vehicle) | 810.49±178.27 | 1351.16±397.31 | 745.25±56.49 |
| (2) LNP699 G502 | 842.31±350.65 | 19298.49±11981.14 | 2092.89±171.21 |
| (3) LNP688 G506 | 1190.79±383.64 | 13500.17±12691.60 | 1414.71±422.43 |
| (4) LNP689 G509 | 838.63±284.42 | 14427.7±8715.48 | 1590±813.23 |
| (5) LNP690 G510 | 785.32±108.97 | 52557.24±48034.68 | 6319.77±983.37 |

**Table 17. Complement C3a measurements from Study 1.**

| **Treatment Group** | **Pre-Bleed** | **6 hour** | **day 7** |
|---|---|---|---|
| (1) TSS (vehicle) | 23.9±11.95 | 25.51±14.79 | 30.67±18.36 |
| (2) LNP699 G502 | 32.36±11.29 | 94.33±58.45 | 38.50±12.69 |
| (3) LNP688 G506 | 22.30±1.73 | 127.00±22.34 | 37.80±6.86 |
| (4) LNP689 G509 | 35.83±21.94 | 174.00±44.51 | 50.83±21.92 |
| (5) LNP690 G510 | 36.30±8.21 | 163.00±40.60 | 42.50±12.44 |

**Table 18. Complement bb measurements from Study 1.**

| **Treatment Group** | **04-bb** | **Pre-Bleed** | **6 hour** | **day 7** |
|---|---|---|---|---|
| (1) TSS (vehicle) | Control | 1.53±0.19 | 3.37±2.13 | 1.43±0.71 |
| (2) LNP699 G502 | G502 | 1.45±0.39 | 9.01±5.28 | 1.57±0.54 |
| (3) LNP688 G506 | G506 | 1.45±0.78 | 11.78±2.33 | 1.78±0.84 |
| (4) LNP689 G509 | G509 | 1.95±0.99 | 15.73±2.23 | 2.83±0.88 |
| (5) LNP690 G510 | G510 | 2.12±0.44 | 13.57±1.23 | 2.21±0.72 |

**Table 19. IL-6 measurements from Study 2.**

| **Treatment group** | **Pre-Bleed** | **90 min** | **6 hour** | **24 hour** | **Day 7** |
|---|---|---|---|---|---|
| (1) TSS (vehicle) | 1.77 | 11.46 | 4.2 | 2.76 | 3.01 |
| (2) TSS (vehicle) | 5.23 | 18.11 | 20.36 | 13.2 | 6.36 |
| (3) LNP898 G502 | 2.02 | 1305.75 | 1138.22 | 383.32 | 16.02 |
| (4) LNP898 G502 | 2.34 | 37.19 | 91.59 | 14.11 | 3.07 |
| (5) LNP897 G502 | 2.1 | 55.79 | 6.89 | 2.26 | 2.01 |
| (6) LNP897 G502 | 6.8 | 10.1 | 44.72 | 5.4 | 2.01 |
| (7) LNP897 G502 | 1.97 | 44.87 | 32.61 | 2.97 | 1.11 |
| (8) LNP897 G502 | 3.14 | 37.68 | 73.41 | 8.58 | 2.22 |
| (9) LNP916 GFP | 1.6 | BLQ | 95.32 | 27.58 | BLQ |
| (10) LNP916 GFP | 2.43 | BLQ | 883.01 | 66.71 | BLQ |

**Table 20. MCP-1 measurements from Study 2.**

| **Treatment group** | **Pre-Bleed** | **90 min** | **6 hour** | **24 hour** | **Day 7** |
|---|---|---|---|---|---|
| (1) TSS (vehicle) | 312.12 | 197.24 | 145.36 | 177.02 | 403.82 |
| (2) TSS (vehicle) | 232.44 | 175.08 | 187.72 | 136.64 | 325.69 |
| (3) LNP898 G502 | 249.1 | 2183.5 | 1814.64 | 1887.41 | 372.38 |
| (4) LNP898 G502 | 349.51 | 430.49 | 5635.55 | 953.05 | 236.6 |
| (5) LNP897 G502 | 492.3 | 989.98 | 409.08 | 302.97 | 506.82 |
| (6) LNP897 G502 | 283.79 | 225.1 | 1141.08 | 484.59 | 259.46 |
| (7) LNP897 G502 | 223.16 | 349.79 | 398.57 | 172.67 | 287.09 |
| (8) LNP897 G502 | 584.42 | 853.51 | 3880.81 | 1588.46 | 692.99 |
| (9) LNP916 GFP | 325.84 | BLQ | 1189.97 | 2279.82 | BLQ |
| (10) LNP916 GFP | 175.47 | BLQ | 3284.16 | 2023.53 | BLQ |

**Table 21. Complement C3a measurements from Study 2.**

| **Treatment group** | **Pre-Bleed** | **90 min** | **6 hour** | **24 hour** | **Day 7** |
|---|---|---|---|---|---|
| (1) TSS (vehicle) | 0.087 | 0.096 | 0.048 | 0.033 | 0.038 |
| (2) TSS (vehicle) | 0.369 | 0.311 | 0.146 | 0.1 | 0.106 |
| (3) LNP898 G502 | 0.087 | 0.953 | 0.647 | 0.277 | 0.065 |
| (4) LNP898 G502 | 0.099 | 0.262 | 0.123 | 0.049 | 0.044 |
| (5) LNP897 G502 | 0.067 | 0.479 | 0.209 | 0.036 | 0.036 |
| (6) LNP897 G502 | 0.141 | 0.433 | 0.34 | 0.11 | 0.074 |
| (7) LNP897 G502 | 0.1 | 0.345 | 0.396 | 0.096 | 0.127 |
| (8) LNP897 G502 | 0.261 | 0.458 | 0.409 | 0.244 | 0.313 |
| (9) LNP916 GFP | 0.149 | BLQ | 0.714 | 0.382 | BLQ |
| (10) LNP916 GFP | 0.117 | BLQ | 0.752 | 0.723 | BLQ |

**Table 22. Complement bb measurements from Study 2.**

| **Treatment group** | **Pre-Bleed** | **90 min** | **6 hour** | **24 hour** | **Day 7** |
|---|---|---|---|---|---|
| (1) TSS (vehicle) | 0.087 | 0.096 | 0.048 | 0.033 | 0.038 |
| (2) TSS (vehicle) | 0.369 | 0.311 | 0.146 | 0.1 | 0.106 |
| (3) LNP898 G502 | 0.087 | 0.953 | 0.647 | 0.277 | 0.065 |
| (4) LNP898 G502 | 0.099 | 0.262 | 0.123 | 0.049 | 0.044 |
| (5) LNP897 G502 | 0.067 | 0.479 | 0.209 | 0.036 | 0.036 |
| (6) LNP897 G502 | 0.141 | 0.433 | 0.34 | 0.11 | 0.074 |
| (7) LNP897 G502 | 0.1 | 0.345 | 0.396 | 0.096 | 0.127 |
| (8) LNP897 G502 | 0.261 | 0.458 | 0.409 | 0.244 | 0.313 |
| (9) LNP916 GFP | 0.149 | BLQ | 0.714 | 0.382 | BLQ |
| (10) LNP916 GFP | 0.117 | BLQ | 0.752 | 0.723 | BLQ |

**Table 23. IL-6 measurements from Study 3.**

| **Treatment group** | **Pre-bleed** | **90 min** | **6 hour** | **24 hour** | **Day 7** |
|---|---|---|---|---|---|
| (1) TSS | 1.89±0.97 | 2.56±1.4 1 | 0.90±0.7 1 | BLQ | 0.08 |
| (2) LNP1021 G502 | 210±0.35 | 7.44±5.1 6 | 6.94±8.4 5 | 1.07±1.11 | 1.76±0.98 |
| (3) LNP1021 G502 | 0.79 | 2.96 | 4.25 | 0.67 | 0.27 |
| (4) LNP1022 G502 | 1.54±1.32 | 20.42±31 .60 | 13.94±10 .10 | 0.98±0.41 | 2.04±0.65 |
| (5) LNP1023 G502 | 2.92±1.68 | 6.28±7.1 8 | 6.06±2.3 1 | 3.62±4.68 | 2.00±1.21 |
| (6) LNP1024 G509 | 1.43±0.62 | 2.64±1.9 2 | 7.72±11. 96 | 0.45±0.19 | 0.88±0.79 |
| (7) LNP1024 G509 | 1.35±0.74 | 2.64±2.3 5 | 1.71±0.4 1 | 0.36±0.58 | 0.51±0.32 |
| (8) LNP1025 G509 | 1.64 | 2.68 | 25.65 | 0.58 | 2.00 |
| (9) LNP1021 G502 | 0.56 | 6.15 | 28.80 | 0.85 | 0.61 |
| (10) LNP1022 G502 | 1.76 | 8.66 | 2907.86 | 11.26 | 1.72 |

**Table 24. MCP-1 measurements from Study 2.**

| **Treatment group** | **Pre-bleed** | **90 min** | **6 hour** | **24 hour** | **Day 7** |
|---|---|---|---|---|---|
| (1) TSS | 204.01±46. 39 | 197.62±19.5 4 | 310.84±45.8 7 | 179.07±20.7 7 | 234.61±71.79 |
| (2) LNP1021 G502 | 303.67±36. 37 | 337.63±195. 18 | 755.20±581. 45 | 339.75±206. 20 | 214.82±40.81 |
| (3) LNP1021 G502 | 229.30 | 358.10 | 3182.00 | 413.56 | 178.30 |
| (4) LNP1022 G502 | 393.63±18 7.81 | 467.72±221. 61 | 1852.94±219 9.66 | 497.12±412. 30 | 382.19±67.27 |
| (5) LNP1023 G502 | 213.72±8.8 5 | 196.18±62.8 1 | 1722.18±141 3.90 | 197.83±74.0 1 | 156.16±18.87 |
| (6) LNP1024 G509 | 237.76±96. 36 | 210.37±95.1 7 | 468.53±250. 42 | 22.32±69.06 | 141.20±71.90 |
| (7) LNP1024 G509 | 207.36 | 183.07 | 1885.66 | 235.70 | 163.11 |
| (8) LNP1025 G509 | 259.57±11 2.98 | 299.21±304. 89 | 1193.10±974 .04 | 258.82±88.5 3 | 219.86±219.86 |
| (9) LNP1021 G502 | 199.29 | 286.04 | 2001.23 | 197.57 | 196.44 |
| (10) LNP1022 G502 | 305.81 | 970.65 | 7039.06 | 8379.05 | 203.47 |

**Table 25. Complement C3a measurements from Study 3.**

| **Treatment group** | **Pre-bleed** | **90 min** | **6 hour** | **24 hour** | **Day 7** |
|---|---|---|---|---|---|
| (1) TSS | 42.47±10.30 | 55.40±13.58 | 29.30±14.46 | 41.70±23.65 | 27.43±12.43 |
| (2) LNP1021 G502 | 34.37±0.50 | 86.50±3.66 | 90.07±4.85 | 56.60±2.25 | 32.53±0.93 |
| (3) LNP1021 G502 | 34.30 | 128.00 | 93.30 | 33.40 | 28.20 |
| (4) LNP1022 G502 | 41.55±13.51 | 151.37±109.98 | 82.00±31.82 | 45.57±18.58 | 32.77±6.45 |
| (5) LNP1023 G502 | 31.67±3.19 | 74.40±22.08 | 74.13±48.61 | 33.83±9.75 | 27.70±8.05 |
| (6) LNP1024 G509 | 56.60±25.61 | 100.37±77.95 | 74.73±70.15 | 55.20±48.34 | 49.97±39.94 |
| (7) LNP1024 G509 | 33.80 | 33.90 | 33.70 | 26.10 | 20.90 |
| (8) LNP1025 G509 | 39.90±13.01 | 75.73±1.38 | 46.13±30.56 | 25.00±3.80 | 23.90±7.18 |
| (9) LNP1021 G502 | 34 | 85.70 | 133.00 | 62.00 | 25.50 |
| (10) LNP1022 G502 | 29.8 | 68.10 | 113.00 | 71.70 | 23.30 |

**Table 26. Complement bb measurements from Study 3.**

| **Treatment group** | **Pre-bleed** | **90 min** | **6 hour** | **24 hour** | **Day 7** |
|---|---|---|---|---|---|
| (1) TSS | 1.46±0.70 | 2.18±0.78 | 1.96±0.64 | 0.945±0.15 | 1.34±0.50 |
| (2) LNP1021 G502 | 1.77±0.60 | 6.51±3.66 | 11.00±4.85 | 3.59±2.25 | 2.07±0.93 |
| (3) LNP1021 G502 | 1.24 | 2.90 | 11.50 | 2.97 | 1.24 |
| (4) LNP1022 G502 | 1.52±0.34 | 5.67±2.28 | 10.2±3.36 | 3.66±1.68 | 1.84±0.24 |
| (5) LNP1023 G502 | 1.65±0.94 | 4.4±1 | 7.68±4.67 | 2.64±1.18 | 2.08±1.32 |
| (6) LNP1024 G509 | 1.61±0.13 | 4.52±1.81 | 4.50±3.22 | 1.63±0.84 | 1.63±0.32 |
| (7) LNP1024 G509 | 0.96 | 2.99 | 2.64 | 1.13 | 1.07 |
| (8) LNP1025 G509 | 1.37±0.17 | 4.9±4.51 | 3.79±3.84 | 1.66±1.43 | 1.35±0.44 |
| (9) LNP1021 G502 | 1.41 | 5.67 | 11.50 | 4.64 | 1.38 |
| (10) LNP1022 G502 | 1.28 | 5.22 | 14.10 | 5.64 | 1.87 |

### Example 6 - PEG Lipid Screen

In another study, alternative PEG lipids were compared in LNP formulations comprising 2 mol-% or 3 mol-% of the PEG lipid.

Three PEG lipids were used in the study: Lipid 1 (DMG-PEG2k; Nof), is depicted as:

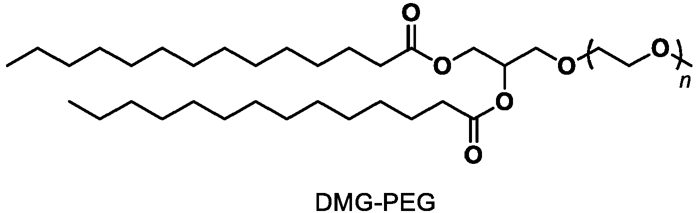

Lipid 2, synthesized as described in Heyes, et al., J. Controlled Release, 107 (2005), pp. 278-279 (See "Synthesis of PEG2000-C-DMA"), can be depicted as: 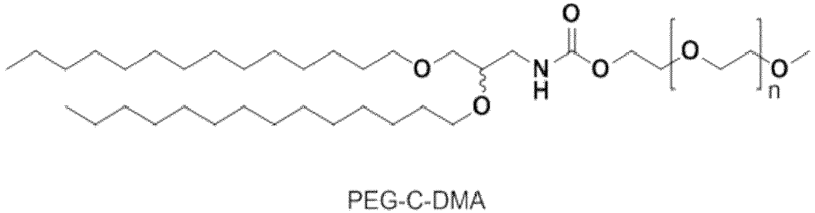 **and Lipid 3, disclosed in** WO2016/010840 **(see compound S027, paragraphs [00240] to [00244]) and** WO2011/076807**, can be depicted as:**

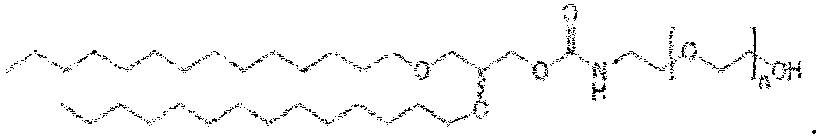

Lipid A was formulated with each PEG lipid at 2 mol-% and 3 mol-%. The lipid nanoparticle components were dissolved in 100% ethanol with the lipid component molar ratios set forth above. In brief, the RNA cargos were prepared in 25 mM citrate, 100 mM NaCl, pH 5.0, resulting in a concentration of RNA cargo of approximately 0.45 mg/mL. The LNPs were formulated with a lipid amine to RNA phosphate (N:P) molar ratio of about 4.5 with the ratio of mRNA to gRNA at 1:1 by weight.

**Table 27.**

| **LNP** # | **LNP 784** | **LNP 785** | **LNP 786** | **LNP 787** | **LNP 788** | **LNP 789** |
|---|---|---|---|---|---|---|
| CL/chol./DSPC/PEG (theoretical mol-%) | 45/44/9/ 2 | 45/43/9/ 3 | 45/44/9/ 2 | 45/43/9/ 3 | 45/44/9/ 2 | 45/43/9/ 3 |
| mRNA | Cas9 SEQ ID NO:48 | Cas9 SEQ ID NO:48 | Cas9 SEQ ID NO:48 | Cas9 SEQ ID NO:48 | Cas9 SEQ ID NO:48 | Cas9 SEQ ID NO:48 |
| gRNA | G282 SEQ ID NO:42 | G282 SEQ ID NO:42 | G282 SEQ ID NO:42 | G282, SEQ ID NO:42 | G282, SEQ ID NO:42 | G282 SEQ ID NO:42 |
| PEG Type | Lipid 1 | Lipid 1 | Lipid 2 | Lipid 2 | Lipid 3 | Lipid 3 |
| N/P | 4.5 | 4.5 | 4.5 | 4.5 | 4.5 | 4.5 |

Cas9 mRNA, sg282, and LNPs were prepared as described in Example 1.

LNP compositions with Lipid 1, Lipid 2, or Lipid 3 were were administered to female CD-1 mice and assessed as described in Example 1 at 1 mg/kg and 0.5 mg/kg of the body weight. Cohorts of mice were measured for liver editing by Next-Generation Sequencing (NGS) and serum TTR levels according to the methods of Example 1.

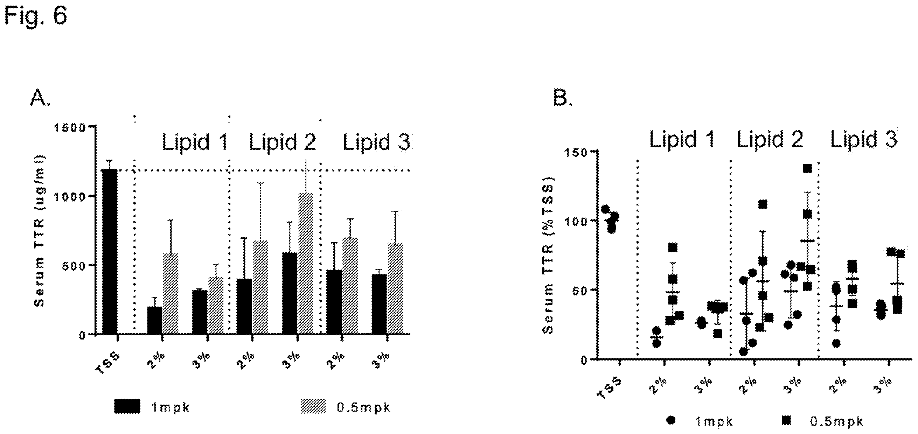

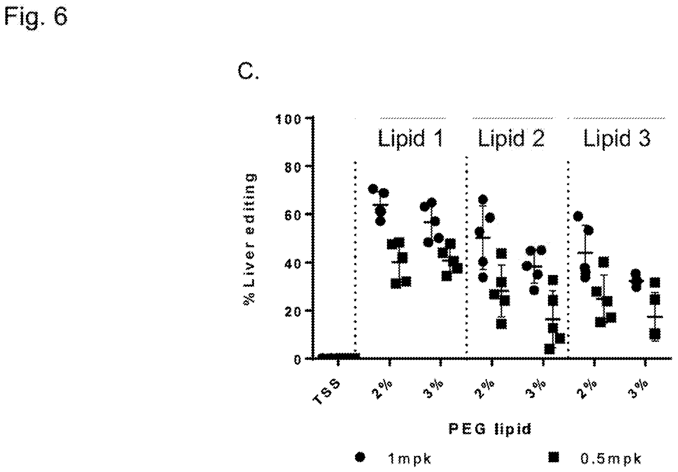

Fig. 6A and Fig. 6B compare serum TTR levels between PEG lipid formulations. Fig. 6A shows serum TTR in µg/mL, and Fig. 6B shows the data as a percent knockdown (%TSS). Fig. 6C shows percent editing achieved in the liver. The data indicate that LNP compositions with each of the tested PEG lipids tested potency at 2 mol-% and 3 mol-%, with Lipid 1 consistently performing slightly better than Lipid 2 and Lipid 3.

### Example 7 - Lipid A Analogs

A number of structural analogs of Lipid A were synthesized and tested in the LNP compositions described herein.

Synthesis: Lipid A is made by reacting 4,4-bis(octyloxy)butanoic acid ("Intermediate 13b" in Example 13 of WO2015/095340) with (9Z,12Z)-3-hydroxy-2-(hydroxymethyl)propyl octadeca-9,12-dienoate ("Intermediate 13c"), prior to addition of the head group by reacting the product of Intermediate 13b and Intermediate 13c with 3-diethylamino-1-propanol. (See pp. 84-86 of WO2015/095340.)

Intermediate 13b from WO2015/095340 (4,4 bis(octyloxy)butanoic acid) was synthesized via 4,4-bis(octyloxy)butanenitrile as follows:

### Intermediate 13a: 4,4-bis(octyloxy)butanenitrile

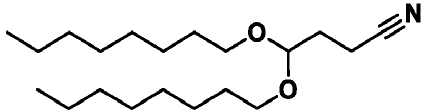

To a mixture of 4,4-diethoxybutanenitrile (9.4 g, 60 mmol) and octan-1-ol (23.1 g, 178 mmol) was added pyridinium *p*-toluenesulfonate (748 mg, 3.0 mmol) at rt. The mixture was warmed to 105°C and stirred for 18 hours with the reaction vessel open to air and not fitted with a refluxing condenser. The reaction mixture was then cooled to room temperature and purified on silica gel (0-5% gradient of ethyl acetate in hexanes) to provide 10.1 g (31.0 mmol) of Intermediate 13a as a clear oil. ¹H NMR (400 MHz, CDCl₃) δ 4.55 (t, *J* = 5.3 Hz, 1H), 3.60 (dt, *J* = 9.2, 6.6 Hz, 2H), 3.43 (dt, *J* = 9.2, 6.6 Hz, 2H), 2.42 (t, *J* = 7.4 Hz, 2H), 1.94 (td, *J* = 7.4, 5.3 Hz, 2H), 1.63 - 1.50 (m, 4H), 1.38 - 1.19 (m, 20H), 0.93 - 0.82 (m, 6H) ppm.

Next, to a solution of Intermediate 13a (8.42 g, 31 mmol) in ethanol (30 mL) was added 31 mL of aqueous potassium hydroxide (2.5 M, 30.9 mL, 77.3 mmol) at room temperature. Upon fitting the vessel with a reflux condenser, the mixture was heated to 110 °C and stirred for 24 hours. The mixture was then cooled to room temperature, acidified with aqueous hydrochloride acid (1N) to pH 5, and extracted into hexanes three times. The combined organic extracts were washed with water (twice) and brine, dried over anhydrous magnesium sulfate, and concentrated in vacuo to afford 8.15 g (23.6 mmol) of Intermediate 13b as a clear oil, which was used without further purification. ¹H NMR (400 MHz, CDCl₃) δ 4.50 (t, *J* = 5.5 Hz, 1H), 3.57 (dt, *J* = 9.4, 6.7 Hz, 2H), 3.41 (dt, *J* = 9.3, 6.7 Hz, 2H), 2.40 (t, *J* = 7.4 Hz, 2H), 1.92 (td, *J* = 7.4, 5.3 Hz, 2H), 1.56 (m, 4H), 1.37 - 1.21 (m, 20H), 0.92 - 0.83 (m, 6H) ppm (structure below).

### Intermediate 13b

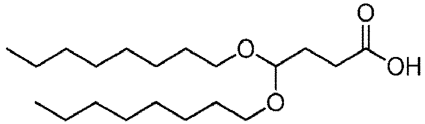

Using the methods described above, the C(5, 6, 7, 9, and 10)-acetal acid intermediates, called Intermediates B3-F3 and depicted below, were prepared using the appropriate alkan-1-ol reagents.

### Intermediate B3 4,4-bis(pentyloxy)butanoic acid

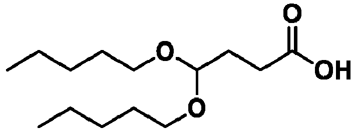

¹H NMR (400 MHz, CDCl₃) δ 4.52 (t, *J* = 5.5 Hz, 1H), 3.58 (dt, *J* = 9.3, 6.6 Hz, 2H), 3.41 (dt, *J* = 9.3, 6.7 Hz, 2H), 2.45 (t, *J* = 7.4 Hz, 2H), 1.94 (m, 2H), 1.57 (m, 4H), 1.32 (m, *J* = 3.7 Hz, 8H), 0.95 - 0.83 (m, 6H) ppm.

### Intermediate C3: 4,4-bis(hexyloxy)butanoic acid

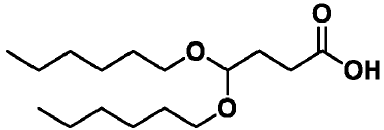

¹H NMR (400 MHz, CDCl₃) δ 4.44 (t, *J* = 5.6 Hz, 1H), 3.49 (dt, *J* = 9.3, 6.9 Hz, 2H), 3.39 (dt, *J* = 9.3, 6.8 Hz, 2H), 2.12 (t, *J* = 7.6 Hz, 2H), 1.79 (q, *J* = 7.0 Hz, 2H), 1.54 (m, 4H), 1.29 (m, 12H), 0.94 - 0.82 (m, 6H) ppm.

### Intermediate D3: 4,4-bis(heptyloxy)butanoic acid

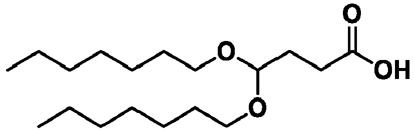

¹H NMR (400 MHz, CDCl₃) δ 8.85 (br s, 1H), 4.46 (t, *J* = 5.6 Hz, 1H), 3.52 (dt, *J* = 9.4, 6.8 Hz, 2H), 3.39 (dt, *J* = 9.3, 6.8 Hz, 2H), 2.26 (t, *J* = 7.6 Hz, 2H), 1.85 (q, *J* = 7.0 Hz, 2H), 1.53 (m, 4H), 1.29 (m, 16H), 0.94 - 0.80 (m, 6H) ppm.

### Intermediate E3: 4,4-bis(nonyloxy)butanoic acid

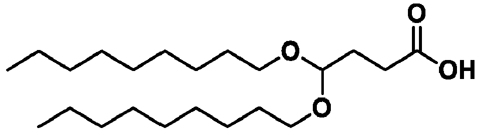

¹H NMR (400 MHz, CDCl₃) δ 5.32 (br s, 1H), 4.44 (t, *J* = 5.6 Hz, 1H), 3.49 (dt, *J* = 9.3, 6.9 Hz, 2H), 3.38 (dt, *J* = 9.4, 6.9 Hz, 2H), 2.10 (t, *J* = 7.6 Hz, 2H), 1.78 (q, *J* = 7.0 Hz, 2H), 1.53 (m, 4H), 1.27 (m, 24H), 0.88 (t, *J* = 6.6 Hz, 6H) ppm.

### Intermediate F3: 4,4-bis(decyloxy)butanoic acid:

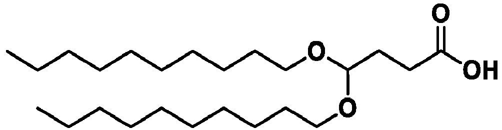

¹H NMR (400 MHz, CDCl₃) δ 4.48 (t, *J* = 5.5 Hz, 1H), 3.55 (m, 2H), 3.42 (m, 2H), 2.29 (dd, *J* = 10.8, 7.5 Hz, 2H), 1.90 - 1.82 (m, 2H), 1.55 (m, 4H), 1.27 (m, 28H), 0.88 (t, *J* = 6.7 Hz, 6H) ppm.

Acetal analogs of Lipid A (C(8)) were synthesized by reacting the C(5, 6, 7, 9, or 10)-acetal acid intermediates (B3-F3) with Intermediate 13c, prior to reacting the product of that step with 3-diethylamino-1-propanol. (See pp 84-86 of WO2015/095340.) Each analog was synthesized and characterized by ¹H NMR (data not shown).

The C7, C9, and C10 analogs were formulated at 45 mol-% Lipid A Analog, 2 mol-% DMG-PEG2k, 9 mol-% DSPC, and 44 mol-% cholesterol, with an N:P ratio of 4.5. Each analog was also formulated at 55 mol-% Lipid A Analog, 2.5 mol-% DMG-PEG2k, 9 mol-% DSPC, and 38.5 mol-% cholesterol, with an N:P ratio of 6. The lipid nanoparticle components were dissolved in 100% ethanol with the lipid component molar ratios set forth above. The RNA cargos were prepared in 25 mM citrate, 100 mM NaCl, pH 5.0, resulting in a concentration of RNA cargo of approximately 0.45 mg/mL.

The RNA cargo included Cas9 mRNA comprising SEQ ID NO:43 and sg282, prepared as described above. The LNPs were formed as described in Example 1.

An expanded panel of acetal analogs, including LNP compositions comprising the C(5) and C(6) Lipid A analogs were tested alongside the prior panel. The two new analogs were formulated at 55 mol-% Lipid A Analog, 2.5 mol-% DMG-PEG2k, 9 mol-% DPSC, and 33.5 mol-% cholesterol, with an N/P ratio of 6, as described above. Analysis indicated that sizes for all LNPs is below 120 nm, PDI is below 0.2 and %-encapsulated RNA is higher than 80%. Analytical results for the formulations are in Table 28, below.

**Table 28.**

| **LNP ID** | **Lipid A Analogs** | **Analog mol-%** | **PEG%** | **N/P** | **RNA Conc (mg/ mL)** | **%EE** | **Z-avg. (nm)** | **PDI** | **Number mean (nm)** |
|---|---|---|---|---|---|---|---|---|---|
| **LNP 1122** | C5 analog (LP000030-001) | 55 | 2.5 | 6 | 0.063 | 88 | 118.8 | 0.103 | 88.17 |
| **LNP 1123** | C6 analog (LP000031-001) | 55 | 2.5 | 6 | 0.067 | 95 | 107.6 | 0.038 | 88.1 |
| **LNP 1004** | C7 analog (LP000020-001) | 55 | 2.5 | 6 | 0.068 | 98 | 100 | 0.012 | 81.55 |
| **LNP 1002** | LP000001-011 | 55 | 2.5 | 6 | 0.067 | 98 | 95.06 | 0.01 | 78.95 |
| **LNP 1006** | C9 analog (LP000021-001) | 55 | 2.5 | 6 | 0.067 | 97 | 95.43 | 0.022 | 80.35 |
| **LNP 1008** | C10 analog (LP000022-001) | 55 | 2.5 | 6 | 0.069 | 95 | 103.9 | 0.008 | 86.79 |

The analogs were assessed for pKa using 6-(p-toluidino)-6-napthalene sulfonic acid ("TNS") dissolved in water. In this assay 0.1 M phosphate buffer was prepared at different pH values ranging from 4.5 to 10.5. Each analog was individually prepared in 100% ethanol. The lipid and TNS were then added in individual pH buffer and transferred to a plate to analyze at 321-488 nm wavelength on the SpectraMax plate reader. Values were plotted to generate pKa, logIC₅₀ is used as pKa.

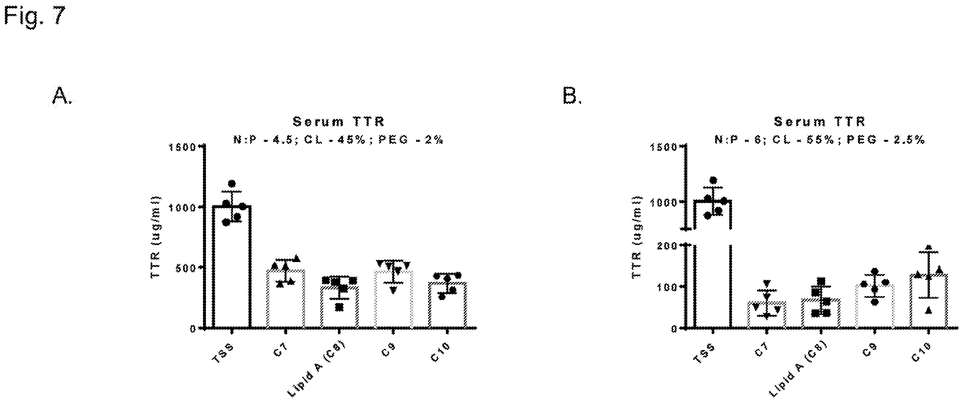

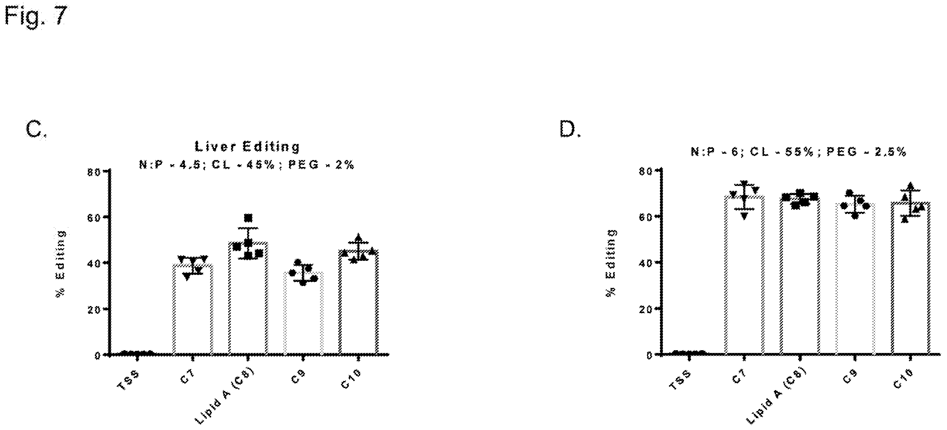

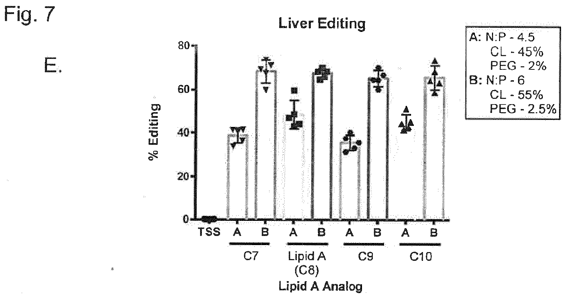

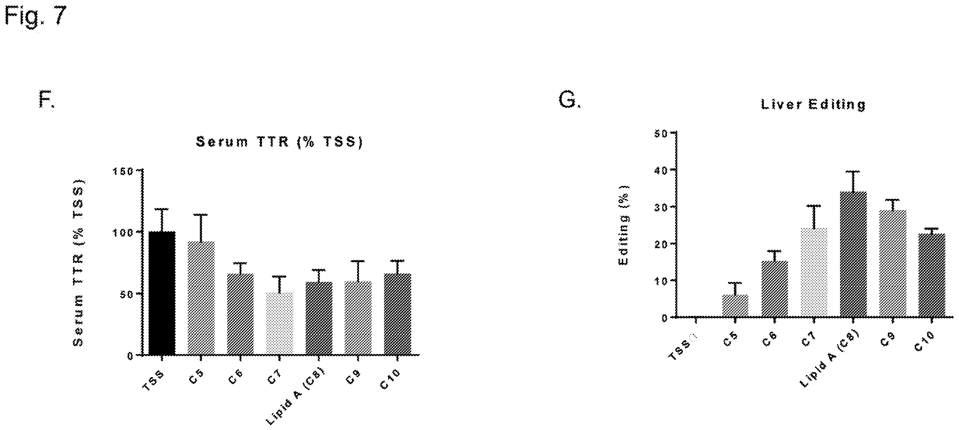

Female CD-1 mice were dosed as described in Example 1 with 0.3 mg/kg (Fig. 7A-Fig. 7E), or with 0.1 mg per kg (Fig. 7F-Fig. 7G). In brief, CD-1 female mice from Charles River Laboratories, n=5 per group, were administered the LNP compositions at varying doses. At necropsy (7 days post dose), serum was collected for TTR analysis and liver was collected for editing analysis. Serum TTR and percent editing assays were performed as described in Example 1. The serum TTR levels and liver editing from Fig. 7A-Fig. 7E indicate that all the analogs performed comparably to Lipid A at 0.3 milligrams per kilogram body weight. Fig. 7F-Fig. 7G indicate that while Lipid A had the highest potency, the newly synthesized analogs all have suitable TTR knockdown and liver editing.

### Example 8 - Dose Response Curve - Primary Cyno hepatocytes

*Primary liver hepatocytes.* Primary cynomolgus liver hepatocytes (PCH) (Gibco) were thawed and resuspended in hepatocyte thawing medium with supplements (Gibco, Cat. CM7000) followed by centrifugation at 80 g for 4 minutes. The supernatant was discarded and the pelleted cells resuspended in hepatocyte plating medium plus supplement pack (Invitrogen, Cat. A1217601 and CM3000). Cells were counted and plated on Bio-coat collagen I coated 96-well plates (ThermoFisher, Cat. 877272) at a density of 50,000 cells/well. Plated cells were allowed to settle and adhere for 24 hours in a tissue culture incubator (37°C and 5% CO₂ atmosphere) prior to LNP administration. After incubation cells were checked for monolayer formation and media was replaced with hepatocyte culture medium with serum-free supplement pack (Invitrogen, Cat. A1217601 and CM4000).

LNP formulations for this study (LNP1021, LNP1022, LNP1023, LNP1024, LNP1025, and LNP897) were prepared as described above.

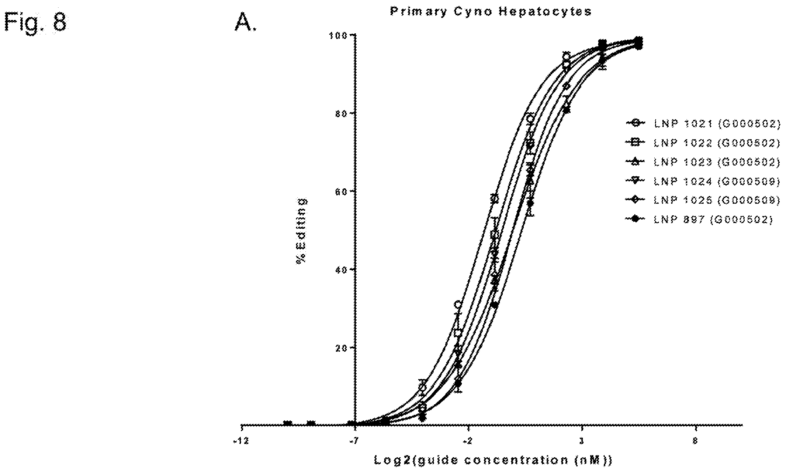

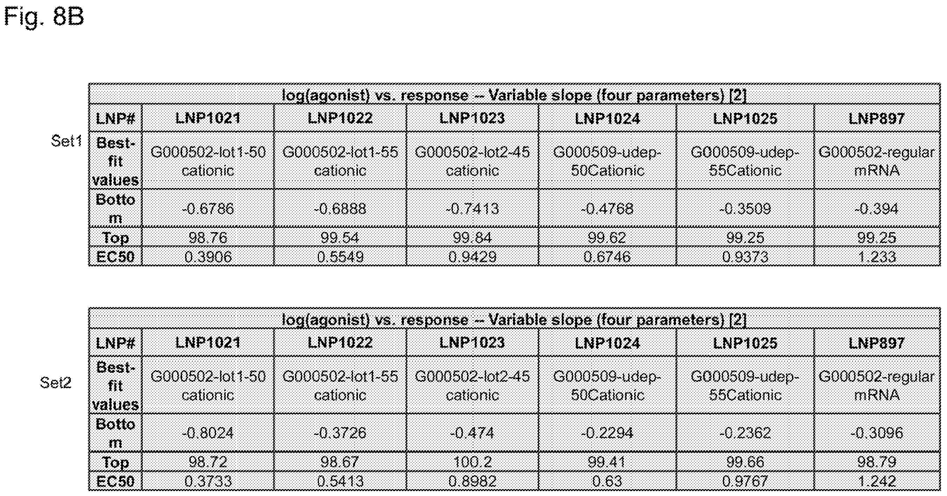

Various doses of lipid nanoparticle formulations containing modified sgRNAs were tested on primary cyno hepatocytes to generate a dose response curve. After plating and 24 hours in culture, LNPs were incubated in hepatocyte maintenance media containing 6% cyno serum at 37°C for 5 minutes. Post-incubation the LNPs were added onto the primary cyno hepatocytes in an 8 point 2-fold dose response curve starting at 100 ng mRNA. The cells were lysed 72 hours post treatment for NGS analysis as described in Example 1. Percent editing was determined for various LNP compositions and the data are graphed in Fig. 8A. The % editing with Cas9 mRNA (SEQ ID NO 48) and U-depleted Cas9 mRNA (SEQ I NO:43) is presented in Fig. 8B. LNP compositions are described in Table 2 (LNP 897) and Table 5 (LNP 1021, 1022, 1023, 1024, and 1025).

The results show a quantitative assay for comparative potency assessements, demonstrating both mRNA and LNP composition affect potency.

### Example 9 - RNA Cargo: mRNA and gRNA Coformulations

This study evaluated *in vivo* efficacy in mice of different ratios of gRNA to mRNA. CleanCap^{™} capped Cas9 mRNAs with the ORF of SEQ ID NO: 4, HSD 5' UTR, human albumin 3' UTR, a Kozak sequence, and a poly-A tail were made by IVT synthesis as indicated in Example 1 with N1-methylpseudouridine triphosphate in place of uridine triphosphate.

LNP formulations prepared from the mRNA described and sg282 (SEQ ID NO: 42; G282) as described in Example 2 with Lipid A, cholesterol, DSPC, and PEG2k-DMG in a 50:38:9:3 molar ratio and with an N:P ratio of 6. The gRNA:Cas9 mRNA weight ratios of the formulations were as shown in Table 29.

**Table 29.**

| **LNP ID** | **Guide : Cas9 m RNA Ratio (w/w)** | **RNA Cone (mg/mL)** | **EE (%)** | **Z-Ave Size (nm)** | **Particle PDI** | **Number Ave (nm)** |
|---|---|---|---|---|---|---|
| **1110** | 8:1 | 0.92 | 99 | 69.52 | 0.022 | 56.47 |
| **1111** | 4:1 | 0.86 | 97 | 76.65 | 0.065 | 57.36 |
| **1112** | 2:1 | 0.90 | 99 | 76.58 | 0.036 | 63.11 |
| **1113** | 1:1 | 0.97 | 99 | 76.60 | 0.071 | 58.92 |
| **1114** | 1:2 | 1.05 | 99 | 76.34 | 0.018 | 62.82 |
| **1115** | 1:4 | 0.65 | 99 | 82.64 | 0.018 | 66.63 |
| **1116** | 1:8 | 0.75 | 100 | 82.01 | 0.039 | 65.05 |

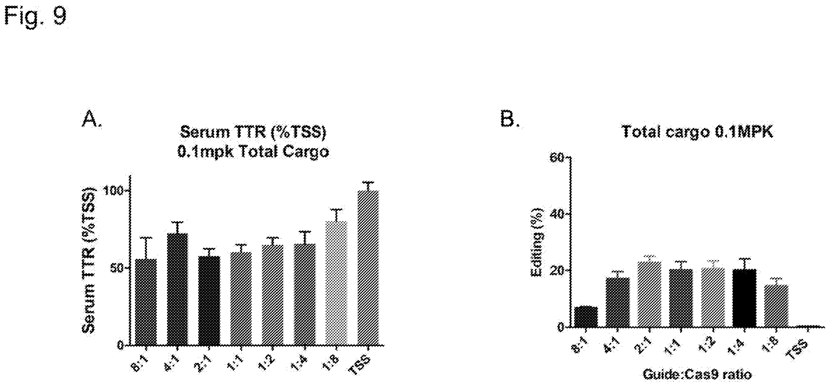

For *in vivo* characterization, the above LNPs were administered to mice at 0.1 mg total RNA (mg guide RNA + mg mRNA) per kg (n=5 per group). At 7 to 9 days post-dose, animals were sacrificed, blood and the liver were collected, and serum TTR and liver editing were measured as described in Example 1. Serum TTR and liver editing results are shown in Fig. 9A and 9B. Negative control mice were dosed with TSS vehicle.

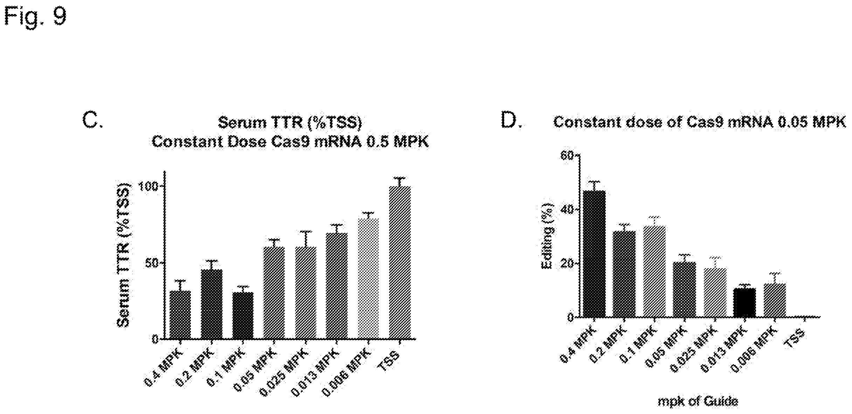

In addition, the above LNPs were administered to mice at a constant mRNA dose of 0.05 mg mRNA per kg (n=5 per group), while varying the gRNA dose from 0.06 mg per kg to 0.4 mg per kg. At 7 to 9 days post-dose, animals were sacrificed, blood and the liver were collected, and serum TTR and liver editing were measured. Serum TTR and liver editing results are shown in Fig. 9C and Fig. 9D. Negative control mice were dosed with TSS vehicle.

### Example 10 - Neutral Lipids

To evaluate the *in vivo* efficacy of LNPs, LNP formulations were prepared with the mRNA of Example 2 and sg534 (SEQ ID NO: 72; G534), as described in Example 2. The lipid nanoparticle components were dissolved in 100% ethanol with the lipid component molar ratios set forth below. In brief, the RNA cargos were prepared in a buffer of 25 mM citrate and 100 mM NaCl at pH 5.0, resulting in a concentration of RNA cargo of approximately 0.45 mg/mL. The LNPs were formulated with a lipid amine to RNA phosphate (N:P) molar ratio of about 6 with the ratio of gRNA to mRNA at 1:2 by weight.

LNP formulations were analyzed for average particle size, polydispersity (pdi), total RNA content and encapsulation efficiency of RNA as described in Example 1. Analysis of average particle size, polydispersity (PDI), total RNA content and encapsulation efficiency of RNA are shown in Table 30. Molar ratios of lipid are provided as amine lipid (Lipid A)/neutral lipid/helper lipid (cholesterol)/PEG lipid (PEG2k-DMG). The neutral lipid was DSP, DPPC, or absent, as specified.

**Table 30. LNP compositions and data. (Molar ratios of lipid are provided as amine lipid (Lipid A)/neutral lipid/helper lipid (cholesterol)/PEG lipid (PEG2k-DMG).)**

| **Sample ID** | **Neutral Lipid** | **Molar Ratios** | **RNA Conc (mg/ mL)** | **%EE** | **Z-avg. (nm)** | **PDI** | **Number mean (nm)** | **% Liver Editing** | **Serum TTR (µg/mL)** | **% TTR KD** |
|---|---|---|---|---|---|---|---|---|---|---|
| TSS control | | | | | | | | 0.0 | 1248.9 | |
| CO241 | - | 50.0/0.0/ 47.0/3.0 | 1.46 | 94 | 75.64 | 0.090 | 54.21 | 1.8 | 1070.2 | 14.3 |
| CO242 | - | 59.0/0.0/ 38.0/3.0 | 1.51 | 94 | 92.25 | 0.019 | 75.56 | 12.0 | 819.6 | 34.4 |
| CO243 | - | 54.5/0.0/ 42.5/3.0 | 1.62 | 94 | 78.90 | 0.052 | 61.49 | 3.3 | 1260.5 | -0.9 |
| CO244 | DSPC | 50.0/9.0/ 39.0/2.0 | 1.50 | 93 | 101.3 | 0.044 | 80.73 | 27.4 | 741.0 | 40.7 |
| CO034 | DSPC | 50.0/9.0/ 38.0/3.0 | 1.48 | 97 | 84.23 | 0.040 | 66.96 | 34.2 | 630.1 | 49.6 |
| CO245 | DSPC | 52.5/4.0/ 42.5/3.0 | 1.55 | 95 | 81.88 | 0.054 | 64.54 | 5.8 | 846.6 | 32.2 |
| CO246 | DPPC | 50.0/9.0/ 38.0/3.0 | 1.52 | 96 | 87.11 | 0.040 | 70.04 | 35.9 | 528.6 | 57.7 |
| CO247 | DPPC | 52.5/4.0/ 42.5/3.0 | 1.54 | 97 | 83.67 | 0.050 | 66.43 | 18.3 | 726.6 | 41.8 |

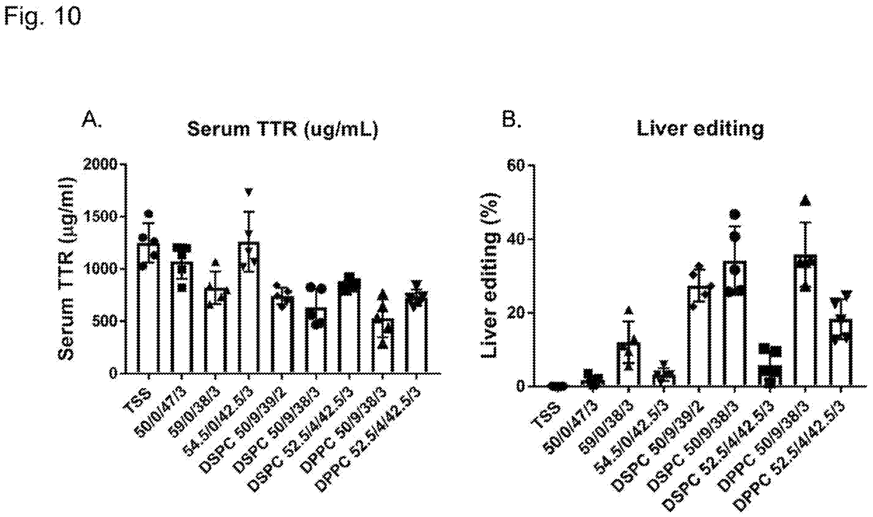

For *in vivo* characterization, the above LNPs were administered intravenously to female Sprague Dawley rats at 0.3 mg total RNA (guide RNA and mRNA) per kg bodyweight. There were five rats per group. At seven days post-dosing, animals were sacrificed, blood and the liver were collected, and serum TTR and liver editing were measured as described in Example 1. Negative control animals were dosed with TSS vehicle. Serum TTR and liver editing results are shown in Fig. 10A and 10B, and in Table 30 (above).

### EMBODIMENTS

The present invention provides the following embodiments:
1. A lipid nanoparticle ("LNP") composition comprising:
   an RNA component; and
   a lipid component, wherein the lipid component comprises:
      about 50-60 mol-% amine lipid;
      about 8-10 mol-% neutral lipid; and
      about 2.5-4 mol-% PEG lipid,
   wherein the remainder of the lipid component is helper lipid, and
   wherein the N/P ratio of the LNP composition is about 6.
2. An LNP composition comprising:
   an RNA component;
      about 50-60 mol-% amine lipid;
      about 27-39.5 mol-% helper lipid;
      about 8-10 mol-% neutral lipid; and
      about 2.5-4 mol-% PEG lipid,
   wherein the N/P ratio of the LNP composition is about 5-7.
3. The LNP composition of embodiment 2, wherein the N/P ratio is about 6.
4. An LNP composition comprising:
   an RNA component; and
   a lipid component, wherein the lipid component comprises
      about 50-60 mol-% amine lipid;
      about 5-15 mol-% neutral lipid; and
      about 2.5-4 mol-% PEG lipid,
   wherein the remainder of the lipid component is helper lipid, and
   wherein the N/P ratio of the LNP composition is about 3-10.
5. An LNP composition comprising:
   an RNA component; and
   a lipid component, wherein the lipid component comprises
      about 40-60 mol-% amine lipid;
      about 5-15 mol-% neutral lipid; and
      about 2.5-4 mol-% PEG lipid,
   wherein the remainder of the lipid component is helper lipid, and
   wherein the N/P ratio of the LNP composition is about 6.
6. An LNP composition comprising:
   an RNA component; and
   a lipid component, wherein the lipid component comprises
      about 50-60 mol-% amine lipid;
      about 5-15 mol-% neutral lipid; and
      about 1.5-10 mol-% PEG lipid,
   wherein the remainder of the lipid component is helper lipid, and
   wherein the N/P ratio of the LNP composition is about 6.
7. An LNP composition comprising:
   an RNA component; and
   a lipid component, wherein the lipid component comprises
      about 40-60 mol-% amine lipid;
      about 0-10 mol-% neutral lipid; and
      about 1.5-10 mol-% PEG lipid,
   wherein the remainder of the lipid component is helper lipid, and
   wherein the N/P ratio of the LNP composition is about 3-10.
8. An LNP composition comprising:
   an RNA component; and
   a lipid component, wherein the lipid component comprises:
      about 40-60 mol-% amine lipid;
      less than about 1 mol-% neutral lipid; and
      about 1.5-10 mol-% PEG lipid,
   wherein the remainder of the lipid component is helper lipid, and
   wherein the N/P ratio of the LNP composition is about 3-10.
9. An LNP composition comprising:
   an RNA component; and
   a lipid component, wherein the lipid component comprises:
      about 40-60 mol-% amine lipid; and
      about 1.5-10 mol-% PEG lipid,
   wherein the remainder of the lipid component is helper lipid,
   wherein the N/P ratio of the LNP composition is about 3-10, and
   wherein the LNP composition is essentially free of or free of neutral phospholipid.
10. An LNP composition comprising:
   an RNA component; and
   a lipid component, wherein the lipid component comprises:
      about 50-60 mol-% amine lipid;
      about 8-10 mol-% neutral lipid; and
      about 2.5-4 mol-% PEG lipid,
   wherein the remainder of the lipid component is helper lipid, and
   wherein the N/P ratio of the LNP composition is about 3-7.
11. The composition of any preceding embodiment, wherein the RNA component comprises an mRNA.
12. The composition of any preceding embodiment, wherein the RNA component comprises an RNA-guided DNA-binding agent, such as a Cas nuclease mRNA.
13. The composition of any preceding embodiment, wherein the RNA component comprises a Class 2 Cas nuclease mRNA.
14. The composition of any preceding embodiment, wherein the RNA component comprises a Cas9 nuclease mRNA.
15. The composition of any of embodiments 11-14, wherein the mRNA is a modified mRNA.
16. The composition of any preceding embodiment, wherein the RNA component comprises an RNA comprising an open reading frame encoding an RNA-guided DNA-binding agent, wherein the open reading frame has a uridine content ranging from its minimum uridine content to 150% of the minimum uridine content.
17. The composition of any preceding embodiment, wherein the RNA component comprises an mRNA comprising an open reading frame encoding an RNA-guided DNA-binding agent, wherein the open reading frame has a uridine dinucleotide content ranging from its minimum uridine dinucleotide content to 150% of the minimum uridine dinucleotide content.
18. The composition of any preceding embodiment, wherein the RNA component comprises an mRNA comprising a sequence with at least 90% identity to any one of SEQ ID NO: 1, 4, 7, 9, 10, 11, 12, 14, 15, 17, 18, 20, 21, 23, 24, 26, 27, 29, 30, 50, 52, 54, 65, or 66, wherein the mRNA comprises an open reading frame encoding an RNA-guided DNA-binding agent.
19. The composition of any of any preceding embodiment, wherein the RNA component comprises a gRNA nucleic acid.
20. The composition of embodiment 19, wherein the gRNA nucleic acid is a gRNA.
21. The composition of any preceding embodiment, wherein the RNA component comprises a Class 2 Cas nuclease mRNA and a gRNA.
22. The composition of any of embodiments 19-21, wherein the gRNA nucleic acid is or encodes a dual-guide RNA (dgRNA).
23. The composition of any of embodiments 19-21, wherein the gRNA nucleic acid is or encodes an sgRNA.
24. The composition of any of embodiments 19-23, wherein the gRNA is modified.
25. The composition of embodiment 24, wherein the gRNA comprises a modification chosen from 2'-O-methyl (2'-O-Me) modified nucleotide, a phosphorothioate (PS) bond between nucleotides; and a 2'-fluoro (2'-F) modified nucleotide.
26. The composition of any of embodiments 24-25, wherein the gRNA comprises a modification at one or more of the first five nucleotides at the 5' end.
27. The composition of any of embodiments 24-26, wherein the gRNA comprises a modification at one or more of the last five nucleotides at the 3' end.
28. The composition of any of embodiments 24-27, wherein the gRNA comprises PS bonds between the first four nucleotides.
29. The composition of any of embodiments 24-28, wherein the gRNA comprises PS bonds between the last four nucleotides.
30. The composition of any of embodiments 24-29, further comprising 2'-O-Me modified nucleotides at the first three nucleotides at the 5' end.
31. The composition of any of embodiments 24-30, further comprising 2'-O-Me modified nucleotides at the last three nucleotides at the 3' end.
32. The composition of any of embodiments 19-31, wherein the gRNA and Class 2 Cas nuclease mRNA are present in a ratio ranging from about 10:1 to about 1:10 by weight.
33. The composition of any of embodiments 19-31, wherein the gRNA and Class 2 Cas nuclease mRNA are present in a ratio ranging from about 5:1 to about 1:5 by weight
34. The composition of any of embodiments 19-33, wherein the gRNA and Class 2 Cas nuclease mRNA are present in a ratio ranging from about 3:1 to about 1:1 by weight.
35. The composition of any of embodiments 19-34, wherein the gRNA and Class 2 Cas nuclease mRNA are present in a ratio ranging from about 2:1 to about 1:1 by weight.
36. The composition of any of embodiments 19-35, wherein the gRNA and Class 2 Cas nuclease mRNA are present in a ratio of about 2:1 by weight
37. The composition of any of embodiments 19-35, wherein the gRNA and Class 2 Cas nuclease mRNA are present in a ratio of about 1:1 by weight.
38. The composition of any preceding embodiment, further comprising at least one template.
39. The composition of any preceding embodiment, wherein the mol-% PEG lipid is about 3.
40. The composition of any preceding embodiment, wherein the mol-% amine lipid is about 50.
41. The composition of any preceding embodiment, wherein the mol-% amine lipid is about 55.
42. The composition of any preceding embodiment, wherein the mol-% amime lipid is ± 3 mol-%.
43. The composition of any preceding embodiment, wherein the mol-% amine lipid is ± 2 mol-%.
44. The composition of any preceding embodiment, wherein the mol-% amine lipid is 47-53 mol-%.
45. The composition of any preceding embodiment, wherein the mol-% amine lipid is 48-53 mol-%.
46. The composition of any preceding embodiment, wherein the mol-% amine lipid is 53-57 mol-%.
47. The composition of any preceding embodiment, wherein the N/P ratio is 6 ± 1.
48. The composition of any preceding embodiment, wherein the N/P ratio is 6 ± 0.5.
49. The composition of any preceding embodiment, wherein the amine lipid is Lipid A.
50. The composition of any preceding embodiment, wherein the amine lipid is an analog of Lipid A.
51. The composition of embodiment 50, wherein the analog is an acetal analog.
52. The composition of embodiment 51, wherein the acetal analog is a C4-C12 acetal analog.
53. The composition of embodiment 50, wherein the acetal analog is a C5-C12 acetal analog.
54. The composition of embodiment 50, wherein the acetal analog is a C5-C10 acetal analog.
55. The compositionof embodiment 50, wherein the acetal analog is chosenfrom a C4, C5, C6, C7, C9, C10, C11, and C12 analog.
56. The composition of any preceding embodiment, wherein the helper lipid is cholesterol.
57. The composition of any preceding embodiment, wherein the neutral lipid is DSPC.
58. The composition of any preceding embodiment, wherein the neutral lipid is DPPC.
59. The composition of any preceding embodiment, wherein the PEG lipid comprises dimyristoylglycerol (DMG).
60. The composition of any preceding embodiment, wherein the PEG lipid comprises a PEG-2k.
61. The composition of any preceding embodiment, wherein the PEG lipid is a PEG-DMG.
62. The composition of embodiment 61, wherein the PEG-DMG is a PEG2k-DMG.
63. The composition of embodiment 9, wherein the LNP composition is essentially free of neutral lipid.
64. The composition of embodiment 63, wherein the neutral lipid is a phospholipid.
65. A method of gene editing, comprising contacting a cell with an LNP composition of any of embodiments 12-64.
66. A method of gene editing, comprising delivering a Class 2 Cas nuclease mRNA and a guide RNA nucleic acid to a cell, wherein the Class 2 Cas mRNA and the guide RNA nucleic acid are formulated as at least one LNP composition of any of embodiments 13-64.
67. A method of producing a genetically engineered cell, comprising contacting a cell with at least one LNP composition of any of embodiments 12-64.
68. The method of any of embodiments 65-67, wherein the LNP composition is administered at least two times.
69. The method of embodiment 68, wherein the LNP composition is administered 2-5 times.
70. The method of embodiment 68 or 69, wherein editing improves upon readministration.
71. The method of any of embodiments 65-70, further comprising introducing at least one template nucleic acid to the cell.
72. The method of any of embodiments 65-71, wherein the mRNA is formulated in a first LNP composition and the guide RNA nucleic acid is formulated in a second LNP composition.
73. The method of embodiment 72, wherein the first and second LNP compositions are administered simultaneously.
74. The method of embodiment 72, wherein the first and second LNP compositions are administered sequentially.
75. The method of any of embodiments 65-73, wherein the mRNA and the guide RNA nucleic acid are formulated in a single LNP composition.

### BRIEF DESCRIPTION OF DISCLOSED SEQUENCES

| **SEQ ID NO** | **Description** |
|---|---|
| 1 | DNA coding sequence of Cas9 using the thymidine analog of the minimal uridine codons listed in Table 3, with start and stop codons |
| 2 | DNA coding sequence of Cas9 using codons with generally high expression in humans |
| 3 | Amino acid sequence of Cas9 with one nuclear localization signal (1xNLS) as the C-terminal 7 amino acids |
| 4 | Cas9 mRNA ORF using minimal uridine codons as listed in Table 3, with start and stop codons |
| 5 | Cas9 mRNA ORF using codons with generally high expression in humans, with start and stop codons |
| 6 | Amino acid sequence of Cas9 nickase with 1xNLS as the C-terminal 7 amino acids |
| 7 | Cas9 nickase mRNA ORF encoding SEQ ID NO: 6 using minimal uridine codons as listed in Table 3, with start and stop codons |
| 8 | Amino acid sequence of dCas9 with 1xNLS as the C-terminal 7 amino acids |
| 9 | dCas9 mRNA ORF encoding SEQ ID NO: 8 using minimal uridine codons as listed in Table 3, with start and stop codons |
| 10 | Cas9 mRNA coding sequence using minimal uridine codons as listed in Table 3 (no start or stop codons; suitable for inclusion in fusion protein coding sequence) |
| 11 | Cas9 nickase mRNA coding sequence using minimal uridine codons as listed in Table 3 (no start or stop codons; suitable for inclusion in fusion protein coding sequence) |
| 12 | dCas9 mRNA coding sequence using minimal uridine codons as listed in Table 3 (no start or stop codons; suitable for inclusion in fusion protein coding sequence) |
| 13 | Amino acid sequence of Cas9 (without NLS) |
| 14 | Cas9 mRNA ORF encoding SEQ ID NO: 13 using minimal uridine codons as listed in Table 3, with start and stop codons |
| 15 | Cas9 coding sequence encoding SEQ ID NO: 13 using minimal uridine codons as listed in Table 3 (no start or stop codons; suitable for inclusion in fusion protein coding sequence) |
| 16 | Amino acid sequence of Cas9 nickase (without NLS) |
| 17 | Cas9 nickase mRNA ORF encoding SEQ ID NO: 16 using minimal uridine codons as listed in Table 3, with start and stop codons |
| 18 | Cas9 nickase coding sequence encoding SEQ ID NO: 16 using minimal uridine codons as listed in Table 3 (no start or stop codons; suitable for inclusion in fusion protein coding sequence) |
| 19 | Amino acid sequence of dCas9 (without NLS) |
| 20 | dCas9 mRNA ORF encoding SEQ ID NO: 13 using minimal uridine codons as listed in Table 3, with start and stop codons |
| 21 | dCas9 coding sequence encoding SEQ ID NO: 13 using minimal uridine codons as listed in Table 3 (no start or stop codons; suitable for inclusion in fusion protein coding sequence) |
| 22 | Amino acid sequence of Cas9 with two nuclear localization signals (2xNLS) as the C-terminal amino acids |
| 23 | Cas9 mRNA ORF encoding SEQ ID NO: 13 using minimal uridine codons as listed in Table 3, with start and stop codons |
| 24 | Cas9 coding sequence encoding SEQ ID NO: 13 using minimal uridine codons as listed in Table 3 (no start or stop codons; suitable for inclusion in fusion protein coding sequence) |
| 25 | Amino acid sequence of Cas9 nickase with two nuclear localization signals as the C-terminal amino acids |
| 26 | Cas9 nickase mRNA ORF encoding SEQ ID NO: 16 using minimal uridine codons as listed in Table 3, with start and stop codons |
| 27 | Cas9 nickase coding sequence encoding SEQ ID NO: 16 using minimal uridine codons as listed in Table 3 (no start or stop codons; suitable for inclusion in fusion protein coding sequence) |
| 28 | Amino acid sequence of dCas9 with two nuclear localization signals as the C-terminal amino acids |
| 29 | dCas9 mRNA ORF encoding SEQ ID NO: 13 using minimal uridine codons as listed in Table 3, with start and stop codons |
| 30 | dCas9 coding sequence encoding SEQ ID NO: 13 using minimal uridine codons as listed in Table 3 (no start or stop codons; suitable for inclusion in fusion protein coding sequence) |
| 31 | T7 Promoter |
| 32 | Human beta-globin 5' UTR |
| 33 | Human beta-globin 3' UTR |
| 34 | Human alpha-globin 5' UTR |
| 35 | Human alpha-globin 3' UTR |
| 36 | *Xenopus laevis* beta-globin 5' UTR |
| 37 | *Xenopus laevis* beta-globin 3' UTR |
| 38 | Bovine Growth Hormone 5' UTR |
| 39 | Bovine Growth Hormone 3' UTR |
| 40 | Mus musculus hemoglobin alpha, adult chain 1 (Hba-a1), 3'UTR |
| 41 | HSD17B4 5' UTR |
| 42 | G282 single guide RNA targeting the mouse TTR gene |
| 43 | Cas9 transcript with 5' UTR of HSD, ORF corresponding to SEQ ID NO: 4, Kozak sequence, and 3' UTR of ALB |
| 44 | Cas9 transcript with 5' UTR of HSD, ORF corresponding to SEQ ID NO: 4, and 3' UTR of ALB |
| 45 | Alternative Cas9 ORF with 19.36% U content |
| 46 | Cas9 transcript with 5' UTR of HSD, ORF corresponding to SEQ ID NO: 45, Kozak sequence, and 3' UTR of ALB |
| 47 | Cas9 transcript with 5' UTR of HSD, ORF corresponding to SEQ ID NO: 45, and 3' UTR of ALB |
| 48 | Cas9 transcript comprising Cas9 ORF using codons with generally high expression in humans |
| 49 | Cas9 transcript comprising Kozak sequence with Cas9 ORF using codons with generally high expression in humans |
| 50 | Cas9 ORF with splice junctions removed; 12.75% U content |
| 51 | Cas9 transcript with 5' UTR of HSD, ORF corresponding to SEQ ID NO: 50, Kozak sequence, and 3' UTR of ALB |
| 52 | Cas9 ORF with minimal uridine codons frequently used in humans in general; 12.75% U content |
| 53 | Cas9 transcript with 5' UTR of HSD, ORF corresponding to SEQ ID NO: 52, Kozak sequence, and 3' UTR of ALB |
| 54 | Cas9 ORF with minimal uridine codons infrequently used in humans in general; 12.75% U content |
| 55 | Cas9 transcript with 5' UTR of HSD, ORF corresponding to SEQ ID NO: 54, Kozak sequence, and 3' UTR of ALB |
| 56 | Cas9 transcript with AGG as first three nucleotides for use with CleanCap^{™}, 5' UTR of HSD, ORF corresponding to SEQ ID NO: 4, Kozak sequence, and 3' UTR of ALB |
| 57 | Cas9 transcript with 5' UTR from CMV, ORF corresponding to SEQ ID NO: 4, Kozak sequence, and 3' UTR of ALB |
| 58 | Cas9 transcript with 5' UTR from HBB, ORF corresponding to SEQ ID NO: 4, Kozak sequence, and 3' UTR of HBB |
| 59 | Cas9 transcript with 5' UTR from XBG, ORF corresponding to SEQ ID NO: 4, Kozak sequence, and 3' UTR of XBG |
| 60 | Cas9 transcript with AGG as first three nucleotides for use with CleanCap^{™}, 5' UTR from XBG, ORF corresponding to SEQ ID NO: 4, Kozak sequence, and 3' UTR of XBG |
| 61 | Cas9 transcript with AGG as first three nucleotides for use with CleanCap^{™}, 5' UTR from HSD, ORF corresponding to SEQ ID NO: 4, Kozak sequence, and 3' UTR of ALB |
| 62 | 30/30/39 poly-A sequence |
| 63 | poly-A 100 sequence |
| 64 | G209 single guide RNA targeting the mouse TTR gene |
| 65 | ORF encoding Neisseria meningitidis Cas9 using minimal uridine codons as listed in Table 3, with start and stop codons |
| 66 | ORF encoding Neisseria meningitidis Cas9 using minimal uridine codons as listed in Table 3 (no start or stop codons; suitable for inclusion in fusion protein coding sequence) |
| 67 | Transcript comprising SEQ ID NO: 65 (encoding Neisseria meningitidis Cas9) |
| 68 | Amino acid sequence of Neisseria meningitidis Cas9 |
| 69 | G390 single guide RNA targeting the rat TTR gene |
| 70 | G502 single guide RNA targeting the cynomolgus monkey TTR gene |
| 71 | G509 single guide RNA targeting the cynomolgus monkey TTR gene |
| 72 | G534 single guide RNA targeting the rat TTR gene |

See the Sequence Table below for the sequences themselves. Transcript sequences generally include GGG as the first three nucleotides for use with ARCA or AGG as the first three nucleotides for use with CleanCap^{™}. Accordingly, the first three nucleotides can be modified for use with other capping approaches, such as Vaccinia capping enzyme. Promoters and poly-A sequences are not included in the transcript sequences. A promoter such as a T7 promoter (SEQ ID NO: 31) and a poly-A sequence such as SEQ ID NO: 62 or 63 can be appended to the disclosed transcript sequences at the 5' and 3' ends, respectively. Most nucleotide sequences are provided as DNA but can be readily converted to RNA by changing Ts to Us.

### Sequence Table

The following sequence table provides a listing of sequences disclosed herein. It is understood that if a DNA sequence (comprising Ts) is referenced with respect to an RNA, then Ts should be replaced with Us (which may be modified or unmodified depending on the context), and vice versa.

| **Description** | **Sequence** | **SEQ** ID No. |
|---|---|---|
| **Cas9 DNA coding sequence 2** | ATGGACAAGAAGTACAGCATCGGACTGGACATCGGAACAAAC AGCGTCGGATGGGCAGTCATCACAGACGAATACAAGGTCCCG AGCAAGAAGTTCAAGGTCCTGGGAAACACAGACAGACACAGC ATCAAGAAGAACCTGATCGGAGCACTGCTGTTCGACAGCGGA GAAACAGCAGAAGCAACAAGACTGAAGAGAACAGCAAGAAG AAGATACACAAGAAGAAAGAACAGAATCTGCTACCTGCAGGA AATCTTCAGCAACGAAATGGCAAAGGTCGACGACAGCTTCTTC CACAGACTGGAAGAAAGCTTCCTGGTCGAAGAAGACAAGAAG CACGAAAGACACCCGATCTTCGGAAACATCGTCGACGAAGTC GCATACCACGAAAAGTACCCGACAATCTACCACCTGAGAAAG AAGCTGGTCGACAGCACAGACAAGGCAGACCTGAGACTGATC TACCTGGCACTGGCACACATGATCAAGTTCAGAGGACACTTCC TGATCGAAGGAGACCTGAACCCGGACAACAGCGACGTCGACA AGCTGTTCATCCAGCTGGTCCAGACATACAACCAGCTGTTCGA AGAAAACCCGATCAACGCAAGCGGAGTCGACGCAAAGGCAAT CCTGAGCGCAAGACTGAGCAAGAGCAGAAGACTGGAAAACCT GATCGCACAGCTGCCGGGAGAAAAGAAGAACGGACTGTTCGG AAACCTGATCGCACTGAGCCTGGGACTGACACCGAACTTCAA GAGCAACTTCGACCTGGCAGAAGACGCAAAGCTGCAGCTGAG CAAGGACACATACGACGACGACCTGGACAACCTGCTGGCACA GATCGGAGACCAGTACGCAGACCTGTTCCTGGCAGCAAAGAA CCTGAGCGACGCAATCCTGCTGAGCGACATCCTGAGAGTCAAC ACAGAAATCACAAAGGCACCGCTGAGCGCAAGCATGATCAAG AGATACGACGAACACCACCAGGACCTGACACTGCTGAAGGCA CTGGTCAGACAGCAGCTGCCGGAAAAGTACAAGGAAATCTTC TTCGACCAGAGCAAGAACGGATACGCAGGATACATCGACGGA GGAGCAAGCCAGGAAGAATTCTACAAGTTCATCAAGCCGATC CTGGAAAAGATGGACGGAACAGAAGAACTGCTGGTCAAGCTG AACAGAGAAGACCTGCTGAGAAAGCAGAGAACATTCGACAAC GGAAGCATCCCGCACCAGATCCACCTGGGAGAACTGCACGCA ATCCTGAGAAGACAGGAAGACTTCTACCCGTTCCTGAAGGAC AACAGAGAAAAGATCGAAAAGATCCTGACATTCAGAATCCCG TACTACGTCGGACCGCTGGCAAGAGGAAACAGCAGATTCGCA TGGATGACAAGAAAGAGCGAAGAAACAATCACACCGTGGAAC TTCGAAGAAGTCGTCGACAAGGGAGCAAGCGCACAGAGCTTC ATCGAAAGAATGACAAACTTCGACAAGAACCTGCCGAACGAA AAGGTCCTGCCGAAGCACAGCCTGCTGTACGAATACTTCACAG TCTACAACGAACTGACAAAGGTCAAGTACGTCACAGAAGGAA TGAGAAAGCCGGCATTCCTGAGCGGAGAACAGAAGAAGGCAA TCGTCGACCTGCTGTTCAAGACAAACAGAAAGGTCACAGTCA AGCAGCTGAAGGAAGACTACTTCAAGAAGATCGAATGCTTCG ACAGCGTCGAAATCAGCGGAGTCGAAGACAGATTCAACGCAA GCCTGGGAACATACCACGACCTGCTGAAGATCATCAAGGACA AGGACTTCCTGGACAACGAAGAAAACGAAGACATCCTGGAAG ACATCGTCCTGACACTGACACTGTTCGAAGACAGAGAAATGAT CGAAGAAAGACTGAAGACATACGCACACCTGTTCGACGACAA GGTCATGAAGCAGCTGAAGAGAAGAAGATACACAGGATGGGG AAGACTGAGCAGAAAGCTGATCAACGGAATCAGAGACAAGCA | 1 |
| | GAGCGGAAAGACAATCCTGGACTTCCTGAAGAGCGACGGATT CGCAAACAGAAACTTCATGCAGCTGATCCACGACGACAGCCT GACATTCAAGGAAGACATCCAGAAGGCACAGGTCAGCGGACA GGGAGACAGCCTGCACGAACACATCGCAAACCTGGCAGGAAG CCCGGCAATCAAGAAGGGAATCCTGCAGACAGTCAAGGTCGT CGACGAACTGGTCAAGGTCATGGGAAGACACAAGCCGGAAAA CATCGTCATCGAAATGGCAAGAGAAAACCAGACAACACAGAA GGGACAGAAGAACAGCAGAGAAAGAATGAAGAGAATCGAAG AAGGAATCAAGGAACTGGGAAGCCAGATCCTGAAGGAACACC CGGTCGAAAACACACAGCTGCAGAACGAAAAGCTGTACCTGT ACTACCTGCAGAACGGAAGAGACATGTACGTCGACCAGGAAC TGGACATCAACAGACTGAGCGACTACGACGTCGACCACATCG TCCCGCAGAGCTTCCTGAAGGACGACAGCATCGACAACAAGG TCCTGACAAGAAGCGACAAGAACAGAGGAAAGAGCGACAAC GTCCCGAGCGAAGAAGTCGTCAAGAAGATGAAGAACTACTGG AGACAGCTGCTGAACGCAAAGCTGATCACACAGAGAAAGTTC GACAACCTGACAAAGGCAGAGAGAGGAGGACTGAGCGAACT GGACAAGGCAGGATTCATCAAGAGACAGCTGGTCGAAACAAG ACAGATCACAAAGCACGTCGCACAGATCCTGGACAGCAGAAT GAACACAAAGTACGACGAAAACGACAAGCTGATCAGAGAAGT CAAGGTCATCACACTGAAGAGCAAGCTGGTCAGCGACTTCAG AAAGGACTTCCAGTTCTACAAGGTCAGAGAAATCAACAACTA CCACCACGCACACGACGCATACCTGAACGCAGTCGTCGGAAC AGCACTGATCAAGAAGTACCCGAAGCTGGAAAGCGAATTCGT CTACGGAGACTACAAGGTCTACGACGTCAGAAAGATGATCGC AAAGAGCGAACAGGAAATCGGAAAGGCAACAGCAAAGTACTT CTTCTACAGCAACATCATGAACTTCTTCAAGACAGAAATCACA CTGGCAAACGGAGAAATCAGAAAGAGACCGCTGATCGAAACA AACGGAGAAACAGGAGAAATCGTCTGGGACAAGGGAAGAGA CTTCGCAACAGTCAGAAAGGTCCTGAGCATGCCGCAGGTCAA CATCGTCAAGAAGACAGAAGTCCAGACAGGAGGATTCAGCAA GGAAAGCATCCTGCCGAAGAGAAACAGCGACAAGCTGATCGC AAGAAAGAAGGACTGGGACCCGAAGAAGTACGGAGGATTCG ACAGCCCGACAGTCGCATACAGCGTCCTGGTCGTCGCAAAGGT CGAAAAGGGAAAGAGCAAGAAGCTGAAGAGCGTCAAGGAAC TGCTGGGAATCACAATCATGGAAAGAAGCAGCTTCGAAAAGA ACCCGATCGACTTCCTGGAAGCAAAGGGATACAAGGAAGTCA AGAAGGACCTGATCATCAAGCTGCCGAAGTACAGCCTGTTCG AACTGGAAAACGGAAGAAAGAGAATGCTGGCAAGCGCAGGA GAACTGCAGAAGGGAAACGAACTGGCACTGCCGAGCAAGTAC GTCAACTTCCTGTACCTGGCAAGCCACTACGAAAAGCTGAAGG GAAGCCCGGAAGACAACGAACAGAAGCAGCTGTTCGTCGAAC AGCACAAGCACTACCTGGACGAAATCATCGAACAGATCAGCG AATTCAGCAAGAGAGTCATCCTGGCAGACGCAAACCTGGACA AGGTCCTGAGCGCATACAACAAGCACAGAGACAAGCCGATCA GAGAACAGGCAGAAAACATCATCCACCTGTTCACACTGACAA ACCTGGGAGCACCGGCAGCATTCAAGTACTTCGACACAACAA TCGACAGAAAGAGATACACAAGCACAAAGGAAGTCCTGGACG CAACACTGATCCACCAGAGCATCACAGGACTGTACGAAACAA GAATCGACCTGAGCCAGCTGGGAGGAGACGGAGGAGGAAGCC CGAAGAAGAAGAGAAAGGTCTAG | |
| **Cas9 DNA coding sequence 1** | ATGGATAAGAAGTACTCAATCGGGCTGGATATCGGAACTAATT CCGTGGGTTGGGCAGTGATCACGGATGAATACAAAGTGCCGT CCAAGAAGTTCAAGGTCCTGGGGAACACCGATAGACACAGCA TCAAGAAAAATCTCATCGGAGCCCTGCTGTTTGACTCCGGCGA AACCGCAGAAGCGACCCGGCTCAAACGTACCGCGAGGCGACG CTACACCCGGCGGAAGAATCGCATCTGCTATCTGCAAGAGATC TTTTCGAACGAAATGGCAAAGGTCGACGACAGCTTCTTCCACC GCCTGGAAGAATCTTTCCTGGTGGAGGAGGACAAGAAGCATG AACGGCATCCTATCTTTGGAAACATCGTCGACGAAGTGGCGTA | 2 |
| | CCACGAAAAGTACCCGACCATCTACCATCTGCGGAAGAAGTT GGTTGACTCAACTGACAAGGCCGACCTCAGATTGATCTACTTG GCCCTCGCCCATATGATCAAATTCCGCGGACACTTCCTGATCG AAGGCGATCTGAACCCTGATAACTCCGACGTGGATAAGCTTTT CATTCAACTGGTGCAGACCTACAACCAACTGTTCGAAGAAAAC CCAATCAATGCTAGCGGCGTCGATGCCAAGGCCATCCTGTCCG CCCGGCTGTCGAAGTCGCGGCGCCTCGAAAACCTGATCGCACA GCTGCCGGGAGAGAAAAAGAACGGACTTTTCGGCAACTTGAT CGCTCTCTCACTGGGACTCACTCCCAATTTCAAGTCCAATTTTG ACCTGGCCGAGGACGCGAAGCTGCAACTCTCAAAGGACACCT ACGACGACGACTTGGACAATTTGCTGGCACAAATTGGCGATCA GTACGCGGATCTGTTCCTTGCCGCTAAGAACCTTTCGGACGCA ATCTTGCTGTCCGATATCCTGCGCGTGAACACCGAAATAACCA AAGCGCCGCTTAGCGCCTCGATGATTAAGCGGTACGACGAGC ATCACCAGGATCTCACGCTGCTCAAAGCGCTCGTGAGACAGCA ACTGCCTGAAAAGTACAAGGAGATCTTCTTCGACCAGTCCAAG AATGGGTACGCAGGGTACATCGATGGAGGCGCTAGCCAGGAA GAGTTCTATAAGTTCATCAAGCCAATCCTGGAAAAGATGGACG GAACCGAAGAACTGCTGGTCAAGCTGAACAGGGAGGATCTGC TCCGGAAACAGAGAACCTTTGACAACGGATCCATTCCCCACCA GATCCATCTGGGTGAGCTGCACGCCATCTTGCGGCGCCAGGAG GACTTTTACCCATTCCTCAAGGACAACCGGGAAAAGATCGAG AAAATTCTGACGTTCCGCATCCCGTATTACGTGGGCCCACTGG CGCGCGGCAATTCGCGCTTCGCGTGGATGACTAGAAAATCAG AGGAAACCATCACTCCTTGGAATTTCGAGGAAGTTGTGGATAA GGGAGCTTCGGCACAAAGCTTCATCGAACGAATGACCAACTTC GACAAGAATCTCCCAAACGAGAAGGTGCTTCCTAAGCACAGC CTCCTTTACGAATACTTCACTGTCTACAACGAACTGACTAAAG TGAAATACGTTACTGAAGGAATGAGGAAGCCGGCCTTTCTGTC CGGAGAACAGAAGAAAGCAATTGTCGATCTGCTGTTCAAGAC CAACCGCAAGGTGACCGTCAAGCAGCTTAAAGAGGACTACTT CAAGAAGATCGAGTGTTTCGACTCAGTGGAAATCAGCGGGGT GGAGGACAGATTCAACGCTTCGCTGGGAACCTATCATGATCTC CTGAAGATCATCAAGGACAAGGACTTCCTTGACAACGAGGAG AACGAGGACATCCTGGAAGATATCGTCCTGACCTTGACCCTTT TCGAGGATCGCGAGATGATCGAGGAGAGGCTTAAGACCTACG CTCATCTCTTCGACGATAAGGTCATGAAACAACTCAAGCGCCG CCGGTACACTGGTTGGGGCCGCCTCTCCCGCAAGCTGATCAAC GGTATTCGCGATAAACAGAGCGGTAAAACTATCCTGGATTTCC TCAAATCGGATGGCTTCGCTAATCGTAACTTCATGCAATTGAT CCACGACGACAGCCTGACCTTTAAGGAGGACATCCAAAAAGC ACAAGTGTCCGGACAGGGAGACTCACTCCATGAACACATCGC GAATCTGGCCGGTTCGCCGGCGATTAAGAAGGGAATTCTGCA AACTGTGAAGGTGGTCGACGAGCTGGTGAAGGTCATGGGACG GCACAAACCGGAGAATATCGTGATTGAAATGGCCCGAGAAAA CCAGACTACCCAGAAGGGCCAGAAAAACTCCCGCGAAAGGAT GAAGCGGATCGAAGAAGGAATCAAGGAGCTGGGCAGCCAGAT CCTGAAAGAGCACCCGGTGGAAAACACGCAGCTGCAGAACGA GAAGCTCTACCTGTACTATTTGCAAAATGGACGGGACATGTAC GTGGACCAAGAGCTGGACATCAATCGGTTGTCTGATTACGACG TGGACCACATCGTTCCACAGTCCTTTCTGAAGGATGACTCGAT CGATAACAAGGTGTTGACTCGCAGCGACAAGAACAGAGGGAA GTCAGATAATGTGCCATCGGAGGAGGTCGTGAAGAAGATGAA GAATTACTGGCGGCAGCTCCTGAATGCGAAGCTGATTACCCAG AGAAAGTTTGACAATCTCACTAAAGCCGAGCGCGGCGGACTC TCAGAGCTGGATAAGGCTGGATTCATCAAACGGCAGCTGGTC GAGACTCGGCAGATTACCAAGCACGTGGCGCAGATCTTGGAC TCCCGCATGAACACTAAATACGACGAGAACGATAAGCTCATC CGGGAAGTGAAGGTGATTACCCTGAAAAGCAAACTTGTGTCG GACTTTCGGAAGGACTTTCAGTTTTACAAAGTGAGAGAAATCA ACAACTACCATCACGCGCATGACGCATACCTCAACGCTGTGGT | |
| | CGGTACCGCCCTGATCAAAAAGTACCCTAAACTTGAATCGGAG TTTGTGTACGGAGACTACAAGGTCTACGACGTGAGGAAGATG ATAGCCAAGTCCGAACAGGAAATCGGGAAAGCAACTGCGAAA TACTTCTTTTACTCAAACATCATGAACTTTTTCAAGACTGAAAT TACGCTGGCCAATGGAGAAATCAGGAAGAGGCCACTGATCGA AACTAACGGAGAAACGGGCGAAATCGTGTGGGACAAGGGCAG GGACTTCGCAACTGTTCGCAAAGTGCTCTCTATGCCGCAAGTC AATATTGTGAAGAAAACCGAAGTGCAAACCGGCGGATTTTCA AAGGAATCGATCCTCCCAAAGAGAAATAGCGACAAGCTCATT GCACGCAAGAAAGACTGGGACCCGAAGAAGTACGGAGGATTC GATTCGCCGACTGTCGCATACTCCGTCCTCGTGGTGGCCAAGG TGGAGAAGGGAAAGAGCAAAAAGCTCAAATCCGTCAAAGAGC TGCTGGGGATTACCATCATGGAACGATCCTCGTTCGAGAAGAA CCCGATTGATTTCCTCGAGGCGAAGGGTTACAAGGAGGTGAA GAAGGATCTGATCATCAAACTCCCCAAGTACTCACTGTTCGAA CTGGAAAATGGTCGGAAGCGCATGCTGGCTTCGGCCGGAGAA CTCCAAAAAGGAAATGAGCTGGCCTTGCCTAGCAAGTACGTC AACTTCCTCTATCTTGCTTCGCACTACGAAAAACTCAAAGGGT CACCGGAAGATAACGAACAGAAGCAGCTTTTCGTGGAGCAGC ACAAGCATTATCTGGATGAAATCATCGAACAAATCTCCGAGTT TTCAAAGCGCGTGATCCTCGCCGACGCCAACCTCGACAAAGTC CTGTCGGCCTACAATAAGCATAGAGATAAGCCGATCAGAGAA CAGGCCGAGAACATTATCCACTTGTTCACCCTGACTAACCTGG GAGCCCCAGCCGCCTTCAAGTACTTCGATACTACTATCGATCG CAAAAGATACACGTCCACCAAGGAAGTTCTGGACGCGACCCT GATCCACCAAAGCATCACTGGACTCTACGAAACTAGGATCGAT CTGTCGCAGCTGGGTGGCGATGGCGGTGGATCTCCGAAAAAG AAGAGAAAGGTGTAATGA | |
| **Cas9 amino acid sequence** | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDHIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGDGGSPKKKRKV | 3 |
| | | |
| **Cas9 mRNA open reading frame (ORF) 2** | AUGGACAAGAAGUACAGCAUCGGACUGGACAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA | 4 |
| | GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACGGAGGAGGAAGCCCGAAGAAGAAGAGAAAGGUCUAG | |
| **Cas9 mRNA ORF 1** | AUGGAUAAGAAGUACUCAAUCGGGCUGGAUAUCGGAACUAA UUCCGUGGGUUGGGCAGUGAUCACGGAUGAAUACAAAGUGC CGUCCAAGAAGUUCAAGGUCCUGGGGAACACCGAUAGACAC AGCAUCAAGAAAAAUCUCAUCGGAGCCCUGCUGUUUGACUC CGGCGAAACCGCAGAAGCGACCCGGCUCAAACGUACCGCGAG GCGACGCUACACCCGGCGGAAGAAUCGCAUCUGCUAUCUGC AAGAGAUCUUUUCGAACGAAAUGGCAAAGGUCGACGACAGC UUCUUCCACCGCCUGGAAGAAUCUUUCCUGGUGGAGGAGGA CAAGAAGCAUGAACGGCAUCCUAUCUUUGGAAACAUCGUCG ACGAAGUGGCGUACCACGAAAAGUACCCGACCAUCUACCAU CUGCGGAAGAAGUUGGUUGACUCAACUGACAAGGCCGACCU CAGAUUGAUCUACUUGGCCCUCGCCCAUAUGAUCAAAUUCC GCGGACACUUCCUGAUCGAAGGCGAUCUGAACCCUGAUAAC UCCGACGUGGAUAAGCUUUUCAUUCAACUGGUGCAGACCUA CAACCAACUGUUCGAAGAAAACCCAAUCAAUGCUAGCGGCG UCGAUGCCAAGGCCAUCCUGUCCGCCCGGCUGUCGAAGUCGC GGCGCCUCGAAAACCUGAUCGCACAGCUGCCGGGAGAGAAA | 5 |
| | AAGAACGGACUUUUCGGCAACUUGAUCGCUCUCUCACUGGG ACUCACUCCCAAUUUCAAGUCCAAUUUUGACCUGGCCGAGG ACGCGAAGCUGCAACUCUCAAAGGACACCUACGACGACGAC UUGGACAAUUUGCUGGCACAAAUUGGCGAUCAGUACGCGGA UCUGUUCCUUGCCGCUAAGAACCUUUCGGACGCAAUCUUGC UGUCCGAUAUCCUGCGCGUGAACACCGAAAUAACCAAAGCG CCGCUUAGCGCCUCGAUGAUUAAGCGGUACGACGAGCAUCA CCAGGAUCUCACGCUGCUCAAAGCGCUCGUGAGACAGCAAC UGCCUGAAAAGUACAAGGAGAUCUUCUUCGACCAGUCCAAG AAUGGGUACGCAGGGUACAUCGAUGGAGGCGCUAGCCAGGA AGAGUUCUAUAAGUUCAUCAAGCCAAUCCUGGAAAAGAUGG ACGGAACCGAAGAACUGCUGGUCAAGCUGAACAGGGAGGAU CUGCUCCGGAAACAGAGAACCUUUGACAACGGAUCCAUUCC CCACCAGAUCCAUCUGGGUGAGCUGCACGCCAUCUUGCGGCG CCAGGAGGACUUUUACCCAUUCCUCAAGGACAACCGGGAAA AGAUCGAGAAAAUUCUGACGUUCCGCAUCCCGUAUUACGUG GGCCCACUGGCGCGCGGCAAUUCGCGCUUCGCGUGGAUGAC UAGAAAAUCAGAGGAAACCAUCACUCCUUGGAAUUUCGAGG AAGUUGUGGAUAAGGGAGCUUCGGCACAAAGCUUCAUCGAA CGAAUGACCAACUUCGACAAGAAUCUCCCAAACGAGAAGGU GCUUCCUAAGCACAGCCUCCUUUACGAAUACUUCACUGUCU ACAACGAACUGACUAAAGUGAAAUACGUUACUGAAGGAAUG AGGAAGCCGGCCUUUCUGUCCGGAGAACAGAAGAAAGCAAU UGUCGAUCUGCUGUUCAAGACCAACCGCAAGGUGACCGUCA AGCAGCUUAAAGAGGACUACUUCAAGAAGAUCGAGUGUUUC GACUCAGUGGAAAUCAGCGGGGUGGAGGACAGAUUCAACGC UUCGCUGGGAACCUAUCAUGAUCUCCUGAAGAUCAUCAAGG ACAAGGACUUCCUUGACAACGAGGAGAACGAGGACAUCCUG GAAGAUAUCGUCCUGACCUUGACCCUUUUCGAGGAUCGCGA GAUGAUCGAGGAGAGGCUUAAGACCUACGCUCAUCUCUUCG ACGAUAAGGUCAUGAAACAACUCAAGCGCCGCCGGUACACU GGUUGGGGCCGCCUCUCCCGCAAGCUGAUCAACGGUAUUCG CGAUAAACAGAGCGGUAAAACUAUCCUGGAUUUCCUCAAAU CGGAUGGCUUCGCUAAUCGUAACUUCAUGCAAUUGAUCCAC GACGACAGCCUGACCUUUAAGGAGGACAUCCAAAAAGCACA AGUGUCCGGACAGGGAGACUCACUCCAUGAACACAUCGCGA AUCUGGCCGGUUCGCCGGCGAUUAAGAAGGGAAUUCUGCAA ACUGUGAAGGUGGUCGACGAGCUGGUGAAGGUCAUGGGACG GCACAAACCGGAGAAUAUCGUGAUUGAAAUGGCCCGAGAAA ACCAGACUACCCAGAAGGGCCAGAAAAACUCCCGCGAAAGG AUGAAGCGGAUCGAAGAAGGAAUCAAGGAGCUGGGCAGCCA GAUCCUGAAAGAGCACCCGGUGGAAAACACGCAGCUGCAGA ACGAGAAGCUCUACCUGUACUAUUUGCAAAAUGGACGGGAC AUGUACGUGGACCAAGAGCUGGACAUCAAUCGGUUGUCUGA UUACGACGUGGACCACAUCGUUCCACAGUCCUUUCUGAAGG AUGACUCGAUCGAUAACAAGGUGUUGACUCGCAGCGACAAG AACAGAGGGAAGUCAGAUAAUGUGCCAUCGGAGGAGGUCGU GAAGAAGAUGAAGAAUUACUGGCGGCAGCUCCUGAAUGCGA AGCUGAUUACCCAGAGAAAGUUUGACAAUCUCACUAAAGCC GAGCGCGGCGGACUCUCAGAGCUGGAUAAGGCUGGAUUCAU CAAACGGCAGCUGGUCGAGACUCGGCAGAUUACCAAGCACG UGGCGCAGAUCUUGGACUCCCGCAUGAACACUAAAUACGAC GAGAACGAUAAGCUCAUCCGGGAAGUGAAGGUGAUUACCCU GAAAAGCAAACUUGUGUCGGACUUUCGGAAGGACUUUCAGU UUUACAAAGUGAGAGAAAUCAACAACUACCAUCACGCGCAU GACGCAUACCUCAACGCUGUGGUCGGUACCGCCCUGAUCAA AAAGUACCCUAAACUUGAAUCGGAGUUUGUGUACGGAGACU ACAAGGUCUACGACGUGAGGAAGAUGAUAGCCAAGUCCGAA CAGGAAAUCGGGAAAGCAACUGCGAAAUACUUCUUUUACUC AAACAUCAUGAACUUUUUCAAGACUGAAAUUACGCUGGCCA AUGGAGAAAUCAGGAAGAGGCCACUGAUCGAAACUAACGGA | |
| | GAAACGGGCGAAAUCGUGUGGGACAAGGGCAGGGACUUCGC AACUGUUCGCAAAGUGCUCUCUAUGCCGCAAGUCAAUAUUG UGAAGAAAACCGAAGUGCAAACCGGCGGAUUUUCAAAGGAA UCGAUCCUCCCAAAGAGAAAUAGCGACAAGCUCAUUGCACG CAAGAAAGACUGGGACCCGAAGAAGUACGGAGGAUUCGAUU CGCCGACUGUCGCAUACUCCGUCCUCGUGGUGGCCAAGGUG GAGAAGGGAAAGAGCAAAAAGCUCAAAUCCGUCAAAGAGCU GCUGGGGAUUACCAUCAUGGAACGAUCCUCGUUCGAGAAGA ACCCGAUUGAUUUCCUCGAGGCGAAGGGUUACAAGGAGGUG AAGAAGGAUCUGAUCAUCAAACUCCCCAAGUACUCACUGUU CGAACUGGAAAAUGGUCGGAAGCGCAUGCUGGCUUCGGCCG GAGAACUCCAAAAAGGAAAUGAGCUGGCCUUGCCUAGCAAG UACGUCAACUUCCUCUAUCUUGCUUCGCACUACGAAAAACU CAAAGGGUCACCGGAAGAUAACGAACAGAAGCAGCUUUUCG UGGAGCAGCACAAGCAUUAUCUGGAUGAAAUCAUCGAACAA AUCUCCGAGUUUUCAAAGCGCGUGAUCCUCGCCGACGCCAAC CUCGACAAAGUCCUGUCGGCCUACAAUAAGCAUAGAGAUAA GCCGAUCAGAGAACAGGCCGAGAACAUUAUCCACUUGUUCA CCCUGACUAACCUGGGAGCCCCAGCCGCCUUCAAGUACUUCG AUACUACUAUCGAUCGCAAAAGAUACACGUCCACCAAGGAA GUUCUGGACGCGACCCUGAUCCACCAAAGCAUCACUGGACUC UACGAAACUAGGAUCGAUCUGUCGCAGCUGGGUGGCGAUGG CGGUGGAUCUCCGAAAAAGAAGAGAAAGGUGUAAUGA | |
| **Cas9 nickase (D10A) amino acid sequence** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDHIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGDGGGSPKKKRKV | 6 |
| **Cas9 nickase (D10A) mRNA ORF** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC | 7 |
| | AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG | |
| | UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACGGAGGAGGAAGCCCGAAGAAGAAGAGAAAGGUCUAG | |
| **dCas9 (D10A H840A) amino acid sequence** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDAIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK | 8 |
| | ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGDGGGSPKKKRKV | |
| **dCas9 (D10A H840A) mRNA ORF** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC | 9 |
| | ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACGCAAUCGUCCCGCAGAGCUUCCUGAA GGACGACAGCAUCGACAACAAGGUCCUGACAAGAAGCGACA AGAACAGAGGAAAGAGCGACAACGUCCCGAGCGAAGAAGUC GUCAAGAAGAUGAAGAACUACUGGAGACAGCUGCUGAACGC AAAGCUGAUCACACAGAGAAAGUUCGACAACCUGACAAAGG CAGAGAGAGGAGGACUGAGCGAACUGGACAAGGCAGGAUUC AUCAAGAGACAGCUGGUCGAAACAAGACAGAUCACAAAGCA CGUCGCACAGAUCCUGGACAGCAGAAUGAACACAAAGUACG ACGAAAACGACAAGCUGAUCAGAGAAGUCAAGGUCAUCACA CUGAAGAGCAAGCUGGUCAGCGACUUCAGAAAGGACUUCCA GUUCUACAAGGUCAGAGAAAUCAACAACUACCACCACGCAC ACGACGCAUACCUGAACGCAGUCGUCGGAACAGCACUGAUC AAGAAGUACCCGAAGCUGGAAAGCGAAUUCGUCUACGGAGA CUACAAGGUCUACGACGUCAGAAAGAUGAUCGCAAAGAGCG AACAGGAAAUCGGAAAGGCAACAGCAAAGUACUUCUUCUAC AGCAACAUCAUGAACUUCUUCAAGACAGAAAUCACACUGGC AAACGGAGAAAUCAGAAAGAGACCGCUGAUCGAAACAAACG GAGAAACAGGAGAAAUCGUCUGGGACAAGGGAAGAGACUUC GCAACAGUCAGAAAGGUCCUGAGCAUGCCGCAGGUCAACAU CGUCAAGAAGACAGAAGUCCAGACAGGAGGAUUCAGCAAGG AAAGCAUCCUGCCGAAGAGAAACAGCGACAAGCUGAUCGCA AGAAAGAAGGACUGGGACCCGAAGAAGUACGGAGGAUUCGA CAGCCCGACAGUCGCAUACAGCGUCCUGGUCGUCGCAAAGG UCGAAAAGGGAAAGAGCAAGAAGCUGAAGAGCGUCAAGGAA CUGCUGGGAAUCACAAUCAUGGAAAGAAGCAGCUUCGAAAA GAACCCGAUCGACUUCCUGGAAGCAAAGGGAUACAAGGAAG UCAAGAAGGACCUGAUCAUCAAGCUGCCGAAGUACAGCCUG UUCGAACUGGAAAACGGAAGAAAGAGAAUGCUGGCAAGCGC AGGAGAACUGCAGAAGGGAAACGAACUGGCACUGCCGAGCA AGUACGUCAACUUCCUGUACCUGGCAAGCCACUACGAAAAG CUGAAGGGAAGCCCGGAAGACAACGAACAGAAGCAGCUGUU CGUCGAACAGCACAAGCACUACCUGGACGAAAUCAUCGAAC AGAUCAGCGAAUUCAGCAAGAGAGUCAUCCUGGCAGACGCA AACCUGGACAAGGUCCUGAGCGCAUACAACAAGCACAGAGA CAAGCCGAUCAGAGAACAGGCAGAAAACAUCAUCCACCUGU UCACACUGACAAACCUGGGAGCACCGGCAGCAUUCAAGUAC UUCGACACAACAAUCGACAGAAAGAGAUACACAAGCACAAA GGAAGUCCUGGACGCAACACUGAUCCACCAGAGCAUCACAG GACUGUACGAAACAAGAAUCGACCUGAGCCAGCUGGGAGGA GACGGAGGAGGAAGCCCGAAGAAGAAGAGAAAGGUCUAG | |
| **Cas9 bare coding sequence** | GACAAGAAGUACAGCAUCGGACUGGACAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU | 10 |
| | GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU | |
| | CUACAAGGUCAGAGAAAUCAACAACUACCACCACGCACACG ACGCAUACCUGAACGCAGUCGUCGGAACAGCACUGAUCAAG AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC GGAGGAGGAAGCCCGAAGAAGAAGAGAAAGGUC | |
| **Cas9 nickase bare coding sequence** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC | **11** |
| | UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU CUACAAGGUCAGAGAAAUCAACAACUACCACCACGCACACG ACGCAUACCUGAACGCAGUCGUCGGAACAGCACUGAUCAAG AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU | |
| | ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC GGAGGAGGAAGCCCGAAGAAGAAGAGAAAGGUC | |
| **dCas9 bare coding sequence** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG | **12** |
| | AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACGCAAUCGUCCCGCAGAGCUUCCUGAAGG ACGACAGCAUCGACAACAAGGUCCUGACAAGAAGCGACAAG AACAGAGGAAAGAGCGACAACGUCCCGAGCGAAGAAGUCGU CAAGAAGAUGAAGAACUACUGGAGACAGCUGCUGAACGCAA AGCUGAUCACACAGAGAAAGUUCGACAACCUGACAAAGGCA GAGAGAGGAGGACUGAGCGAACUGGACAAGGCAGGAUUCAU CAAGAGACAGCUGGUCGAAACAAGACAGAUCACAAAGCACG UCGCACAGAUCCUGGACAGCAGAAUGAACACAAAGUACGAC GAAAACGACAAGCUGAUCAGAGAAGUCAAGGUCAUCACACU GAAGAGCAAGCUGGUCAGCGACUUCAGAAAGGACUUCCAGU UCUACAAGGUCAGAGAAAUCAACAACUACCACCACGCACAC GACGCAUACCUGAACGCAGUCGUCGGAACAGCACUGAUCAA GAAGUACCCGAAGCUGGAAAGCGAAUUCGUCUACGGAGACU ACAAGGUCUACGACGUCAGAAAGAUGAUCGCAAAGAGCGAA CAGGAAAUCGGAAAGGCAACAGCAAAGUACUUCUUCUACAG CAACAUCAUGAACUUCUUCAAGACAGAAAUCACACUGGCAA ACGGAGAAAUCAGAAAGAGACCGCUGAUCGAAACAAACGGA GAAACAGGAGAAAUCGUCUGGGACAAGGGAAGAGACUUCGC AACAGUCAGAAAGGUCCUGAGCAUGCCGCAGGUCAACAUCG UCAAGAAGACAGAAGUCCAGACAGGAGGAUUCAGCAAGGAA AGCAUCCUGCCGAAGAGAAACAGCGACAAGCUGAUCGCAAG AAAGAAGGACUGGGACCCGAAGAAGUACGGAGGAUUCGACA GCCCGACAGUCGCAUACAGCGUCCUGGUCGUCGCAAAGGUC GAAAAGGGAAAGAGCAAGAAGCUGAAGAGCGUCAAGGAACU GCUGGGAAUCACAAUCAUGGAAAGAAGCAGCUUCGAAAAGA ACCCGAUCGACUUCCUGGAAGCAAAGGGAUACAAGGAAGUC AAGAAGGACCUGAUCAUCAAGCUGCCGAAGUACAGCCUGUU CGAACUGGAAAACGGAAGAAAGAGAAUGCUGGCAAGCGCAG GAGAACUGCAGAAGGGAAACGAACUGGCACUGCCGAGCAAG UACGUCAACUUCCUGUACCUGGCAAGCCACUACGAAAAGCU GAAGGGAAGCCCGGAAGACAACGAACAGAAGCAGCUGUUCG UCGAACAGCACAAGCACUACCUGGACGAAAUCAUCGAACAG AUCAGCGAAUUCAGCAAGAGAGUCAUCCUGGCAGACGCAAA CCUGGACAAGGUCCUGAGCGCAUACAACAAGCACAGAGACA AGCCGAUCAGAGAACAGGCAGAAAACAUCAUCCACCUGUUC ACACUGACAAACCUGGGAGCACCGGCAGCAUUCAAGUACUU CGACACAACAAUCGACAGAAAGAGAUACACAAGCACAAAGG AAGUCCUGGACGCAACACUGAUCCACCAGAGCAUCACAGGA CUGUACGAAACAAGAAUCGACCUGAGCCAGCUGGGAGGAGA CGGAGGAGGAAGCCCGAAGAAGAAGAGAAAGGUC | |
| **Amino acid sequence of Cas9 (without NLS)** | | **13** |
| | | |
| **Cas9 mRNA ORF encoding SEQ ID NO: 13 using minimal uridine codons as listed in Table 3, with start and stop codons** | AUGGACAAGAAGUACAGCAUCGGACUGGACAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG | **14** |
| | AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU | |
| | UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACUAG | |
| **Cas9 coding sequence encoding SEQ ID NO: 13 using minimal uridine codons as listed in Table 3 (no start or stop codons; suitable for inclusion in fusion protein coding sequence)** | GACAAGAAGUACAGCAUCGGACUGGACAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG | **15** |
| | ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU CUACAAGGUCAGAGAAAUCAACAACUACCACCACGCACACG ACGCAUACCUGAACGCAGUCGUCGGAACAGCACUGAUCAAG AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC | |
| **Amino acid sequence of Cas9 nickase (without NLS)** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI | **16** |
| | EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDHIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGD | |
| **Cas9 nickase mRNA ORF encoding SEQ ID NO: 16 using minimal uridine codons as listed in Table 3, with start and stop codons** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU | **17** |
| | UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACUAG | |
| **Cas9 nickase coding sequence encoding SEQ ID NO: 16 using minimal uridine codons as listed in Table 3 (no start or stop codons; suitable for inclusion in fusion protein coding sequence)** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC | **18** |
| | AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU CUACAAGGUCAGAGAAAUCAACAACUACCACCACGCACACG ACGCAUACCUGAACGCAGUCGUCGGAACAGCACUGAUCAAG AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC | |
| **Amino acid sequence of dCas9 (without NLS)** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDAIVPQSFLKDDSIDNK VLTRSDKNRGKSDNVPSEEVVKKMKNYWRQLLNAKLITQRKFD | **19** |
| | NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGD | |
| **dCas9 mRNA ORF encoding SEQ ID NO: 19 using minimal uridine codons as listed in Table 3, with start and stop codons** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA | **20** |
| | CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACGCAAUCGUCCCGCAGAGCUUCCUGAA GGACGACAGCAUCGACAACAAGGUCCUGACAAGAAGCGACA AGAACAGAGGAAAGAGCGACAACGUCCCGAGCGAAGAAGUC GUCAAGAAGAUGAAGAACUACUGGAGACAGCUGCUGAACGC AAAGCUGAUCACACAGAGAAAGUUCGACAACCUGACAAAGG CAGAGAGAGGAGGACUGAGCGAACUGGACAAGGCAGGAUUC AUCAAGAGACAGCUGGUCGAAACAAGACAGAUCACAAAGCA CGUCGCACAGAUCCUGGACAGCAGAAUGAACACAAAGUACG ACGAAAACGACAAGCUGAUCAGAGAAGUCAAGGUCAUCACA CUGAAGAGCAAGCUGGUCAGCGACUUCAGAAAGGACUUCCA GUUCUACAAGGUCAGAGAAAUCAACAACUACCACCACGCAC ACGACGCAUACCUGAACGCAGUCGUCGGAACAGCACUGAUC AAGAAGUACCCGAAGCUGGAAAGCGAAUUCGUCUACGGAGA CUACAAGGUCUACGACGUCAGAAAGAUGAUCGCAAAGAGCG AACAGGAAAUCGGAAAGGCAACAGCAAAGUACUUCUUCUAC AGCAACAUCAUGAACUUCUUCAAGACAGAAAUCACACUGGC AAACGGAGAAAUCAGAAAGAGACCGCUGAUCGAAACAAACG GAGAAACAGGAGAAAUCGUCUGGGACAAGGGAAGAGACUUC GCAACAGUCAGAAAGGUCCUGAGCAUGCCGCAGGUCAACAU CGUCAAGAAGACAGAAGUCCAGACAGGAGGAUUCAGCAAGG AAAGCAUCCUGCCGAAGAGAAACAGCGACAAGCUGAUCGCA AGAAAGAAGGACUGGGACCCGAAGAAGUACGGAGGAUUCGA CAGCCCGACAGUCGCAUACAGCGUCCUGGUCGUCGCAAAGG UCGAAAAGGGAAAGAGCAAGAAGCUGAAGAGCGUCAAGGAA CUGCUGGGAAUCACAAUCAUGGAAAGAAGCAGCUUCGAAAA GAACCCGAUCGACUUCCUGGAAGCAAAGGGAUACAAGGAAG UCAAGAAGGACCUGAUCAUCAAGCUGCCGAAGUACAGCCUG UUCGAACUGGAAAACGGAAGAAAGAGAAUGCUGGCAAGCGC AGGAGAACUGCAGAAGGGAAACGAACUGGCACUGCCGAGCA AGUACGUCAACUUCCUGUACCUGGCAAGCCACUACGAAAAG CUGAAGGGAAGCCCGGAAGACAACGAACAGAAGCAGCUGUU CGUCGAACAGCACAAGCACUACCUGGACGAAAUCAUCGAAC AGAUCAGCGAAUUCAGCAAGAGAGUCAUCCUGGCAGACGCA AACCUGGACAAGGUCCUGAGCGCAUACAACAAGCACAGAGA CAAGCCGAUCAGAGAACAGGCAGAAAACAUCAUCCACCUGU UCACACUGACAAACCUGGGAGCACCGGCAGCAUUCAAGUAC UUCGACACAACAAUCGACAGAAAGAGAUACACAAGCACAAA GGAAGUCCUGGACGCAACACUGAUCCACCAGAGCAUCACAG GACUGUACGAAACAAGAAUCGACCUGAGCCAGCUGGGAGGA GACUAG | |
| **dCas9 coding sequence encoding SEQ ID NO: 19 using minimal** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU | **21** |
| **uridine codons as listed in Table 3 (no start or stop codons; suitable for inclusion in fusion protein coding sequence)** | CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACGCAAUCGUCCCGCAGAGCUUCCUGAAGG ACGACAGCAUCGACAACAAGGUCCUGACAAGAAGCGACAAG AACAGAGGAAAGAGCGACAACGUCCCGAGCGAAGAAGUCGU CAAGAAGAUGAAGAACUACUGGAGACAGCUGCUGAACGCAA AGCUGAUCACACAGAGAAAGUUCGACAACCUGACAAAGGCA GAGAGAGGAGGACUGAGCGAACUGGACAAGGCAGGAUUCAU CAAGAGACAGCUGGUCGAAACAAGACAGAUCACAAAGCACG | |
| | UCGCACAGAUCCUGGACAGCAGAAUGAACACAAAGUACGAC GAAAACGACAAGCUGAUCAGAGAAGUCAAGGUCAUCACACU GAAGAGCAAGCUGGUCAGCGACUUCAGAAAGGACUUCCAGU UCUACAAGGUCAGAGAAAUCAACAACUACCACCACGCACAC GACGCAUACCUGAACGCAGUCGUCGGAACAGCACUGAUCAA GAAGUACCCGAAGCUGGAAAGCGAAUUCGUCUACGGAGACU ACAAGGUCUACGACGUCAGAAAGAUGAUCGCAAAGAGCGAA CAGGAAAUCGGAAAGGCAACAGCAAAGUACUUCUUCUACAG CAACAUCAUGAACUUCUUCAAGACAGAAAUCACACUGGCAA ACGGAGAAAUCAGAAAGAGACCGCUGAUCGAAACAAACGGA GAAACAGGAGAAAUCGUCUGGGACAAGGGAAGAGACUUCGC AACAGUCAGAAAGGUCCUGAGCAUGCCGCAGGUCAACAUCG UCAAGAAGACAGAAGUCCAGACAGGAGGAUUCAGCAAGGAA AGCAUCCUGCCGAAGAGAAACAGCGACAAGCUGAUCGCAAG AAAGAAGGACUGGGACCCGAAGAAGUACGGAGGAUUCGACA GCCCGACAGUCGCAUACAGCGUCCUGGUCGUCGCAAAGGUC GAAAAGGGAAAGAGCAAGAAGCUGAAGAGCGUCAAGGAACU GCUGGGAAUCACAAUCAUGGAAAGAAGCAGCUUCGAAAAGA ACCCGAUCGACUUCCUGGAAGCAAAGGGAUACAAGGAAGUC AAGAAGGACCUGAUCAUCAAGCUGCCGAAGUACAGCCUGUU CGAACUGGAAAACGGAAGAAAGAGAAUGCUGGCAAGCGCAG GAGAACUGCAGAAGGGAAACGAACUGGCACUGCCGAGCAAG UACGUCAACUUCCUGUACCUGGCAAGCCACUACGAAAAGCU GAAGGGAAGCCCGGAAGACAACGAACAGAAGCAGCUGUUCG UCGAACAGCACAAGCACUACCUGGACGAAAUCAUCGAACAG AUCAGCGAAUUCAGCAAGAGAGUCAUCCUGGCAGACGCAAA CCUGGACAAGGUCCUGAGCGCAUACAACAAGCACAGAGACA AGCCGAUCAGAGAACAGGCAGAAAACAUCAUCCACCUGUUC ACACUGACAAACCUGGGAGCACCGGCAGCAUUCAAGUACUU CGACACAACAAUCGACAGAAAGAGAUACACAAGCACAAAGG AAGUCCUGGACGCAACACUGAUCCACCAGAGCAUCACAGGA CUGUACGAAACAAGAAUCGACCUGAGCCAGCUGGGAGGAGA CGGAGGAGGAAGC | |
| **Amino acid sequence of Cas9 with two nuclear localization signals as the C-terminal amino acids** | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDHIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR | **22** |
| | MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGD GSGSPKKKRKVDGSPKKKRKVDSG | |
| **Cas9 mRNA ORF encoding SEQ ID NO: 22 using minimal uridine codons as listed in Table 3, with start and stop codons** | AUGGACAAGAAGUACAGCAUCGGACUGGACAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA | **23** |
| | AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACGGAAGCGGAAGCCCGAAGAAGAAGAGAAAGGUCGACGGA AGCCCGAAGAAGAAGAGAAAGGUCGACAGCGGAUAG | |
| **Cas9 coding sequence encoding SEQ ID NO: 23 using minimal uridine codons as listed in Table 3 (no start or stop codons;** | GACAAGAAGUACAGCAUCGGACUGGACAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA | **24** |
| **suitable for inclusion in fusion protein coding sequence)** | GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU CUACAAGGUCAGAGAAAUCAACAACUACCACCACGCACACG ACGCAUACCUGAACGCAGUCGUCGGAACAGCACUGAUCAAG | |
| | AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC GGAAGCGGAAGCCCGAAGAAGAAGAGAAAGGUCGACGGAAG CCCGAAGAAGAAGAGAAAGGUCGACAGCGGA | |
| **Amino acid sequence of Cas9 nickase with two nuclear localization signals as the C-terminal amino acids** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDHIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI | **25** |
| | REQAENIIHLFTLTNLGAPAAFKYFDTTIDRKRYTSTKEVLDATLI HQSITGLYETRIDLSQLGGDGSGSPKKKRKVDGSPKKKRKVDSG | |
| **Cas9 nickase mRNA ORF encoding SEQ ID NO: 25 using minimal uridine codons as listed in Table 3, with start and stop codons** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA | **26** |
| | GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG ACAUGUACGUCGACCAGGAACUGGACAUCAACAGACUGAGC GACUACGACGUCGACCACAUCGUCCCGCAGAGCUUCCUGAAG GACGACAGCAUCGACAACAAGGUCCUGACAAGAAGCGACAA GAACAGAGGAAAGAGCGACAACGUCCCGAGCGAAGAAGUCG UCAAGAAGAUGAAGAACUACUGGAGACAGCUGCUGAACGCA AAGCUGAUCACACAGAGAAAGUUCGACAACCUGACAAAGGC AGAGAGAGGAGGACUGAGCGAACUGGACAAGGCAGGAUUCA UCAAGAGACAGCUGGUCGAAACAAGACAGAUCACAAAGCAC GUCGCACAGAUCCUGGACAGCAGAAUGAACACAAAGUACGA CGAAAACGACAAGCUGAUCAGAGAAGUCAAGGUCAUCACAC UGAAGAGCAAGCUGGUCAGCGACUUCAGAAAGGACUUCCAG UUCUACAAGGUCAGAGAAAUCAACAACUACCACCACGCACA CGACGCAUACCUGAACGCAGUCGUCGGAACAGCACUGAUCA AGAAGUACCCGAAGCUGGAAAGCGAAUUCGUCUACGGAGAC UACAAGGUCUACGACGUCAGAAAGAUGAUCGCAAAGAGCGA ACAGGAAAUCGGAAAGGCAACAGCAAAGUACUUCUUCUACA GCAACAUCAUGAACUUCUUCAAGACAGAAAUCACACUGGCA AACGGAGAAAUCAGAAAGAGACCGCUGAUCGAAACAAACGG AGAAACAGGAGAAAUCGUCUGGGACAAGGGAAGAGACUUCG CAACAGUCAGAAAGGUCCUGAGCAUGCCGCAGGUCAACAUC GUCAAGAAGACAGAAGUCCAGACAGGAGGAUUCAGCAAGGA AAGCAUCCUGCCGAAGAGAAACAGCGACAAGCUGAUCGCAA GAAAGAAGGACUGGGACCCGAAGAAGUACGGAGGAUUCGAC AGCCCGACAGUCGCAUACAGCGUCCUGGUCGUCGCAAAGGU CGAAAAGGGAAAGAGCAAGAAGCUGAAGAGCGUCAAGGAAC UGCUGGGAAUCACAAUCAUGGAAAGAAGCAGCUUCGAAAAG AACCCGAUCGACUUCCUGGAAGCAAAGGGAUACAAGGAAGU CAAGAAGGACCUGAUCAUCAAGCUGCCGAAGUACAGCCUGU UCGAACUGGAAAACGGAAGAAAGAGAAUGCUGGCAAGCGCA GGAGAACUGCAGAAGGGAAACGAACUGGCACUGCCGAGCAA GUACGUCAACUUCCUGUACCUGGCAAGCCACUACGAAAAGC UGAAGGGAAGCCCGGAAGACAACGAACAGAAGCAGCUGUUC GUCGAACAGCACAAGCACUACCUGGACGAAAUCAUCGAACA GAUCAGCGAAUUCAGCAAGAGAGUCAUCCUGGCAGACGCAA ACCUGGACAAGGUCCUGAGCGCAUACAACAAGCACAGAGAC AAGCCGAUCAGAGAACAGGCAGAAAACAUCAUCCACCUGUU CACACUGACAAACCUGGGAGCACCGGCAGCAUUCAAGUACU UCGACACAACAAUCGACAGAAAGAGAUACACAAGCACAAAG GAAGUCCUGGACGCAACACUGAUCCACCAGAGCAUCACAGG ACUGUACGAAACAAGAAUCGACCUGAGCCAGCUGGGAGGAG ACGGAAGCGGAAGCCCGAAGAAGAAGAGAAAGGUCGACGGA AGCCCGAAGAAGAAGAGAAAGGUCGACAGCGGAUAG | |
| **Cas9 nickase coding sequence encoding SEQ ID NO: 25 using minimal uridine codons as listed in Table 3 (no start or stop codons; suitable for inclusion in** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC | **27** |
| **fusion protein coding sequence)** | GACGCAAAGGCAAUCCUGAGCGCAAGACUGAGCAAGAGCAG AAGACUGGAAAACCUGAUCGCACAGCUGCCGGGAGAAAAGA AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACCACAUCGUCCCGCAGAGCUUCCUGAAGGA CGACAGCAUCGACAACAAGGUCCUGACAAGAAGCGACAAGA ACAGAGGAAAGAGCGACAACGUCCCGAGCGAAGAAGUCGUC AAGAAGAUGAAGAACUACUGGAGACAGCUGCUGAACGCAAA GCUGAUCACACAGAGAAAGUUCGACAACCUGACAAAGGCAG AGAGAGGAGGACUGAGCGAACUGGACAAGGCAGGAUUCAUC AAGAGACAGCUGGUCGAAACAAGACAGAUCACAAAGCACGU CGCACAGAUCCUGGACAGCAGAAUGAACACAAAGUACGACG AAAACGACAAGCUGAUCAGAGAAGUCAAGGUCAUCACACUG AAGAGCAAGCUGGUCAGCGACUUCAGAAAGGACUUCCAGUU CUACAAGGUCAGAGAAAUCAACAACUACCACCACGCACACG ACGCAUACCUGAACGCAGUCGUCGGAACAGCACUGAUCAAG AAGUACCCGAAGCUGGAAAGCGAAUUCGUCUACGGAGACUA CAAGGUCUACGACGUCAGAAAGAUGAUCGCAAAGAGCGAAC AGGAAAUCGGAAAGGCAACAGCAAAGUACUUCUUCUACAGC | |
| | AACAUCAUGAACUUCUUCAAGACAGAAAUCACACUGGCAAA CGGAGAAAUCAGAAAGAGACCGCUGAUCGAAACAAACGGAG AAACAGGAGAAAUCGUCUGGGACAAGGGAAGAGACUUCGCA ACAGUCAGAAAGGUCCUGAGCAUGCCGCAGGUCAACAUCGU CAAGAAGACAGAAGUCCAGACAGGAGGAUUCAGCAAGGAAA GCAUCCUGCCGAAGAGAAACAGCGACAAGCUGAUCGCAAGA AAGAAGGACUGGGACCCGAAGAAGUACGGAGGAUUCGACAG CCCGACAGUCGCAUACAGCGUCCUGGUCGUCGCAAAGGUCG AAAAGGGAAAGAGCAAGAAGCUGAAGAGCGUCAAGGAACUG CUGGGAAUCACAAUCAUGGAAAGAAGCAGCUUCGAAAAGAA CCCGAUCGACUUCCUGGAAGCAAAGGGAUACAAGGAAGUCA AGAAGGACCUGAUCAUCAAGCUGCCGAAGUACAGCCUGUUC GAACUGGAAAACGGAAGAAAGAGAAUGCUGGCAAGCGCAGG AGAACUGCAGAAGGGAAACGAACUGGCACUGCCGAGCAAGU ACGUCAACUUCCUGUACCUGGCAAGCCACUACGAAAAGCUG AAGGGAAGCCCGGAAGACAACGAACAGAAGCAGCUGUUCGU CGAACAGCACAAGCACUACCUGGACGAAAUCAUCGAACAGA UCAGCGAAUUCAGCAAGAGAGUCAUCCUGGCAGACGCAAAC CUGGACAAGGUCCUGAGCGCAUACAACAAGCACAGAGACAA GCCGAUCAGAGAACAGGCAGAAAACAUCAUCCACCUGUUCA CACUGACAAACCUGGGAGCACCGGCAGCAUUCAAGUACUUC GACACAACAAUCGACAGAAAGAGAUACACAAGCACAAAGGA AGUCCUGGACGCAACACUGAUCCACCAGAGCAUCACAGGAC UGUACGAAACAAGAAUCGACCUGAGCCAGCUGGGAGGAGAC GGAAGCGGAAGCCCGAAGAAGAAGAGAAAGGUCGACGGAAG CCCGAAGAAGAAGAGAAAGGUCGACAGCGGA | |
| **Amino acid sequence of dCas9 with two nuclear localization signals as the C-terminal amino acids** | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLENL IAQLPGEKKNGLFGNLIALSLGLTPNFKSNFDLAEDAKLQLSKDT YDDDLDNLLAQIGDQYADLFLAAKNLSDAILLSDILRVNTEITKA PLSASMIKRYDEHHQDLTLLKALVRQQLPEKYKEIFFDQSKNGYA GYIDGGASQEEFYKFIKPILEKMDGTEELLVKLNREDLLRKQRTF DNGSIPHQIHLGELHAILRRQEDFYPFLKDNREKIEKILTFRIPYYV GPLARGNSRFAWMTRKSEETITPWNFEEVVDKGASAQSFIERMT NFDKNLPNEKVLPKHSLLYEYFTVYNELTKVKYVTEGMRKPAFL SGEQKKAIVDLLFKTNRKVTVKQLKEDYFKKIECFDSVEISGVED RFNASLGTYHDLLKIIKDKDFLDNEENEDILEDIVLTLTLFEDREMI EERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDAIVPQSFLKDDSIDNK VLTRSDKNRGKSDNVPSEEVVKKMKNYWRQLLNAKLITQRKFD NLTKAERGGLSELDKAGFIKRQLVETRQITKHVAQILDSRMNTKY DENDKLIREVKVITLKSKLVSDFRKDFQFYKVREINNYHHAHDA YLNAVVGTALIKKYPKLESEFVYGDYKVYDVRKMIAKSEQEIGK ATAKYFFYSNIMNFFKTEITLANGEIRKRPLIETNGETGEIVWDKG RDFATVRKVLSMPQVNIVKKTEVQTGGFSKESILPKRNSDKLIAR KKDWDPKKYGGFDSPTVAYSVLVVAKVEKGKSKKLKSVKELLG ITIMERSSFEKNPIDFLEAKGYKEVKKDLIIKLPKYSLFELENGRKR MLASAGELQKGNELALPSKYVNFLYLASHYEKLKGSPEDNEQKQ LFVEQHKHYLDEIIEQISEFSKRVILADANLDKVLSAYNKHRDKPI REQAENIIHLFTLTNLGAPAAFKYFDTTIDRKRYTSTKEVLDATLI HQSITGLYETRIDLSQLGGDGSGSPKKKRKVDGSPKKKRKVDSG | **28** |
| **dCas9 mRNA ORF encoding SEQ ID NO: 28 using minimal uridine codons as listed in Table 3, with start and stop codons** | AUGGACAAGAAGUACAGCAUCGGACUGGCAAUCGGAACAAA CAGCGUCGGAUGGGCAGUCAUCACAGACGAAUACAAGGUCC CGAGCAAGAAGUUCAAGGUCCUGGGAAACACAGACAGACAC AGCAUCAAGAAGAACCUGAUCGGAGCACUGCUGUUCGACAG CGGAGAAACAGCAGAAGCAACAAGACUGAAGAGAACAGCAA GAAGAAGAUACACAAGAAGAAAGAACAGAAUCUGCUACCUG CAGGAAAUCUUCAGCAACGAAAUGGCAAAGGUCGACGACAG CUUCUUCCACAGACUGGAAGAAAGCUUCCUGGUCGAAGAAG ACAAGAAGCACGAAAGACACCCGAUCUUCGGAAACAUCGUC GACGAAGUCGCAUACCACGAAAAGUACCCGACAAUCUACCA CCUGAGAAAGAAGCUGGUCGACAGCACAGACAAGGCAGACC UGAGACUGAUCUACCUGGCACUGGCACACAUGAUCAAGUUC AGAGGACACUUCCUGAUCGAAGGAGACCUGAACCCGGACAA CAGCGACGUCGACAAGCUGUUCAUCCAGCUGGUCCAGACAU ACAACCAGCUGUUCGAAGAAAACCCGAUCAACGCAAGCGGA GUCGACGCAAAGGCAAUCCUGAGCGCAAGACUGAGCAAGAG CAGAAGACUGGAAAACCUGAUCGCACAGCUGCCGGGAGAAA AGAAGAACGGACUGUUCGGAAACCUGAUCGCACUGAGCCUG GGACUGACACCGAACUUCAAGAGCAACUUCGACCUGGCAGA AGACGCAAAGCUGCAGCUGAGCAAGGACACAUACGACGACG ACCUGGACAACCUGCUGGCACAGAUCGGAGACCAGUACGCA GACCUGUUCCUGGCAGCAAAGAACCUGAGCGACGCAAUCCU GCUGAGCGACAUCCUGAGAGUCAACACAGAAAUCACAAAGG CACCGCUGAGCGCAAGCAUGAUCAAGAGAUACGACGAACAC CACCAGGACCUGACACUGCUGAAGGCACUGGUCAGACAGCA GCUGCCGGAAAAGUACAAGGAAAUCUUCUUCGACCAGAGCA AGAACGGAUACGCAGGAUACAUCGACGGAGGAGCAAGCCAG GAAGAAUUCUACAAGUUCAUCAAGCCGAUCCUGGAAAAGAU GGACGGAACAGAAGAACUGCUGGUCAAGCUGAACAGAGAAG ACCUGCUGAGAAAGCAGAGAACAUUCGACAACGGAAGCAUC CCGCACCAGAUCCACCUGGGAGAACUGCACGCAAUCCUGAGA AGACAGGAAGACUUCUACCCGUUCCUGAAGGACAACAGAGA AAAGAUCGAAAAGAUCCUGACAUUCAGAAUCCCGUACUACG UCGGACCGCUGGCAAGAGGAAACAGCAGAUUCGCAUGGAUG ACAAGAAAGAGCGAAGAAACAAUCACACCGUGGAACUUCGA AGAAGUCGUCGACAAGGGAGCAAGCGCACAGAGCUUCAUCG AAAGAAUGACAAACUUCGACAAGAACCUGCCGAACGAAAAG GUCCUGCCGAAGCACAGCCUGCUGUACGAAUACUUCACAGU CUACAACGAACUGACAAAGGUCAAGUACGUCACAGAAGGAA UGAGAAAGCCGGCAUUCCUGAGCGGAGAACAGAAGAAGGCA AUCGUCGACCUGCUGUUCAAGACAAACAGAAAGGUCACAGU CAAGCAGCUGAAGGAAGACUACUUCAAGAAGAUCGAAUGCU UCGACAGCGUCGAAAUCAGCGGAGUCGAAGACAGAUUCAAC GCAAGCCUGGGAACAUACCACGACCUGCUGAAGAUCAUCAA GGACAAGGACUUCCUGGACAACGAAGAAAACGAAGACAUCC UGGAAGACAUCGUCCUGACACUGACACUGUUCGAAGACAGA GAAAUGAUCGAAGAAAGACUGAAGACAUACGCACACCUGUU CGACGACAAGGUCAUGAAGCAGCUGAAGAGAAGAAGAUACA CAGGAUGGGGAAGACUGAGCAGAAAGCUGAUCAACGGAAUC AGAGACAAGCAGAGCGGAAAGACAAUCCUGGACUUCCUGAA GAGCGACGGAUUCGCAAACAGAAACUUCAUGCAGCUGAUCC ACGACGACAGCCUGACAUUCAAGGAAGACAUCCAGAAGGCA CAGGUCAGCGGACAGGGAGACAGCCUGCACGAACACAUCGC AAACCUGGCAGGAAGCCCGGCAAUCAAGAAGGGAAUCCUGC AGACAGUCAAGGUCGUCGACGAACUGGUCAAGGUCAUGGGA AGACACAAGCCGGAAAACAUCGUCAUCGAAAUGGCAAGAGA AAACCAGACAACACAGAAGGGACAGAAGAACAGCAGAGAAA GAAUGAAGAGAAUCGAAGAAGGAAUCAAGGAACUGGGAAGC CAGAUCCUGAAGGAACACCCGGUCGAAAACACACAGCUGCA GAACGAAAAGCUGUACCUGUACUACCUGCAGAACGGAAGAG | **29** |
| | ACAUGUACGUCGACCAGGAACUGGACAUCAACAGACUGAGC GACUACGACGUCGACGCAAUCGUCCCGCAGAGCUUCCUGAA GGACGACAGCAUCGACAACAAGGUCCUGACAAGAAGCGACA AGAACAGAGGAAAGAGCGACAACGUCCCGAGCGAAGAAGUC GUCAAGAAGAUGAAGAACUACUGGAGACAGCUGCUGAACGC AAAGCUGAUCACACAGAGAAAGUUCGACAACCUGACAAAGG CAGAGAGAGGAGGACUGAGCGAACUGGACAAGGCAGGAUUC AUCAAGAGACAGCUGGUCGAAACAAGACAGAUCACAAAGCA CGUCGCACAGAUCCUGGACAGCAGAAUGAACACAAAGUACG ACGAAAACGACAAGCUGAUCAGAGAAGUCAAGGUCAUCACA CUGAAGAGCAAGCUGGUCAGCGACUUCAGAAAGGACUUCCA GUUCUACAAGGUCAGAGAAAUCAACAACUACCACCACGCAC ACGACGCAUACCUGAACGCAGUCGUCGGAACAGCACUGAUC AAGAAGUACCCGAAGCUGGAAAGCGAAUUCGUCUACGGAGA CUACAAGGUCUACGACGUCAGAAAGAUGAUCGCAAAGAGCG AACAGGAAAUCGGAAAGGCAACAGCAAAGUACUUCUUCUAC AGCAACAUCAUGAACUUCUUCAAGACAGAAAUCACACUGGC AAACGGAGAAAUCAGAAAGAGACCGCUGAUCGAAACAAACG GAGAAACAGGAGAAAUCGUCUGGGACAAGGGAAGAGACUUC GCAACAGUCAGAAAGGUCCUGAGCAUGCCGCAGGUCAACAU CGUCAAGAAGACAGAAGUCCAGACAGGAGGAUUCAGCAAGG AAAGCAUCCUGCCGAAGAGAAACAGCGACAAGCUGAUCGCA AGAAAGAAGGACUGGGACCCGAAGAAGUACGGAGGAUUCGA CAGCCCGACAGUCGCAUACAGCGUCCUGGUCGUCGCAAAGG UCGAAAAGGGAAAGAGCAAGAAGCUGAAGAGCGUCAAGGAA CUGCUGGGAAUCACAAUCAUGGAAAGAAGCAGCUUCGAAAA GAACCCGAUCGACUUCCUGGAAGCAAAGGGAUACAAGGAAG UCAAGAAGGACCUGAUCAUCAAGCUGCCGAAGUACAGCCUG UUCGAACUGGAAAACGGAAGAAAGAGAAUGCUGGCAAGCGC AGGAGAACUGCAGAAGGGAAACGAACUGGCACUGCCGAGCA AGUACGUCAACUUCCUGUACCUGGCAAGCCACUACGAAAAG CUGAAGGGAAGCCCGGAAGACAACGAACAGAAGCAGCUGUU CGUCGAACAGCACAAGCACUACCUGGACGAAAUCAUCGAAC AGAUCAGCGAAUUCAGCAAGAGAGUCAUCCUGGCAGACGCA AACCUGGACAAGGUCCUGAGCGCAUACAACAAGCACAGAGA CAAGCCGAUCAGAGAACAGGCAGAAAACAUCAUCCACCUGU UCACACUGACAAACCUGGGAGCACCGGCAGCAUUCAAGUAC UUCGACACAACAAUCGACAGAAAGAGAUACACAAGCACAAA GGAAGUCCUGGACGCAACACUGAUCCACCAGAGCAUCACAG GACUGUACGAAACAAGAAUCGACCUGAGCCAGCUGGGAGGA GAC GGAAGCGGAAGCCCGAAGAAGAAGAGAAAGGUCGACGGAAG CCCGAAGAAGAAGAGAAAGGUCGACAGCGGAUAG | |
| **dCas9 coding sequence encoding SEQ ID NO: 28 using minimal uridine codons as listed in Table 3 (no start or stop codons; suitable for inclusion in fusion protein** | GACAAGAAGUACAGCAUCGGACUGGCAAUCGGAACAAACAG CGUCGGAUGGGCAGUCAUCACAGACGAAUACAAGGUCCCGA GCAAGAAGUUCAAGGUCCUGGGAAACACAGACAGACACAGC AUCAAGAAGAACCUGAUCGGAGCACUGCUGUUCGACAGCGG AGAAACAGCAGAAGCAACAAGACUGAAGAGAACAGCAAGAA GAAGAUACACAAGAAGAAAGAACAGAAUCUGCUACCUGCAG GAAAUCUUCAGCAACGAAAUGGCAAAGGUCGACGACAGCUU CUUCCACAGACUGGAAGAAAGCUUCCUGGUCGAAGAAGACA AGAAGCACGAAAGACACCCGAUCUUCGGAAACAUCGUCGAC GAAGUCGCAUACCACGAAAAGUACCCGACAAUCUACCACCU GAGAAAGAAGCUGGUCGACAGCACAGACAAGGCAGACCUGA GACUGAUCUACCUGGCACUGGCACACAUGAUCAAGUUCAGA GGACACUUCCUGAUCGAAGGAGACCUGAACCCGGACAACAG CGACGUCGACAAGCUGUUCAUCCAGCUGGUCCAGACAUACA ACCAGCUGUUCGAAGAAAACCCGAUCAACGCAAGCGGAGUC GACGCAAAGGCAAUCCUGAGCGCAAGACUGAGCAAGAGCAG AAGACUGGAAAACCUGAUCGCACAGCUGCCGGGAGAAAAGA | **30** |
| **coding sequence)** | AGAACGGACUGUUCGGAAACCUGAUCGCACUGAGCCUGGGA CUGACACCGAACUUCAAGAGCAACUUCGACCUGGCAGAAGA CGCAAAGCUGCAGCUGAGCAAGGACACAUACGACGACGACC UGGACAACCUGCUGGCACAGAUCGGAGACCAGUACGCAGAC CUGUUCCUGGCAGCAAAGAACCUGAGCGACGCAAUCCUGCU GAGCGACAUCCUGAGAGUCAACACAGAAAUCACAAAGGCAC CGCUGAGCGCAAGCAUGAUCAAGAGAUACGACGAACACCAC CAGGACCUGACACUGCUGAAGGCACUGGUCAGACAGCAGCU GCCGGAAAAGUACAAGGAAAUCUUCUUCGACCAGAGCAAGA ACGGAUACGCAGGAUACAUCGACGGAGGAGCAAGCCAGGAA GAAUUCUACAAGUUCAUCAAGCCGAUCCUGGAAAAGAUGGA CGGAACAGAAGAACUGCUGGUCAAGCUGAACAGAGAAGACC UGCUGAGAAAGCAGAGAACAUUCGACAACGGAAGCAUCCCG CACCAGAUCCACCUGGGAGAACUGCACGCAAUCCUGAGAAG ACAGGAAGACUUCUACCCGUUCCUGAAGGACAACAGAGAAA AGAUCGAAAAGAUCCUGACAUUCAGAAUCCCGUACUACGUC GGACCGCUGGCAAGAGGAAACAGCAGAUUCGCAUGGAUGAC AAGAAAGAGCGAAGAAACAAUCACACCGUGGAACUUCGAAG AAGUCGUCGACAAGGGAGCAAGCGCACAGAGCUUCAUCGAA AGAAUGACAAACUUCGACAAGAACCUGCCGAACGAAAAGGU CCUGCCGAAGCACAGCCUGCUGUACGAAUACUUCACAGUCU ACAACGAACUGACAAAGGUCAAGUACGUCACAGAAGGAAUG AGAAAGCCGGCAUUCCUGAGCGGAGAACAGAAGAAGGCAAU CGUCGACCUGCUGUUCAAGACAAACAGAAAGGUCACAGUCA AGCAGCUGAAGGAAGACUACUUCAAGAAGAUCGAAUGCUUC GACAGCGUCGAAAUCAGCGGAGUCGAAGACAGAUUCAACGC AAGCCUGGGAACAUACCACGACCUGCUGAAGAUCAUCAAGG ACAAGGACUUCCUGGACAACGAAGAAAACGAAGACAUCCUG GAAGACAUCGUCCUGACACUGACACUGUUCGAAGACAGAGA AAUGAUCGAAGAAAGACUGAAGACAUACGCACACCUGUUCG ACGACAAGGUCAUGAAGCAGCUGAAGAGAAGAAGAUACACA GGAUGGGGAAGACUGAGCAGAAAGCUGAUCAACGGAAUCAG AGACAAGCAGAGCGGAAAGACAAUCCUGGACUUCCUGAAGA GCGACGGAUUCGCAAACAGAAACUUCAUGCAGCUGAUCCAC GACGACAGCCUGACAUUCAAGGAAGACAUCCAGAAGGCACA GGUCAGCGGACAGGGAGACAGCCUGCACGAACACAUCGCAA ACCUGGCAGGAAGCCCGGCAAUCAAGAAGGGAAUCCUGCAG ACAGUCAAGGUCGUCGACGAACUGGUCAAGGUCAUGGGAAG ACACAAGCCGGAAAACAUCGUCAUCGAAAUGGCAAGAGAAA ACCAGACAACACAGAAGGGACAGAAGAACAGCAGAGAAAGA AUGAAGAGAAUCGAAGAAGGAAUCAAGGAACUGGGAAGCCA GAUCCUGAAGGAACACCCGGUCGAAAACACACAGCUGCAGA ACGAAAAGCUGUACCUGUACUACCUGCAGAACGGAAGAGAC AUGUACGUCGACCAGGAACUGGACAUCAACAGACUGAGCGA CUACGACGUCGACGCAAUCGUCCCGCAGAGCUUCCUGAAGG ACGACAGCAUCGACAACAAGGUCCUGACAAGAAGCGACAAG AACAGAGGAAAGAGCGACAACGUCCCGAGCGAAGAAGUCGU CAAGAAGAUGAAGAACUACUGGAGACAGCUGCUGAACGCAA AGCUGAUCACACAGAGAAAGUUCGACAACCUGACAAAGGCA GAGAGAGGAGGACUGAGCGAACUGGACAAGGCAGGAUUCAU CAAGAGACAGCUGGUCGAAACAAGACAGAUCACAAAGCACG UCGCACAGAUCCUGGACAGCAGAAUGAACACAAAGUACGAC GAAAACGACAAGCUGAUCAGAGAAGUCAAGGUCAUCACACU GAAGAGCAAGCUGGUCAGCGACUUCAGAAAGGACUUCCAGU UCUACAAGGUCAGAGAAAUCAACAACUACCACCACGCACAC GACGCAUACCUGAACGCAGUCGUCGGAACAGCACUGAUCAA GAAGUACCCGAAGCUGGAAAGCGAAUUCGUCUACGGAGACU ACAAGGUCUACGACGUCAGAAAGAUGAUCGCAAAGAGCGAA CAGGAAAUCGGAAAGGCAACAGCAAAGUACUUCUUCUACAG CAACAUCAUGAACUUCUUCAAGACAGAAAUCACACUGGCAA ACGGAGAAAUCAGAAAGAGACCGCUGAUCGAAACAAACGGA | |
| | GAAACAGGAGAAAUCGUCUGGGACAAGGGAAGAGACUUCGC AACAGUCAGAAAGGUCCUGAGCAUGCCGCAGGUCAACAUCG UCAAGAAGACAGAAGUCCAGACAGGAGGAUUCAGCAAGGAA AGCAUCCUGCCGAAGAGAAACAGCGACAAGCUGAUCGCAAG AAAGAAGGACUGGGACCCGAAGAAGUACGGAGGAUUCGACA GCCCGACAGUCGCAUACAGCGUCCUGGUCGUCGCAAAGGUC GAAAAGGGAAAGAGCAAGAAGCUGAAGAGCGUCAAGGAACU GCUGGGAAUCACAAUCAUGGAAAGAAGCAGCUUCGAAAAGA ACCCGAUCGACUUCCUGGAAGCAAAGGGAUACAAGGAAGUC AAGAAGGACCUGAUCAUCAAGCUGCCGAAGUACAGCCUGUU CGAACUGGAAAACGGAAGAAAGAGAAUGCUGGCAAGCGCAG GAGAACUGCAGAAGGGAAACGAACUGGCACUGCCGAGCAAG UACGUCAACUUCCUGUACCUGGCAAGCCACUACGAAAAGCU GAAGGGAAGCCCGGAAGACAACGAACAGAAGCAGCUGUUCG UCGAACAGCACAAGCACUACCUGGACGAAAUCAUCGAACAG AUCAGCGAAUUCAGCAAGAGAGUCAUCCUGGCAGACGCAAA CCUGGACAAGGUCCUGAGCGCAUACAACAAGCACAGAGACA AGCCGAUCAGAGAACAGGCAGAAAACAUCAUCCACCUGUUC ACACUGACAAACCUGGGAGCACCGGCAGCAUUCAAGUACUU CGACACAACAAUCGACAGAAAGAGAUACACAAGCACAAAGG AAGUCCUGGACGCAACACUGAUCCACCAGAGCAUCACAGGA CUGUACGAAACAAGAAUCGACCUGAGCCAGCUGGGAGGAGA C GGAAGCGGAAGCCCGAAGAAGAAGAGAAAGGUCGACGGAAG CCCGAAGAAGAAGAGAAAGGUCGACAGCGGA | |
| **T7 promoter** | TAATACGACTCACTATA | **31** |
| **Human beta-globin 5' UTR** | ACATTTGCTTCTGACACAACTGTGTTCACTAGCAACCTCAAAC AGACACC | **32** |
| **Human beta-globin 3' UTR** | GCTCGCTTTCTTGCTGTCCAATTTCTATTAAAGGTTCCTTTGTT CCCTAAGTCCAACTACTAAACTGGGGGATATTATGAAGGGCCT TGAGCATCTGGATTCTGCCTAATAAAAAACATTTATTTTCATTG C | **33** |
| **Human alpha-globin 5' UTR** | CATAAACCCTGGCGCGCTCGCGGCCCGGCACTCTTCTGGTCCC CACAGACTCAGAGAGAACCCACC | **34** |
| **Human alpha-globin 3'UTR** | GCTGGAGCCTCGGTGGCCATGCTTCTTGCCCCTTGGGCCTCCC CCCAGCCCCTCCTCCCCTTCCTGCACCCGTACCCCCGTGGTCTT TGAATAAAGTCTGAGTGGGCGGC | **35** |
| ***Xenopus laevis* beta-globin 5' UTR** | AAGCTCAGAATAAACGCTCAACTTTGGCC | **36** |
| ***Xenopus laevis* beta-globin 3' UTR** | ACCAGCCTCAAGAACACCCGAATGGAGTCTCTAAGCTACATA ATACCAACTTACACTTTACAAAATGTTGTCCCCCAAAATGTAG CCATTCGTATCTGCTCCTAATAAAAAGAAAGTTTCTTCACATTC T | **37** |
| **Bovine Growth Hormone 5' UTR** | CAGGGTCCTGTGGACAGCTCACCAGCT | **38** |
| **Bovine Growth Hormone 3' UTR** | TTGCCAGCCATCTGTTGTTTGCCCCTCCCCCGTGCCTTCCTTGA CCCTGGAAGGTGCCACTCCCACTGTCCTTTCCTAATAAAATGA GGAAATTGCATCGCA | **39** |
| **Mus musculus hemoglobin alpha, adult chain 1 (Hba-a1), 3'UTR** | GCTGCCTTCTGCGGGGCTTGCCTTCTGGCCATGCCCTTCTTCTC TCCCTTGCACCTGTACCTCTTGGTCTTTGAATAAAGCCTGAGTA GGAAG | **40** |
| **HSD17B4 5' UTR** | TCCCGCAGTCGGCGTCCAGCGGCTCTGCTTGTTCGTGTGTGTGT CGTTGCAGGCCTTATTC | **41** |
| **G282 guide RNA targeting TTR** | mU*mU*mA*CAGCCACGUCUACAGCAGUUUUAGAmGm CmUmAmGmAmAmAmUmAmGmCAAGUUAAAAUAAGG CUAGUCCGUUAUCAmAmCmUmUmGmAmAmAmAmAm GmUmGmGmCmAmCmCmGmAmGmUmCmGmGmUmGmC mU*mU*mU*mU | **42** |
| **Cas9 transcript with 5' UTR of HSD, ORF correspondi ng to SEQ ID NO: 4, Kozak sequence, and 3' UTR of ALB** | GGGTCCCGCAGTCGGCGTCCAGCGGCTCTGCTTGTTCGTGTGT GTGTCGTTGCAGGCCTTATTCGGATCCGCCACCATGGACAAGA AGTACAGCATCGGACTGGACATCGGAACAAACAGCGTCGGAT GGGCAGTCATCACAGACGAATACAAGGTCCCGAGCAAGAAGT TCAAGGTCCTGGGAAACACAGACAGACACAGCATCAAGAAGA ACCTGATCGGAGCACTGCTGTTCGACAGCGGAGAAACAGCAG AAGCAACAAGACTGAAGAGAACAGCAAGAAGAAGATACACA AGAAGAAAGAACAGAATCTGCTACCTGCAGGAAATCTTCAGC AACGAAATGGCAAAGGTCGACGACAGCTTCTTCCACAGACTG GAAGAAAGCTTCCTGGTCGAAGAAGACAAGAAGCACGAAAGA CACCCGATCTTCGGAAACATCGTCGACGAAGTCGCATACCACG AAAAGTACCCGACAATCTACCACCTGAGAAAGAAGCTGGTCG ACAGCACAGACAAGGCAGACCTGAGACTGATCTACCTGGCAC TGGCACACATGATCAAGTTCAGAGGACACTTCCTGATCGAAGG AGACCTGAACCCGGACAACAGCGACGTCGACAAGCTGTTCAT CCAGCTGGTCCAGACATACAACCAGCTGTTCGAAGAAAACCC GATCAACGCAAGCGGAGTCGACGCAAAGGCAATCCTGAGCGC AAGACTGAGCAAGAGCAGAAGACTGGAAAACCTGATCGCACA GCTGCCGGGAGAAAAGAAGAACGGACTGTTCGGAAACCTGAT CGCACTGAGCCTGGGACTGACACCGAACTTCAAGAGCAACTTC GACCTGGCAGAAGACGCAAAGCTGCAGCTGAGCAAGGACACA TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA | **43** |
| | AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAGAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG ACCTGAGCCAGCTGGGAGGAGACGGAGGAGGAAGCCCGAAG AAGAAGAGAAAGGTCTAGCTAGCCATCACATTTAAAAGCATC TCAGCCTACCATGAGAATAAGAGAAAGAAAATGAAGATCAAT AGCTTATTCATCTCTTTTTCTTTTTCGTTGGTGTAAAGCCAACA | |
| | CCCTGTCTAAAAAACATAAATTTCTTTAATCATTTTGCCTCTTT TCTCTGTGCTTCAATTAATAAAAAATGGAAAGAACCTCGAG | |
| **Cas9 transcript with 5' UTR of HSD, ORF correspondi ng to SEQ ID NO: 4, and 3' UTR of ALB** | GGGTCCCGCAGTCGGCGTCCAGCGGCTCTGCTTGTTCGTGT GTGTGTCGTTGCAGGCCTTATTCGGATCCATGGACAAGAAGTA CAGCATCGGACTGGACATCGGAACAAACAGCGTCGGATGGGC AGTCATCACAGACGAATACAAGGTCCCGAGCAAGAAGTTCAA GGTCCTGGGAAACACAGACAGACACAGCATCAAGAAGAACCT GATCGGAGCACTGCTGTTCGACAGCGGAGAAACAGCAGAAGC AACAAGACTGAAGAGAACAGCAAGAAGAAGATACACAAGAA GAAAGAACAGAATCTGCTACCTGCAGGAAATCTTCAGCAACG AAATGGCAAAGGTCGACGACAGCTTCTTCCACAGACTGGAAG AAAGCTTCCTGGTCGAAGAAGACAAGAAGCACGAAAGACACC CGATCTTCGGAAACATCGTCGACGAAGTCGCATACCACGAAA AGTACCCGACAATCTACCACCTGAGAAAGAAGCTGGTCGACA GCACAGACAAGGCAGACCTGAGACTGATCTACCTGGCACTGG CACACATGATCAAGTTCAGAGGACACTTCCTGATCGAAGGAG ACCTGAACCCGGACAACAGCGACGTCGACAAGCTGTTCATCC AGCTGGTCCAGACATACAACCAGCTGTTCGAAGAAAACCCGA TCAACGCAAGCGGAGTCGACGCAAAGGCAATCCTGAGCGCAA GACTGAGCAAGAGCAGAAGACTGGAAAACCTGATCGCACAGC TGCCGGGAGAAAAGAAGAACGGACTGTTCGGAAACCTGATCG CACTGAGCCTGGGACTGACACCGAACTTCAAGAGCAACTTCG ACCTGGCAGAAGACGCAAAGCTGCAGCTGAGCAAGGACACAT ACGACGACGACCTGGACAACCTGCTGGCACAGATCGGAGACC AGTACGCAGACCTGTTCCTGGCAGCAAAGAACCTGAGCGACG CAATCCTGCTGAGCGACATCCTGAGAGTCAACACAGAAATCA CAAAGGCACCGCTGAGCGCAAGCATGATCAAGAGATACGACG AACACCACCAGGACCTGACACTGCTGAAGGCACTGGTCAGAC AGCAGCTGCCGGAAAAGTACAAGGAAATCTTCTTCGACCAGA GCAAGAACGGATACGCAGGATACATCGACGGAGGAGCAAGCC AGGAAGAATTCTACAAGTTCATCAAGCCGATCCTGGAAAAGA TGGACGGAACAGAAGAACTGCTGGTCAAGCTGAACAGAGAAG ACCTGCTGAGAAAGCAGAGAACATTCGACAACGGAAGCATCC CGCACCAGATCCACCTGGGAGAACTGCACGCAATCCTGAGAA GACAGGAAGACTTCTACCCGTTCCTGAAGGACAACAGAGAAA AGATCGAAAAGATCCTGACATTCAGAATCCCGTACTACGTCGG ACCGCTGGCAAGAGGAAACAGCAGATTCGCATGGATGACAAG AAAGAGCGAAGAAACAATCACACCGTGGAACTTCGAAGAAGT CGTCGACAAGGGAGCAAGCGCACAGAGCTTCATCGAAAGAAT GACAAACTTCGACAAGAACCTGCCGAACGAAAAGGTCCTGCC GAAGCACAGCCTGCTGTACGAATACTTCACAGTCTACAACGAA CTGACAAAGGTCAAGTACGTCACAGAAGGAATGAGAAAGCCG GCATTCCTGAGCGGAGAACAGAAGAAGGCAATCGTCGACCTG CTGTTCAAGACAAACAGAAAGGTCACAGTCAAGCAGCTGAAG GAAGACTACTTCAAGAAGATCGAATGCTTCGACAGCGTCGAA ATCAGCGGAGTCGAAGACAGATTCAACGCAAGCCTGGGAACA TACCACGACCTGCTGAAGATCATCAAGGACAAGGACTTCCTGG ACAACGAAGAAAACGAAGACATCCTGGAAGACATCGTCCTGA CACTGACACTGTTCGAAGACAGAGAAATGATCGAAGAAAGAC TGAAGACATACGCACACCTGTTCGACGACAAGGTCATGAAGC AGCTGAAGAGAAGAAGATACACAGGATGGGGAAGACTGAGC AGAAAGCTGATCAACGGAATCAGAGACAAGCAGAGCGGAAA GACAATCCTGGACTTCCTGAAGAGCGACGGATTCGCAAACAG AAACTTCATGCAGCTGATCCACGACGACAGCCTGACATTCAAG GAAGACATCCAGAAGGCACAGGTCAGCGGACAGGGAGACAG CCTGCACGAACACATCGCAAACCTGGCAGGAAGCCCGGCAAT CAAGAAGGGAATCCTGCAGACAGTCAAGGTCGTCGACGAACT GGTCAAGGTCATGGGAAGACACAAGCCGGAAAACATCGTCAT CGAAATGGCAAGAGAAAACCAGACAACACAGAAGGGACAGA | **44** |
| | AGAACAGCAGAGAAAGAATGAAGAGAATCGAAGAAGGAATC AAGGAACTGGGAAGCCAGATCCTGAAGGAACACCCGGTCGAA AACACACAGCTGCAGAACGAAAAGCTGTACCTGTACTACCTG CAGAACGGAAGAGACATGTACGTCGACCAGGAACTGGACATC AACAGACTGAGCGACTACGACGTCGACCACATCGTCCCGCAG AGCTTCCTGAAGGACGACAGCATCGACAACAAGGTCCTGACA AGAAGCGACAAGAACAGAGGAAAGAGCGACAACGTCCCGAG CGAAGAAGTCGTCAAGAAGATGAAGAACTACTGGAGACAGCT GCTGAACGCAAAGCTGATCACACAGAGAAAGTTCGACAACCT GACAAAGGCAGAGAGAGGAGGACTGAGCGAACTGGACAAGG CAGGATTCATCAAGAGACAGCTGGTCGAAACAAGACAGATCA CAAAGCACGTCGCACAGATCCTGGACAGCAGAATGAACACAA AGTACGACGAAAACGACAAGCTGATCAGAGAAGTCAAGGTCA TCACACTGAAGAGCAAGCTGGTCAGCGACTTCAGAAAGGACT TCCAGTTCTACAAGGTCAGAGAAATCAACAACTACCACCACGC ACACGACGCATACCTGAACGCAGTCGTCGGAACAGCACTGAT CAAGAAGTACCCGAAGCTGGAAAGCGAATTCGTCTACGGAGA CTACAAGGTCTACGACGTCAGAAAGATGATCGCAAAGAGCGA ACAGGAAATCGGAAAGGCAACAGCAAAGTACTTCTTCTACAG CAACATCATGAACTTCTTCAAGACAGAAATCACACTGGCAAAC GGAGAAATCAGAAAGAGACCGCTGATCGAAACAAACGGAGA AACAGGAGAAATCGTCTGGGACAAGGGAAGAGACTTCGCAAC AGTCAGAAAGGTCCTGAGCATGCCGCAGGTCAACATCGTCAA GAAGACAGAAGTCCAGACAGGAGGATTCAGCAAGGAAAGCAT CCTGCCGAAGAGAAACAGCGACAAGCTGATCGCAAGAAAGAA GGACTGGGACCCGAAGAAGTACGGAGGATTCGACAGCCCGAC AGTCGCATACAGCGTCCTGGTCGTCGCAAAGGTCGAAAAGGG AAAGAGCAAGAAGCTGAAGAGCGTCAAGGAACTGCTGGGAAT CACAATCATGGAAAGAAGCAGCTTCGAAAAGAACCCGATCGA CTTCCTGGAAGCAAAGGGATACAAGGAAGTCAAGAAGGACCT GATCATCAAGCTGCCGAAGTACAGCCTGTTCGAACTGGAAAA CGGAAGAAAGAGAATGCTGGCAAGCGCAGGAGAACTGCAGA AGGGAAACGAACTGGCACTGCCGAGCAAGTACGTCAACTTCC TGTACCTGGCAAGCCACTACGAAAAGCTGAAGGGAAGCCCGG AAGACAACGAACAGAAGCAGCTGTTCGTCGAACAGCACAAGC ACTACCTGGACGAAATCATCGAACAGATCAGCGAATTCAGCA AGAGAGTCATCCTGGCAGACGCAAACCTGGACAAGGTCCTGA GCGCATACAACAAGCACAGAGACAAGCCGATCAGAGAACAGG CAGAAAACATCATCCACCTGTTCACACTGACAAACCTGGGAGC ACCGGCAGCATTCAAGTACTTCGACACAACAATCGACAGAAA GAGATACACAAGCACAAAGGAAGTCCTGGACGCAACACTGAT CCACCAGAGCATCACAGGACTGTACGAAACAAGAATCGACCT GAGCCAGCTGGGAGGAGACGGAGGAGGAAGCCCGAAGAAGA AGAGAAAGGTCTAGCTAGCCATCACATTTAAAAGCATCTCAGC CTACCATGAGAATAAGAGAAAGAAAATGAAGATCAATAGCTT ATTCATCTCTTTTTCTTTTTCGTTGGTGTAAAGCCAACACCCTG TCTAAAAAACATAAATTTCTTTAATCATTTTGCCTCTTTTCTCT GTGCTTCAATTAATAAAAAATGGAAAGAACCTCGAG | |
| Alternative Cas9 ORF with 19.36% U content | ATGGATAAGAAGTACTCGATCGGGCTGGATATCGGAACTAATT CCGTGGGTTGGGCAGTGATCACGGATGAATACAAAGTGCCGT CCAAGAAGTTCAAGGTCCTGGGGAACACCGATAGACACAGCA TCAAGAAGAATCTCATCGGAGCCCTGCTGTTTGACTCCGGCGA AACCGCAGAAGCGACCCGGCTCAAACGTACCGCGAGGCGACG CTACACCCGGCGGAAGAATCGCATCTGCTATCTGCAAGAAATC TTTTCGAACGAAATGGCAAAGGTGGACGACAGCTTCTTCCACC GCCTGGAAGAATCTTTCCTGGTGGAGGAGGACAAGAAGCATG AACGGCATCCTATCTTTGGAAACATCGTGGACGAAGTGGCGTA CCACGAAAAGTACCCGACCATCTACCATCTGCGGAAGAAGTT GGTTGACTCAACTGACAAGGCCGACCTCAGATTGATCTACTTG | **45** |
| | GCCCTCGCCCATATGATCAAATTCCGCGGACACTTCCTGATCG AAGGCGATCTGAACCCTGATAACTCCGACGTGGATAAGCTGTT CATTCAACTGGTGCAGACCTACAACCAACTGTTCGAAGAAAAC CCAATCAATGCCAGCGGCGTCGATGCCAAGGCCATCCTGTCCG CCCGGCTGTCGAAGTCGCGGCGCCTCGAAAACCTGATCGCACA GCTGCCGGGAGAGAAGAAGAACGGACTTTTCGGCAACTTGAT CGCTCTCTCACTGGGACTCACTCCCAATTTCAAGTCCAATTTTG ACCTGGCCGAGGACGCGAAGCTGCAACTCTCAAAGGACACCT ACGACGACGACTTGGACAATTTGCTGGCACAAATTGGCGATCA GTACGCGGATCTGTTCCTTGCCGCTAAGAACCTTTCGGACGCA ATCTTGCTGTCCGATATCCTGCGCGTGAACACCGAAATAACCA AAGCGCCGCTTAGCGCCTCGATGATTAAGCGGTACGACGAGC ATCACCAGGATCTCACGCTGCTCAAAGCGCTCGTGAGACAGCA ACTGCCTGAAAAGTACAAGGAGATTTTCTTCGACCAGTCCAAG AATGGGTACGCAGGGTACATCGATGGAGGCGCCAGCCAGGAA GAGTTCTATAAGTTCATCAAGCCAATCCTGGAAAAGATGGACG GAACCGAAGAACTGCTGGTCAAGCTGAACAGGGAGGATCTGC TCCGCAAACAGAGAACCTTTGACAACGGAAGCATTCCACACC AGATCCATCTGGGTGAGCTGCACGCCATCTTGCGGCGCCAGGA GGACTTTTACCCATTCCTCAAGGACAACCGGGAAAAGATCGA GAAAATTCTGACGTTCCGCATCCCGTATTACGTGGGCCCACTG GCGCGCGGCAATTCGCGCTTCGCGTGGATGACTAGAAAATCA GAGGAAACCATCACTCCTTGGAATTTCGAGGAAGTTGTGGATA AGGGAGCTTCGGCACAATCCTTCATCGAACGAATGACCAACTT CGACAAGAATCTCCCAAACGAGAAGGTGCTTCCTAAGCACAG CCTCCTTTACGAATACTTCACTGTCTACAACGAACTGACTAAA GTGAAATACGTTACTGAAGGAATGAGGAAGCCGGCCTTTCTG AGCGGAGAACAGAAGAAAGCGATTGTCGATCTGCTGTTCAAG ACCAACCGCAAGGTGACCGTCAAGCAGCTTAAAGAGGACTAC TTCAAGAAGATCGAGTGTTTCGACTCAGTGGAAATCAGCGGA GTGGAGGACAGATTCAACGCTTCGCTGGGAACCTATCATGATC TCCTGAAGATCATCAAGGACAAGGACTTCCTTGACAACGAGG AGAACGAGGACATCCTGGAAGATATCGTCCTGACCTTGACCCT TTTCGAGGATCGCGAGATGATCGAGGAGAGGCTTAAGACCTA CGCTCATCTCTTCGACGATAAGGTCATGAAACAACTCAAGCGC CGCCGGTACACTGGTTGGGGCCGCCTCTCCCGCAAGCTGATCA ACGGTATTCGCGATAAACAGAGCGGTAAAACTATCCTGGATTT CCTCAAATCGGATGGCTTCGCTAATCGTAACTTCATGCAGTTG ATCCACGACGACAGCCTGACCTTTAAGGAGGACATCCAGAAA GCACAAGTGAGCGGACAGGGAGACTCACTCCATGAACACATC GCGAATCTGGCCGGTTCGCCGGCGATTAAGAAGGGAATCCTG CAAACTGTGAAGGTGGTGGACGAGCTGGTGAAGGTCATGGGA CGGCACAAACCGGAGAATATCGTGATTGAAATGGCCCGAGAA AACCAGACTACCCAGAAGGGCCAGAAGAACTCCCGCGAAAGG ATGAAGCGGATCGAAGAAGGAATCAAGGAGCTGGGCAGCCAG ATCCTGAAAGAGCACCCGGTGGAAAACACGCAGCTGCAGAAC GAGAAGCTCTACCTGTACTATTTGCAAAATGGACGGGACATGT ACGTGGACCAAGAGCTGGACATCAATCGGTTGTCTGATTACGA CGTGGACCACATCGTTCCACAGTCCTTTCTGAAGGATGACTCC ATCGATAACAAGGTGTTGACTCGCAGCGACAAGAACAGAGGG AAGTCAGATAATGTGCCATCGGAGGAGGTCGTGAAGAAGATG AAGAATTACTGGCGGCAGCTCCTGAATGCGAAGCTGATTACCC AGAGAAAGTTTGACAATCTCACTAAAGCCGAGCGCGGCGGAC TCTCAGAGCTGGATAAGGCTGGATTCATCAAACGGCAGCTGGT CGAGACTCGGCAGATTACCAAGCACGTGGCGCAGATCCTGGA CTCCCGCATGAACACTAAATACGACGAGAACGATAAGCTCAT CCGGGAAGTGAAGGTGATTACCCTGAAAAGCAAACTTGTGTC GGACTTTCGGAAGGACTTTCAGTTTTACAAAGTGAGAGAAATC AACAACTACCATCACGCGCATGACGCATACCTCAACGCTGTGG TCGGCACCGCCCTGATCAAGAAGTACCCTAAACTTGAATCGGA GTTTGTGTACGGAGACTACAAGGTCTACGACGTGAGGAAGAT | |
| | GATAGCCAAGTCCGAACAGGAAATCGGGAAAGCAACTGCGAA ATACTTCTTTTACTCAAACATCATGAACTTCTTCAAGACTGAA ATTACGCTGGCCAATGGAGAAATCAGGAAGAGGCCACTGATC GAAACTAACGGAGAAACGGGCGAAATCGTGTGGGACAAGGGC AGGGACTTCGCAACTGTTCGCAAAGTGCTCTCTATGCCGCAAG TCAATATTGTGAAGAAAACCGAAGTGCAAACCGGCGGATTTTC AAAGGAATCGATCCTCCCAAAGAGAAATAGCGACAAGCTCAT TGCACGCAAGAAAGACTGGGACCCGAAGAAGTACGGAGGATT CGATTCGCCGACTGTCGCATACTCCGTCCTCGTGGTGGCCAAG GTGGAGAAGGGAAAGAGCAAGAAGCTCAAATCCGTCAAAGA GCTGCTGGGGATTACCATCATGGAACGATCCTCGTTCGAGAAG AACCCGATTGATTTCCTGGAGGCGAAGGGTTACAAGGAGGTG AAGAAGGATCTGATCATCAAACTGCCCAAGTACTCACTGTTCG AACTGGAAAATGGTCGGAAGCGCATGCTGGCTTCGGCCGGAG AACTCCAGAAAGGAAATGAGCTGGCCTTGCCTAGCAAGTACG TCAACTTCCTCTATCTTGCTTCGCACTACGAAAACTCAAAGG GTCACCGGAAGATAACGAACAGAAGCAGCTTTTCGTGGAGCA GCACAAGCATTATCTGGATGAAATCATCGAACAAATCTCCGAG TTTTCAAAGCGCGTGATCCTCGCCGACGCCAACCTCGACAAAG TCCTGTCGGCCTACAATAAGCATAGAGATAAGCCGATCAGAG AACAGGCCGAGAACATTATCCACTTGTTCACCCTGACTAACCT GGGAGCTCCAGCCGCCTTCAAGTACTTCGATACTACTATCGAC CGCAAAAGATACACGTCCACCAAGGAAGTTCTGGACGCGACC CTGATCCACCAAAGCATCACTGGACTCTACGAAACTAGGATCG ATCTGTCGCAGCTGGGTGGCGATGGTGGCGGTGGATCCTACCC ATACGACGTGCCTGACTACGCCTCCGGAGGTGGTGGCCCCAAG AAGAAACGGAAGGTGTGATAG | |
| **Cas9 transcript with 5' UTR of HSD, ORF correspondi ng to SEQ ID NO: 45, Kozak sequence, and 3' UTR of ALB** | GGGTCCCGCAGTCGGCGTCCAGCGGCTCTGCTTGTTCGTGTGT GTGTCGTTGCAGGCCTTATTCGGATCTGCCACCATGGATAAGA AGTACTCGATCGGGCTGGATATCGGAACTAATTCCGTGGGTTG GGCAGTGATCACGGATGAATACAAAGTGCCGTCCAAGAAGTT CAAGGTCCTGGGGAACACCGATAGACACAGCATCAAGAAGAA TCTCATCGGAGCCCTGCTGTTTGACTCCGGCGAAACCGCAGAA GCGACCCGGCTCAAACGTACCGCGAGGCGACGCTACACCCGG CGGAAGAATCGCATCTGCTATCTGCAAGAAATCTTTTCGAACG AAATGGCAAAGGTGGACGACAGCTTCTTCCACCGCCTGGAAG AATCTTTCCTGGTGGAGGAGGACAAGAAGCATGAACGGCATC CTATCTTTGGAAACATCGTGGACGAAGTGGCGTACCACGAAA AGTACCCGACCATCTACCATCTGCGGAAGAAGTTGGTTGACTC AACTGACAAGGCCGACCTCAGATTGATCTACTTGGCCCTCGCC CATATGATCAAATTCCGCGGACACTTCCTGATCGAAGGCGATC TGAACCCTGATAACTCCGACGTGGATAAGCTGTTCATTCAACT GGTGCAGACCTACAACCAACTGTTCGAAGAAAACCCAATCAA TGCCAGCGGCGTCGATGCCAAGGCCATCCTGTCCGCCCGGCTG TCGAAGTCGCGGCGCCTCGAAAACCTGATCGCACAGCTGCCG GGAGAGAAGAAGAACGGACTTTTCGGCAACTTGATCGCTCTCT CACTGGGACTCACTCCCAATTTCAAGTCCAATTTTGACCTGGC CGAGGACGCGAAGCTGCAACTCTCAAAGGACACCTACGACGA CGACTTGGACAATTTGCTGGCACAAATTGGCGATCAGTACGCG GATCTGTTCCTTGCCGCTAAGAACCTTTCGGACGCAATCTTGCT GTCCGATATCCTGCGCGTGAACACCGAAATAACCAAAGCGCC GCTTAGCGCCTCGATGATTAAGCGGTACGACGAGCATCACCAG GATCTCACGCTGCTCAAAGCGCTCGTGAGACAGCAACTGCCTG AAAAGTACAAGGAGATTTTCTTCGACCAGTCCAAGAATGGGT ACGCAGGGTACATCGATGGAGGCGCCAGCCAGGAAGAGTTCT ATAAGTTCATCAAGCCAATCCTGGAAAAGATGGACGGAACCG AAGAACTGCTGGTCAAGCTGAACAGGGAGGATCTGCTCCGCA AACAGAGAACCTTTGACAACGGAAGCATTCCACACCAGATCC ATCTGGGTGAGCTGCACGCCATCTTGCGGCGCCAGGAGGACTT | **46** |
| | TTACCCATTCCTCAAGGACAACCGGGAAAAGATCGAGAAAAT TCTGACGTTCCGCATCCCGTATTACGTGGGCCCACTGGCGCGC GGCAATTCGCGCTTCGCGTGGATGACTAGAAAATCAGAGGAA ACCATCACTCCTTGGAATTTCGAGGAAGTTGTGGATAAGGGAG CTTCGGCACAATCCTTCATCGAACGAATGACCAACTTCGACAA GAATCTCCCAAACGAGAAGGTGCTTCCTAAGCACAGCCTCCTT TACGAATACTTCACTGTCTACAACGAACTGACTAAAGTGAAAT ACGTTACTGAAGGAATGAGGAAGCCGGCCTTTCTGAGCGGAG AACAGAAGAAAGCGATTGTCGATCTGCTGTTCAAGACCAACC GCAAGGTGACCGTCAAGCAGCTTAAAGAGGACTACTTCAAGA AGATCGAGTGTTTCGACTCAGTGGAAATCAGCGGAGTGGAGG ACAGATTCAACGCTTCGCTGGGAACCTATCATGATCTCCTGAA GATCATCAAGGACAAGGACTTCCTTGACAACGAGGAGAACGA GGACATCCTGGAAGATATCGTCCTGACCTTGACCCTTTTCGAG GATCGCGAGATGATCGAGGAGAGGCTTAAGACCTACGCTCAT CTCTTCGACGATAAGGTCATGAAACAACTCAAGCGCCGCCGGT ACACTGGTTGGGGCCGCCTCTCCCGCAAGCTGATCAACGGTAT TCGCGATAAACAGAGCGGTAAAACTATCCTGGATTTCCTCAAA TCGGATGGCTTCGCTAATCGTAACTTCATGCAGTTGATCCACG ACGACAGCCTGACCTTTAAGGAGGACATCCAGAAAGCACAAG TGAGCGGACAGGGAGACTCACTCCATGAACACATCGCGAATC TGGCCGGTTCGCCGGCGATTAAGAAGGGAATCCTGCAAACTGT GAAGGTGGTGGACGAGCTGGTGAAGGTCATGGGACGGCACAA ACCGGAGAATATCGTGATTGAAATGGCCCGAGAAAACCAGAC TACCCAGAAGGGCCAGAAGAACTCCCGCGAAAGGATGAAGCG GATCGAAGAAGGAATCAAGGAGCTGGGCAGCCAGATCCTGAA AGAGCACCCGGTGGAAAACACGCAGCTGCAGAACGAGAAGCT CTACCTGTACTATTTGCAAAATGGACGGGACATGTACGTGGAC CAAGAGCTGGACATCAATCGGTTGTCTGATTACGACGTGGACC ACATCGTTCCACAGTCCTTTCTGAAGGATGACTCCATCGATAA CAAGGTGTTGACTCGCAGCGACAAGAACAGAGGGAAGTCAGA TAATGTGCCATCGGAGGAGGTCGTGAAGAAGATGAAGAATTA CTGGCGGCAGCTCCTGAATGCGAAGCTGATTACCCAGAGAAA GTTTGACAATCTCACTAAAGCCGAGCGCGGCGGACTCTCAGAG CTGGATAAGGCTGGATTCATCAAACGGCAGCTGGTCGAGACTC GGCAGATTACCAAGCACGTGGCGCAGATCCTGGACTCCCGCAT GAACACTAAATACGACGAGAACGATAAGCTCATCCGGGAAGT GAAGGTGATTACCCTGAAAAGCAAACTTGTGTCGGACTTTCGG AAGGACTTTCAGTTTTACAAAGTGAGAGAAATCAACAACTACC ATCACGCGCATGACGCATACCTCAACGCTGTGGTCGGCACCGC CCTGATCAAGAAGTACCCTAAACTTGAATCGGAGTTTGTGTAC GGAGACTACAAGGTCTACGACGTGAGGAAGATGATAGCCAAG TCCGAACAGGAAATCGGGAAAGCAACTGCGAAATACTTCTTTT ACTCAAACATCATGAACTTCTTCAAGACTGAAATTACGCTGGC CAATGGAGAAATCAGGAAGAGGCCACTGATCGAAACTAACGG AGAAACGGGCGAAATCGTGTGGGACAAGGGCAGGGACTTCGC AACTGTTCGCAAAGTGCTCTCTATGCCGCAAGTCAATATTGTG AAGAAAACCGAAGTGCAAACCGGCGGATTTTCAAAGGAATCG ATCCTCCCAAAGAGAAATAGCGACAAGCTCATTGCACGCAAG AAAGACTGGGACCCGAAGAAGTACGGAGGATTCGATTCGCCG ACTGTCGCATACTCCGTCCTCGTGGTGGCCAAGGTGGAGAAGG GAAAGAGCAAGAAGCTCAAATCCGTCAAAGAGCTGCTGGGGA TTACCATCATGGAACGATCCTCGTTCGAGAAGAACCCGATTGA TTTCCTGGAGGCGAAGGGTTACAAGGAGGTGAAGAAGGATCT GATCATCAAACTGCCCAAGTACTCACTGTTCGAACTGGAAAAT GGTCGGAAGCGCATGCTGGCTTCGGCCGGAGAACTCCAGAAA GGAAATGAGCTGGCCTTGCCTAGCAAGTACGTCAACTTCCTCT ATCTTGCTTCGCACTACGAGAAACTCAAAGGGTCACCGGAAG ATAACGAACAGAAGCAGCTTTTCGTGGAGCAGCACAAGCATT ATCTGGATGAAATCATCGAACAAATCTCCGAGTTTTCAAAGCG CGTGATCCTCGCCGACGCCAACCTCGACAAAGTCCTGTCGGCC | |
| | TACAATAAGCATAGAGATAAGCCGATCAGAGAACAGGCCGAG AACATTATCCACTTGTTCACCCTGACTAACCTGGGAGCTCCAG CCGCCTTCAAGTACTTCGATACTACTATCGACCGCAAAAGATA CACGTCCACCAAGGAAGTTCTGGACGCGACCCTGATCCACCAA AGCATCACTGGACTCTACGAAACTAGGATCGATCTGTCGCAGC TGGGTGGCGATGGTGGCGGTGGATCCTACCCATACGACGTGCC TGACTACGCCTCCGGAGGTGGTGGCCCCAAGAAGAAACGGAA GGTGTGATAGCTAGCCATCACATTTAAAAGCATCTCAGCCTAC CATGAGAATAAGAGAAAGAAAATGAAGATCAATAGCTTATTC ATCTCTTTTTCTTTTTCGTTGGTGTAAAGCCAACACCCTGTCTA AAAAACATAAATTTCTTTAATCATTTTGCCTCTTTTCTCTGTGC TTCAATTAATAAAAAATGGAAAGAACCTCGAG | |
| **Cas9 transcript with 5' UTR of HSD, ORF correspondi ng to SEQ ID NO: 45, and 3' UTR of ALB** | GGGTCCCGCAGTCGGCGTCCAGCGGCTCTGCTTGTTCGTGTGT GTGTCGTTGCAGGCCTTATTCGGATCTATGGATAAGAAGTACT CGATCGGGCTGGATATCGGAACTAATTCCGTGGGTTGGGCAGT GATCACGGATGAATACAAAGTGCCGTCCAAGAAGTTCAAGGT CCTGGGGAACACCGATAGACACAGCATCAAGAAGAATCTCAT CGGAGCCCTGCTGTTTGACTCCGGCGAAACCGCAGAAGCGAC CCGGCTCAAACGTACCGCGAGGCGACGCTACACCCGGCGGAA GAATCGCATCTGCTATCTGCAAGAAATCTTTTCGAACGAAATG GCAAAGGTGGACGACAGCTTCTTCCACCGCCTGGAAGAATCTT TCCTGGTGGAGGAGGACAAGAAGCATGAACGGCATCCTATCT TTGGAAACATCGTGGACGAAGTGGCGTACCACGAAAAGTACC CGACCATCTACCATCTGCGGAAGAAGTTGGTTGACTCAACTGA CAAGGCCGACCTCAGATTGATCTACTTGGCCCTCGCCCATATG ATCAAATTCCGCGGACACTTCCTGATCGAAGGCGATCTGAACC CTGATAACTCCGACGTGGATAAGCTGTTCATTCAACTGGTGCA GACCTACAACCAACTGTTCGAAGAAAACCCAATCAATGCCAG CGGCGTCGATGCCAAGGCCATCCTGTCCGCCCGGCTGTCGAAG TCGCGGCGCCTCGAAAACCTGATCGCACAGCTGCCGGGAGAG AAGAAGAACGGACTTTTCGGCAACTTGATCGCTCTCTCACTGG GACTCACTCCCAATTTCAAGTCCAATTTTGACCTGGCCGAGGA CGCGAAGCTGCAACTCTCAAAGGACACCTACGACGACGACTT GGACAATTTGCTGGCACAAATTGGCGATCAGTACGCGGATCTG TTCCTTGCCGCTAAGAACCTTTCGGACGCAATCTTGCTGTCCG ATATCCTGCGCGTGAACACCGAAATAACCAAAGCGCCGCTTA GCGCCTCGATGATTAAGCGGTACGACGAGCATCACCAGGATCT CACGCTGCTCAAAGCGCTCGTGAGACAGCAACTGCCTGAAAA GTACAAGGAGATTTTCTTCGACCAGTCCAAGAATGGGTACGCA GGGTACATCGATGGAGGCGCCAGCCAGGAAGAGTTCTATAAG TTCATCAAGCCAATCCTGGAAAAGATGGACGGAACCGAAGAA CTGCTGGTCAAGCTGAACAGGGAGGATCTGCTCCGCAAACAG AGAACCTTTGACAACGGAAGCATTCCACACCAGATCCATCTGG GTGAGCTGCACGCCATCTTGCGGCGCCAGGAGGACTTTTACCC ATTCCTCAAGGACAACCGGGAAAAGATCGAGAAAATTCTGAC GTTCCGCATCCCGTATTACGTGGGCCCACTGGCGCGCGGCAAT TCGCGCTTCGCGTGGATGACTAGAAAATCAGAGGAAACCATC ACTCCTTGGAATTTCGAGGAAGTTGTGGATAAGGGAGCTTCGG CACAATCCTTCATCGAACGAATGACCAACTTCGACAAGAATCT CCCAAACGAGAAGGTGCTTCCTAAGCACAGCCTCCTTTACGAA TACTTCACTGTCTACAACGAACTGACTAAAGTGAAATACGTTA CTGAAGGAATGAGGAAGCCGGCCTTTCTGAGCGGAGAACAGA AGAAAGCGATTGTCGATCTGCTGTTCAAGACCAACCGCAAGGT GACCGTCAAGCAGCTTAAAGAGGACTACTTCAAGAAGATCGA GTGTTTCGACTCAGTGGAAATCAGCGGAGTGGAGGACAGATT CAACGCTTCGCTGGGAACCTATCATGATCTCCTGAAGATCATC AAGGACAAGGACTTCCTTGACAACGAGGAGAACGAGGACATC CTGGAAGATATCGTCCTGACCTTGACCCTTTTCGAGGATCGCG AGATGATCGAGGAGAGGCTTAAGACCTACGCTCATCTCTTCGA | **47** |
| | CGATAAGGTCATGAAACAACTCAAGCGCCGCCGGTACACTGG TTGGGGCCGCCTCTCCCGCAAGCTGATCAACGGTATTCGCGAT AAACAGAGCGGTAAAACTATCCTGGATTTCCTCAAATCGGATG GCTTCGCTAATCGTAACTTCATGCAGTTGATCCACGACGACAG CCTGACCTTTAAGGAGGACATCCAGAAAGCACAAGTGAGCGG ACAGGGAGACTCACTCCATGAACACATCGCGAATCTGGCCGG TTCGCCGGCGATTAAGAAGGGAATCCTGCAAACTGTGAAGGT GGTGGACGAGCTGGTGAAGGTCATGGGACGGCACAAACCGGA GAATATCGTGATTGAAATGGCCCGAGAAAACCAGACTACCCA GAAGGGCCAGAAGAACTCCCGCGAAAGGATGAAGCGGATCGA AGAAGGAATCAAGGAGCTGGGCAGCCAGATCCTGAAAGAGCA CCCGGTGGAAAACACGCAGCTGCAGAACGAGAAGCTCTACCT GTACTATTTGCAAAATGGACGGGACATGTACGTGGACCAAGA GCTGGACATCAATCGGTTGTCTGATTACGACGTGGACCACATC GTTCCACAGTCCTTTCTGAAGGATGACTCCATCGATAACAAGG TGTTGACTCGCAGCGACAAGAACAGAGGGAAGTCAGATAATG TGCCATCGGAGGAGGTCGTGAAGAAGATGAAGAATTACTGGC GGCAGCTCCTGAATGCGAAGCTGATTACCCAGAGAAAGTTTG ACAATCTCACTAAAGCCGAGCGCGGCGGACTCTCAGAGCTGG ATAAGGCTGGATTCATCAAACGGCAGCTGGTCGAGACTCGGC AGATTACCAAGCACGTGGCGCAGATCCTGGACTCCCGCATGA ACACTAAATACGACGAGAACGATAAGCTCATCCGGGAAGTGA AGGTGATTACCCTGAAAAGCAAACTTGTGTCGGACTTTCGGAA GGACTTTCAGTTTTACAAAGTGAGAGAAATCAACAACTACCAT CACGCGCATGACGCATACCTCAACGCTGTGGTCGGCACCGCCC TGATCAAGAAGTACCCTAAACTTGAATCGGAGTTTGTGTACGG AGACTACAAGGTCTACGACGTGAGGAAGATGATAGCCAAGTC CGAACAGGAAATCGGGAAAGCAACTGCGAAATACTTCTTTTA CTCAAACATCATGAACTTCTTCAAGACTGAAATTACGCTGGCC AATGGAGAAATCAGGAAGAGGCCACTGATCGAAACTAACGGA GAAACGGGCGAAATCGTGTGGGACAAGGGCAGGGACTTCGCA ACTGTTCGCAAAGTGCTCTCTATGCCGCAAGTCAATATTGTGA AGAAAACCGAAGTGCAAACCGGCGGATTTTCAAAGGAATCGA TCCTCCCAAAGAGAAATAGCGACAAGCTCATTGCACGCAAGA AAGACTGGGACCCGAAGAAGTACGGAGGATTCGATTCGCCGA CTGTCGCATACTCCGTCCTCGTGGTGGCCAAGGTGGAGAAGGG AAAGAGCAAGAAGCTCAAATCCGTCAAAGAGCTGCTGGGGAT TACCATCATGGAACGATCCTCGTTCGAGAAGAACCCGATTGAT TTCCTGGAGGCGAAGGGTTACAAGGAGGTGAAGAAGGATCTG ATCATCAAACTGCCCAAGTACTCACTGTTCGAACTGGAAAATG GTCGGAAGCGCATGCTGGCTTCGGCCGGAGAACTCCAGAAAG GAAATGAGCTGGCCTTGCCTAGCAAGTACGTCAACTTCCTCTA TCTTGCTTCGCACTACGAGAAACTCAAAGGGTCACCGGAAGAT AACGAACAGAAGCAGCTTTTCGTGGAGCAGCACAAGCATTAT CTGGATGAAATCATCGAACAAATCTCCGAGTTTTCAAAGCGCG TGATCCTCGCCGACGCCAACCTCGACAAAGTCCTGTCGGCCTA CAATAAGCATAGAGATAAGCCGATCAGAGAACAGGCCGAGAA CATTATCCACTTGTTCACCCTGACTAACCTGGGAGCTCCAGCC GCCTTCAAGTACTTCGATACTACTATCGACCGCAAAAGATACA CGTCCACCAAGGAAGTTCTGGACGCGACCCTGATCCACCAAA GCATCACTGGACTCTACGAAACTAGGATCGATCTGTCGCAGCT GGGTGGCGATGGTGGCGGTGGATCCTACCCATACGACGTGCCT GACTACGCCTCCGGAGGTGGTGGCCCCAAGAAGAAACGGAAG GTGTGATAGCTAGCCATCACATTTAAAAGCATCTCAGCCTACC ATGAGAATAAGAGAAAGAAAATGAAGATCAATAGCTTATTCA TCTCTTTTTCTTTTTCGTTGGTGTAAAGCCAACACCCTGTCTAA AAAACATAAATTTCTTTAATCATTTTGCCTCTTTTCTCTGTGCT TCAATTAATAAAAAATGGAAAGAACCTCGAG | |
| Cas9 transcript comprising Cas9 ORF using codons with generally high expression in humans | GGGTCCCGCAGTCGGCGTCCAGCGGCTCTGCTTGTTCGTGTGT GTGTCGTTGCAGGCCTTATTCGGATCCATGCCTAAGAAAAAGC GGAAGGTCGACGGGGATAAGAAGTACTCAATCGGGCTGGATA TCGGAACTAATTCCGTGGGTTGGGCAGTGATCACGGATGAATA CAAAGTGCCGTCCAAGAAGTTCAAGGTCCTGGGGAACACCGA TAGACACAGCATCAAGAAAAATCTCATCGGAGCCCTGCTGTTT GACTCCGGCGAAACCGCAGAAGCGACCCGGCTCAAACGTACC GCGAGGCGACGCTACACCCGGCGGAAGAATCGCATCTGCTAT CTGCAAGAGATCTTTTCGAACGAAATGGCAAAGGTCGACGAC AGCTTCTTCCACCGCCTGGAAGAATCTTTCCTGGTGGAGGAGG ACAAGAAGCATGAACGGCATCCTATCTTTGGAAACATCGTCGA CGAAGTGGCGTACCACGAAAAGTACCCGACCATCTACCATCTG CGGAAGAAGTTGGTTGACTCAACTGACAAGGCCGACCTCAGA TTGATCTACTTGGCCCTCGCCCATATGATCAAATTCCGCGGAC ACTTCCTGATCGAAGGCGATCTGAACCCTGATAACTCCGACGT GGATAAGCTTTTCATTCAACTGGTGCAGACCTACAACCAACTG TTCGAAGAAAACCCAATCAATGCTAGCGGCGTCGATGCCAAG GCCATCCTGTCCGCCCGGCTGTCGAAGTCGCGGCGCCTCGAAA ACCTGATCGCACAGCTGCCGGGAGAGAAAAAGAACGGACTTT TCGGCAACTTGATCGCTCTCTCACTGGGACTCACTCCCAATTTC AAGTCCAATTTTGACCTGGCCGAGGACGCGAAGCTGCAACTCT CAAAGGACACCTACGACGACGACTTGGACAATTTGCTGGCAC AAATTGGCGATCAGTACGCGGATCTGTTCCTTGCCGCTAAGAA CCTTTCGGACGCAATCTTGCTGTCCGATATCCTGCGCGTGAAC ACCGAAATAACCAAAGCGCCGCTTAGCGCCTCGATGATTAAG CGGTACGACGAGCATCACCAGGATCTCACGCTGCTCAAAGCG CTCGTGAGACAGCAACTGCCTGAAAAGTACAAGGAGATCTTCT TCGACCAGTCCAAGAATGGGTACGCAGGGTACATCGATGGAG GCGCTAGCCAGGAAGAGTTCTATAAGTTCATCAAGCCAATCCT GGAAAAGATGGACGGAACCGAAGAACTGCTGGTCAAGCTGAA CAGGGAGGATCTGCTCCGGAAACAGAGAACCTTTGACAACGG ATCCATTCCCCACCAGATCCATCTGGGTGAGCTGCACGCCATC TTGCGGCGCCAGGAGGACTTTTACCCATTCCTCAAGGACAACC GGGAAAAGATCGAGAAAATTCTGACGTTCCGCATCCCGTATTA CGTGGGCCCACTGGCGCGCGGCAATTCGCGCTTCGCGTGGATG ACTAGAAAATCAGAGGAAACCATCACTCCTTGGAATTTCGAG GAAGTTGTGGATAAGGGAGCTTCGGCACAAAGCTTCATCGAA CGAATGACCAACTTCGACAAGAATCTCCCAAACGAGAAGGTG CTTCCTAAGCACAGCCTCCTTTACGAATACTTCACTGTCTACAA CGAACTGACTAAAGTGAAATACGTTACTGAAGGAATGAGGAA GCCGGCCTTTCTGTCCGGAGAACAGAAGAAAGCAATTGTCGAT CTGCTGTTCAAGACCAACCGCAAGGTGACCGTCAAGCAGCTTA AAGAGGACTACTTCAAGAAGATCGAGTGTTTCGACTCAGTGG AAATCAGCGGGGTGGAGGACAGATTCAACGCTTCGCTGGGAA CCTATCATGATCTCCTGAAGATCATCAAGGACAAGGACTTCCT TGACAACGAGGAGAACGAGGACATCCTGGAAGATATCGTCCT GACCTTGACCCTTTTCGAGGATCGCGAGATGATCGAGGAGAG GCTTAAGACCTACGCTCATCTCTTCGACGATAAGGTCATGAAA CAACTCAAGCGCCGCCGGTACACTGGTTGGGGCCGCCTCTCCC GCAAGCTGATCAACGGTATTCGCGATAAACAGAGCGGTAAAA CTATCCTGGATTTCCTCAAATCGGATGGCTTCGCTAATCGTAA CTTCATGCAATTGATCCACGACGACAGCCTGACCTTTAAGGAG GACATCCAAAAAGCACAAGTGTCCGGACAGGGAGACTCACTC CATGAACACATCGCGAATCTGGCCGGTTCGCCGGCGATTAAGA AGGGAATTCTGCAAACTGTGAAGGTGGTCGACGAGCTGGTGA AGGTCATGGGACGGCACAAACCGGAGAATATCGTGATTGAAA TGGCCCGAGAAAACCAGACTACCCAGAAGGGCCAGAAAAACT CCCGCGAAAGGATGAAGCGGATCGAAGAAGGAATCAAGGAG CTGGGCAGCCAGATCCTGAAAGAGCACCCGGTGGAAAACACG CAGCTGCAGAACGAGAAGCTCTACCTGTACTATTTGCAAAATG | **48** |
| | GACGGGACATGTACGTGGACCAAGAGCTGGACATCAATCGGT TGTCTGATTACGACGTGGACCACATCGTTCCACAGTCCTTTCTG AAGGATGACTCGATCGATAACAAGGTGTTGACTCGCAGCGAC AAGAACAGAGGGAAGTCAGATAATGTGCCATCGGAGGAGGTC GTGAAGAAGATGAAGAATTACTGGCGGCAGCTCCTGAATGCG AAGCTGATTACCCAGAGAAAGTTTGACAATCTCACTAAAGCCG AGCGCGGCGGACTCTCAGAGCTGGATAAGGCTGGATTCATCA AACGGCAGCTGGTCGAGACTCGGCAGATTACCAAGCACGTGG CGCAGATCTTGGACTCCCGCATGAACACTAAATACGACGAGA ACGATAAGCTCATCCGGGAAGTGAAGGTGATTACCCTGAAAA GCAAACTTGTGTCGGACTTTCGGAAGGACTTTCAGTTTTACAA AGTGAGAGAAATCAACAACTACCATCACGCGCATGACGCATA CCTCAACGCTGTGGTCGGTACCGCCCTGATCAAAAAGTACCCT AAACTTGAATCGGAGTTTGTGTACGGAGACTACAAGGTCTACG ACGTGAGGAAGATGATAGCCAAGTCCGAACAGGAAATCGGGA AAGCAACTGCGAAATACTTCTTTTACTCAAACATCATGAACTT TTTCAAGACTGAAATTACGCTGGCCAATGGAGAAATCAGGAA GAGGCCACTGATCGAAACTAACGGAGAAACGGGCGAAATCGT GTGGGACAAGGGCAGGGACTTCGCAACTGTTCGCAAAGTGCT CTCTATGCCGCAAGTCAATATTGTGAAGAAAACCGAAGTGCA AACCGGCGGATTTTCAAAGGAATCGATCCTCCCAAAGAGAAA TAGCGACAAGCTCATTGCACGCAAGAAAGACTGGGACCCGAA GAAGTACGGAGGATTCGATTCGCCGACTGTCGCATACTCCGTC CTCGTGGTGGCCAAGGTGGAGAAGGGAAAGAGCAAAAAGCTC AAATCCGTCAAAGAGCTGCTGGGGATTACCATCATGGAACGA TCCTCGTTCGAGAAGAACCCGATTGATTTCCTCGAGGCGAAGG GTTACAAGGAGGTGAAGAAGGATCTGATCATCAAACTCCCCA AGTACTCACTGTTCGAACTGGAAAATGGTCGGAAGCGCATGCT GGCTTCGGCCGGAGAACTCCAAAAAGGAAATGAGCTGGCCTT GCCTAGCAAGTACGTCAACTTCCTCTATCTTGCTTCGCACTACG AAAAACTCAAAGGGTCACCGGAAGATAACGAACAGAAGCAGC TTTTCGTGGAGCAGCACAAGCATTATCTGGATGAAATCATCGA ACAAATCTCCGAGTTTTCAAAGCGCGTGATCCTCGCCGACGCC AACCTCGACAAAGTCCTGTCGGCCTACAATAAGCATAGAGAT AAGCCGATCAGAGAACAGGCCGAGAACATTATCCACTTGTTC ACCCTGACTAACCTGGGAGCCCCAGCCGCCTTCAAGTACTTCG ATACTACTATCGATCGCAAAAGATACACGTCCACCAAGGAAG TTCTGGACGCGACCCTGATCCACCAAAGCATCACTGGACTCTA CGAAACTAGGATCGATCTGTCGCAGCTGGGTGGCGATTGATAG TCTAGCCATCACATTTAAAAGCATCTCAGCCTACCATGAGAAT AAGAGAAAGAAAATGAAGATCAATAGCTTATTCATCTCTTTTT CTTTTTCGTTGGTGTAAAGCCAACACCCTGTCTAAAAAACATA AATTTCTTTAATCATTTTGCCTCTTTTCTCTGTGCTTCAATTAAT AAAAAATGGAAAGAACCTCGAG | |
| **Cas9 transcript comprising Kozak sequence with Cas9 ORF using codons with generally high expression in humans** | GGGTCCCGCAGTCGGCGTCCAGCGGCTCTGCTTGTTCGTGTGT GTGTCGTTGCAGGCCTTATTCGGATCCGCCACCATGCCTAAGA AAAAGCGGAAGGTCGACGGGGATAAGAAGTACTCAATCGGGC TGGATATCGGAACTAATTCCGTGGGTTGGGCAGTGATCACGGA TGAATACAAAGTGCCGTCCAAGAAGTTCAAGGTCCTGGGGAA CACCGATAGACACAGCATCAAGAAAAATCTCATCGGAGCCCT GCTGTTTGACTCCGGCGAAACCGCAGAAGCGACCCGGCTCAA ACGTACCGCGAGGCGACGCTACACCCGGCGGAAGAATCGCAT CTGCTATCTGCAAGAGATCTTTTCGAACGAAATGGCAAAGGTC GACGACAGCTTCTTCCACCGCCTGGAAGAATCTTTCCTGGTGG AGGAGGACAAGAAGCATGAACGGCATCCTATCTTTGGAAACA TCGTCGACGAAGTGGCGTACCACGAAAAGTACCCGACCATCT ACCATCTGCGGAAGAAGTTGGTTGACTCAACTGACAAGGCCG ACCTCAGATTGATCTACTTGGCCCTCGCCCATATGATCAAATT CCGCGGACACTTCCTGATCGAAGGCGATCTGAACCCTGATAAC | **49** |
| | TCCGACGTGGATAAGCTTTTCATTCAACTGGTGCAGACCTACA ACCAACTGTTCGAAGAAAACCCAATCAATGCTAGCGGCGTCG ATGCCAAGGCCATCCTGTCCGCCCGGCTGTCGAAGTCGCGGCG CCTCGAAAACCTGATCGCACAGCTGCCGGGAGAGAAAAAGAA CGGACTTTTCGGCAACTTGATCGCTCTCTCACTGGGACTCACTC CCAATTTCAAGTCCAATTTTGACCTGGCCGAGGACGCGAAGCT GCAACTCTCAAAGGACACCTACGACGACGACTTGGACAATTTG CTGGCACAAATTGGCGATCAGTACGCGGATCTGTTCCTTGCCG CTAAGAACCTTTCGGACGCAATCTTGCTGTCCGATATCCTGCG CGTGAACACCGAAATAACCAAAGCGCCGCTTAGCGCCTCGAT GATTAAGCGGTACGACGAGCATCACCAGGATCTCACGCTGCTC AAAGCGCTCGTGAGACAGCAACTGCCTGAAAAGTACAAGGAG ATCTTCTTCGACCAGTCCAAGAATGGGTACGCAGGGTACATCG ATGGAGGCGCTAGCCAGGAAGAGTTCTATAAGTTCATCAAGC CAATCCTGGAAAAGATGGACGGAACCGAAGAACTGCTGGTCA AGCTGAACAGGGAGGATCTGCTCCGGAAACAGAGAACCTTTG ACAACGGATCCATTCCCCACCAGATCCATCTGGGTGAGCTGCA CGCCATCTTGCGGCGCCAGGAGGACTTTTACCCATTCCTCAAG GACAACCGGGAAAAGATCGAGAAAATTCTGACGTTCCGCATC CCGTATTACGTGGGCCCACTGGCGCGCGGCAATTCGCGCTTCG CGTGGATGACTAGAAAATCAGAGGAAACCATCACTCCTTGGA ATTTCGAGGAAGTTGTGGATAAGGGAGCTTCGGCACAAAGCTT CATCGAACGAATGACCAACTTCGACAAGAATCTCCCAAACGA GAAGGTGCTTCCTAAGCACAGCCTCCTTTACGAATACTTCACT GTCTACAACGAACTGACTAAAGTGAAATACGTTACTGAAGGA ATGAGGAAGCCGGCCTTTCTGTCCGGAGAACAGAAGAAAGCA ATTGTCGATCTGCTGTTCAAGACCAACCGCAAGGTGACCGTCA AGCAGCTTAAAGAGGACTACTTCAAGAAGATCGAGTGTTTCG ACTCAGTGGAAATCAGCGGGGTGGAGGACAGATTCAACGCTT CGCTGGGAACCTATCATGATCTCCTGAAGATCATCAAGGACAA GGACTTCCTTGACAACGAGGAGAACGAGGACATCCTGGAAGA TATCGTCCTGACCTTGACCCTTTTCGAGGATCGCGAGATGATC GAGGAGAGGCTTAAGACCTACGCTCATCTCTTCGACGATAAGG TCATGAAACAACTCAAGCGCCGCCGGTACACTGGTTGGGGCC GCCTCTCCCGCAAGCTGATCAACGGTATTCGCGATAAACAGAG CGGTAAAACTATCCTGGATTTCCTCAAATCGGATGGCTTCGCT AATCGTAACTTCATGCAATTGATCCACGACGACAGCCTGACCT TTAAGGAGGACATCCAAAAAGCACAAGTGTCCGGACAGGGAG ACTCACTCCATGAACACATCGCGAATCTGGCCGGTTCGCCGGC GATTAAGAAGGGAATTCTGCAAACTGTGAAGGTGGTCGACGA GCTGGTGAAGGTCATGGGACGGCACAAACCGGAGAATATCGT GATTGAAATGGCCCGAGAAAACCAGACTACCCAGAAGGGCCA GAAAAACTCCCGCGAAAGGATGAAGCGGATCGAAGAAGGAAT CAAGGAGCTGGGCAGCCAGATCCTGAAAGAGCACCCGGTGGA AAACACGCAGCTGCAGAACGAGAAGCTCTACCTGTACTATTTG CAAAATGGACGGGACATGTACGTGGACCAAGAGCTGGACATC AATCGGTTGTCTGATTACGACGTGGACCACATCGTTCCACAGT CCTTTCTGAAGGATGACTCGATCGATAACAAGGTGTTGACTCG CAGCGACAAGAACAGAGGGAAGTCAGATAATGTGCCATCGGA GGAGGTCGTGAAGAAGATGAAGAATTACTGGCGGCAGCTCCT GAATGCGAAGCTGATTACCCAGAGAAAGTTTGACAATCTCACT AAAGCCGAGCGCGGCGGACTCTCAGAGCTGGATAAGGCTGGA TTCATCAAACGGCAGCTGGTCGAGACTCGGCAGATTACCAAGC ACGTGGCGCAGATCTTGGACTCCCGCATGAACACTAAATACGA CGAGAACGATAAGCTCATCCGGGAAGTGAAGGTGATTACCCT GAAAAGCAAACTTGTGTCGGACTTTCGGAAGGACTTTCAGTTT TACAAAGTGAGAGAAATCAACAACTACCATCACGCGCATGAC GCATACCTCAACGCTGTGGTCGGTACCGCCCTGATCAAAAAGT ACCCTAAACTTGAATCGGAGTTTGTGTACGGAGACTACAAGGT CTACGACGTGAGGAAGATGATAGCCAAGTCCGAACAGGAAAT CGGGAAAGCAACTGCGAAATACTTCTTTTACTCAAACATCATG | |
| | AACTTTTTCAAGACTGAAATTACGCTGGCCAATGGAGAAATCA GGAAGAGGCCACTGATCGAAACTAACGGAGAAACGGGCGAA ATCGTGTGGGACAAGGGCAGGGACTTCGCAACTGTTCGCAAA GTGCTCTCTATGCCGCAAGTCAATATTGTGAAGAAAACCGAAG TGCAAACCGGCGGATTTTCAAAGGAATCGATCCTCCCAAAGA GAAATAGCGACAAGCTCATTGCACGCAAGAAAGACTGGGACC CGAAGAAGTACGGAGGATTCGATTCGCCGACTGTCGCATACTC CGTCCTCGTGGTGGCCAAGGTGGAGAAGGGAAAGAGCAAAAA GCTCAAATCCGTCAAAGAGCTGCTGGGGATTACCATCATGGAA CGATCCTCGTTCGAGAAGAACCCGATTGATTTCCTCGAGGCGA AGGGTTACAAGGAGGTGAAGAAGGATCTGATCATCAAACTCC CCAAGTACTCACTGTTCGAACTGGAAAATGGTCGGAAGCGCAT GCTGGCTTCGGCCGGAGAACTCCAAAAAGGAAATGAGCTGGC CTTGCCTAGCAAGTACGTCAACTTCCTCTATCTTGCTTCGCACT ACGAAAAACTCAAAGGGTCACCGGAAGATAACGAACAGAAGC AGCTTTTCGTGGAGCAGCACAAGCATTATCTGGATGAAATCAT CGAACAAATCTCCGAGTTTTCAAAGCGCGTGATCCTCGCCGAC GCCAACCTCGACAAAGTCCTGTCGGCCTACAATAAGCATAGA GATAAGCCGATCAGAGAACAGGCCGAGAACATTATCCACTTG TTCACCCTGACTAACCTGGGAGCCCCAGCCGCCTTCAAGTACT TCGATACTACTATCGATCGCAAAAGATACACGTCCACCAAGGA AGTTCTGGACGCGACCCTGATCCACCAAAGCATCACTGGACTC TACGAAACTAGGATCGATCTGTCGCAGCTGGGTGGCGATTGAT AGTCTAGCCATCACATTTAAAAGCATCTCAGCCTACCATGAGA ATAAGAGAAAGAAAATGAAGATCAATAGCTTATTCATCTCTTT TTCTTTTTCGTTGGTGTAAAGCCAACACCCTGTCTAAAAAACA TAAATTTCTTTAATCATTTTGCCTCTTTTCTCTGTGCTTCAATTA ATAAAAAATGGAAAGAACCTCGAG | |
| **Cas9 ORF with splice junctions removed; 12.75% U content** | ATGGACAAGAAGTACAGCATCGGACTGGACATCGGAACAAAC AGCGTCGGATGGGCAGTCATCACAGACGAATACAAGGTCCCG AGCAAGAAGTTCAAGGTCCTGGGAAACACAGACAGACACAGC ATCAAGAAGAACCTGATCGGAGCACTGCTGTTCGACAGCGGA GAAACAGCAGAAGCAACAAGACTGAAGAGAACAGCAAGAAG AAGATACACAAGAAGAAAGAACAGAATCTGCTACCTGCAGGA AATCTTCAGCAACGAAATGGCAAAGGTCGACGACAGCTTCTTC CACcggCTGGAAGAAAGCTTCCTGGTCGAAGAAGACAAGAAGC ACGAAAGACACCCGATCTTCGGAAACATCGTCGACGAAGTCG CATACCACGAAAAGTACCCGACAATCTACCACCTGAGAAAGA AGCTGGTCGACAGCACAGACAAGGCAGACCTGAGACTGATCT ACCTGGCACTGGCACACATGATCAAGTTCAGAGGACACTTCCT GATCGAAGGAGACCTGAACCCGGACAACAGCGACGTCGACAA GCTGTTCATCCAGCTGGTCCAGACATACAACCAGCTGTTCGAA GAAAACCCGATCAACGCAAGCGGAGTCGACGCAAAGGCAATC CTGAGCGCAAGACTGAGCAAGAGCAGAAGACTGGAAAACCTG ATCGCACAGCTGCCGGGAGAAAAGAAGAACGGACTGTTCGGA AACCTGATCGCACTGAGCCTGGGACTGACACCGAACTTCAAG AGCAACTTCGACCTGGCAGAAGACGCAAAGCTGCAGCTGAGC AAGGACACATACGACGACGACCTGGACAACCTGCTGGCACAG ATCGGAGACCAGTACGCAGACCTGTTCCTGGCAGCAAAGAAC CTGAGCGACGCAATCCTGCTGAGCGACATCCTGAGAGTCAAC ACAGAAATCACAAAGGCACCGCTGAGCGCAAGCATGATCAAG AGATACGACGAACACCACCAGGACCTGACACTGCTGAAGGCA CTGGTCAGACAGCAGCTGCCGGAAAAGTACAAGGAAATCTTC TTCGACCAGAGCAAGAACGGATACGCAGGATACATCGACGGA GGAGCAAGCCAGGAAGAATTCTACAAGTTCATCAAGCCGATC CTGGAAAAGATGGACGGAACAGAAGAACTGCTGGTCAAGCTG AACAGAGAAGACCTGCTGAGAAAGCAGAGAACATTCGACAAC GGAAGCATCCCGCACCAGATCCACCTGGGAGAACTGCACGCA ATCCTGAGAAGACAGGAAGACTTCTACCCGTTCCTGAAGGAC | **50** |
| | AACAGAGAAAAGATCGAAAAGATCCTGACATTCAGAATCCCG TACTACGTCGGACCGCTGGCAAGAGGAAACAGCAGATTCGCA TGGATGACAAGAAAGAGCGAAGAAACAATCACACCGTGGAAC TTCGAAGAAGTCGTCGACAAGGGAGCAAGCGCACAGAGCTTC ATCGAAAGAATGACAAACTTCGACAAGAACCTGCCGAACGAA AAGGTCCTGCCGAAGCACAGCCTGCTGTACGAATACTTCACAG TCTACAACGAACTGACAAAGGTCAAGTACGTCACAGAAGGAA TGAGAAAGCCGGCATTCCTGAGCGGAGAACAGAAGAAGGCAA TCGTCGACCTGCTGTTCAAGACAAACAGAAAGGTCACAGTCA AGCAGCTGAAGGAAGACTACTTCAAGAAGATCGAATGCTTCG ACAGCGTCGAAATCAGCGGAGTCGAAGACAGATTCAACGCAA GCCTGGGAACATACCACGACCTGCTGAAGATCATCAAGGACA AGGACTTCCTGGACAACGAAGAAAACGAAGACATCCTGGAAG ACATCGTCCTGACACTGACACTGTTCGAAGACAGAGAAATGAT CGAAGAAAGACTGAAGACATACGCACACCTGTTCGACGACAA GGTCATGAAGCAGCTGAAGAGAAGAAGATACACAGGATGGGG AAGACTGAGCAGAAAGCTGATCAACGGAATCAGAGACAAGCA GAGCGGAAAGACAATCCTGGACTTCCTGAAGAGCGACGGATT CGCAAACAGAAACTTCATGCAGCTGATCCACGACGACAGCCT GACATTCAAGGAAGACATCCAGAAGGCACAGGTCAGCGGACA GGGAGACAGCCTGCACGAACACATCGCAAACCTGGCAGGAAG CCCGGCAATCAAGAAGGGAATCCTGCAGACAGTCAAGGTCGT CGACGAACTGGTCAAGGTCATGGGAAGACACAAGCCGGAAAA CATCGTCATCGAAATGGCAAGAGAAAACCAGACAACACAGAA GGGACAGAAGAACAGCAGAGAAAGAATGAAGAGAATCGAAG AAGGAATCAAGGAACTGGGAAGCCAGATCCTGAAGGAACACC CGGTCGAAAACACACAGCTGCAGAACGAAAAGCTGTACCTGT ACTACCTGCAaAACGGAAGAGACATGTACGTCGACCAGGAACT GGACATCAACAGACTGAGCGACTACGACGTCGACCACATCGT CCCGCAGAGCTTCCTGAAGGACGACAGCATCGACAACAAGGT CCTGACAAGAAGCGACAAGAACAGAGGAAAGAGCGACAACG TCCCGAGCGAAGAAGTCGTCAAGAAGATGAAGAACTACTGGA GACAGCTGCTGAACGCAAAGCTGATCACACAGAGAAAGTTCG ACAACCTGACAAAGGCAGAGAGAGGAGGACTGAGCGAACTG GACAAGGCAGGATTCATCAAGAGACAGCTGGTCGAAACAAGA CAGATCACAAAGCACGTCGCACAGATCCTGGACAGCAGAATG AACACAAAGTACGACGAAAACGACAAGCTGATCAGAGAAGTC AAGGTCATCACACTGAAGAGCAAGCTGGTCAGCGACTTCAGA AAGGACTTCCAGTTCTACAAGGTCAGAGAAATCAACAACTAC CACCACGCACACGACGCATACCTGAACGCAGTCGTCGGAACA GCACTGATCAAGAAGTACCCGAAGCTGGAAAGCGAATTCGTC TACGGAGACTACAAGGTCTACGACGTCAGAAAGATGATCGCA AAGAGCGAACAGGAAATCGGAAAGGCAACAGCAAAGTACTTC TTCTACAGCAACATCATGAACTTCTTCAAGACAGAAATCACAC TGGCAAACGGAGAAATCAGAAAGAGACCGCTGATCGAAACAA ACGGAGAAACAGGAGAAATCGTCTGGGACAAGGGAAGAGAC TTCGCAACAGTCAGAAAGGTCCTGAGCATGCCGCAGGTCAAC ATCGTCAAGAAGACAGAAGTCCAGACAGGAGGATTCAGCAAG GAAAGCATCCTGCCGAAGAGAAACAGCGACAAGCTGATCGCA AGAAAGAAGGACTGGGACCCGAAGAAGTACGGAGGATTCGAC AGCCCGACAGTCGCATACAGCGTCCTGGTCGTCGCAAAGGTCG AAAAGGGAAAGAGCAAGAAGCTGAAGAGCGTCAAGGAACTG CTGGGAATCACAATCATGGAAAGAAGCAGCTTCGAAAAGAAC CCGATCGACTTCCTGGAAGCAAAGGGATACAAGGAAGTCAAG AAGGACCTGATCATCAAGCTGCCGAAGTACAGCCTGTTCGAAC TGGAAAACGGAAGAAAGAGAATGCTGGCAAGCGCAGGAGAA CTGCAGAAGGGAAACGAACTGGCACTGCCGAGCAAGTACGTC AACTTCCTGTACCTGGCAAGCCACTACGAAAAGCTGAAGGGA AGCCCGGAAGACAACGAACAGAAGCAGCTGTTCGTCGAACAG CACAAGCACTACCTGGACGAAATCATCGAACAGATCAGCGAA TTCAGCAAGAGAGTCATCCTGGCAGACGCAAACCTGGACAAG | |
| | GTCCTGAGCGCATACAACAAGCACAGAGACAAGCCGATCAGA GAACAGGCAGAAAACATCATCCACCTGTTCACACTGACAAAC CTGGGAGCACCGGCAGCATTCAAGTACTTCGACACAACAATC GACAGAAAGAGATACACAAGCACAAAGGAAGTCCTGGACGCA ACACTGATCCACCAGAGCATCACAGGACTGTACGAAACAAGA ATCGACCTGAGCCAGCTGGGAGGAGACGGAGGAGGAAGCCCG AAGAAGAAGAGAAAGGTCTAG | |
| **Cas9 transcript with 5' UTR of HSD, ORF correspondi ng to SEQ ID NO: 50, Kozak sequence, and 3' UTR of ALB** | GGGTCCCGCAGTCGGCGTCCAGCGGCTCTGCTTGTTCGTGTGT GTGTCGTTGCAGGCCTTATTCGGATCCGCCACCATGGACAAGA AGTACAGCATCGGACTGGACATCGGAACAAACAGCGTCGGAT GGGCAGTCATCACAGACGAATACAAGGTCCCGAGCAAGAAGT TCAAGGTCCTGGGAAACACAGACAGACACAGCATCAAGAAGA ACCTGATCGGAGCACTGCTGTTCGACAGCGGAGAAACAGCAG AAGCAACAAGACTGAAGAGAACAGCAAGAAGAAGATACACA AGAAGAAAGAACAGAATCTGCTACCTGCAGGAAATCTTCAGC AACGAAATGGCAAAGGTCGACGACAGCTTCTTCCACcggCTGG AAGAAAGCTTCCTGGTCGAAGAAGACAAGAAGCACGAAAGAC ACCCGATCTTCGGAAACATCGTCGACGAAGTCGCATACCACGA AAAGTACCCGACAATCTACCACCTGAGAAAGAAGCTGGTCGA CAGCACAGACAAGGCAGACCTGAGACTGATCTACCTGGCACT GGCACACATGATCAAGTTCAGAGGACACTTCCTGATCGAAGG AGACCTGAACCCGGACAACAGCGACGTCGACAAGCTGTTCAT CCAGCTGGTCCAGACATACAACCAGCTGTTCGAAGAAAACCC GATCAACGCAAGCGGAGTCGACGCAAAGGCAATCCTGAGCGC AAGACTGAGCAAGAGCAGAAGACTGGAAAACCTGATCGCACA GCTGCCGGGAGAAAAGAAGAACGGACTGTTCGGAAACCTGAT CGCACTGAGCCTGGGACTGACACCGAACTTCAAGAGCAACTTC GACCTGGCAGAAGACGCAAAGCTGCAGCTGAGCAAGGACACA TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA | **51** |
| | GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAaAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG ACCTGAGCCAGCTGGGAGGAGACGGAGGAGGAAGCCCGAAG AAGAAGAGAAAGGTCTAGCTAGCCATCACATTTAAAAGCATC TCAGCCTACCATGAGAATAAGAGAAAGAAAATGAAGATCAAT AGCTTATTCATCTCTTTTTCTTTTTCGTTGGTGTAAAGCCAACA CCCTGTCTAAAAAACATAAATTTCTTTAATCATTTTGCCTCTTT TCTCTGTGCTTCAATTAATAAAAAATGGAAAGAACCTCGAG | |
| **Cas9 ORF with minimal uridine codons frequently** | ATGGACAAGAAGTACAGCATCGGCCTGGACATCGGCACCAAC AGCGTGGGCTGGGCCGTGATCACCGACGAGTACAAGGTGCCC AGCAAGAAGTTCAAGGTGCTGGGCAACACCGACAGACACAGC ATCAAGAAGAACCTGATCGGCGCCCTGCTGTTCGACAGCGGC GAGACCGCCGAGGCCACCAGACTGAAGAGAACCGCCAGAAGA AGATACACCAGAAGAAAGAACAGAATCTGCTACCTGCAGGAG | **52** |
| **used in humans in general; 12.75% U content** | ATCTTCAGCAACGAGATGGCCAAGGTGGACGACAGCTTCTTCC ACAGACTGGAGGAGAGCTTCCTGGTGGAGGAGGACAAGAAGC ACGAGAGACACCCCATCTTCGGCAACATCGTGGACGAGGTGG CCTACCACGAGAAGTACCCCACCATCTACCACCTGAGAAAGA AGCTGGTGGACAGCACCGACAAGGCCGACCTGAGACTGATCT ACCTGGCCCTGGCCCACATGATCAAGTTCAGAGGCCACTTCCT GATCGAGGGCGACCTGAACCCCGACAACAGCGACGTGGACAA GCTGTTCATCCAGCTGGTGCAGACCTACAACCAGCTGTTCGAG GAGAACCCCATCAACGCCAGCGGCGTGGACGCCAAGGCCATC CTGAGCGCCAGACTGAGCAAGAGCAGAAGACTGGAGAACCTG ATCGCCCAGCTGCCCGGCGAGAAGAAGAACGGCCTGTTCGGC AACCTGATCGCCCTGAGCCTGGGCCTGACCCCCAACTTCAAGA GCAACTTCGACCTGGCCGAGGACGCCAAGCTGCAGCTGAGCA AGGACACCTACGACGACGACCTGGACAACCTGCTGGCCCAGA TCGGCGACCAGTACGCCGACCTGTTCCTGGCCGCCAAGAACCT GAGCGACGCCATCCTGCTGAGCGACATCCTGAGAGTGAACAC CGAGATCACCAAGGCCCCCCTGAGCGCCAGCATGATCAAGAG ATACGACGAGCACCACCAGGACCTGACCCTGCTGAAGGCCCT GGTGAGACAGCAGCTGCCCGAGAAGTACAAGGAGATCTTCTT CGACCAGAGCAAGAACGGCTACGCCGGCTACATCGACGGCGG CGCCAGCCAGGAGGAGTTCTACAAGTTCATCAAGCCCATCCTG GAGAAGATGGACGGCACCGAGGAGCTGCTGGTGAAGCTGAAC AGAGAGGACCTGCTGAGAAAGCAGAGAACCTTCGACAACGGC AGCATCCCCCACCAGATCCACCTGGGCGAGCTGCACGCCATCC TGAGAAGACAGGAGGACTTCTACCCCTTCCTGAAGGACAACA GAGAGAAGATCGAGAAGATCCTGACCTTCAGAATCCCCTACT ACGTGGGCCCCCTGGCCAGAGGCAACAGCAGATTCGCCTGGA TGACCAGAAAGAGCGAGGAGACCATCACCCCCTGGAACTTCG AGGAGGTGGTGGACAAGGGCGCCAGCGCCCAGAGCTTCATCG AGAGAATGACCAACTTCGACAAGAACCTGCCCAACGAGAAGG TGCTGCCCAAGCACAGCCTGCTGTACGAGTACTTCACCGTGTA CAACGAGCTGACCAAGGTGAAGTACGTGACCGAGGGCATGAG AAAGCCCGCCTTCCTGAGCGGCGAGCAGAAGAAGGCCATCGT GGACCTGCTGTTCAAGACCAACAGAAAGGTGACCGTGAAGCA GCTGAAGGAGGACTACTTCAAGAAGATCGAGTGCTTCGACAG CGTGGAGATCAGCGGCGTGGAGGACAGATTCAACGCCAGCCT GGGCACCTACCACGACCTGCTGAAGATCATCAAGGACAAGGA CTTCCTGGACAACGAGGAGAACGAGGACATCCTGGAGGACAT CGTGCTGACCCTGACCCTGTTCGAGGACAGAGAGATGATCGA GGAGAGACTGAAGACCTACGCCCACCTGTTCGACGACAAGGT GATGAAGCAGCTGAAGAGAAGAAGATACACCGGCTGGGGCAG ACTGAGCAGAAAGCTGATCAACGGCATCAGAGACAAGCAGAG CGGCAAGACCATCCTGGACTTCCTGAAGAGCGACGGCTTCGCC AACAGAAACTTCATGCAGCTGATCCACGACGACAGCCTGACCT TCAAGGAGGACATCCAGAAGGCCCAGGTGAGCGGCCAGGGCG ACAGCCTGCACGAGCACATCGCCAACCTGGCCGGCAGCCCCG CCATCAAGAAGGGCATCCTGCAGACCGTGAAGGTGGTGGACG AGCTGGTGAAGGTGATGGGCAGACACAAGCCCGAGAACATCG TGATCGAGATGGCCAGAGAGAACCAGACCACCCAGAAGGGCC AGAAGAACAGCAGAGAGAGAATGAAGAGAATCGAGGAGGGC ATCAAGGAGCTGGGCAGCCAGATCCTGAAGGAGCACCCCGTG GAGAACACCCAGCTGCAGAACGAGAAGCTGTACCTGTACTAC CTGCAGAACGGCAGAGACATGTACGTGGACCAGGAGCTGGAC ATCAACAGACTGAGCGACTACGACGTGGACCACATCGTGCCC CAGAGCTTCCTGAAGGACGACAGCATCGACAACAAGGTGCTG ACCAGAAGCGACAAGAACAGAGGCAAGAGCGACAACGTGCC CAGCGAGGAGGTGGTGAAGAAGATGAAGAACTACTGGAGACA GCTGCTGAACGCCAAGCTGATCACCCAGAGAAAGTTCGACAA CCTGACCAAGGCCGAGAGAGGCGGCCTGAGCGAGCTGGACAA GGCCGGCTTCATCAAGAGACAGCTGGTGGAGACCAGACAGAT CACCAAGCACGTGGCCCAGATCCTGGACAGCAGAATGAACAC | |
| | CAAGTACGACGAGAACGACAAGCTGATCAGAGAGGTGAAGGT GATCACCCTGAAGAGCAAGCTGGTGAGCGACTTCAGAAAGGA CTTCCAGTTCTACAAGGTGAGAGAGATCAACAACTACCACCAC GCCCACGACGCCTACCTGAACGCCGTGGTGGGCACCGCCCTGA TCAAGAAGTACCCCAAGCTGGAGAGCGAGTTCGTGTACGGCG ACTACAAGGTGTACGACGTGAGAAAGATGATCGCCAAGAGCG AGCAGGAGATCGGCAAGGCCACCGCCAAGTACTTCTTCTACA GCAACATCATGAACTTCTTCAAGACCGAGATCACCCTGGCCAA CGGCGAGATCAGAAAGAGACCCCTGATCGAGACCAACGGCGA GACCGGCGAGATCGTGTGGGACAAGGGCAGAGACTTCGCCAC CGTGAGAAAGGTGCTGAGCATGCCCCAGGTGAACATCGTGAA GAAGACCGAGGTGCAGACCGGCGGCTTCAGCAAGGAGAGCAT CCTGCCCAAGAGAAACAGCGACAAGCTGATCGCCAGAAAGAA GGACTGGGACCCCAAGAAGTACGGCGGCTTCGACAGCCCCAC CGTGGCCTACAGCGTGCTGGTGGTGGCCAAGGTGGAGAAGGG CAAGAGCAAGAAGCTGAAGAGCGTGAAGGAGCTGCTGGGCAT CACCATCATGGAGAGAAGCAGCTTCGAGAAGAACCCCATCGA CTTCCTGGAGGCCAAGGGCTACAAGGAGGTGAAGAAGGACCT GATCATCAAGCTGCCCAAGTACAGCCTGTTCGAGCTGGAGAAC GGCAGAAAGAGAATGCTGGCCAGCGCCGGCGAGCTGCAGAAG GGCAACGAGCTGGCCCTGCCCAGCAAGTACGTGAACTTCCTGT ACCTGGCCAGCCACTACGAGAAGCTGAAGGGCAGCCCCGAGG ACAACGAGCAGAAGCAGCTGTTCGTGGAGCAGCACAAGCACT ACCTGGACGAGATCATCGAGCAGATCAGCGAGTTCAGCAAGA GAGTGATCCTGGCCGACGCCAACCTGGACAAGGTGCTGAGCG CCTACAACAAGCACAGAGACAAGCCCATCAGAGAGCAGGCCG AGAACATCATCCACCTGTTCACCCTGACCAACCTGGGCGCCCC CGCCGCCTTCAAGTACTTCGACACCACCATCGACAGAAAGAG ATACACCAGCACCAAGGAGGTGCTGGACGCCACCCTGATCCA CCAGAGCATCACCGGCCTGTACGAGACCAGAATCGACCTGAG CCAGCTGGGCGGCGACGGCGGCGGCAGCCCCAAGAAGAAGAG AAAGGTGTGA | |
| **Cas9 transcript with 5' UTR of HSD, ORF correspondi ng to SEQ ID NO: 52, Kozak sequence, and 3' UTR of ALB** | GGGTCCCGCAGTCGGCGTCCAGCGGCTCTGCTTGTTCGTGTGT GTGTCGTTGCAGGCCTTATTCGGATCCGCCACCATGGACAAGA AGTACAGCATCGGCCTGGACATCGGCACCAACAGCGTGGGCT GGGCCGTGATCACCGACGAGTACAAGGTGCCCAGCAAGAAGT TCAAGGTGCTGGGCAACACCGACAGACACAGCATCAAGAAGA ACCTGATCGGCGCCCTGCTGTTCGACAGCGGCGAGACCGCCGA GGCCACCAGACTGAAGAGAACCGCCAGAAGAAGATACACCAG AAGAAAGAACAGAATCTGCTACCTGCAGGAGATCTTCAGCAA CGAGATGGCCAAGGTGGACGACAGCTTCTTCCACAGACTGGA GGAGAGCTTCCTGGTGGAGGAGGACAAGAAGCACGAGAGACA CCCCATCTTCGGCAACATCGTGGACGAGGTGGCCTACCACGAG AAGTACCCCACCATCTACCACCTGAGAAAGAAGCTGGTGGAC AGCACCGACAAGGCCGACCTGAGACTGATCTACCTGGCCCTG GCCCACATGATCAAGTTCAGAGGCCACTTCCTGATCGAGGGCG ACCTGAACCCCGACAACAGCGACGTGGACAAGCTGTTCATCC AGCTGGTGCAGACCTACAACCAGCTGTTCGAGGAGAACCCCA TCAACGCCAGCGGCGTGGACGCCAAGGCCATCCTGAGCGCCA GACTGAGCAAGAGCAGAAGACTGGAGAACCTGATCGCCCAGC TGCCCGGCGAGAAGAAGAACGGCCTGTTCGGCAACCTGATCG CCCTGAGCCTGGGCCTGACCCCCAACTTCAAGAGCAACTTCGA CCTGGCCGAGGACGCCAAGCTGCAGCTGAGCAAGGACACCTA CGACGACGACCTGGACAACCTGCTGGCCCAGATCGGCGACCA GTACGCCGACCTGTTCCTGGCCGCCAAGAACCTGAGCGACGCC ATCCTGCTGAGCGACATCCTGAGAGTGAACACCGAGATCACC AAGGCCCCCCTGAGCGCCAGCATGATCAAGAGATACGACGAG CACCACCAGGACCTGACCCTGCTGAAGGCCCTGGTGAGACAG CAGCTGCCCGAGAAGTACAAGGAGATCTTCTTCGACCAGAGC | **53** |
| | AAGAACGGCTACGCCGGCTACATCGACGGCGGCGCCAGCCAG GAGGAGTTCTACAAGTTCATCAAGCCCATCCTGGAGAAGATG GACGGCACCGAGGAGCTGCTGGTGAAGCTGAACAGAGAGGAC CTGCTGAGAAAGCAGAGAACCTTCGACAACGGCAGCATCCCC CACCAGATCCACCTGGGCGAGCTGCACGCCATCCTGAGAAGA CAGGAGGACTTCTACCCCTTCCTGAAGGACAACAGAGAGAAG ATCGAGAAGATCCTGACCTTCAGAATCCCCTACTACGTGGGCC CCCTGGCCAGAGGCAACAGCAGATTCGCCTGGATGACCAGAA AGAGCGAGGAGACCATCACCCCCTGGAACTTCGAGGAGGTGG TGGACAAGGGCGCCAGCGCCCAGAGCTTCATCGAGAGAATGA CCAACTTCGACAAGAACCTGCCCAACGAGAAGGTGCTGCCCA AGCACAGCCTGCTGTACGAGTACTTCACCGTGTACAACGAGCT GACCAAGGTGAAGTACGTGACCGAGGGCATGAGAAAGCCCGC CTTCCTGAGCGGCGAGCAGAAGAAGGCCATCGTGGACCTGCT GTTCAAGACCAACAGAAAGGTGACCGTGAAGCAGCTGAAGGA GGACTACTTCAAGAAGATCGAGTGCTTCGACAGCGTGGAGAT CAGCGGCGTGGAGGACAGATTCAACGCCAGCCTGGGCACCTA CCACGACCTGCTGAAGATCATCAAGGACAAGGACTTCCTGGA CAACGAGGAGAACGAGGACATCCTGGAGGACATCGTGCTGAC CCTGACCCTGTTCGAGGACAGAGAGATGATCGAGGAGAGACT GAAGACCTACGCCCACCTGTTCGACGACAAGGTGATGAAGCA GCTGAAGAGAAGAAGATACACCGGCTGGGGCAGACTGAGCAG AAAGCTGATCAACGGCATCAGAGACAAGCAGAGCGGCAAGAC CATCCTGGACTTCCTGAAGAGCGACGGCTTCGCCAACAGAAAC TTCATGCAGCTGATCCACGACGACAGCCTGACCTTCAAGGAGG ACATCCAGAAGGCCCAGGTGAGCGGCCAGGGCGACAGCCTGC ACGAGCACATCGCCAACCTGGCCGGCAGCCCCGCCATCAAGA AGGGCATCCTGCAGACCGTGAAGGTGGTGGACGAGCTGGTGA AGGTGATGGGCAGACACAAGCCCGAGAACATCGTGATCGAGA TGGCCAGAGAGAACCAGACCACCCAGAAGGGCCAGAAGAAC AGCAGAGAGAGAATGAAGAGAATCGAGGAGGGCATCAAGGA GCTGGGCAGCCAGATCCTGAAGGAGCACCCCGTGGAGAACAC CCAGCTGCAGAACGAGAAGCTGTACCTGTACTACCTGCAGAA CGGCAGAGACATGTACGTGGACCAGGAGCTGGACATCAACAG ACTGAGCGACTACGACGTGGACCACATCGTGCCCCAGAGCTTC CTGAAGGACGACAGCATCGACAACAAGGTGCTGACCAGAAGC GACAAGAACAGAGGCAAGAGCGACAACGTGCCCAGCGAGGA GGTGGTGAAGAAGATGAAGAACTACTGGAGACAGCTGCTGAA CGCCAAGCTGATCACCCAGAGAAAGTTCGACAACCTGACCAA GGCCGAGAGAGGCGGCCTGAGCGAGCTGGACAAGGCCGGCTT CATCAAGAGACAGCTGGTGGAGACCAGACAGATCACCAAGCA CGTGGCCCAGATCCTGGACAGCAGAATGAACACCAAGTACGA CGAGAACGACAAGCTGATCAGAGAGGTGAAGGTGATCACCCT GAAGAGCAAGCTGGTGAGCGACTTCAGAAAGGACTTCCAGTT CTACAAGGTGAGAGAGATCAACAACTACCACCACGCCCACGA CGCCTACCTGAACGCCGTGGTGGGCACCGCCCTGATCAAGAA GTACCCCAAGCTGGAGAGCGAGTTCGTGTACGGCGACTACAA GGTGTACGACGTGAGAAAGATGATCGCCAAGAGCGAGCAGGA GATCGGCAAGGCCACCGCCAAGTACTTCTTCTACAGCAACATC ATGAACTTCTTCAAGACCGAGATCACCCTGGCCAACGGCGAG ATCAGAAAGAGACCCCTGATCGAGACCAACGGCGAGACCGGC GAGATCGTGTGGGACAAGGGCAGAGACTTCGCCACCGTGAGA AAGGTGCTGAGCATGCCCCAGGTGAACATCGTGAAGAAGACC GAGGTGCAGACCGGCGGCTTCAGCAAGGAGAGCATCCTGCCC AAGAGAAACAGCGACAAGCTGATCGCCAGAAAGAAGGACTG GGACCCCAAGAAGTACGGCGGCTTCGACAGCCCCACCGTGGC CTACAGCGTGCTGGTGGTGGCCAAGGTGGAGAAGGGCAAGAG CAAGAAGCTGAAGAGCGTGAAGGAGCTGCTGGGCATCACCAT CATGGAGAGAAGCAGCTTCGAGAAGAACCCCATCGACTTCCT GGAGGCCAAGGGCTACAAGGAGGTGAAGAAGGACCTGATCAT CAAGCTGCCCAAGTACAGCCTGTTCGAGCTGGAGAACGGCAG | |
| | AAAGAGAATGCTGGCCAGCGCCGGCGAGCTGCAGAAGGGCAA CGAGCTGGCCCTGCCCAGCAAGTACGTGAACTTCCTGTACCTG GCCAGCCACTACGAGAAGCTGAAGGGCAGCCCCGAGGACAAC GAGCAGAAGCAGCTGTTCGTGGAGCAGCACAAGCACTACCTG GACGAGATCATCGAGCAGATCAGCGAGTTCAGCAAGAGAGTG ATCCTGGCCGACGCCAACCTGGACAAGGTGCTGAGCGCCTAC AACAAGCACAGAGACAAGCCCATCAGAGAGCAGGCCGAGAA CATCATCCACCTGTTCACCCTGACCAACCTGGGCGCCCCCGCC GCCTTCAAGTACTTCGACACCACCATCGACAGAAAGAGATAC ACCAGCACCAAGGAGGTGCTGGACGCCACCCTGATCCACCAG AGCATCACCGGCCTGTACGAGACCAGAATCGACCTGAGCCAG CTGGGCGGCGACGGCGGCGGCAGCCCCAAGAAGAAGAGAAA GGTGTGACTAGCCATCACATTTAAAAGCATCTCAGCCTACCAT GAGAATAAGAGAAAGAAAATGAAGATCAATAGCTTATTCATC TCTTTTTCTTTTTCGTTGGTGTAAAGCCAACACCCTGTCTAAAA AACATAAATTTCTTTAATCATTTTGCCTCTTTTCTCTGTGCTTCA ATTAATAAAAAATGGAAAGAACCTCGAG | |
| **Cas9 ORF with minimal uridine codons infrequently used in humans in general; 12.75% U content** | ATGGACAAAAAATACAGCATAGGGCTAGACATAGGGACGAAC AGCGTAGGGTGGGCGGTAATAACGGACGAATACAAAGTACCG AGCAAAAAATTCAAAGTACTAGGGAACACGGACCGACACAGC ATAAAAAAAAACCTAATAGGGGCGCTACTATTCGACAGCGGG GAAACGGCGGAAGCGACGCGACTAAAACGAACGGCGCGACG ACGATACACGCGACGAAAAAACCGAATATGCTACCTACAAGA AATATTCAGCAACGAAATGGCGAAAGTAGACGACAGCTTCTT CCACCGACTAGAAGAAAGCTTCCTAGTAGAAGAAGACAAAAA ACACGAACGACACCCGATATTCGGGAACATAGTAGACGAAGT AGCGTACCACGAAAAATACCCGACGATATACCACCTACGAAA AAAACTAGTAGACAGCACGGACAAAGCGGACCTACGACTAAT ATACCTAGCGCTAGCGCACATGATAAAATTCCGAGGGCACTTC CTAATAGAAGGGGACCTAAACCCGGACAACAGCGACGTAGAC AAACTATTCATACAACTAGTACAAACGTACAACCAACTATTCG AAGAAAACCCGATAAACGCGAGCGGGGTAGACGCGAAAGCG ATACTAAGCGCGCGACTAAGCAAAAGCCGACGACTAGAAAAC CTAATAGCGCAACTACCGGGGGAAAAAAAAAACGGGCTATTC GGGAACCTAATAGCGCTAAGCCTAGGGCTAACGCCGAACTTC AAAAGCAACTTCGACCTAGCGGAAGACGCGAAACTACAACTA AGCAAAGACACGTACGACGACGACCTAGACAACCTACTAGCG CAAATAGGGGACCAATACGCGGACCTATTCCTAGCGGCGAAA AACCTAAGCGACGCGATACTACTAAGCGACATACTACGAGTA AACACGGAAATAACGAAAGCGCCGCTAAGCGCGAGCATGATA AAACGATACGACGAACACCACCAAGACCTAACGCTACTAAAA GCGCTAGTACGACAACAACTACCGGAAAAATACAAAGAAATA TTCTTCGACCAAAGCAAAAACGGGTACGCGGGGTACATAGAC GGGGGGGCGAGCCAAGAAGAATTCTACAAATTCATAAAACCG ATACTAGAAAAAATGGACGGGACGGAAGAACTACTAGTAAAA CTAAACCGAGAAGACCTACTACGAAAACAACGAACGTTCGAC AACGGGAGCATACCGCACCAAATACACCTAGGGGAACTACAC GCGATACTACGACGACAAGAAGACTTCTACCCGTTCCTAAAAG ACAACCGAGAAAAAATAGAAAAAATACTAACGTTCCGAATAC CGTACTACGTAGGGCCGCTAGCGCGAGGGAACAGCCGATTCG CGTGGATGACGCGAAAAAGCGAAGAAACGATAACGCCGTGGA ACTTCGAAGAAGTAGTAGACAAAGGGGCGAGCGCGCAAAGCT TCATAGAACGAATGACGAACTTCGACAAAAACCTACCGAACG AAAAAGTACTACCGAAACACAGCCTACTATACGAATACTTCAC GGTATACAACGAACTAACGAAAGTAAAATACGTAACGGAAGG GATGCGAAAACCGGCGTTCCTAAGCGGGGAACAAAAAAAAGC GATAGTAGACCTACTATTCAAAACGAACCGAAAAGTAACGGT AAAACAACTAAAAGAAGACTACTTCAAAAAAATAGAATGCTT CGACAGCGTAGAAATAAGCGGGGTAGAAGACCGATTCAACGC | **54** |
| | GAGCCTAGGGACGTACCACGACCTACTAAAAATAATAAAAGA CAAAGACTTCCTAGACAACGAAGAAAACGAAGACATACTAGA AGACATAGTACTAACGCTAACGCTATTCGAAGACCGAGAAAT GATAGAAGAACGACTAAAAACGTACGCGCACCTATTCGACGA CAAAGTAATGAAACAACTAAAACGACGACGATACACGGGGTG GGGGCGACTAAGCCGAAAACTAATAAACGGGATACGAGACAA ACAAAGCGGGAAAACGATACTAGACTTCCTAAAAAGCGACGG GTTCGCGAACCGAAACTTCATGCAACTAATACACGACGACAG CCTAACGTTCAAAGAAGACATACAAAAAGCGCAAGTAAGCGG GCAAGGGGACAGCCTACACGAACACATAGCGAACCTAGCGGG GAGCCCGGCGATAAAAAAAGGGATACTACAAACGGTAAAAGT AGTAGACGAACTAGTAAAAGTAATGGGGCGACACAAACCGGA AAACATAGTAATAGAAATGGCGCGAGAAAACCAAACGACGCA AAAAGGGCAAAAAAACAGCCGAGAACGAATGAAACGAATAG AAGAAGGGATAAAAGAACTAGGGAGCCAAATACTAAAAGAA CACCCGGTAGAAAACACGCAACTACAAAACGAAAAACTATAC CTATACTACCTACAAAACGGGCGAGACATGTACGTAGACCAA GAACTAGACATAAACCGACTAAGCGACTACGACGTAGACCAC ATAGTACCGCAAAGCTTCCTAAAAGACGACAGCATAGACAAC AAAGTACTAACGCGAAGCGACAAAAACCGAGGGAAAAGCGA CAACGTACCGAGCGAAGAAGTAGTAAAAAAAATGAAAAACTA CTGGCGACAACTACTAAACGCGAAACTAATAACGCAACGAAA ATTCGACAACCTAACGAAAGCGGAACGAGGGGGGCTAAGCGA ACTAGACAAAGCGGGGTTCATAAAACGACAACTAGTAGAAAC GCGACAAATAACGAAACACGTAGCGCAAATACTAGACAGCCG AATGAACACGAAATACGACGAAAACGACAAACTAATACGAGA AGTAAAAGTAATAACGCTAAAAAGCAAACTAGTAAGCGACTT CCGAAAAGACTTCCAATTCTACAAAGTACGAGAAATAAACAA CTACCACCACGCGCACGACGCGTACCTAAACGCGGTAGTAGG GACGGCGCTAATAAAAAAATACCCGAAACTAGAAAGCGAATT CGTATACGGGGACTACAAAGTATACGACGTACGAAAAATGAT AGCGAAAAGCGAACAAGAAATAGGGAAAGCGACGGCGAAAT ACTTCTTCTACAGCAACATAATGAACTTCTTCAAAACGGAAAT AACGCTAGCGAACGGGGAAATACGAAAACGACCGCTAATAGA AACGAACGGGGAAACGGGGGAAATAGTATGGGACAAAGGGC GAGACTTCGCGACGGTACGAAAAGTACTAAGCATGCCGCAAG TAAACATAGTAAAAAAAACGGAAGTACAAACGGGGGGGTTCA GCAAAGAAAGCATACTACCGAAACGAAACAGCGACAAACTAA TAGCGCGAAAAAAAGACTGGGACCCGAAAAAATACGGGGGGT TCGACAGCCCGACGGTAGCGTACAGCGTACTAGTAGTAGCGA AAGTAGAAAAAGGGAAAAGCAAAAAACTAAAAAGCGTAAAA GAACTACTAGGGATAACGATAATGGAACGAAGCAGCTTCGAA AAAAACCCGATAGACTTCCTAGAAGCGAAAGGGTACAAAGAA GTAAAAAAAGACCTAATAATAAAACTACCGAAATACAGCCTA TTCGAACTAGAAAACGGGCGAAAACGAATGCTAGCGAGCGCG GGGGAACTACAAAAAGGGAACGAACTAGCGCTACCGAGCAAA TACGTAAACTTCCTATACCTAGCGAGCCACTACGAAAAACTAA AAGGGAGCCCGGAAGACAACGAACAAAAACAACTATTCGTAG AACAACACAAACACTACCTAGACGAAATAATAGAACAAATAA GCGAATTCAGCAAACGAGTAATACTAGCGGACGCGAACCTAG ACAAAGTACTAAGCGCGTACAACAAACACCGAGACAAACCGA TACGAGAACAAGCGGAAAACATAATACACCTATTCACGCTAA CGAACCTAGGGGCGCCGGCGGCGTTCAAATACTTCGACACGA CGATAGACCGAAAACGATACACGAGCACGAAAGAAGTACTAG ACGCGACGCTAATACACCAAAGCATAACGGGGCTATACGAAA CGCGAATAGACCTAAGCCAACTAGGGGGGGACGGGGGGGGG AGCCCGAAAAAAAAACGAAAAGTATGA | |
| **Cas9 transcript** | GGGTCCCGCAGTCGGCGTCCAGCGGCTCTGCTTGTTCGTGTGT GTGTCGTTGCAGGCCTTATTCGGATCCGCCACCATGGACAAAA | **55** |
| **with 5' UTR of HSD, ORF correspondi ng to SEQ ID NO: 54, Kozak sequence, and 3' UTR of ALB** | AATACAGCATAGGGCTAGACATAGGGACGAACAGCGTAGGGT GGGCGGTAATAACGGACGAATACAAAGTACCGAGCAAAAAAT TCAAAGTACTAGGGAACACGGACCGACACAGCATAAAAAAAA ACCTAATAGGGGCGCTACTATTCGACAGCGGGGAAACGGCGG AAGCGACGCGACTAAAACGAACGGCGCGACGACGATACACGC GACGAAAAAACCGAATATGCTACCTACAAGAAATATTCAGCA ACGAAATGGCGAAAGTAGACGACAGCTTCTTCCACCGACTAG AAGAAAGCTTCCTAGTAGAAGAAGACAAAAAACACGAACGAC ACCCGATATTCGGGAACATAGTAGACGAAGTAGCGTACCACG AAAAATACCCGACGATATACCACCTACGAAAAAAACTAGTAG ACAGCACGGACAAAGCGGACCTACGACTAATATACCTAGCGC TAGCGCACATGATAAAATTCCGAGGGCACTTCCTAATAGAAG GGGACCTAAACCCGGACAACAGCGACGTAGACAAACTATTCA TACAACTAGTACAAACGTACAACCAACTATTCGAAGAAAACC CGATAAACGCGAGCGGGGTAGACGCGAAAGCGATACTAAGCG CGCGACTAAGCAAAAGCCGACGACTAGAAAACCTAATAGCGC AACTACCGGGGGAAAAAAAAAACGGGCTATTCGGGAACCTAA TAGCGCTAAGCCTAGGGCTAACGCCGAACTTCAAAAGCAACTT CGACCTAGCGGAAGACGCGAAACTACAACTAAGCAAAGACAC GTACGACGACGACCTAGACAACCTACTAGCGCAAATAGGGGA CCAATACGCGGACCTATTCCTAGCGGCGAAAAACCTAAGCGA CGCGATACTACTAAGCGACATACTACGAGTAAACACGGAAAT AACGAAAGCGCCGCTAAGCGCGAGCATGATAAAACGATACGA CGAACACCACCAAGACCTAACGCTACTAAAAGCGCTAGTACG ACAACAACTACCGGAAAAATACAAAGAAATATTCTTCGACCA AAGCAAAAACGGGTACGCGGGGTACATAGACGGGGGGGCGA GCCAAGAAGAATTCTACAAATTCATAAAACCGATACTAGAAA AAATGGACGGGACGGAAGAACTACTAGTAAAACTAAACCGAG AAGACCTACTACGAAAACAACGAACGTTCGACAACGGGAGCA TACCGCACCAAATACACCTAGGGGAACTACACGCGATACTAC GACGACAAGAAGACTTCTACCCGTTCCTAAAAGACAACCGAG AAAAAATAGAAAAAATACTAACGTTCCGAATACCGTACTACG TAGGGCCGCTAGCGCGAGGGAACAGCCGATTCGCGTGGATGA CGCGAAAAAGCGAAGAAACGATAACGCCGTGGAACTTCGAAG AAGTAGTAGACAAAGGGGCGAGCGCGCAAAGCTTCATAGAAC GAATGACGAACTTCGACAAAAACCTACCGAACGAAAAAGTAC TACCGAAACACAGCCTACTATACGAATACTTCACGGTATACAA CGAACTAACGAAAGTAAAATACGTAACGGAAGGGATGCGAAA ACCGGCGTTCCTAAGCGGGGAACAAAAAAAAGCGATAGTAGA CCTACTATTCAAAACGAACCGAAAAGTAACGGTAAAACAACT AAAAGAAGACTACTTCAAAAAAATAGAATGCTTCGACAGCGT AGAAATAAGCGGGGTAGAAGACCGATTCAACGCGAGCCTAGG GACGTACCACGACCTACTAAAAATAATAAAAGACAAAGACTT CCTAGACAACGAAGAAAACGAAGACATACTAGAAGACATAGT ACTAACGCTAACGCTATTCGAAGACCGAGAAATGATAGAAGA ACGACTAAAAACGTACGCGCACCTATTCGACGACAAAGTAAT GAAACAACTAAAACGACGACGATACACGGGGTGGGGGCGACT AAGCCGAAAACTAATAAACGGGATACGAGACAAACAAAGCG GGAAAACGATACTAGACTTCCTAAAAAGCGACGGGTTCGCGA ACCGAAACTTCATGCAACTAATACACGACGACAGCCTAACGTT CAAAGAAGACATACAAAAAGCGCAAGTAAGCGGGCAAGGGG ACAGCCTACACGAACACATAGCGAACCTAGCGGGGAGCCCGG CGATAAAAAAAGGGATACTACAAACGGTAAAAGTAGTAGACG AACTAGTAAAAGTAATGGGGCGACACAAACCGGAAAACATAG TAATAGAAATGGCGCGAGAAAACCAAACGACGCAAAAAGGG CAAAAAAACAGCCGAGAACGAATGAAACGAATAGAAGAAGG GATAAAAGAACTAGGGAGCCAAATACTAAAAGAACACCCGGT AGAAAACACGCAACTACAAAACGAAAAACTATACCTATACTA CCTACAAAACGGGCGAGACATGTACGTAGACCAAGAACTAGA CATAAACCGACTAAGCGACTACGACGTAGACCACATAGTACC GCAAAGCTTCCTAAAAGACGACAGCATAGACAACAAAGTACT | |
| | AACGCGAAGCGACAAAAACCGAGGGAAAAGCGACAACGTAC CGAGCGAAGAAGTAGTAAAAAAAATGAAAAACTACTGGCGAC AACTACTAAACGCGAAACTAATAACGCAACGAAAATTCGACA ACCTAACGAAAGCGGAACGAGGGGGGCTAAGCGAACTAGACA AAGCGGGGTTCATAAAACGACAACTAGTAGAAACGCGACAAA TAACGAAACACGTAGCGCAAATACTAGACAGCCGAATGAACA CGAAATACGACGAAAACGACAAACTAATACGAGAAGTAAAAG TAATAACGCTAAAAAGCAAACTAGTAAGCGACTTCCGAAAAG ACTTCCAATTCTACAAAGTACGAGAAATAAACAACTACCACCA CGCGCACGACGCGTACCTAAACGCGGTAGTAGGGACGGCGCT AATAAAAAAATACCCGAAACTAGAAAGCGAATTCGTATACGG GGACTACAAAGTATACGACGTACGAAAAATGATAGCGAAAAG CGAACAAGAAATAGGGAAAGCGACGGCGAAATACTTCTTCTA CAGCAACATAATGAACTTCTTCAAAACGGAAATAACGCTAGC GAACGGGGAAATACGAAAACGACCGCTAATAGAAACGAACG GGGAAACGGGGGAAATAGTATGGGACAAAGGGCGAGACTTCG CGACGGTACGAAAAGTACTAAGCATGCCGCAAGTAAACATAG TAAAAAAAACGGAAGTACAAACGGGGGGGTTCAGCAAAGAA AGCATACTACCGAAACGAAACAGCGACAAACTAATAGCGCGA AAAAAAGACTGGGACCCGAAAAAATACGGGGGGTTCGACAGC CCGACGGTAGCGTACAGCGTACTAGTAGTAGCGAAAGTAGAA AAAGGGAAAAGCAAAAAACTAAAAAGCGTAAAAGAACTACT AGGGATAACGATAATGGAACGAAGCAGCTTCGAAAAAAACCC GATAGACTTCCTAGAAGCGAAAGGGTACAAAGAAGTAAAAAA AGACCTAATAATAAAACTACCGAAATACAGCCTATTCGAACTA GAAAACGGGCGAAAACGAATGCTAGCGAGCGCGGGGGAACT ACAAAAAGGGAACGAACTAGCGCTACCGAGCAAATACGTAAA CTTCCTATACCTAGCGAGCCACTACGAAAAACTAAAAGGGAG CCCGGAAGACAACGAACAAAAACAACTATTCGTAGAACAACA CAAACACTACCTAGACGAAATAATAGAACAAATAAGCGAATT CAGCAAACGAGTAATACTAGCGGACGCGAACCTAGACAAAGT ACTAAGCGCGTACAACAAACACCGAGACAAACCGATACGAGA ACAAGCGGAAAACATAATACACCTATTCACGCTAACGAACCT AGGGGCGCCGGCGGCGTTCAAATACTTCGACACGACGATAGA CCGAAAACGATACACGAGCACGAAAGAAGTACTAGACGCGAC GCTAATACACCAAAGCATAACGGGGCTATACGAAACGCGAAT AGACCTAAGCCAACTAGGGGGGGACGGGGGGGGGAGCCCGA AAAAAAAACGAAAAGTATGACTAGCCATCACATTTAAAAGCA TCTCAGCCTACCATGAGAATAAGAGAAAGAAAATGAAGATCA ATAGCTTATTCATCTCTTTTTCTTTTTCGTTGGTGTAAAGCCAA CACCCTGTCTAAAAAACATAAATTTCTTTAATCATTTTGCCTCT TTTCTCTGTGCTTCAATTAATAAAAAATGGAAAGAACCTCGAG | |
| **Cas9 transcript with AGG as first three nucleotides for use with CleanCap^{™}, 5' UTR of HSD, ORF correspondi ng to SEQ ID NO: 4, Kozak sequence, and 3' UTR of ALB** | AGGTCCCGCAGTCGGCGTCCAGCGGCTCTGCTTGTTCGTGTGT GTGTCGTTGCAGGCCTTATTCGGATCCGCCACCATGGACAAGA AGTACAGCATCGGACTGGACATCGGAACAAACAGCGTCGGAT GGGCAGTCATCACAGACGAATACAAGGTCCCGAGCAAGAAGT TCAAGGTCCTGGGAAACACAGACAGACACAGCATCAAGAAGA ACCTGATCGGAGCACTGCTGTTCGACAGCGGAGAAACAGCAG AAGCAACAAGACTGAAGAGAACAGCAAGAAGAAGATACACA AGAAGAAAGAACAGAATCTGCTACCTGCAGGAAATCTTCAGC AACGAAATGGCAAAGGTCGACGACAGCTTCTTCCACAGACTG GAAGAAAGCTTCCTGGTCGAAGAAGACAAGAAGCACGAAAGA CACCCGATCTTCGGAAACATCGTCGACGAAGTCGCATACCACG AAAAGTACCCGACAATCTACCACCTGAGAAAGAAGCTGGTCG ACAGCACAGACAAGGCAGACCTGAGACTGATCTACCTGGCAC TGGCACACATGATCAAGTTCAGAGGACACTTCCTGATCGAAGG AGACCTGAACCCGGACAACAGCGACGTCGACAAGCTGTTCAT CCAGCTGGTCCAGACATACAACCAGCTGTTCGAAGAAAACCC GATCAACGCAAGCGGAGTCGACGCAAAGGCAATCCTGAGCGC | **56** |
| | AAGACTGAGCAAGAGCAGAAGACTGGAAAACCTGATCGCACA GCTGCCGGGAGAAAAGAAGAACGGACTGTTCGGAAACCTGAT CGCACTGAGCCTGGGACTGACACCGAACTTCAAGAGCAACTTC GACCTGGCAGAAGACGCAAAGCTGCAGCTGAGCAAGGACACA TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAGAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA | |
| | GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG ACCTGAGCCAGCTGGGAGGAGACGGAGGAGGAAGCCCGAAG AAGAAGAGAAAGGTCTAGCTAGCCATCACATTTAAAAGCATC TCAGCCTACCATGAGAATAAGAGAAAGAAAATGAAGATCAAT AGCTTATTCATCTCTTTTTCTTTTTCGTTGGTGTAAAGCCAACA CCCTGTCTAAAAAACATAAATTTCTTTAATCATTTTGCCTCTTT TCTCTGTGCTTCAATTAATAAAAAATGGAAAGAACCTCGAG | |
| **Cas9 transcript with 5' UTR from CMV, ORF correspondi ng to SEQ ID NO: 4, Kozak sequence, and 3' UTR of ALB** | GGGCAGATCGCCTGGAGACGCCATCCACGCTGTTTTGACCTCC ATAGAAGACACCGGGACCGATCCAGCCTCCGCGGCCGGGAAC GGTGCATTGGAACGCGGATTCCCCGTGCCAAGAGTGACTCACC GTCCTTGACACGGCCACCATGGACAAGAAGTACAGCATCGGA CTGGACATCGGAACAAACAGCGTCGGATGGGCAGTCATCACA GACGAATACAAGGTCCCGAGCAAGAAGTTCAAGGTCCTGGGA AACACAGACAGACACAGCATCAAGAAGAACCTGATCGGAGCA CTGCTGTTCGACAGCGGAGAAACAGCAGAAGCAACAAGACTG AAGAGAACAGCAAGAAGAAGATACACAAGAAGAAAGAACAG AATCTGCTACCTGCAGGAAATCTTCAGCAACGAAATGGCAAA GGTCGACGACAGCTTCTTCCACAGACTGGAAGAAAGCTTCCTG GTCGAAGAAGACAAGAAGCACGAAAGACACCCGATCTTCGGA AACATCGTCGACGAAGTCGCATACCACGAAAAGTACCCGACA ATCTACCACCTGAGAAAGAAGCTGGTCGACAGCACAGACAAG GCAGACCTGAGACTGATCTACCTGGCACTGGCACACATGATCA AGTTCAGAGGACACTTCCTGATCGAAGGAGACCTGAACCCGG ACAACAGCGACGTCGACAAGCTGTTCATCCAGCTGGTCCAGAC ATACAACCAGCTGTTCGAAGAAAACCCGATCAACGCAAGCGG AGTCGACGCAAAGGCAATCCTGAGCGCAAGACTGAGCAAGAG CAGAAGACTGGAAAACCTGATCGCACAGCTGCCGGGAGAAAA GAAGAACGGACTGTTCGGAAACCTGATCGCACTGAGCCTGGG ACTGACACCGAACTTCAAGAGCAACTTCGACCTGGCAGAAGA CGCAAAGCTGCAGCTGAGCAAGGACACATACGACGACGACCT GGACAACCTGCTGGCACAGATCGGAGACCAGTACGCAGACCT GTTCCTGGCAGCAAAGAACCTGAGCGACGCAATCCTGCTGAG CGACATCCTGAGAGTCAACACAGAAATCACAAAGGCACCGCT GAGCGCAAGCATGATCAAGAGATACGACGAACACCACCAGGA CCTGACACTGCTGAAGGCACTGGTCAGACAGCAGCTGCCGGA AAAGTACAAGGAAATCTTCTTCGACCAGAGCAAGAACGGATA CGCAGGATACATCGACGGAGGAGCAAGCCAGGAAGAATTCTA CAAGTTCATCAAGCCGATCCTGGAAAAGATGGACGGAACAGA AGAACTGCTGGTCAAGCTGAACAGAGAAGACCTGCTGAGAAA | **57** |
| | GCAGAGAACATTCGACAACGGAAGCATCCCGCACCAGATCCA CCTGGGAGAACTGCACGCAATCCTGAGAAGACAGGAAGACTT CTACCCGTTCCTGAAGGACAACAGAGAAAAGATCGAAAAGAT CCTGACATTCAGAATCCCGTACTACGTCGGACCGCTGGCAAGA GGAAACAGCAGATTCGCATGGATGACAAGAAAGAGCGAAGA AACAATCACACCGTGGAACTTCGAAGAAGTCGTCGACAAGGG AGCAAGCGCACAGAGCTTCATCGAAAGAATGACAAACTTCGA CAAGAACCTGCCGAACGAAAAGGTCCTGCCGAAGCACAGCCT GCTGTACGAATACTTCACAGTCTACAACGAACTGACAAAGGTC AAGTACGTCACAGAAGGAATGAGAAAGCCGGCATTCCTGAGC GGAGAACAGAAGAAGGCAATCGTCGACCTGCTGTTCAAGACA AACAGAAAGGTCACAGTCAAGCAGCTGAAGGAAGACTACTTC AAGAAGATCGAATGCTTCGACAGCGTCGAAATCAGCGGAGTC GAAGACAGATTCAACGCAAGCCTGGGAACATACCACGACCTG CTGAAGATCATCAAGGACAAGGACTTCCTGGACAACGAAGAA AACGAAGACATCCTGGAAGACATCGTCCTGACACTGACACTGT TCGAAGACAGAGAAATGATCGAAGAAAGACTGAAGACATACG CACACCTGTTCGACGACAAGGTCATGAAGCAGCTGAAGAGAA GAAGATACACAGGATGGGGAAGACTGAGCAGAAAGCTGATCA ACGGAATCAGAGACAAGCAGAGCGGAAAGACAATCCTGGACT TCCTGAAGAGCGACGGATTCGCAAACAGAAACTTCATGCAGC TGATCCACGACGACAGCCTGACATTCAAGGAAGACATCCAGA AGGCACAGGTCAGCGGACAGGGAGACAGCCTGCACGAACACA TCGCAAACCTGGCAGGAAGCCCGGCAATCAAGAAGGGAATCC TGCAGACAGTCAAGGTCGTCGACGAACTGGTCAAGGTCATGG GAAGACACAAGCCGGAAAACATCGTCATCGAAATGGCAAGAG AAAACCAGACAACACAGAAGGGACAGAAGAACAGCAGAGAA AGAATGAAGAGAATCGAAGAAGGAATCAAGGAACTGGGAAG CCAGATCCTGAAGGAACACCCGGTCGAAAACACACAGCTGCA GAACGAAAAGCTGTACCTGTACTACCTGCAGAACGGAAGAGA CATGTACGTCGACCAGGAACTGGACATCAACAGACTGAGCGA CTACGACGTCGACCACATCGTCCCGCAGAGCTTCCTGAAGGAC GACAGCATCGACAACAAGGTCCTGACAAGAAGCGACAAGAAC AGAGGAAAGAGCGACAACGTCCCGAGCGAAGAAGTCGTCAAG AAGATGAAGAACTACTGGAGACAGCTGCTGAACGCAAAGCTG ATCACACAGAGAAAGTTCGACAACCTGACAAAGGCAGAGAGA GGAGGACTGAGCGAACTGGACAAGGCAGGATTCATCAAGAGA CAGCTGGTCGAAACAAGACAGATCACAAAGCACGTCGCACAG ATCCTGGACAGCAGAATGAACACAAAGTACGACGAAAACGAC AAGCTGATCAGAGAAGTCAAGGTCATCACACTGAAGAGCAAG CTGGTCAGCGACTTCAGAAAGGACTTCCAGTTCTACAAGGTCA GAGAAATCAACAACTACCACCACGCACACGACGCATACCTGA ACGCAGTCGTCGGAACAGCACTGATCAAGAAGTACCCGAAGC TGGAAAGCGAATTCGTCTACGGAGACTACAAGGTCTACGACG TCAGAAAGATGATCGCAAAGAGCGAACAGGAAATCGGAAAG GCAACAGCAAAGTACTTCTTCTACAGCAACATCATGAACTTCT TCAAGACAGAAATCACACTGGCAAACGGAGAAATCAGAAAGA GACCGCTGATCGAAACAAACGGAGAAACAGGAGAAATCGTCT GGGACAAGGGAAGAGACTTCGCAACAGTCAGAAAGGTCCTGA GCATGCCGCAGGTCAACATCGTCAAGAAGACAGAAGTCCAGA CAGGAGGATTCAGCAAGGAAAGCATCCTGCCGAAGAGAAACA GCGACAAGCTGATCGCAAGAAAGAAGGACTGGGACCCGAAGA AGTACGGAGGATTCGACAGCCCGACAGTCGCATACAGCGTCC TGGTCGTCGCAAAGGTCGAAAAGGGAAAGAGCAAGAAGCTGA AGAGCGTCAAGGAACTGCTGGGAATCACAATCATGGAAAGAA GCAGCTTCGAAAAGAACCCGATCGACTTCCTGGAAGCAAAGG GATACAAGGAAGTCAAGAAGGACCTGATCATCAAGCTGCCGA AGTACAGCCTGTTCGAACTGGAAAACGGAAGAAAGAGAATGC TGGCAAGCGCAGGAGAACTGCAGAAGGGAAACGAACTGGCAC TGCCGAGCAAGTACGTCAACTTCCTGTACCTGGCAAGCCACTA CGAAAAGCTGAAGGGAAGCCCGGAAGACAACGAACAGAAGC | |
| | AGCTGTTCGTCGAACAGCACAAGCACTACCTGGACGAAATCAT CGAACAGATCAGCGAATTCAGCAAGAGAGTCATCCTGGCAGA CGCAAACCTGGACAAGGTCCTGAGCGCATACAACAAGCACAG AGACAAGCCGATCAGAGAACAGGCAGAAAACATCATCCACCT GTTCACACTGACAAACCTGGGAGCACCGGCAGCATTCAAGTA CTTCGACACAACAATCGACAGAAAGAGATACACAAGCACAAA GGAAGTCCTGGACGCAACACTGATCCACCAGAGCATCACAGG ACTGTACGAAACAAGAATCGACCTGAGCCAGCTGGGAGGAGA CGGAGGAGGAAGCCCGAAGAAGAAGAGAAAGGTCTAGCTAG CCATCACATTTAAAAGCATCTCAGCCTACCATGAGAATAAGAG AAAGAAAATGAAGATCAATAGCTTATTCATCTCTTTTTCTTTTT CGTTGGTGTAAAGCCAACACCCTGTCTAAAAAACATAAATTTC TTTAATCATTTTGCCTCTTTTCTCTGTGCTTCAATTAATAAAAA ATGGAAAGAACCTCGAG | |
| **Cas9 transcript with 5' UTR from HBB, ORF correspondi ng to SEQ ID NO: 4, Kozak sequence, and 3' UTR of HBB** | GGGacatttgcttctgacacaactgtgttcactagcaacctcaaacagacaccggatctgccaccAT GGACAAGAAGTACAGCATCGGACTGGACATCGGAACAAACAG CGTCGGATGGGCAGTCATCACAGACGAATACAAGGTCCCGAG CAAGAAGTTCAAGGTCCTGGGAAACACAGACAGACACAGCAT CAAGAAGAACCTGATCGGAGCACTGCTGTTCGACAGCGGAGA AACAGCAGAAGCAACAAGACTGAAGAGAACAGCAAGAAGAA GATACACAAGAAGAAAGAACAGAATCTGCTACCTGCAGGAAA TCTTCAGCAACGAAATGGCAAAGGTCGACGACAGCTTCTTCCA CAGACTGGAAGAAAGCTTCCTGGTCGAAGAAGACAAGAAGCA CGAAAGACACCCGATCTTCGGAAACATCGTCGACGAAGTCGC ATACCACGAAAAGTACCCGACAATCTACCACCTGAGAAAGAA GCTGGTCGACAGCACAGACAAGGCAGACCTGAGACTGATCTA CCTGGCACTGGCACACATGATCAAGTTCAGAGGACACTTCCTG ATCGAAGGAGACCTGAACCCGGACAACAGCGACGTCGACAAG CTGTTCATCCAGCTGGTCCAGACATACAACCAGCTGTTCGAAG AAAACCCGATCAACGCAAGCGGAGTCGACGCAAAGGCAATCC TGAGCGCAAGACTGAGCAAGAGCAGAAGACTGGAAAACCTGA TCGCACAGCTGCCGGGAGAAAAGAAGAACGGACTGTTCGGAA ACCTGATCGCACTGAGCCTGGGACTGACACCGAACTTCAAGA GCAACTTCGACCTGGCAGAAGACGCAAAGCTGCAGCTGAGCA AGGACACATACGACGACGACCTGGACAACCTGCTGGCACAGA TCGGAGACCAGTACGCAGACCTGTTCCTGGCAGCAAAGAACC TGAGCGACGCAATCCTGCTGAGCGACATCCTGAGAGTCAACA CAGAAATCACAAAGGCACCGCTGAGCGCAAGCATGATCAAGA GATACGACGAACACCACCAGGACCTGACACTGCTGAAGGCAC TGGTCAGACAGCAGCTGCCGGAAAAGTACAAGGAAATCTTCT TCGACCAGAGCAAGAACGGATACGCAGGATACATCGACGGAG GAGCAAGCCAGGAAGAATTCTACAAGTTCATCAAGCCGATCC TGGAAAAGATGGACGGAACAGAAGAACTGCTGGTCAAGCTGA ACAGAGAAGACCTGCTGAGAAAGCAGAGAACATTCGACAACG GAAGCATCCCGCACCAGATCCACCTGGGAGAACTGCACGCAA TCCTGAGAAGACAGGAAGACTTCTACCCGTTCCTGAAGGACA ACAGAGAAAAGATCGAAAAGATCCTGACATTCAGAATCCCGT ACTACGTCGGACCGCTGGCAAGAGGAAACAGCAGATTCGCAT GGATGACAAGAAAGAGCGAAGAAACAATCACACCGTGGAACT TCGAAGAAGTCGTCGACAAGGGAGCAAGCGCACAGAGCTTCA TCGAAAGAATGACAAACTTCGACAAGAACCTGCCGAACGAAA AGGTCCTGCCGAAGCACAGCCTGCTGTACGAATACTTCACAGT CTACAACGAACTGACAAAGGTCAAGTACGTCACAGAAGGAAT GAGAAAGCCGGCATTCCTGAGCGGAGAACAGAAGAAGGCAAT CGTCGACCTGCTGTTCAAGACAAACAGAAAGGTCACAGTCAA GCAGCTGAAGGAAGACTACTTCAAGAAGATCGAATGCTTCGA CAGCGTCGAAATCAGCGGAGTCGAAGACAGATTCAACGCAAG CCTGGGAACATACCACGACCTGCTGAAGATCATCAAGGACAA GGACTTCCTGGACAACGAAGAAAACGAAGACATCCTGGAAGA | **58** |
| | CATCGTCCTGACACTGACACTGTTCGAAGACAGAGAAATGATC GAAGAAAGACTGAAGACATACGCACACCTGTTCGACGACAAG GTCATGAAGCAGCTGAAGAGAAGAAGATACACAGGATGGGGA AGACTGAGCAGAAAGCTGATCAACGGAATCAGAGACAAGCAG AGCGGAAAGACAATCCTGGACTTCCTGAAGAGCGACGGATTC GCAAACAGAAACTTCATGCAGCTGATCCACGACGACAGCCTG ACATTCAAGGAAGACATCCAGAAGGCACAGGTCAGCGGACAG GGAGACAGCCTGCACGAACACATCGCAAACCTGGCAGGAAGC CCGGCAATCAAGAAGGGAATCCTGCAGACAGTCAAGGTCGTC GACGAACTGGTCAAGGTCATGGGAAGACACAAGCCGGAAAAC ATCGTCATCGAAATGGCAAGAGAAAACCAGACAACACAGAAG GGACAGAAGAACAGCAGAGAAAGAATGAAGAGAATCGAAGA AGGAATCAAGGAACTGGGAAGCCAGATCCTGAAGGAACACCC GGTCGAAAACACACAGCTGCAGAACGAAAAGCTGTACCTGTA CTACCTGCAGAACGGAAGAGACATGTACGTCGACCAGGAACT GGACATCAACAGACTGAGCGACTACGACGTCGACCACATCGT CCCGCAGAGCTTCCTGAAGGACGACAGCATCGACAACAAGGT CCTGACAAGAAGCGACAAGAACAGAGGAAAGAGCGACAACG TCCCGAGCGAAGAAGTCGTCAAGAAGATGAAGAACTACTGGA GACAGCTGCTGAACGCAAAGCTGATCACACAGAGAAAGTTCG ACAACCTGACAAAGGCAGAGAGAGGAGGACTGAGCGAACTG GACAAGGCAGGATTCATCAAGAGACAGCTGGTCGAAACAAGA CAGATCACAAAGCACGTCGCACAGATCCTGGACAGCAGAATG AACACAAAGTACGACGAAAACGACAAGCTGATCAGAGAAGTC AAGGTCATCACACTGAAGAGCAAGCTGGTCAGCGACTTCAGA AAGGACTTCCAGTTCTACAAGGTCAGAGAAATCAACAACTAC CACCACGCACACGACGCATACCTGAACGCAGTCGTCGGAACA GCACTGATCAAGAAGTACCCGAAGCTGGAAAGCGAATTCGTC TACGGAGACTACAAGGTCTACGACGTCAGAAAGATGATCGCA AAGAGCGAACAGGAAATCGGAAAGGCAACAGCAAAGTACTTC TTCTACAGCAACATCATGAACTTCTTCAAGACAGAAATCACAC TGGCAAACGGAGAAATCAGAAAGAGACCGCTGATCGAAACAA ACGGAGAAACAGGAGAAATCGTCTGGGACAAGGGAAGAGAC TTCGCAACAGTCAGAAAGGTCCTGAGCATGCCGCAGGTCAAC ATCGTCAAGAAGACAGAAGTCCAGACAGGAGGATTCAGCAAG GAAAGCATCCTGCCGAAGAGAAACAGCGACAAGCTGATCGCA AGAAAGAAGGACTGGGACCCGAAGAAGTACGGAGGATTCGAC AGCCCGACAGTCGCATACAGCGTCCTGGTCGTCGCAAAGGTCG AAAAGGGAAAGAGCAAGAAGCTGAAGAGCGTCAAGGAACTG CTGGGAATCACAATCATGGAAAGAAGCAGCTTCGAAAAGAAC CCGATCGACTTCCTGGAAGCAAAGGGATACAAGGAAGTCAAG AAGGACCTGATCATCAAGCTGCCGAAGTACAGCCTGTTCGAAC TGGAAAACGGAAGAAAGAGAATGCTGGCAAGCGCAGGAGAA CTGCAGAAGGGAAACGAACTGGCACTGCCGAGCAAGTACGTC AACTTCCTGTACCTGGCAAGCCACTACGAAAAGCTGAAGGGA AGCCCGGAAGACAACGAACAGAAGCAGCTGTTCGTCGAACAG CACAAGCACTACCTGGACGAAATCATCGAACAGATCAGCGAA TTCAGCAAGAGAGTCATCCTGGCAGACGCAAACCTGGACAAG GTCCTGAGCGCATACAACAAGCACAGAGACAAGCCGATCAGA GAACAGGCAGAAAACATCATCCACCTGTTCACACTGACAAAC CTGGGAGCACCGGCAGCATTCAAGTACTTCGACACAACAATC GACAGAAAGAGATACACAAGCACAAAGGAAGTCCTGGACGCA ACACTGATCCACCAGAGCATCACAGGACTGTACGAAACAAGA ATCGACCTGAGCCAGCTGGGAGGAGACGGAGGAGGAAGCCCG AAGAAGAAGAGAAAGGTCTAGctagcgctcgctttcttgctgtccaatttctattaaa ggttcctttgttccctaagtccaactactaaactgggggatattatgaagggccttgagcatctggattctg cctaataaaaaacatttattttcattgcctcgag | |
| **Cas9 transcript** | GGGaagctcagaataaacgctcaactttggccggatctgccacCATGGACAAGAAGT ACAGCATCGGACTGGACATCGGAACAAACAGCGTCGGATGGG | **59** |
| **with 5' UTR from XBG, ORF correspondi ng to SEQ ID NO: 4, Kozak sequence, and 3' UTR of XBG** | CAGTCATCACAGACGAATACAAGGTCCCGAGCAAGAAGTTCA AGGTCCTGGGAAACACAGACAGACACAGCATCAAGAAGAACC TGATCGGAGCACTGCTGTTCGACAGCGGAGAAACAGCAGAAG CAACAAGACTGAAGAGAACAGCAAGAAGAAGATACACAAGA AGAAAGAACAGAATCTGCTACCTGCAGGAAATCTTCAGCAAC GAAATGGCAAAGGTCGACGACAGCTTCTTCCACAGACTGGAA GAAAGCTTCCTGGTCGAAGAAGACAAGAAGCACGAAAGACAC CCGATCTTCGGAAACATCGTCGACGAAGTCGCATACCACGAA AAGTACCCGACAATCTACCACCTGAGAAAGAAGCTGGTCGAC AGCACAGACAAGGCAGACCTGAGACTGATCTACCTGGCACTG GCACACATGATCAAGTTCAGAGGACACTTCCTGATCGAAGGA GACCTGAACCCGGACAACAGCGACGTCGACAAGCTGTTCATC CAGCTGGTCCAGACATACAACCAGCTGTTCGAAGAAAACCCG ATCAACGCAAGCGGAGTCGACGCAAAGGCAATCCTGAGCGCA AGACTGAGCAAGAGCAGAAGACTGGAAAACCTGATCGCACAG CTGCCGGGAGAAAAGAAGAACGGACTGTTCGGAAACCTGATC GCACTGAGCCTGGGACTGACACCGAACTTCAAGAGCAACTTC GACCTGGCAGAAGACGCAAAGCTGCAGCTGAGCAAGGACACA TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAGAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA | |
| | GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG ACCTGAGCCAGCTGGGAGGAGACGGAGGAGGAAGCCCGAAG AAGAAGAGAAAGGTCTAGctagcaccagcctcaagaacacccgaatggagtctctaa gctacataataccaacttacactttacaaaatgttgtcccccaaaatgtagccattcgtatctgctcctaata aaaagaaagtttcttcacattctctcgag | |
| **Cas9 transcript with AGG as first three nucleotides for use with CleanCap^{™}, 5' UTR from XBG, ORF correspondi ng to SEQ ID NO: 4, Kozak sequence, and 3' UTR of XBG** | AGGaagctcagaataaacgctcaactttggccggatctgccacCATGGACAAGAAGT ACAGCATCGGACTGGACATCGGAACAAACAGCGTCGGATGGG CAGTCATCACAGACGAATACAAGGTCCCGAGCAAGAAGTTCA AGGTCCTGGGAAACACAGACAGACACAGCATCAAGAAGAACC TGATCGGAGCACTGCTGTTCGACAGCGGAGAAACAGCAGAAG CAACAAGACTGAAGAGAACAGCAAGAAGAAGATACACAAGA AGAAAGAACAGAATCTGCTACCTGCAGGAAATCTTCAGCAAC GAAATGGCAAAGGTCGACGACAGCTTCTTCCACAGACTGGAA GAAAGCTTCCTGGTCGAAGAAGACAAGAAGCACGAAAGACAC CCGATCTTCGGAAACATCGTCGACGAAGTCGCATACCACGAA AAGTACCCGACAATCTACCACCTGAGAAAGAAGCTGGTCGAC AGCACAGACAAGGCAGACCTGAGACTGATCTACCTGGCACTG GCACACATGATCAAGTTCAGAGGACACTTCCTGATCGAAGGA GACCTGAACCCGGACAACAGCGACGTCGACAAGCTGTTCATC CAGCTGGTCCAGACATACAACCAGCTGTTCGAAGAAAACCCG ATCAACGCAAGCGGAGTCGACGCAAAGGCAATCCTGAGCGCA AGACTGAGCAAGAGCAGAAGACTGGAAAACCTGATCGCACAG CTGCCGGGAGAAAAGAAGAACGGACTGTTCGGAAACCTGATC GCACTGAGCCTGGGACTGACACCGAACTTCAAGAGCAACTTC GACCTGGCAGAAGACGCAAAGCTGCAGCTGAGCAAGGACACA | **60** |
| | TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAGAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA | |
| | GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG ACCTGAGCCAGCTGGGAGGAGACGGAGGAGGAAGCCCGAAG AAGAAGAGAAAGGTCTAGctagcaccagcctcaagaacacccgaatggagtctctaa gctacataataccaacttacactttacaaaatgttgtcccccaaaatgtagccattcgtatctgctcctaata aaaagaaagtttcttcacattctctcgag | |
| **Cas9 transcript with AGG as first three nucleotides for use with CleanCap^{™}, 5' UTR from HSD, ORF correspondi ng to SEQ ID NO: 4, Kozak sequence, and 3' UTR of ALB** | AGGTCCCGCAGTCGGCGTCCAGCGGCTCTGCTTGTTCGTGTGT GTGTCGTTGCAGGCCTTATTCGGATCCGCCACCATGGACAAGA AGTACAGCATCGGACTGGACATCGGAACAAACAGCGTCGGAT GGGCAGTCATCACAGACGAATACAAGGTCCCGAGCAAGAAGT TCAAGGTCCTGGGAAACACAGACAGACACAGCATCAAGAAGA ACCTGATCGGAGCACTGCTGTTCGACAGCGGAGAAACAGCAG AAGCAACAAGACTGAAGAGAACAGCAAGAAGAAGATACACA AGAAGAAAGAACAGAATCTGCTACCTGCAGGAAATCTTCAGC AACGAAATGGCAAAGGTCGACGACAGCTTCTTCCACAGACTG GAAGAAAGCTTCCTGGTCGAAGAAGACAAGAAGCACGAAAGA CACCCGATCTTCGGAAACATCGTCGACGAAGTCGCATACCACG AAAAGTACCCGACAATCTACCACCTGAGAAAGAAGCTGGTCG ACAGCACAGACAAGGCAGACCTGAGACTGATCTACCTGGCAC TGGCACACATGATCAAGTTCAGAGGACACTTCCTGATCGAAGG AGACCTGAACCCGGACAACAGCGACGTCGACAAGCTGTTCAT CCAGCTGGTCCAGACATACAACCAGCTGTTCGAAGAAAACCC GATCAACGCAAGCGGAGTCGACGCAAAGGCAATCCTGAGCGC AAGACTGAGCAAGAGCAGAAGACTGGAAAACCTGATCGCACA GCTGCCGGGAGAAAAGAAGAACGGACTGTTCGGAAACCTGAT CGCACTGAGCCTGGGACTGACACCGAACTTCAAGAGCAACTTC GACCTGGCAGAAGACGCAAAGCTGCAGCTGAGCAAGGACACA TACGACGACGACCTGGACAACCTGCTGGCACAGATCGGAGAC CAGTACGCAGACCTGTTCCTGGCAGCAAAGAACCTGAGCGAC GCAATCCTGCTGAGCGACATCCTGAGAGTCAACACAGAAATC ACAAAGGCACCGCTGAGCGCAAGCATGATCAAGAGATACGAC GAACACCACCAGGACCTGACACTGCTGAAGGCACTGGTCAGA CAGCAGCTGCCGGAAAAGTACAAGGAAATCTTCTTCGACCAG AGCAAGAACGGATACGCAGGATACATCGACGGAGGAGCAAGC CAGGAAGAATTCTACAAGTTCATCAAGCCGATCCTGGAAAAG ATGGACGGAACAGAAGAACTGCTGGTCAAGCTGAACAGAGAA GACCTGCTGAGAAAGCAGAGAACATTCGACAACGGAAGCATC CCGCACCAGATCCACCTGGGAGAACTGCACGCAATCCTGAGA AGACAGGAAGACTTCTACCCGTTCCTGAAGGACAACAGAGAA AAGATCGAAAAGATCCTGACATTCAGAATCCCGTACTACGTCG GACCGCTGGCAAGAGGAAACAGCAGATTCGCATGGATGACAA GAAAGAGCGAAGAAACAATCACACCGTGGAACTTCGAAGAAG TCGTCGACAAGGGAGCAAGCGCACAGAGCTTCATCGAAAGAA TGACAAACTTCGACAAGAACCTGCCGAACGAAAAGGTCCTGC | **61** |
| | CGAAGCACAGCCTGCTGTACGAATACTTCACAGTCTACAACGA ACTGACAAAGGTCAAGTACGTCACAGAAGGAATGAGAAAGCC GGCATTCCTGAGCGGAGAACAGAAGAAGGCAATCGTCGACCT GCTGTTCAAGACAAACAGAAAGGTCACAGTCAAGCAGCTGAA GGAAGACTACTTCAAGAAGATCGAATGCTTCGACAGCGTCGA AATCAGCGGAGTCGAAGACAGATTCAACGCAAGCCTGGGAAC ATACCACGACCTGCTGAAGATCATCAAGGACAAGGACTTCCTG GACAACGAAGAAAACGAAGACATCCTGGAAGACATCGTCCTG ACACTGACACTGTTCGAAGACAGAGAAATGATCGAAGAAAGA CTGAAGACATACGCACACCTGTTCGACGACAAGGTCATGAAG CAGCTGAAGAGAAGAAGATACACAGGATGGGGAAGACTGAG CAGAAAGCTGATCAACGGAATCAGAGACAAGCAGAGCGGAA AGACAATCCTGGACTTCCTGAAGAGCGACGGATTCGCAAACA GAAACTTCATGCAGCTGATCCACGACGACAGCCTGACATTCAA GGAAGACATCCAGAAGGCACAGGTCAGCGGACAGGGAGACA GCCTGCACGAACACATCGCAAACCTGGCAGGAAGCCCGGCAA TCAAGAAGGGAATCCTGCAGACAGTCAAGGTCGTCGACGAAC TGGTCAAGGTCATGGGAAGACACAAGCCGGAAAACATCGTCA TCGAAATGGCAAGAGAAAACCAGACAACACAGAAGGGACAG AAGAACAGCAGAGAAAGAATGAAGAGAATCGAAGAAGGAAT CAAGGAACTGGGAAGCCAGATCCTGAAGGAACACCCGGTCGA AAACACACAGCTGCAGAACGAAAAGCTGTACCTGTACTACCT GCAGAACGGAAGAGACATGTACGTCGACCAGGAACTGGACAT CAACAGACTGAGCGACTACGACGTCGACCACATCGTCCCGCA GAGCTTCCTGAAGGACGACAGCATCGACAACAAGGTCCTGAC AAGAAGCGACAAGAACAGAGGAAAGAGCGACAACGTCCCGA GCGAAGAAGTCGTCAAGAAGATGAAGAACTACTGGAGACAGC TGCTGAACGCAAAGCTGATCACACAGAGAAAGTTCGACAACC TGACAAAGGCAGAGAGAGGAGGACTGAGCGAACTGGACAAG GCAGGATTCATCAAGAGACAGCTGGTCGAAACAAGACAGATC ACAAAGCACGTCGCACAGATCCTGGACAGCAGAATGAACACA AAGTACGACGAAAACGACAAGCTGATCAGAGAAGTCAAGGTC ATCACACTGAAGAGCAAGCTGGTCAGCGACTTCAGAAAGGAC TTCCAGTTCTACAAGGTCAGAGAAATCAACAACTACCACCACG CACACGACGCATACCTGAACGCAGTCGTCGGAACAGCACTGA TCAAGAAGTACCCGAAGCTGGAAAGCGAATTCGTCTACGGAG ACTACAAGGTCTACGACGTCAGAAAGATGATCGCAAAGAGCG AACAGGAAATCGGAAAGGCAACAGCAAAGTACTTCTTCTACA GCAACATCATGAACTTCTTCAAGACAGAAATCACACTGGCAA ACGGAGAAATCAGAAAGAGACCGCTGATCGAAACAAACGGA GAAACAGGAGAAATCGTCTGGGACAAGGGAAGAGACTTCGCA ACAGTCAGAAAGGTCCTGAGCATGCCGCAGGTCAACATCGTC AAGAAGACAGAAGTCCAGACAGGAGGATTCAGCAAGGAAAG CATCCTGCCGAAGAGAAACAGCGACAAGCTGATCGCAAGAAA GAAGGACTGGGACCCGAAGAAGTACGGAGGATTCGACAGCCC GACAGTCGCATACAGCGTCCTGGTCGTCGCAAAGGTCGAAAA GGGAAAGAGCAAGAAGCTGAAGAGCGTCAAGGAACTGCTGG GAATCACAATCATGGAAAGAAGCAGCTTCGAAAAGAACCCGA TCGACTTCCTGGAAGCAAAGGGATACAAGGAAGTCAAGAAGG ACCTGATCATCAAGCTGCCGAAGTACAGCCTGTTCGAACTGGA AAACGGAAGAAAGAGAATGCTGGCAAGCGCAGGAGAACTGC AGAAGGGAAACGAACTGGCACTGCCGAGCAAGTACGTCAACT TCCTGTACCTGGCAAGCCACTACGAAAAGCTGAAGGGAAGCC CGGAAGACAACGAACAGAAGCAGCTGTTCGTCGAACAGCACA AGCACTACCTGGACGAAATCATCGAACAGATCAGCGAATTCA GCAAGAGAGTCATCCTGGCAGACGCAAACCTGGACAAGGTCC TGAGCGCATACAACAAGCACAGAGACAAGCCGATCAGAGAAC AGGCAGAAAACATCATCCACCTGTTCACACTGACAAACCTGG GAGCACCGGCAGCATTCAAGTACTTCGACACAACAATCGACA GAAAGAGATACACAAGCACAAAGGAAGTCCTGGACGCAACAC TGATCCACCAGAGCATCACAGGACTGTACGAAACAAGAATCG | |
| | ACCTGAGCCAGCTGGGAGGAGACGGAGGAGGAAGCCCGAAG AAGAAGAGAAAGGTCTAGCTAGCCATCACATTTAAAAGCATC TCAGCCTACCATGAGAATAAGAGAAAGAAAATGAAGATCAAT AGCTTATTCATCTCTTTTTCTTTTTCGTTGGTGTAAAGCCAACA CCCTGTCTAAAAAACATAAATTTCTTTAATCATTTTGCCTCTTT TCTCTGTGCTTCAATTAATAAAAAATGGAAAGAACCTCGAG | |
| **30/30/39 poly-A sequence** | AAAAAAAAAAAAAAAAAAAAAAAAAAAAAGCGAAAAAAA AAAAAAAAAAAAAAAAAAAAAAACCGAAAAAAAAAAAAAA AAAAAAAAAAAAAAAAAAAAAAA | **62** |
| **poly-A 100 sequence** | AAAAAAAAAAAAAAAAAAAAAAAAAAAAAAAAAAAAAAAA AAAAAAAAAAAAAAAAAAAAAAAAAAAAAAAAAAAAAAAA AAAAAAAAAAAAA | **63** |
| **G209 guide RNA** | mC*mC*mA*GUCCAGCGAGGCAAAGGGUUUUAGAGCUAGAAA UAGCAAGUUAAAAUAAGGCUAGUCCGUUAUCAACUUGAAAA AGUGGCACCGAGUCGGUGCmU*mU*mU*U | **64** |
| **ORF encoding Neisseria meningitidis Cas9** | ATGGCAGCATTCAAGCCGAACTCGATCAACTACATCCTGGGAC TGGACATCGGAATCGCATCGGTCGGATGGGCAATGGTCGAAA TCGACGAAGAAGAAAACCCGATCAGACTGATCGACCTGGGAG TCAGAGTCTTCGAAAGAGCAGAAGTCCCGAAGACAGGAGACT CGCTGGCAATGGCAAGAAGACTGGCAAGATCGGTCAGAAGAC TGACAAGAAGAAGAGCACACAGACTGCTGAGAACAAGAAGA CTGCTGAAGAGAGAAGGAGTCCTGCAGGCAGCAAACTTCGAC GAAAACGGACTGATCAAGTCGCTGCCGAACACACCGTGGCAG CTGAGAGCAGCAGCACTGGACAGAAAGCTGACACCGCTGGAA TGGTCGGCAGTCCTGCTGCACCTGATCAAGCACAGAGGATACC TGTCGCAGAGAAAGAACGAAGGAGAAACAGCAGACAAGGAA CTGGGAGCACTGCTGAAGGGAGTCGCAGGAAACGCACACGCA CTGCAGACAGGAGACTTCAGAACACCGGCAGAACTGGCACTG AACAAGTTCGAAAAGGAATCGGGACACATCAGAAACCAGAGA TCGGACTACTCGCACACATTCTCGAGAAAGGACCTGCAGGCA GAACTGATCCTGCTGTTCGAAAAGCAGAAGGAATTCGGAAAC CCGCACGTCTCGGGAGGACTGAAGGAAGGAATCGAAACACTG CTGATGACACAGAGACCGGCACTGTCGGGAGACGCAGTCCAG AAGATGCTGGGACACTGCACATTCGAACCGGCAGAACCGAAG GCAGCAAAGAACACATACACAGCAGAAAGATTCATCTGGCTG ACAAAGCTGAACAACCTGAGAATCCTGGAACAGGGATCGGAA AGACCGCTGACAGACACAGAAAGAGCAACACTGATGGACGAA CCGTACAGAAAGTCGAAGCTGACATACGCACAGGCAAGAAAG CTGCTGGGACTGGAAGACACAGCATTCTTCAAGGGACTGAGA TACGGAAAGGACAACGCAGAAGCATCGACACTGATGGAAATG AAGGCATACCACGCAATCTCGAGAGCACTGGAAAAGGAAGGA CTGAAGGACAAGAAGTCGCCGCTGAACCTGTCGCCGGAACTG CAGGACGAAATCGGAACAGCATTCTCGCTGTTCAAGACAGAC GAAGACATCACAGGAAGACTGAAGGACAGAATCCAGCCGGAA ATCCTGGAAGCACTGCTGAAGCACATCTCGTTCGACAAGTTCG TCCAGATCTCGCTGAAGGCACTGAGAAGAATCGTCCCGCTGAT GGAACAGGGAAAGAGATACGACGAAGCATGCGCAGAAATCTA CGGAGACCACTACGGAAAGAAGAACACAGAAGAAAAGATCT ACCTGCCGCCGATCCCGGCAGACGAAATCAGAAACCCGGTCG TCCTGAGAGCACTGTCGCAGGCAAGAAAGGTCATCAACGGAG TCGTCAGAAGATACGGATCGCCGGCAAGAATCCACATCGAAA CAGCAAGAGAAGTCGGAAAGTCGTTCAAGGACAGAAAGGAA ATCGAAAAGAGACAGGAAGAAAACAGAAAGGACAGAGAAAA GGCAGCAGCAAAGTTCAGAGAATACTTCCCGAACTTCGTCGG AGAACCGAAGTCGAAGGACATCCTGAAGCTGAGACTGTACGA ACAGCAGCACGGAAAGTGCCTGTACTCGGGAAAGGAAATCAA CCTGGGAAGACTGAACGAAAAGGGATACGTCGAAATCGACCA | **65** |
| | CGCACTGCCGTTCTCGAGAACATGGGACGACTCGTTCAACAAC AAGGTCCTGGTCCTGGGATCGGAAAACCAGAACAAGGGAAAC CAGACACCGTACGAATACTTCAACGGAAAGGACAACTCGAGA GAATGGCAGGAATTCAAGGCAAGAGTCGAAACATCGAGATTC CCGAGATCGAAGAAGCAGAGAATCCTGCTGCAGAAGTTCGAC GAAGACGGATTCAAGGAAAGAAACCTGAACGACACAAGATAC GTCAACAGATTCCTGTGCCAGTTCGTCGCAGACAGAATGAGAC TGACAGGAAAGGGAAAGAAGAGAGTCTTCGCATCGAACGGAC AGATCACAAACCTGCTGAGAGGATTCTGGGGACTGAGAAAGG TCAGAGCAGAAAACGACAGACACCACGCACTGGACGCAGTCG TCGTCGCATGCTCGACAGTCGCAATGCAGCAGAAGATCACAA GATTCGTCAGATACAAGGAAATGAACGCATTCGACGGAAAGA CAATCGACAAGGAAACAGGAGAAGTCCTGCACCAGAAGACAC ACTTCCCGCAGCCGTGGGAATTCTTCGCACAGGAAGTCATGAT CAGAGTCTTCGGAAAGCCGGACGGAAAGCCGGAATTCGAAGA AGCAGACACACTGGAAAAGCTGAGAACACTGCTGGCAGAAAA GCTGTCGTCGAGACCGGAAGCAGTCCACGAATACGTCACACC GCTGTTCGTCTCGAGAGCACCGAACAGAAAGATGTCGGGACA GGGACACATGGAAACAGTCAAGTCGGCAAAGAGACTGGACGA AGGAGTCTCGGTCCTGAGAGTCCCGCTGACACAGCTGAAGCTG AAGGACCTGGAAAAGATGGTCAACAGAGAAAGAGAACCGAA GCTGTACGAAGCACTGAAGGCAAGACTGGAAGCACACAAGGA CGACCCGGCAAAGGCATTCGCAGAACCGTTCTACAAGTACGA CAAGGCAGGAAACAGAACACAGCAGGTCAAGGCAGTCAGAGT CGAACAGGTCCAGAAGACAGGAGTCTGGGTCAGAAACCACAA CGGAATCGCAGACAACGCAACAATGGTCAGAGTAGACGTCTT CGAAAAGGGAGACAAGTACTACCTGGTCCCGATCTACTCGTG GCAGGTCGCAAAGGGAATCCTGCCGGACAGAGCAGTCGTCCA GGGAAAGGACGAAGAAGACTGGCAGCTGATCGACGACTCGTT CAACTTCAAGTTCTCGCTGCACCCGAACGACCTGGTCGAAGTC ATCACAAAGAAGGCAAGAATGTTCGGATACTTCGCATCGTGCC ACAGAGGAACAGGAAACATCAACATCAGAATCCACGACCTGG ACCACAAGATCGGAAAGAACGGAATCCTGGAAGGAATCGGAG TCAAGACAGCACTGTCGTTCCAGAAGTACCAGATCGACGAACT GGGAAAGGAAATCAGACCGTGCAGACTGAAGAAGAGACCGCC GGTCAGATCCGGAAAGAGAACAGCAGACGGATCGGAATTCGA ATCGCCGAAGAAGAAGAGAAAGGTCGAATGA | |
| **ORF encoding Neisseria meningitidis Cas9 (no start or stop codons; suitable for inclusion in fusion protein coding sequence)** | GCAGCATTCAAGCCGAACTCGATCAACTACATCCTGGGACTGG ACATCGGAATCGCATCGGTCGGATGGGCAATGGTCGAAATCG ACGAAGAAGAAAACCCGATCAGACTGATCGACCTGGGAGTCA GAGTCTTCGAAAGAGCAGAAGTCCCGAAGACAGGAGACTCGC TGGCAATGGCAAGAAGACTGGCAAGATCGGTCAGAAGACTGA CAAGAAGAAGAGCACACAGACTGCTGAGAACAAGAAGACTGC TGAAGAGAGAAGGAGTCCTGCAGGCAGCAAACTTCGACGAAA ACGGACTGATCAAGTCGCTGCCGAACACACCGTGGCAGCTGA GAGCAGCAGCACTGGACAGAAAGCTGACACCGCTGGAATGGT CGGCAGTCCTGCTGCACCTGATCAAGCACAGAGGATACCTGTC GCAGAGAAAGAACGAAGGAGAAACAGCAGACAAGGAACTGG GAGCACTGCTGAAGGGAGTCGCAGGAAACGCACACGCACTGC AGACAGGAGACTTCAGAACACCGGCAGAACTGGCACTGAACA AGTTCGAAAAGGAATCGGGACACATCAGAAACCAGAGATCGG ACTACTCGCACACATTCTCGAGAAAGGACCTGCAGGCAGAAC TGATCCTGCTGTTCGAAAAGCAGAAGGAATTCGGAAACCCGC ACGTCTCGGGAGGACTGAAGGAAGGAATCGAAACACTGCTGA TGACACAGAGACCGGCACTGTCGGGAGACGCAGTCCAGAAGA TGCTGGGACACTGCACATTCGAACCGGCAGAACCGAAGGCAG CAAAGAACACATACACAGCAGAAAGATTCATCTGGCTGACAA AGCTGAACAACCTGAGAATCCTGGAACAGGGATCGGAAAGAC CGCTGACAGACACAGAAAGAGCAACACTGATGGACGAACCGT ACAGAAAGTCGAAGCTGACATACGCACAGGCAAGAAAGCTGC | **66** |
| | TGGGACTGGAAGACACAGCATTCTTCAAGGGACTGAGATACG GAAAGGACAACGCAGAAGCATCGACACTGATGGAAATGAAGG CATACCACGCAATCTCGAGAGCACTGGAAAAGGAAGGACTGA AGGACAAGAAGTCGCCGCTGAACCTGTCGCCGGAACTGCAGG ACGAAATCGGAACAGCATTCTCGCTGTTCAAGACAGACGAAG ACATCACAGGAAGACTGAAGGACAGAATCCAGCCGGAAATCC TGGAAGCACTGCTGAAGCACATCTCGTTCGACAAGTTCGTCCA GATCTCGCTGAAGGCACTGAGAAGAATCGTCCCGCTGATGGA ACAGGGAAAGAGATACGACGAAGCATGCGCAGAAATCTACGG AGACCACTACGGAAAGAAGAACACAGAAGAAAAGATCTACCT GCCGCCGATCCCGGCAGACGAAATCAGAAACCCGGTCGTCCT GAGAGCACTGTCGCAGGCAAGAAAGGTCATCAACGGAGTCGT CAGAAGATACGGATCGCCGGCAAGAATCCACATCGAAACAGC AAGAGAAGTCGGAAAGTCGTTCAAGGACAGAAAGGAAATCGA AAAGAGACAGGAAGAAAACAGAAAGGACAGAGAAAAGGCAG CAGCAAAGTTCAGAGAATACTTCCCGAACTTCGTCGGAGAACC GAAGTCGAAGGACATCCTGAAGCTGAGACTGTACGAACAGCA GCACGGAAAGTGCCTGTACTCGGGAAAGGAAATCAACCTGGG AAGACTGAACGAAAAGGGATACGTCGAAATCGACCACGCACT GCCGTTCTCGAGAACATGGGACGACTCGTTCAACAACAAGGTC CTGGTCCTGGGATCGGAAAACCAGAACAAGGGAAACCAGACA CCGTACGAATACTTCAACGGAAAGGACAACTCGAGAGAATGG CAGGAATTCAAGGCAAGAGTCGAAACATCGAGATTCCCGAGA TCGAAGAAGCAGAGAATCCTGCTGCAGAAGTTCGACGAAGAC GGATTCAAGGAAAGAAACCTGAACGACACAAGATACGTCAAC AGATTCCTGTGCCAGTTCGTCGCAGACAGAATGAGACTGACAG GAAAGGGAAAGAAGAGAGTCTTCGCATCGAACGGACAGATCA CAAACCTGCTGAGAGGATTCTGGGGACTGAGAAAGGTCAGAG CAGAAAACGACAGACACCACGCACTGGACGCAGTCGTCGTCG CATGCTCGACAGTCGCAATGCAGCAGAAGATCACAAGATTCG TCAGATACAAGGAAATGAACGCATTCGACGGAAAGACAATCG ACAAGGAAACAGGAGAAGTCCTGCACCAGAAGACACACTTCC CGCAGCCGTGGGAATTCTTCGCACAGGAAGTCATGATCAGAGT CTTCGGAAAGCCGGACGGAAAGCCGGAATTCGAAGAAGCAGA CACACTGGAAAAGCTGAGAACACTGCTGGCAGAAAAGCTGTC GTCGAGACCGGAAGCAGTCCACGAATACGTCACACCGCTGTTC GTCTCGAGAGCACCGAACAGAAAGATGTCGGGACAGGGACAC ATGGAAACAGTCAAGTCGGCAAAGAGACTGGACGAAGGAGTC TCGGTCCTGAGAGTCCCGCTGACACAGCTGAAGCTGAAGGAC CTGGAAAAGATGGTCAACAGAGAAAGAGAACCGAAGCTGTAC GAAGCACTGAAGGCAAGACTGGAAGCACACAAGGACGACCCG GCAAAGGCATTCGCAGAACCGTTCTACAAGTACGACAAGGCA GGAAACAGAACACAGCAGGTCAAGGCAGTCAGAGTCGAACAG GTCCAGAAGACAGGAGTCTGGGTCAGAAACCACAACGGAATC GCAGACAACGCAACAATGGTCAGAGTAGACGTCTTCGAAAAG GGAGACAAGTACTACCTGGTCCCGATCTACTCGTGGCAGGTCG CAAAGGGAATCCTGCCGGACAGAGCAGTCGTCCAGGGAAAGG ACGAAGAAGACTGGCAGCTGATCGACGACTCGTTCAACTTCA AGTTCTCGCTGCACCCGAACGACCTGGTCGAAGTCATCACAAA GAAGGCAAGAATGTTCGGATACTTCGCATCGTGCCACAGAGG AACAGGAAACATCAACATCAGAATCCACGACCTGGACCACAA GATCGGAAAGAACGGAATCCTGGAAGGAATCGGAGTCAAGAC AGCACTGTCGTTCCAGAAGTACCAGATCGACGAACTGGGAAA GGAAATCAGACCGTGCAGACTGAAGAAGAGACCGCCGGTCAG ATCCGGAAAGAGAACAGCAGACGGATCGGAATTCGAATCGCC GAAGAAGAAGAGAAAGGTCGAA | |
| **Transcript comprising SEQ ID NO: 65 (encoding** | GGGAGACCCAAGCTGGCTAGCGTTTAAACTTAAGCTTGGATCC GCCACCATGGCAGCATTCAAGCCGAACTCGATCAACTACATCC TGGGACTGGACATCGGAATCGCATCGGTCGGATGGGCAATGG TCGAAATCGACGAAGAAGAAAACCCGATCAGACTGATCGACC | **67** |
| **Neisseria meningitidis Cas9)** | TGGGAGTCAGAGTCTTCGAAAGAGCAGAAGTCCCGAAGACAG GAGACTCGCTGGCAATGGCAAGAAGACTGGCAAGATCGGTCA GAAGACTGACAAGAAGAAGAGCACACAGACTGCTGAGAACA AGAAGACTGCTGAAGAGAGAAGGAGTCCTGCAGGCAGCAAAC TTCGACGAAAACGGACTGATCAAGTCGCTGCCGAACACACCG TGGCAGCTGAGAGCAGCAGCACTGGACAGAAAGCTGACACCG CTGGAATGGTCGGCAGTCCTGCTGCACCTGATCAAGCACAGAG GATACCTGTCGCAGAGAAAGAACGAAGGAGAAACAGCAGAC AAGGAACTGGGAGCACTGCTGAAGGGAGTCGCAGGAAACGCA CACGCACTGCAGACAGGAGACTTCAGAACACCGGCAGAACTG GCACTGAACAAGTTCGAAAAGGAATCGGGACACATCAGAAAC CAGAGATCGGACTACTCGCACACATTCTCGAGAAAGGACCTG CAGGCAGAACTGATCCTGCTGTTCGAAAAGCAGAAGGAATTC GGAAACCCGCACGTCTCGGGAGGACTGAAGGAAGGAATCGAA ACACTGCTGATGACACAGAGACCGGCACTGTCGGGAGACGCA GTCCAGAAGATGCTGGGACACTGCACATTCGAACCGGCAGAA CCGAAGGCAGCAAAGAACACATACACAGCAGAAAGATTCATC TGGCTGACAAAGCTGAACAACCTGAGAATCCTGGAACAGGGA TCGGAAAGACCGCTGACAGACACAGAAAGAGCAACACTGATG GACGAACCGTACAGAAAGTCGAAGCTGACATACGCACAGGCA AGAAAGCTGCTGGGACTGGAAGACACAGCATTCTTCAAGGGA CTGAGATACGGAAAGGACAACGCAGAAGCATCGACACTGATG GAAATGAAGGCATACCACGCAATCTCGAGAGCACTGGAAAAG GAAGGACTGAAGGACAAGAAGTCGCCGCTGAACCTGTCGCCG GAACTGCAGGACGAAATCGGAACAGCATTCTCGCTGTTCAAG ACAGACGAAGACATCACAGGAAGACTGAAGGACAGAATCCAG CCGGAAATCCTGGAAGCACTGCTGAAGCACATCTCGTTCGACA AGTTCGTCCAGATCTCGCTGAAGGCACTGAGAAGAATCGTCCC GCTGATGGAACAGGGAAAGAGATACGACGAAGCATGCGCAGA AATCTACGGAGACCACTACGGAAAGAAGAACACAGAAGAAA AGATCTACCTGCCGCCGATCCCGGCAGACGAAATCAGAAACC CGGTCGTCCTGAGAGCACTGTCGCAGGCAAGAAAGGTCATCA ACGGAGTCGTCAGAAGATACGGATCGCCGGCAAGAATCCACA TCGAAACAGCAAGAGAAGTCGGAAAGTCGTTCAAGGACAGAA AGGAAATCGAAAAGAGACAGGAAGAAAACAGAAAGGACAGA GAAAAGGCAGCAGCAAAGTTCAGAGAATACTTCCCGAACTTC GTCGGAGAACCGAAGTCGAAGGACATCCTGAAGCTGAGACTG TACGAACAGCAGCACGGAAAGTGCCTGTACTCGGGAAAGGAA ATCAACCTGGGAAGACTGAACGAAAAGGGATACGTCGAAATC GACCACGCACTGCCGTTCTCGAGAACATGGGACGACTCGTTCA ACAACAAGGTCCTGGTCCTGGGATCGGAAAACCAGAACAAGG GAAACCAGACACCGTACGAATACTTCAACGGAAAGGACAACT CGAGAGAATGGCAGGAATTCAAGGCAAGAGTCGAAACATCGA GATTCCCGAGATCGAAGAAGCAGAGAATCCTGCTGCAGAAGT TCGACGAAGACGGATTCAAGGAAAGAAACCTGAACGACACAA GATACGTCAACAGATTCCTGTGCCAGTTCGTCGCAGACAGAAT GAGACTGACAGGAAAGGGAAAGAAGAGAGTCTTCGCATCGAA CGGACAGATCACAAACCTGCTGAGAGGATTCTGGGGACTGAG AAAGGTCAGAGCAGAAAACGACAGACACCACGCACTGGACGC AGTCGTCGTCGCATGCTCGACAGTCGCAATGCAGCAGAAGATC ACAAGATTCGTCAGATACAAGGAAATGAACGCATTCGACGGA AAGACAATCGACAAGGAAACAGGAGAAGTCCTGCACCAGAAG ACACACTTCCCGCAGCCGTGGGAATTCTTCGCACAGGAAGTCA TGATCAGAGTCTTCGGAAAGCCGGACGGAAAGCCGGAATTCG AAGAAGCAGACACACTGGAAAAGCTGAGAACACTGCTGGCAG AAAAGCTGTCGTCGAGACCGGAAGCAGTCCACGAATACGTCA CACCGCTGTTCGTCTCGAGAGCACCGAACAGAAAGATGTCGG GACAGGGACACATGGAAACAGTCAAGTCGGCAAAGAGACTGG ACGAAGGAGTCTCGGTCCTGAGAGTCCCGCTGACACAGCTGA AGCTGAAGGACCTGGAAAAGATGGTCAACAGAGAAAGAGAA CCGAAGCTGTACGAAGCACTGAAGGCAAGACTGGAAGCACAC | |
| | AAGGACGACCCGGCAAAGGCATTCGCAGAACCGTTCTACAAG TACGACAAGGCAGGAAACAGAACACAGCAGGTCAAGGCAGTC AGAGTCGAACAGGTCCAGAAGACAGGAGTCTGGGTCAGAAAC CACAACGGAATCGCAGACAACGCAACAATGGTCAGAGTAGAC GTCTTCGAAAAGGGAGACAAGTACTACCTGGTCCCGATCTACT CGTGGCAGGTCGCAAAGGGAATCCTGCCGGACAGAGCAGTCG TCCAGGGAAAGGACGAAGAAGACTGGCAGCTGATCGACGACT CGTTCAACTTCAAGTTCTCGCTGCACCCGAACGACCTGGTCGA AGTCATCACAAAGAAGGCAAGAATGTTCGGATACTTCGCATC GTGCCACAGAGGAACAGGAAACATCAACATCAGAATCCACGA CCTGGACCACAAGATCGGAAAGAACGGAATCCTGGAAGGAAT CGGAGTCAAGACAGCACTGTCGTTCCAGAAGTACCAGATCGA CGAACTGGGAAAGGAAATCAGACCGTGCAGACTGAAGAAGAG ACCGCCGGTCAGATCCGGAAAGAGAACAGCAGACGGATCGGA ATTCGAATCGCCGAAGAAGAAGAGAAAGGTCGAATGATAGCT AGCTCGAGTCTAGAGGGCCCGTTTAAACCCGCTGATCAGCCTC GACTGTGCCTTCTAGTTGCCAGCCATCTGTTGTTTGCCCCTCCC CCGTGCCTTCCTTGACCCTGGAAGGTGCCACTCCCACTGTCCTT TCCTAATAAAATGAGGAAATTGCATCGCATTGTCTGAGTAGGT GTCATTCTATTCTGGGGGGTGGGGTGGGGCAGGACAGCAAGG GGGAGGATTGGGAAGACAATAGCAGGCATGCTGGGGATGCGG TGGGCTCTATGG | |
| **Amino acid sequence of Neisseria meningitidis Cas9** | MAAFKPNSINYILGLDIGIASVGWAMVEIDEEENPIRLIDLGVRVF ERAEVPKTGDSLAMARRLARSVRRLTRRRAHRLLRTRRLLKREG VLQAANFDENGLIKSLPNTPWQLRAAALDRKLTPLEWSAVLLHLI KHRGYLSQRKNEGETADKELGALLKGVAGNAHALQTGDFRTPA ELALNKFEKESGHIRNQRSDYSHTFSRKDLQAELILLFEKQKEFGN PHVSGGLKEGIETLLMTQRPALSGDAVQKMLGHCTFEPAEPKAA KNTYTAERFIWLTKLNNLRILEQGSERPLTDTERATLMDEPYRKS KLTYAQARKLLGLEDTAFFKGLRYGKDNAEASTLMEMKAYHAI SRALEKEGLKDKKSPLNLSPELQDEIGTAFSLFKTDEDITGRLKDR IQPEILEALLKHISFDKFVQISLKALRRIVPLMEQGKRYDEACAEIY GDHYGKKNTEEKIYLPPIPADEIRNPVVLRALSQARKVINGVVRR YGSPARIHIETAREVGKSFKDRKEIEKRQEENRKDREKAAAKFRE YFPNFVGEPKSKDILKLRLYEQQHGKCLYSGKEINLGRLNEKGYV EIDHALPFSRTWDDSFNNKVLVLGSENQNKGNQTPYEYFNGKDN SREWQEFKARVETSRFPRSKKQRILLQKFDEDGFKERNLNDTRYV NRFLCQFVADRMRLTGKGKKRVFASNGQITNLLRGFWGLRKVR AENDRHHALDAVVVACSTVAMQQKITRFVRYKEMNAFDGKTID KETGEVLHQKTHFPQPWEFFAQEVMIRVFGKPDGKPEFEEADTLE KLRTLLAEKLSSRPEAVHEYVTPLFVSRAPNRKMSGQGHMETVK SAKRLDEGVSVLRVPLTQLKLKDLEKMVNREREPKLYEALKARL EAHKDDPAKAFAEPFYKYDKAGNRTQQVKAVRVEQVQKTGVW VRNHNGIADNATMVRVDVFEKGDKYYLVPIYSWQVAKGILPDR AVVQGKDEEDWQLIDDSFNFKFSLHPNDLVEVITKKARMFGYFA SCHRGTGNINIRIHDLDHKIGKNGILEGIGVKTALSFQKYQIDELG KEIRPCRLKKRPPVRSGKRTADGSEFESPKKKRKVE | **68** |
| **G390 guide RNA** | mG*mC*mC*GAGUCUGGAGAGCUGCAGUUUUAGAmGmCmUm AmGmAmAmAmUmAmGmCAAGUUAAAAUAAGGCUAGUCCGU UAUCAmAmCmUmUmGmAmAmAmAmAmGmUmGmGmCmAmC mCmGmAmGmUmCmGmGmUmGmCmU*mU*mU*mU | **69** |
| **G502 guide RNA** | mA*mC*mA*CAAAUACCAGUCCAGCGGUUUUAGAmGmCmUm AmGmAmAmAmUmAmGmCAAGUUAAAAUAAGGCUAGUCCGU UAUCAmAmCmUmUmGmAmAmAmAmAmGmUmGmGmCmAmC mCmGmAmGmUmCmGmGmUmGmCmU*mU*mU*mU | **70** |
| **G509 guide RNA** | mA*mA*mA*GUUCUAGAUGCCGUCCGGUUUUAGAmGmCmUm AmGmAmAmAmUmAmGmCAAGUUAAAAUAAGGCUAGUCCGU UAUCAmAmCmUmUmGmAmAmAmAmAmGmUmGmGmCmAmC mCmGmAmGmUmCmGmGmUmGmCmU*mU*mU*mU | **71** |
| **G534 guide RNA** | mA*mC*mG*CAAAUAUCAGUCCAGCGGUUUUAGAmGmCmUm AmGmAmAmAmUmAmGmCAAGUUAAAAUAAGGCUAGUCCGU UAUCAmAmCmUmUmGmAmAmAmAmAmGmUmGmGmCmAmC mCmGmAmGmUmCmGmGmUmGmCmU*mU*mU*mU | **72** |

| | | |
|---|---|---|
| *** = PS linkage; 'm' = 2'-O-Me nucleotide** | | |

### Mouse G000282 NGS primer sequences

**Forward primer:**
   CACTCTTTCCCTACACGACGCTCTTCCGATCTGTTTTGTTCCAGAGTCTATCACCG
**Reverse primer:**
   GGAGTTCAGACGTGTGCTCTTCCGATCTACACGAATAAGAGCAAATGGGAAC

### Rat G000390 NGS primer sequences

**Forward Primer:**
   CACTCTTTCCCTACACGACGCTCTTCCGATCTTGCATTTCATGAGACCGAAAACA
**Reverse Primer:**
   GGAGTTCAGACGTGTGCTCTTCCGATCTGCTACAGTAGAGCTGTACATAAAACTT

### GFP sequence:

TCGCGCGTTTCGGTGATGACGGTGAAAACCTCTGACACATGCAGCTCCCGGAGAC
GGTCACAGCTTGTCTGTAAGCGGATGCCGGGAGCAGACAAGCCCGTCAGGGCGC
GTCAGCGGGTGTTGGCGGGTGTCGGGGCTGGCTTAACTATGCGGCATCAGAGCA
GATTGTACTGAGAGTGCACCATATGCGGTGTGAAATACCGCACAGATGCGTAAG
GAGAAAATACCGCATCAGGCGCCATTCGCCATTCAGGCTGCGCAACTGTTGGGA
AGGGCGATCGGTGCGGGCCTCTTCGCTATTACGCCAGCTGGCGAAAGGGGGATG
TGCTGCAAGGCGATTAAGTTGGGTAACGCCAGGGTTTTCCCAGTCACGACGTTGT
AAAACGACGGCCAGTGAATTCTAATACGACTCACTATAGGGTCCCGCAGTCGGC
GTCCAGCGGCTCTGCTTGTTCGTGTGTGTGTCGTTGCAGGCCTTATTCGGATCCAT
GGTGAGCAAGGGCGAGGAGCTGTTCACCGGGGTGGTGCCCATCCTGGTCGAGCT
GGACGGCGACGTAAACGGCCACAAGTTCAGCGTGTCCGGCGAGGGCGAGGGCG
ATGCCACCTACGGCAAGCTGACCCTGAAGTTCATCTGCACCACCGGCAAGCTGCC
CGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAA
GGCTACGTCCAGGAGCGCACCATCTTCTTCAAGGACGACGGCAACTACAAGACC
CGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAAC
TACAACAGCCACAACGTCTATATCATGGCCGACAAGCAGAAGAACGGCATCAAG
GTGAACTTCAAGATCCGCCACAACATCGAGGACGGCAGCGTGCAGCTCGCCGAC
CACTACCAGCAGAACACCCCCATCGGCGACGGCCCCGTGCTGCTGCCCGACAAC
CACTACCTGAGCACCCAGTCCGCCCTGAGCAAAGACCCCAACGAGAAGCGCGAT
CACATGGTCCTGCTGGAGTTCGTGACCGCCGCCGGGATCACTCTCGGCATGGACG
AGCTGTACAAGTAATAGGAATTATGCAGTCTAGCCATCACATTTAAAAGCATCTC
AGCCTACCATGAGAATAAGAGAAAGAAAATGAAGATCAATAGCTTATTCATCTC

TTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTC
GAGAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAT
CTAGACTTAAGCTTGATGAGCTCTAGCTTGGCGTAATCATGGTCATAGCTGTTTC
CTGTGTGAAATTGTTATCCGCTCACAATTCCACACAACATACGAGCCGGAAGCAT
AAAGTGTAAAGCCTGGGGTGCCTAATGAGTGAGCTAACTCACATTAATTGCGTTG
CGCTCACTGCCCGCTTTCCAGTCGGGAAACCTGTCGTGCCAGCTGCATTAATGAA
TCGGCCAACGCGCGGGGAGAGGCGGTTTGCGTATTGGGCGCTCTTCCGCTTCCTC
GCTCACTGACTCGCTGCGCTCGGTCGTTCGGCTGCGGCGAGCGGTATCAGCTCAC
TCAAAGGCGGTAATACGGTTATCCACAGAATCAGGGGATAACGCAGGAAAGAAC
ATGTGAGCAAAAGGCCAGCAAAAGGCCAGGAACCGTAAAAAGGCCGCGTTGCT
GGCGTTTTTCCATAGGCTCCGCCCCCCTGACGAGCATCACAAAAATCGACGCTCA
AGTCAGAGGTGGCGAAACCCGACAGGACTATAAAGATACCAGGCGTTTCCCCCT
GGAAGCTCCCTCGTGCGCTCTCCTGTTCCGACCCTGCCGCTTACCGGATACCTGT
CCGCCTTTCTCCCTTCGGGAAGCGTGGCGCTTTCTCATAGCTCACGCTGTAGGTAT
CTCAGTTCGGTGTAGGTCGTTCGCTCCAAGCTGGGCTGTGTGCACGAACCCCCCG
TTCAGCCCGACCGCTGCGCCTTATCCGGTAACTATCGTCTTGAGTCCAACCCGGT
AAGACACGACTTATCGCCACTGGCAGCAGCCACTGGTAACAGGATTAGCAGAGC
GAGGTATGTAGGCGGTGCTACAGAGTTCTTGAAGTGGTGGCCTAACTACGGCTAC
ACTAGAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAGATCC
TTTGATCTTTTCTACGGGGTCTGACGCTCAGTGGAACGAAAACTCACGTTAAGGG
ATTTTGGTCATGAGATTATCAAAAAGGATCTTCACCTAGATCCTTTTAAATTAAA
AATGAAGTTTTAAATCAATCTAAAGTATATATGAGTAAACTTGGTCTGACAGTTA
CCAATGCTTAATCAGTGAGGCACCTATCTCAGCGATCTGTCTATTTCGTTCATCCA
TAGTTGCCTGACTCCCCGTCGTGTAGATAACTACGATACGGGAGGGCTTACCATC
TGGCCCCAGTGCTGCAATGATACCGCGAGACCCACGCTCACCGGCTCCAGATTTA
TCAGCAATAAACCAGCCAGCCGGAAGGGCCGAGCGCAGAAGTGGTCCTGCAACT
TTATCCGCCTCCATCCAGTCTATTAATTGTTGCCGGGAAGCTAGAGTAAGTAGTT
CGCCAGTTAATAGTTTGCGCAACGTTGTTGCCATTGCTACAGGCATCGTGGTGTC
ACGCTCGTCGTTTGGTATGGCTTCATTCAGCTCCGGTTCCCAACGATCAAGGCGA
GTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACT
GCATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCCC
GGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGA
TCCAGTTCGATGTAACCCACTCGTGCACCCAACTGATCTTCAGCATCTTTTACTTT
CACCAGCGTTTCTGGGTGAGCAAAAACAGGAAGGCAAAATGCCGCAAAAAAGG
GAATAAGGGCGACACGGAAATGTTGAATACTCATACTCTTCCTTTTTCAATATTA
TTGAAGCATTTATCAGGGTTATTGTCTCATGAGCGGATACATATTTGAATGTATTT
AGAAAAATAAACAAATAGGGGTTCCGCGCACATTTCCCCGAAAAGTGCCACCTG
ACGTCTAAGAAACCATTATTATCATGACATTAACCTATAAAAATAGGCGTATCAC
GAGGCCCTTTCGTCG

## Claims

1. A lipid nanoparticle ("LNP") composition comprising:
an RNA component, wherein the RNA component comprises (i) an mRNA encoding an RNA-guided DNA-binding agent and (ii) a gRNA nucleic acid; and
a lipid component, wherein the lipid component comprises:
40-60 mol-% amine lipid;
0-10 mol-% neutral lipid; and
1.5-10 mol-% PEG lipid,
wherein the remainder of the lipid component is helper lipid, the N/P ratio of the LNP composition is 6 ± 1, and wherein the amine lipid is Lipid A or an acetal analog of Lipid A, wherein Lipid A is represented by the following structural formula

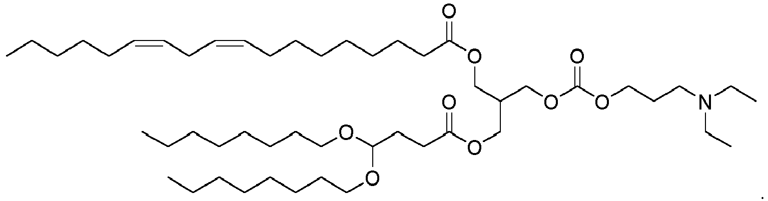

2. The composition of claim 1, wherein the RNA-guided DNA-binding agent is a Cas nuclease.

3. The composition of claim 1 or 2, wherein the RNA-guided DNA-binding agent is a Class 2 Cas nuclease, preferably a Cas9 nuclease.

4. The composition of any one of claims 1-3, wherein the mRNA comprises a sequence with at least 90% identity to any one of SEQ ID NO: 1, 4, 7, 9, 10, 11, 12, 14, 15, 17, 18, 20, 21, 23, 24, 26, 27, 29, 30, 50, 52, 54, 65, or 66, wherein the mRNA comprises an open reading frame encoding the RNA-guided DNA-binding agent.

5. The composition of any one of claims 1-4, wherein the gRNA nucleic acid is a gRNA.

6. The composition of claim 5, wherein the gRNA is modified, such as wherein the gRNA comprises a modification chosen from 2'-O-methyl (2'-O-Me) modified nucleotide, a phosphorothioate (PS) bond between nucleotides; and a 2'-fluoro (2'-F) modified nucleotide.

7. The composition of claim 6, wherein the RNA-guided DNA-binding agent is a Class 2 Cas nuclease, and the gRNA and Class 2 Cas nuclease mRNA are present in a ratio ranging from 10:1 to 1:10 by weight, such as in a ratio from 5:1 to 1:5, from 3:1 to 1:1, or from 2:1 to 1:1, preferably in a ratio of 2:1, 1:1, or 1:2.

8. The composition of any one of claims 1-7, further comprising at least one template nucleic acid.

9. The composition of any one of claims 1-8, wherein the N/P ratio is 6 ± 0.5.

10. The composition of any one of claims 1-9, wherein the mol-% PEG lipid is from 2 mol-% to 10 mol-%, or from 2 mol-% to 8 mol-%, or from 2 mol-% to 4 mol-%, or from 2.5 mol-% to 4 mol-%, preferably 3 mol-%, 2.5 mol-%, or 1.5 mol-%.

11. The composition of any one of claims 1-10, wherein the mol-% amine lipid is from 45 mol-% to 60 mol-%, or from 50 mol-% to 60 mol-%, or from 50 mol-% to 55 mol-%, or 47-53 mol-%, or 48-53 mol-%, or 53-57 mol-%, preferably 50 mol-%, 45 mol-%, 47 mol-%, 48 mol-%, 53 mol-%, or 55 mol-%.

12. The composition of any one of claims 1-11, wherein the mol-% neutral lipid is from 5 mol-% to 10 mol-%, or from 0 mol-% to 5 mol-%, or from 8 mol-% to 10 mol-%, preferably 9 mol-%, 4 mol-%, 5 mol-%, 6 mol-%, 7 mol-%, 8 mol-%, or 10 mol-%.

13. The composition of any one of claims 1-12, wherein the mol-% helper lipid is from 30 mol-% to about 50 mol-%, or from 30 mol-% to about 40 mol-%.

14. The composition of any one of claims 1-13, wherein the amine lipid is Lipid A.

15. The composition of any one of claims 1-13, wherein the amine lipid is represented by the following structural formula 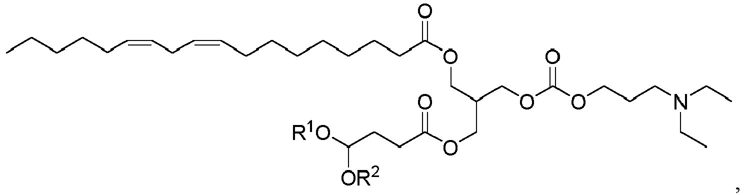 wherein R¹ and R² are each a C4-C12 alkyl.

16. The composition of any one of claims 1-15, wherein the helper lipid is cholesterol.

17. The composition of any one of claims 1-16, wherein the neutral lipid is a phospholipid, such as DSPC or DPPC.

18. The composition of any one of claims 1-17, wherein the PEG lipid comprises dimyristoylglycerol (DMG), a PEG-2k, or PEG-DMG, preferably wherein the PEG lipid is a PEG-DMG, such as a PEG2k-DMG.

19. Use of an LNP composition of any of claims 1-18 for:
(a) gene editing in vitro; or
(b) producing a genetically engineered cell in vitro.

20. An LNP composition of any one of claims 1-18 for use in therapy.

21. An LNP composition for use according to claim 20 wherein the therapy comprises:
(a) gene editing in a subject; or
(b) producing a genetically engineered cell in a subject.

22. The use of claim 19 part (b) or an LNP composition for the use of claim 21 part (b) wherein producing a genetically engineered cell further comprises introducing at least one template nucleic acid into the cell.
